# EUROPEAN PATENT SPECIFICATION

(11) **EP 4 056 570 B1**
(45) Date of publication and mention of the grant of the patent: **07.01.2026**
(21) Application number: 20882541.4
(22) Date of filing: 02.11.2020
(51) Int. Cl.: C07D 487/04, A61K 31/519, A61P 35/00

(54) **EED INHIBITOR, AND PREPARATION METHOD THEREFOR AND USE THEREOF**
EED-INHIBITOR UND HERSTELLUNGSVERFAHREN DAFÜR SOWIE VERWENDUNG DAVON
INHIBITEUR D'EED, SON PROCÉDÉ DE PRÉPARATION ET SON UTILISATION

(30) Priority: 01.11.2019 CN 201911059072
(43) Date of publication of application: 14.09.2022
(73) Proprietor: ShanghaiTech University, Shanghai 201210 (CN); JING MEDICINE TECHNOLOGY (SHANGHAI) LTD., Pudong Shanghai 201210 (CN)
(72) Inventor: QI, Wei, Shanghai 201210 (CN); ZHANG, Haizhen, Shanghai 201314 (CN); MA, Xin, Shanghai 201210 (CN); LEI, Hui, Shanghai 201210 (CN); ZHANG, Yuhua George, Shanghai 201210 (CN); QU, Yuxiu, Shanghai 201210 (CN)
(74) Representative: Synergy IP Group AG
(86) International application number: PCT/CN2020/125873
(87) International publication number: WO 2021/083380

(56) References cited:
- WO-A1-2016/103155
- WO-A1-2016/103155
- WO-A1-2017/219948
- WO-A1-2019/062435
- WO-A1-2019/062435
- WO-A1-2019/158025
- WO-A1-2019/158025
- CN-A- 111 518 100
- JP-A- 2004 238 296

## Description

### FIELD OF THE INVENTION

The present disclosure belongs to the field of medicinal chemistry, and in particular, relates to a new class of EED inhibitors, preparation methods thereof, and pharmaceutical compositions comprising the class of compounds. The class of compounds can be used for the treatment of tumor-related diseases, such as breast cancer, prostate cancer, liver cancer, sarcoma, gastric cancer, diffuse large B-cell lymphoma, follicular lymphoma, and melanoma.

### BACKGROUND OF THE INVENTION

Polycombgroup protein (PcG) is an important family of epigenetic regulators, wherein dysregulation of the members has been found in a variety of tumors. One of important members, Polycomb repressive complex 2 (PRC2), is a protein methyltransferase complex that can methylate the lysine at 27th position of histone H3 (H3K27) near the promoter region of downstream target genes, thereby inhibiting the transcriptional activity of target genes, and playing an important role in development and tissue differentiation and regeneration. The PRC2 complex contains three core subunits: EZH2, EED and SUZ12, all of which are required for PRC2 activity. EZH2 can directly binds to the methyl group donor S-adenosylmethione (SAM) and is an enzymatic subunit; EED can bind to trimethylated H3K27 (H3K27me3) to allosterically activate EZH2, and is an allosteric subunit; and SUZ12 assists in the formation and activity of the complex. EZH1 is a homologous protein of EZH2, and can also participate in the formation of PRC2 but with weak enzymatic activity. In recent years, studies have found that the expressions of EZH2, EED and SUZ12 are up-regulated in a variety of tumors, including but not limited to breast cancer, prostate cancer, liver cancer, sarcoma, and gastric cancer. Activating mutations in EZH2 have also been found in tumors such as diffuse large B lymphoma, follicular lymphoma, and melanoma. Reducing the level and activity of PRC2 by siRNA or PRC2 inhibitor can inhibit the proliferation of tumors such as lymphoma, which reveals that abnormal PRC2 activity is the cause of cancer occurrence and metastasis, and PRC2 can be used as a drug target for treatment. In addition, many studies have reported that PRC2 is also closely related to immune responses mediated by T cells and NK cells. Therefore, PRC2 is a promising drug target for the treatment of lymphoma and other tumors.

At present, there are mainly two drug development mechanisms targeting PRC2: the first is to compete with SAM to bind to EZH2 to exert an inhibitory effect, wherein EPZ-6438 (Epizyme, FDA approved), CPI-1205 (Constellation, Phase II clinical trial) and DS-3201 (Daiichi, Phase II clinical trial) have entered into clinical trials; and another is to allosterically inhibit the activity of PRC2 by targeting EED, wherein only one case, MAK683 (Novartis, Phase I/II clinical trial), has entered into clinical trials. The published tool compounds are A-395 (Abbott) and EED226 (Novartis), which can overcome the resistance problem of the first class of inhibitors due to different binding pockets. To sum up, the PRC2 complex is a proven and promising drug target, and the PRC2 inhibitor targeting EED is still in a relatively early stage of development at present. In-depth research on inhibitors of this target is beneficial to the development of new drugs related to the target, which has broad application value. WO2016/103155, WO2019/158025 and WO2019/062435 discloses triazolopyrimidine derivatives as PRC2 inhibitors, which are structurally different from compounds disclosed herein.

### SUMMARY OF THE INVENTION

Therefore, the object of the present disclosure is to provide a new class of small molecule EED inhibitors, which can be used for the treatment and/or prevention of EED- and/or PRC2-mediated diseases.

In this regard, the present disclosure adopts the following technical solutions:

In one aspect, the present disclosure relates to a compound of formula (X), or a tautomer, stereoisomer, crystal form, pharmaceutically acceptable salt, hydrate or solvate thereof: 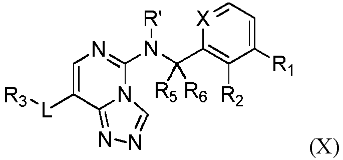 wherein
X is N or C(Rₓ);
wherein Rₓ is selected from H, halogen, C₁₋₆ alkyl, C₁₋₆ haloalkyl or C₁₋₆ alkoxy;
R₁ is selected from H, halogen, C₁₋₆ alkyl, C₁₋₆ haloalkyl or C₁₋₆ alkoxy;
R₂ is selected from H, halogen, C₁₋₆ alkyl, C₁₋₆ haloalkyl or C₁₋₆ alkoxy;
or R₁ and R₂ together form -(CH₂)ₚY-, -Y(CH₂)ₚ- or -Y(CH₂)ₚZ- group;
wherein p is 1, 2, 3, 4 or 5;
Y is selected from O, S, NH or chemical bond;
Z is selected from O, S, NH or chemical bond;
R' is selected from H, C₁₋₆ alkyl or C₁₋₆ haloalkyl;
L is selected from -C(O)-N(R)-, -C(O)-O-, -N(R₃')-C(O)-, -O-C(O)-, -S(O)_{q}-N(R)-, -S(O)_{q}-O-, -O-, -S-, - N(R")-, -C₁₋₄ alkylene- or -C₂₋₄ alkenylene-;
wherein R is selected from H, C₁₋₆ alkyl or C₁₋₆ haloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
R" is selected from H, C₁₋₆ alkyl or C₁₋₆ haloalkyl;
q is 0, 1 or 2;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-NRₐR_{b}, (CH₂)ₙ-ORₐ, (CH₂)ₙ-C₃₋₇ cycloalkyl, (CH₂)ₙ-3- to 10-membered heterocyclyl, (CH₂)ₙ-C₆₋₁₀ aryl or (CH₂)ₙ-5- to 10-membered heteroaryl;
or, R₃, R₃' and the N atom to which they are attached together form a 4- to 7-membered heterocyclyl group; wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₅ and R₆ are independently selected from H, D, C₁₋₆ alkyl or C₁₋₆ haloalkyl;
wherein R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl, -S(O)_{q}-C₁₋₆ alkyl, -S(O)_{q}-C₁₋₆ haloalkyl, -S(O)_{q}-C₁₋₆ alkoxy, -S(O)_{q}-C₃₋₇ cycloalkyl, -S(O)_{q}-4- to 7-membered heterocyclyl, -S(O)_{q}-C₆₋₁₀ aryl or -S(O)_{q}-5- to 10-membered heteroaryl;
wherein R_{c} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
R_{d} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
wherein the alkyl, haloalkyl, alkoxy, haloalkoxy, cycloalkyl, heterocyclyl, aryl, heteroaryl, alkylene and alkenylene are optionally substitued with substituent groups selected from H, D, CN, OH, =O, halogen, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl, or substitued with one or more D, up to complete deuteration.

In another aspect, the present disclosure relates to a compound of formula (I), or a tautomer, stereoisomer, crystal form, pharmaceutically acceptable salt, hydrate or solvate thereof: 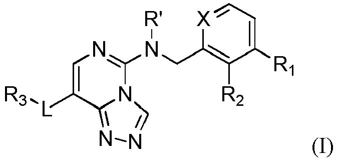 wherein
X is N or C(Rₓ);
wherein Rₓ is selected from H, halogen, C₁₋₆ alkyl, C₁₋₆ haloalkyl or C₁₋₆ alkoxy;
R₁ is selected from H, halogen, C₁₋₆ alkyl, C₁₋₆ haloalkyl or C₁₋₆ alkoxy;
R₂ is selected from H, halogen, C₁₋₆ alkyl, C₁₋₆ haloalkyl or C₁₋₆ alkoxy;
or R₁ and R₂ together form -(CH₂)ₚY-, -Y(CH₂)ₚ- or -Y(CH₂)ₚZ- group;
wherein p is 1, 2, 3, 4 or 5;
Y is selected from O, S, NH or chemical bond;
Z is selected from O, S, NH or chemical bond;
R' is selected from H, C₁₋₆ alkyl or C₁₋₆ haloalkyl;
L is selected from -C(O)-N(R)-, -N(R₃')-C(O)-, -O-, -S-, -N(R")-, -C₁₋₄ alkylene- or -C₂₋₄ alkenylene-;
wherein R is selected from H, C₁₋₆ alkyl or C₁₋₆ haloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
R" is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₇ cycloalkyl, (CH₂)ₙ-3- to 10-membered heterocyclyl, (CH₂)ₙ-C₆₋₁₀ aryl or (CH₂)ₙ-5- to 10-membered heteroaryl;
or, R₃, R₃' and the N atom to which they are attached together form a 4- to 7-membered heterocyclyl group;
wherein m is 0, 1, 2, 3, 4 or 5;
n is 0, 1, 2, 3, 4 or 5.

In another aspect, the present disclosure provides methods for the preparation of compounds of the present disclosure.

In another aspect, the present disclosure provides a pharmaceutical composition, which comprises a compound disclosed herein and pharmaceutically acceptable excipient (s). In a specific embodiment, the compound disclosed herein is provided in a therapeutically effective amount. In a specific embodiment, the compound disclosed herein is provided in a prophylactically effective amount. In another aspect, the pharmaceutical composition of the present disclosure is used in combination with immune checkpoint inhibitor such as PD-1, PD-L1, CTLA-4 antibodies, etc.

In another aspect, the present disclosure provides the use of a compound of the present disclosure or a pharmaceutical composition of the present disclosure in the manufacture of a medicament for the treatment and/or prevention of EED- and/or PRC2-mediated diseases. In another aspect, the present disclosure provides the use of the compound of the present disclosure or the pharmaceutical composition of the present disclosure in the manufacture of a medicament for the treatment and/or prevention of immunodeficiency diseases and autoimmune diseases, etc.

In another aspect, the present disclosure provides a compound of the present disclosure or a pharmaceutical composition thereof for use in the treatment and/or prevention of EED- and/or PRC2-mediated diseases.

In another aspect, the EED- and/or PRC2-mediated disease is a tumor-related disease, an immunodeficiency disease or an autoimmune disease; alternatively, the tumor-related disease is selected from diffuse large B-cell lymphoma, follicular lymphoma, other lymphomas, leukemia, multiple myeloma, mesothelioma, gastric cancer, malignant rhabdomyoma, liver cancer, prostate cancer, breast cancer, bile duct and gallbladder cancer, bladder cancer, brain tumors (including neuroblastoma, schwannoma, glioma, glioblastoma and astrocytoma), cervical cancer, colon cancer, melanoma, endometrial cancer, esophageal cancer, head and neck cancer, lung cancer, nasopharyngeal cancer, ovarian cancer, pancreatic cancer, renal cell cancer, rectal cancer, thyroid cancer, parathyroid tumor, uterine tumor and soft tissue sarcoma; the immunodeficiency disease or autoimmune disease includes, but is not limited to, systemic lupus erythematosus, Crohn's disease, ulcerative colitis, multiple sclerosis, aplastic anemia due to autoimmunity and rejection reaction due to organ transplantation, etc.

In another aspect, the present disclosure provides the use of the compound of the present disclosure or the pharmaceutical composition thereof in the manufacture of a medicament for tumor immunization.

In another aspect, the present disclosure provides a compound of the present disclosure or a pharmaceutical composition thereof for use in tumor immunization.

In another aspect, the present disclosure provides a drug combination comprising a compound of the present disclosure or a pharmaceutical composition thereof, and an immune checkpoint inhibitor.

Other objects and advantages of the present disclosure will be apparent to those skilled in the art from the detailed description, examples and claims that follow.

### Definitions

### Chemical definitions

The definitions of specific functional groups and chemical terms are described in more detail below.

When a range of values is listed, it is intended to encompass each value and sub-range within the range. For example, "C₁₋₆ alkyl" is intended to encompass C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

"C₁₋₆ alkyl" refers to a linear or branched, saturated hydrocarbon group having 1 to 6 carbon atoms, and is also referred to herein as "lower alkyl". In some embodiments, C₁₋₄ alkyl is alternative. Examples of alkyl include, but are not limited to, methyl (C₁), ethyl (C₂), n-propyl (C₃), iso-propyl (C₃), n-butyl (C₄), t-butyl (C₄), sec-butyl (C₄), iso-butyl (C₄), n-pentyl (C₅), 3-pentyl (C₅), pentyl (C₅), neo-pentyl (C₅), 3-methyl-2-butyl (C₅), t-pentyl (C₅) and n-hexyl (C₆). Regardless of whether or not the alkyl group is modified with "substituted", each alkyl group is independently optionally substituted, for example, with 1 to 5 substituents, 1 to 3 substituents or 1 substituent. Appropriate substituents are defined as follows.

"C₂₋₆ alkenyl" refers to a linear or branched, hydrocarbon group having 2-6 carbon atoms and one or more carbon-carbon double bonds (e.g., 1, 2, or 3 carbon-carbon double bonds). One or more carbon-carbon double bonds can be internal (e.g., in 2-butenyl) or terminal (e.g., in 1-butenyl). In some embodiments, C₂₋₄ alkenyl is alternative. Examples of alkenyl include, but are not limited to, vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. Regardless of whether or not the alkenyl group is modified with "substituted", each alkenyl group is independently optionally substituted, for example, with 1 to 5 substituents, 1 to 3 substituents or 1 substituent. Appropriate substituents are defined as follows.

"C₂₋₆ alkynyl" refers to a linear or branched, hydrocarbon group having 2-6 carbon atoms, one or more carbon-carbon triple bonds (e.g., 1, 2 or 3 carbon-carbon triple bonds) and optionally one or more carbon-carbon double bonds (e.g., 1, 2 or 3 carbon-carbon double bonds). In some embodiments, C₂₋₄ alkynyl is alternative. In some embodiments, alkynyl does not contain any double bond. One or more carbon-carbon triple bonds can be internal (e.g., in 2-butynyl) or terminal (e.g., in 1-butynyl). Examples of alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. Regardless of whether or not the alkynyl group is modified with "substituted", each alkynyl group is independently optionally substituted, for example, with 1 to 5 substituents, 1 to 3 substituents or 1 substituent. Appropriate substituents are defined as follows.

"-C₁₋₆ alkylene-, -C₂₋₆ alkenylene- or -C₂₋₆ alkynylene-" refers to a divalent group of "C₁₋₆ alkyl, C₂₋₆ alkenyl or C₂₋₆ alkynyl" as defined above.

"C₁₋₆ alkylene" refers to a divalent group formed by removing another hydrogen from a C₁₋₆ alkyl group, and can be a substituted or unsubstituted alkylene group. In some embodiments, C₁₋₄ alkylene groups are particularly alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Exemplary substituted alkylene groups, e.g., alkylene groups substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, - C(CH₃)₂CH₂-, -CH₂C(CH₃)₂₋), substituted propylene (-CH(CH₃)CH₂CH₂-, -CH₂CH(CH₃)CH₂-, - CH₂CH₂CH(CH₃)-, -C(CH₃)₂CH₂CH₂-, -CH₂C(CH₃)₂CH₂-, -CH₂CH₂C(CH₃)₂-), etc.

"C₂₋₆ alkenylene" refers to a divalent group formed by removing another hydrogen from a C₂₋₆ alkenyl group, and may be a substituted or unsubstituted alkenylene group. In some embodiments, C₂₋₄ alkenylene groups are particularly alternative. Exemplary unsubstituted alkenylene groups include, but are not limited to, vinylene (-CH=CH-) and propylene (e.g., -CH=CHCH₂-, -CH₂-CH=CH-). Exemplary substituted alkenylene groups, e.g., alkenylene groups substituted with one or more alkyl (methyl) groups, include, but are not limited to, substituted ethylene (-C(CH₃)=CH-, -CH=C(CH₃)-), substituted propenylene (-C(CH₃)=CHCH₂-, -CH=C(CH₃)CH₂-, -CH=CHCH(CH₃)-, -CH=CHC(CH₃)₂-, -CH(CH₃)-CH=CH-, - C(CH₃)₂-CH=CH-, -CH₂-C(CH₃)=CH-, -CH₂-CH=C(CH₃)-), etc.

"C₁₋₆ alkoxyl" refers to a -OR group, wherein R is substituted or unsubstituted C₁₋₆ alkyl. In some embodiments, C₁₋₄ alkoxyl is alternative. Specifically, alkoxyl includes, but is not limited to, methoxy, ethoxy, n-propoxy, iso-propoxy, n-butoxy, t-butoxy, sec-butoxy, n-pentyloxy, n-hexyloxy and 1,2-dimethylbutoxy.

"Halo" or "halogen" refers to fluoro (F), chloro (Cl), bromo (Br) and iodo (I). In some embodiments, the halo group is F, Cl or Br. In some embodiments, the halo group is F or Cl. In some embodiments, the halo group is F.

Therefore, "C₁₋₆ haloalkyl" and "C₁₋₆ haloalkoxyl" refer to the above "C₁₋₆ alkyl" and "C₁₋₆ alkoxyl" substituted with one or more halo groups. In some embodiments, C₁₋₄ haloalkyl is alternative, and C₁₋₂haloalkyl is yet alternative. In some embodiments, C₁₋₄haloalkoxyl is alternative, and C₁₋₂haloalkoxyl is yet alternative. Examples of haloalkyl include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, etc. Examples of haloalkoxyl include, but are not limited to, -OCH₂F, -OCHF₂, -OCF₃, etc.

"C₃₋₁₀ cycloalkyl" refers to a non-aromatic cyclic hydrocarbon group having 3-10 ring carbon atoms and zero heteroatoms. In some embodiments, C₃₋₇ cycloalkyl is alternative, C₃₋₆ cycloalkyl is alternative, and C₅₋₆ cycloalkyl and C₃₋₄ cycloalkyl are yet alternative. Cycloalkyl also includes a ring system in which the above cycloalkyl ring is fused with one or more aryl or heteroaryl groups, wherein the point of attachment is on the cycloalkyl ring, and in such instances, the number of carbons continue to designate the number of carbons in the cycloalkyl system. Examples of cycloalkyl include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), cyclononyl (C₉), cyclononenyl (C₉), cyclodecyl (C₁₀), cyclodecenyl (C₁₀), octahydro-1H-indenyl (C₉), decahydronaphthyl (C₁₀), spiro[4.5]decyl (C₁₀), etc. Regardless of whether or not the cycloalkyl group is modified with "substituted", each cycloalkyl group is independently optionally substituted, for example, with 1 to 5 substituents, 1 to 3 substituents or 1 substituent. Appropriate substituents are defined as follows.

"3- to 10-membered heterocyclyl" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon. In heterocyclyl groups that contain one or more nitrogen atoms, the point of attachment can be a carbon or nitrogen atom, as valency permits. In some embodiments, 4- to 7-membered heterocyclyl is alternative, and it is a 4- to 7-membered non-aromatic ring system having ring carbon atoms and 1-3 ring heteroatoms. In some embodiments, 3- to 6-membered heterocyclyl is alternative, and it is a 3- to 6-membered non-aromatic ring system having ring carbon atoms and 1-3 ring heteroatoms. 5- to 6-membered heterocyclyl is yet alternative, and it is a 5- to 6-membered non-aromatic ring system having ring carbon atoms and 1-3 ring heteroatoms. "Heterocyclyl" also includes ring systems wherein the heterocyclyl, as defined above, is fused with one or more cycloalkyl, aryl or heteroaryl groups wherein the point of attachment is on the heterocyclyl ring, and in such instances, the number of ring members continues to designate the number of ring members in the heterocyclyl ring system. Regardless of whether or not the heterocyclyl group is modified with "substituted", each heterocyclyl group is independently optionally substituted, for example, with 1 to 5 substituents, 1 to 3 substituents or 1 substituent. Appropriate substituents are defined as follows.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing three heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclyl) include, without limitation, indolinyl, isoindolinyl, dihydrobenzofuranyl, dihydrobenzothienyl, benzoxazolinonyl, and the like. Exemplary 6-membered heterocyclyl groups fused to a C₆ aryl ring (also referred to herein as a 6,6-bicyclic heterocyclyl) include, without limitation, tetrahydroquinolinyl, tetrahydroisoquinolinyl, and the like.

"C₆₋₁₄ aryl" refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms. In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). In some embodiments, C₆₋₁₀ aryl is alternative, and C₆ aryl is yet alternative. "Aryl" also includes ring systems wherein the aryl ring, as defined above, is fused with one or more cycloalkyl or heterocyclyl groups wherein the point of attachment is on the aryl ring, and in such instances, the number of carbon atoms continues to designate the number of carbon atoms in the aryl ring system. Regardless of whether or not the aryl group is modified with "substituted", each aryl group is independently optionally substituted, for example, with 1 to 5 substituents, 1 to 3 substituents or 1 substituent. Appropriate substituents are defined as follows.

"5- to 10-membered heteroaryl" refers to a radical of a 5- to 10-membered monocyclic or bicyclic 4n+2 aromatic ring system (e.g., having 6 or 10 pi electrons shared in a cyclic array) having ring carbon atoms and 1-4 ring heteroatoms, wherein each heteroatom is independently selected from nitrogen, oxygen and sulfur. In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more cycloalkyl or heterocyclyl groups wherein the point of attachment is on the heteroaryl ring, and in such instances, the number of carbon atoms continues to designate the number of carbon atoms in the heteroaryl ring system. In some embodiments, 5- to 6-membered heteroaryl is alternative, and it is a 5- to 6-membered monocyclic or bicyclic 4n+2 aromatic ring system having ring carbon atoms and 1-4 ring heteroatoms. Regardless of whether or not the heteroaryl group is modified with "substituted", each heteroaryl group is independently optionally substituted, for example, with 1 to 5 substituents, 1 to 3 substituents or 1 substituent. Appropriate substituents are defined as follows.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, , oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Exemplary substituents on carbon atoms include, but are not limited to, halo, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, - CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, - NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, - SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, - P(=O)(NR^{bb} )₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, - OP(R^{cc})₃, -B(R^{aa})₂, -B(OR^{cc})₂, -BR^{aa}(OR^{cc}), alkyl, haloalkyl, alkenyl, alkynyl, carbocyclyl, heterocyclyl, aryl and heteroaryl, wherein each of alkyl, alkenyl, alkynyl, carbocyclyl, heterocyclyl, aryl and heteroaryl is independently substituted with 0, 1, 2, 3, 4 or 5 R^{dd} groups;
or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc};
each R^{aa} is independently selected from alkyl, haloalkyl, alkenyl, alkynyl, carbocyclyl, heterocyclyl, aryl and heteroaryl, or two R^{aa} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups;
each R^{bb} is independently selected from: hydrogen, -OH, -OR^{aa}, -N(R^{cc})₂, -CN, -C(=O)R^{aa}, - C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, - SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, carbocyclyl, heterocyclyl, aryl and heteroaryl, or two R^{bb} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups;
each R^{cc} is independently selected from hydrogen, alkyl, haloalkyl, alkenyl, alkynyl, carbocyclyl, heterocyclyl, aryl and heteroaryl, or two R^{cc} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups;
each R^{dd} is independently selected from: halo, -CN, -NO₂, -N₃, -SO₂H, -SO₃H, -OH, -OR^{ee}, - ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, - OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{cc}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, - C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, carbocyclyl, heterocyclyl, aryl, and heteroaryl, wherein each of alkyl, alkenyl, alkynyl, carbocyclyl, heterocyclyl, aryl and heteroaryl is independently substituted with 0, 1, 2, 3, 4 or 5 R^{gg} groups, or two geminal R^{dd} substituents can be bound to form =O or =S;
each R^{ee} is independently selected from alkyl, haloalkyl, alkenyl, alkynyl, carbocyclyl, aryl, heterocyclyl and heteroaryl, wherein each of alkyl, alkenyl, alkynyl, carbocyclyl, heterocyclyl, aryl and heteroaryl is independently substituted with 0, 1, 2, 3, 4 or 5 R^{gg} groups;
each R^{ff} is independently selected from hydrogen, alkyl, haloalkyl, alkenyl, alkynyl, carbocyclyl, heterocyclyl, aryl and heteroaryl, or two R^{ff} groups are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{gg} groups;
each R^{gg} is independently: halo, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, - NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), - SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, - OP(=O)(OC₁₋₆ alkyl)₂, C₁₋₆ alkyl, C₁₋₆ haloalkyl, C₂-C₆ alkenyl, C₂-C₆ alkynyl, C₃-C₇ carbocyclyl, C₆-C₁₀ aryl, 3- to 7-membered heterocyclyl, or 5- to 10-membered heteroaryl; or two geminal R^{gg} substituents can be bound to form =O or =S, wherein X⁻ is a counter ion.

Exemplary substituents on the nitrogen atom include but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, carbocyclyl, heterocyclyl, aryl and heteroaryl, or two R^{cc} groups connected to the nitrogen atom are bound to form heterocyclyl or heteroaryl ring, wherein each of alkyl, alkenyl, alkynyl, carbocyclyl, heterocyclyl, aryl and heteroaryl is independently substituted with 0, 1, 2, 3, 4 or 5 R^{dd} groups, and wherein R^{aa}, R^{bb}, R^{cc} and R^{dd} are as defined above.

The term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, Berge et al., describe pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid. Salts formed using conventional methods in the art such as ion exchange are also included. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Pharmaceutically acceptable salts derived from appropriate bases include alkali metal, alkaline earth metal, ammonium and N⁺(C₁₋₄alkyl)₄ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

A "subject" to which administration is contemplated includes, but is not limited to, humans (i.e., a male or female of any age group, e.g., a pediatric subject (e.g., infant, child, adolescent) or adult subject (e.g., young adult, middle-aged adult or senior adult)) and/or a non-human animal, e.g., a mammal such as primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease", "disorder" and "condition" are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

"Combination", "combined", and related terms refer to the simultaneous or sequential administration of the therapeutic agents of the present disclosure. For example, the compound disclosed herein may be administered with another therapeutic agent simultaneously or sequentially in separate unit dosage forms, or together in a single unit dosage form.

### BRIEF DESCRIPTION OF THE DRAWINGS

FIG. 1 shows the changes in tumor volume in the Karpas422 CDX efficacy study.
FIG. 2 shows the relative changes in the body weight of mice in the Karpas422 CDX efficacy study.

### DETAILED DESCRIPTION OF THE INVENTION

### Compounds

As used herein, "compounds of the present disclosure" refers to compounds of formula (X) or formulas (I) to (VII) below (including subsets of each formula), or a pharmaceutically acceptable salt, hydrate, or solvate thereof.

In one embodiment, the present disclosure relates to a compound of formula (X), or a tautomer, stereoisomer, crystal form, pharmaceutically acceptable salt, hydrate or solvate thereof: 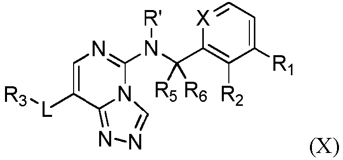 wherein
X is N or C(Rₓ);
wherein Rₓ is selected from H, halogen, C₁₋₆ alkyl, C₁₋₆ haloalkyl or C₁₋₆ alkoxy;
R₁ is selected from H, halogen, C₁₋₆ alkyl, C₁₋₆ haloalkyl or C₁₋₆ alkoxy;
R₂ is selected from H, halogen, C₁₋₆ alkyl, C₁₋₆ haloalkyl or C₁₋₆ alkoxy;
or R₁ and R₂ together form -(CH₂)ₚY-, -Y(CH₂)ₚ- or -Y(CH₂)ₚZ- group;
wherein p is 1, 2, 3, 4 or 5;
Y is selected from O, S, NH or chemical bond;
Z is selected from O, S, NH or chemical bond;
R' is selected from H, C₁₋₆ alkyl or C₁₋₆ haloalkyl;
L is selected from -C(O)-N(R)-, -C(O)-O-, -N(R₃')-C(O)-, -O-C(O)-, -S(O)_{q}-N(R)-, -S(O)_{q}-O-, -O-, -S-, - N(R")-, -C₁₋₄ alkylene- or -C₂₋₄ alkenylene-;
wherein R is selected from H, C₁₋₆ alkyl or C₁₋₆ haloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
R" is selected from H, C₁₋₆ alkyl or C₁₋₆ haloalkyl;
q is 0, 1 or 2;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-NRₐR_{b}, (CH₂)ₙ-ORₐ, (CH₂)ₙ-C₃₋₇ cycloalkyl, (CH₂)ₙ-3- to 10-membered heterocyclyl, (CH₂)ₙ-C₆₋₁₀ aryl or (CH₂)ₙ-5- to 10-membered heteroaryl;
or, R₃, R₃' and the N atom to which they are attached together form a 4- to 7-membered heterocyclyl group; wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₅ and R₆ are independently selected from H, D, C₁₋₆ alkyl or C₁₋₆ haloalkyl;
wherein R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl, -S(O)_{q}-C₁₋₆ alkyl, -S(O)_{q}-C₁₋₆ haloalkyl, -S(O)_{q}-C₁₋₆ alkoxy, -S(O)_{q}-C₃₋₇ cycloalkyl, -S(O)_{q}-4- to 7-membered heterocyclyl, -S(O)_{q}-C₆₋₁₀ aryl or -S(O)_{q}-*5-* to 10-membered heteroaryl;
wherein R_{c} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
R_{d} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
wherein the alkyl, haloalkyl, alkoxy, haloalkoxy, cycloalkyl, heterocyclyl, aryl, heteroaryl, alkylene and alkenylene are optionally substitued with substituent groups selected from H, D, CN, OH, =O, halogen, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl, or substitued with one or more D, up to complete deuteration.

### X

In one embodiment, X is N; in another embodiment, X is C(Rₓ).

In more specific embodiments, Rₓ is selected from H, halogen, C₁₋₆ alkyl, C₁₋₆ haloalkyl or C₁₋₆ alkoxy; in another specific embodiment, Rₓ is H; in another specific embodiment, Rₓ is halogen, alternatively F; in another specific embodiment, Rₓ is C₁₋₆ alkyl: in another specific embodiment, Rₓ is C₁₋₆ haloalkyl; in another specific embodiment, Rₓ is C₁₋₆ alkoxy.

### R₁ and R₂

In one embodiment, R₁ is selected from H, halogen, C₁₋₆ alkyl, C₁₋₆ haloalkyl or C₁₋₆ alkoxy; in another embodiment, R₁ is H; in another embodiment, R₁ is halogen; in another embodiment, R₁ is C₁₋₆ alkyl; in another embodiment, R₁ is C₁₋₆ haloalkyl; in another embodiment, R₁ is C₁₋₆ alkoxy.

In one embodiment, R₂ is selected from H, halogen, C₁₋₆ alkyl, C₁₋₆ haloalkyl or C₁₋₆ alkoxy; in another embodiment, R₂ is H; in another embodiment, R₂ is halogen; in another embodiment, R₂ is C₁₋₆ alkyl; in another embodiment, R₂ is C₁₋₆ haloalkyl; in another embodiment, R₂ is C₁₋₆ alkoxy.

In one embodiment, R₁ and R₂ together form -(CH₂)ₚY-, -Y(CH₂)ₚ- or -Y(CH₂)ₚZ- group; in another embodiment, R₁ and R₂ together form -(CH₂)ₚY-; in another embodiment, R₁ and R₂ together form -Y(CH₂)ₚ-; in another embodiment, R₁ and R₂ together form -Y(CH₂)ₚZ-.

In more specific embodiments, p is 1, 2, 3, 4 or 5; in another specific embodiment, p is 1; in another specific embodiment, p is 2; in another specific embodiment, p is 3; in another specific embodiment, p is 4; in another specific embodiment, p is 5.

In more specific embodiments, Y is selected from O, S, NH or chemical bond; in another specific embodiment, Y is O; in another specific embodiment, Y is S; in another specific embodiment, Y is NH; in another specific embodiment, Y is a chemical bond.

In more specific embodiments, Z is selected from O, S, NH or chemical bond; in another specific embodiment, Z is O; in another specific embodiment, Z is S; in another specific embodiment, Z is NH; in another specific embodiment, Z is a chemical bond.

### R'

In one embodiment, R' is selected from H, C₁₋₆ alkyl or C₁₋₆ haloalkyl; in another embodiment, R' is H; in another embodiment, R' is C₁₋₆ alkyl; in another embodiment, R' is C₁₋₆ haloalkyl; in another specific embodiment, R' is C₁₋₆ alkyl or C₁₋₆ haloalkyl, which is optionally substituted with 1, 2, 3, 4, 5, 6, 7, 8 or 9 D, up to complete deuteration.

### L

In one embodiment, L is selected from -C(O)-N(R)-, -C(O)-O-, -N(R₃')-C(O)-, -O-C(O)-, -S(O)_{q}-N(R)-, -S(O)_{q}-O-, -O-, -S-, -N(R")-, -C₁₋₄ alkylene- or -C₂₋₄ alkenylene-; in another embodiment, L is -C(O)-N(R)-; in another embodiment, L is -C(O)-O-; in another embodiment, L is -N(R₃')-C(O)-; in another embodiment, L is -O-C(O)-; in another embodiment, L is -S(O)_{q}-N(R)-; in another embodiment, L is -S(O)_{q}-O-; in another embodiment, L is -O-; in another embodiment, L is -S-; in another embodiment, L is -N(R")-; in another embodiment, L is -C₁₋₄ alkylene-; in another embodiment, L is -C₂₋₄ alkenylene-.

In a more specific embodiment, L is -C(O)-N(R)-, -C(O)-O-, -S(O)_{q}-N(R)- or -S(O)_{q}-O-.

In a more specific embodiment, L is -C(O)-N(R)- or -C(O)-O-.

In more specific embodiments, R is selected from H, C₁₋₆ alkyl or C₁₋₆ haloalkyl; in another specific embodiment, R is H; in another specific embodiment, R is C₁₋₆ alkyl; in another specific embodiment, R is C₁₋₆ haloalkyl; in another specific embodiment, R is C₁₋₆ alkyl or C₁₋₆ haloalkyl, which is optionally substituted with 1, 2, 3, 4, 5, 6, 7, 8 or 9 D, up to complete deuteration.

In more specific embodiments, R₃' is selected from H, (CH₂)ₘ-C₁₋₆ alkyl, (CH₂)ₘ-C₁₋₆ haloalkyl or (CH₂)ₙ-C₃₋₇ cycloalkyl; in another specific embodiment, R₃' is H; in another specific embodiment, R₃' is (CH₂)ₘ-C₁₋₆ alkyl; in another specific embodiment, R₃' is (CH₂)ₘ-C₁₋₆ haloalkyl; in another specific embodiment, R₃' is (CH₂)ₙ-C₃₋₇ cycloalkyl.

In more specific embodiments, R" is selected from H, C₁₋₆ alkyl or C₁₋₆ haloalkyl; in another specific embodiment, R" is H; in another specific embodiment, R" is C₁₋₆ alkyl; in another specific embodiment, R" is C₁₋₆ haloalkyl.

In more specific embodiments, q is 0; in another specific embodiment, q is 1; in another specific embodiment, q is 2.

### R₃

In one embodiment, R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-NRₐR_{b}, (CH₂)ₙ-ORₐ, (CH₂)ₙ-C₃₋₇ cycloalkyl, (CH₂)ₙ-3- to 10-membered heterocyclyl, (CH₂)ₙ-C₆₋₁₀ aryl or (CH₂)ₙ-5- to 10-membered heteroaryl; in another embodiment, R₃ is (CH₂)ₘ-C₁₋₆ alkyl; in another embodiment, R₃ is (CH₂)ₘ-C₁₋₆ haloalkyl; in another embodiment, R₃ is (CH₂)ₙ-C₁₋₆ alkoxy; in another embodiment, R₃ is (CH₂)ₙ-C₁₋₆ haloalkoxy; in another embodiment, R₃ is (CH₂)ₙ-NRₐR_{b}; in another embodiment, R₃ is (CH₂)ₙ-ORₐ; in another embodiment, R₃ is (CH₂)ₙ-C₃₋₇ cycloalkyl; in another embodiment, R₃ is (CH₂)ₙ-3- to 10-membered heterocyclyl; in another embodiment, R₃ is (CH₂)ₙ-C₆₋₁₀ aryl; in another embodiment, R₃ is (CH₂)ₙ-5- to 10-membered heteroaryl.

In a more specific embodiment, R₃ is selected from (CR₇R₈)ₘ-C₁₋₆ alkyl, (CR₇R₈)ₘ-C₁₋₆ haloalkyl, (CR₇R₈)ₙ-C₁₋₆ alkoxy, (CR₇R₈)ₙ-C₁₋₆ haloalkoxy, (CR₇R₈)ₙ-NRₐR_{b}, (CR₇R₈)ₙ-ORₐ, (CR₇R₈)ₙ-C₃₋₇ cycloalkyl, (CR₇R₈)ₙ-3- to 10-membered heterocyclyl, (CR₇R₈)ₙ-C₆₋₁₀ aryl or (CR₇R₈)ₙ-5- to 10-membered heteroaryl; in another more specific embodiment, R₃ is (CR₇R₈)ₘ-C₁₋₆ alkyl; in another more specific embodiment, R₃ is (CR₇R₈)ₘ-C₁₋₆ haloalkyl; in another more specific embodiment, R₃ is (CR₇R₈)ₙ-C₁₋₆ alkoxy; in another more specific embodiment, R₃ is (CR₇R₈)ₙ-C₁₋₆ haloalkoxy; in another more specific embodiment, R₃ is (CR₇R₈)ₙ-NRₐR_{b}; in another more specific embodiment, R₃ is (CR₇R₈)ₙ-ORₐ; in another more specific embodiment, R₃ is (CR₇R₈)ₙ-C₃₋₇ cycloalkyl; in another more specific embodiment, R₃ is (CR₇R₈)ₙ-3- to 10-membered heterocyclyl; in another more specific embodiment, R₃ is (CR₇R₈)ₙ-C₆₋₁₀ aryl; in another more specific embodiment, R₃ is (CR₇R₈)ₙ-5- to 10-membered heteroaryl.

In a more specific embodiment, R₃ is selected from (CR₇R₈)ₘ-C₁₋₆ alkyl, (CR₇R₈)ₘ-C₁₋₆ haloalkyl, (CR₇R₈)ₙ-C₁₋₆ alkoxy, (CR₇R₈)ₙ-C₁₋₆ haloalkoxy, (CR₇R₈)ₙ-NRₐR_{b}, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl.

In a more specific embodiment, R₃ is selected from (CR₇R₈)ₘ-C₁₋₆ alkyl, (CR₇R₈)ₘ-C₁₋₆ haloalkyl, (CR₇R₈)ₙ-C₁₋₆ alkoxy, (CR₇R₈)ₙ-NRₐR_{b}, (CR₇R₈)ₙ-C₃₋₇ cycloalkyl, (CR₇R₈)ₙ-C₆₋₁₀ aryl or (CR₇R₈)ₙ-5- to 10-membered heteroaryl.

In a more specific embodiment, R₃ is selected from (CR₇R₈)ₘ-C₁₋₆ alkyl, (CR₇R₈)ₘ-C₁₋₆ haloalkyl, (CR₇R₈)ₙ-C₁₋₆ alkoxy, (CR₇R₈)ₙ-NRₐR_{b} or (CR₇R₈)ₙ-C₃₋₄ cycloalkyl.

In a more specific embodiment, R₃ is selected from (CR₇R₈)ₘ-C₁₋₆ haloalkyl or (CR₇R₈)ₙ-C₃₋₄ cycloalkyl.

In a more specific embodiment, R₃ is selected from (CR₇R₈)ₘ-C₁₋₆ alkyl, (CR₇R₈)ₘ₋C₁₋₆ haloalkyl, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl.

In a more specific embodiment, R₃ is selected from (CR₇R₈)ₘ-C₁₋₆ alkyl, (CR₇R₈)ₘ₋C₁₋₆ haloalkyl, (CR₇R₈)ₙ-C₃₋₇ cycloalkyl or (CR₇R₈)ₙ-4- to 7-membered heterocyclyl, only containing O as heteroatom.

In a more specific embodiment, R₃ is selected from (CR₇R₈)ₘ-C₁₋₆ alkyl, (CR₇R₈)ₘ-C₁₋₆ haloalkyl or (CR₇R₈)ₙ-4- to 7-membered heterocyclyl, only containing O as heteroatom.

In another embodiment, R₃, R₃' and the N atom to which they are attached together form a 4- to 7-membered heterocyclyl group.

### m and n

In one embodiment, m is 0, 1, 2, 3, 4 or 5; in another embodiment, m is 0; in another embodiment, m is 1; in another embodiment, m is 2; in another embodiment, m is 3; in another embodiment, m is 4; in another embodiment, m is 5.

In one embodiment, n is 0, 1, 2, 3, 4 or 5; in another embodiment, n is 0; in another embodiment, n is 1; in another embodiment, n is 2; in another embodiment, n is 3; in another embodiment, n is 4; in another embodiment, n is 5.

### Rₐ, R_{b}, R₅, R₆, R₇ and R₈

In one embodiment, Rₐ is H; in another embodiment, Rₐ is C₁₋₆ alkyl; in another embodiment, Rₐ is C₁₋₆ haloalkyl.

In one embodiment, R_{b} is H; in another embodiment, R_{b} is C₁₋₆ alkyl; in another embodiment, R_{b} is C₁₋₆ haloalkyl.

In one embodiment, R₅ is H; in another embodiment, R₅ is D; in another embodiment, R₅ is C₁₋₆ alkyl; in another embodiment, R₅ is C₁₋₆ haloalkyl; in another specific embodiment, R₅ is C₁₋₆ alkyl or C₁₋₆ haloalkyl, which is optionally substituted with 1, 2, 3, 4, 5, 6, 7, 8 or 9 D, up to complete deuteration.

In one embodiment, R₆ is H; in another embodiment, R₆ is D; in another embodiment, R₆ is C₁₋₆ alkyl; in another embodiment, R₆ is C₁₋₆ haloalkyl; in another specific embodiment, R₆ is C₁₋₆ alkyl or C₁₋₆ haloalkyl, which is optionally substituted with 1, 2, 3, 4, 5, 6, 7, 8 or 9 D, up to complete deuteration.

In one embodiment, R₇ is H; in another embodiment, R₇ is D; in another embodiment, R₇ is C₁₋₆ alkyl; in another embodiment, R₇ is C₁₋₆ haloalkyl; in another specific embodiment, R₇ is C₁₋₆ alkyl or C₁₋₆ haloalkyl, which is optionally substituted with 1, 2, 3, 4, 5, 6, 7, 8 or 9 D, up to complete deuteration.

In one embodiment, R₈ is H; in another embodiment, R₈ is D; in another embodiment, R₈ is C₁₋₆ alkyl; in another embodiment, R₈ is C₁₋₆ haloalkyl; in another specific embodiment, R₈ is C₁₋₆ alkyl or C₁₋₆ haloalkyl, which is optionally substituted with 1, 2, 3, 4, 5, 6, 7, 8 or 9 D, up to complete deuteration.

In another specific embodiment, R₇ and R₈ on the same carbon atom are combined to form =O.

### Substituent groups of R₃

In one embodiment, R₃ is optionally substitued with one, two, three or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4-to 7-membered heterocyclyl, C₆₋₁₀ aryl, 5- to 10-membered heteroaryl, (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl, -S(O)_{q}-C₁₋₆ alkyl, -S(O)_{q}-C₁₋₆ haloalkyl, -S(O)_{q}-C₁₋₆ alkoxy, -S(O)_{q}-C₃₋₇ cycloalkyl, -S(O)_{q}-4- to 7-membered heterocyclyl, -S(O)_{q}-C₆₋₁₀ aryl or -S(O)_{q}-5- to 10-membered heteroaryl.

In one embodiment, R₃ is optionally substitued with one, two, three or more substituent groups selected from H, D, halogen, CN, OH, C₁₋₆ alkyl, C₁₋₆ haloalkyl, C₃₋₇ cycloalkyl, 4- to 7-membered heterocyclyl, C₆₋₁₀ aryl, 5- to 10-membered heteroaryl, (CR₇R₈)ₙ-C₁₋₆ alkoxy or -S(O)_{q}-C₁₋₆ alkyl.

In one embodiment, R₃ is optionally substituted with a group selected from H, D, halogen, C₁₋₆ alkyl, C₁₋₆ haloalkyl, C₁₋₆ alkoxy or C₃₋₇ cycloalkyl.

In one embodiment, R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl.

In one embodiment, R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, -C(O)-C₁₋₆ alkyl, - C(O)-C₃₋₇ cycloalkyl or -O-C₆₋₁₀ aryl.

In one embodiment, R₃ is optionally substituted with a group selected from H, D, C₁₋₆ alkyl, C₁₋₆ haloalkyl or C₁₋₆ alkoxy.

Any technical solution in any of the above specific embodiments or any combination thereof can be combined with any technical solution in other specific embodiments or any combination thereof. For example, any technical solution of X or any combination thereof can be combined with any technical solution of R₁, R₂, R', L and R₃, etc. or any combination thereof. The present disclosure is intended to include the combination of all these technical solutions, which is not listed one by one due to space limitation.

In more specific embodiments, the present disclosure relates to a compound of formula (II), or a tautomer, stereoisomer, crystal form, pharmaceutically acceptable salt, hydrate or solvate thereof: 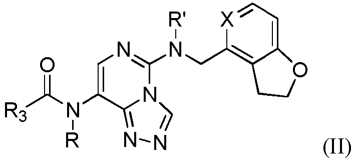 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl;
wherein m is 0, 1, 2, 3, 4 or 5;
n is 0, 1, 2, 3, 4 or 5.

In another more specific embodiment, the present disclosure relates to a compound of formula (II), or a tautomer, stereoisomer, crystal form, pharmaceutically acceptable salt, hydrate or solvate thereof:
wherein
X is N or C(Rₓ); alternatively, X is C(Rₓ);
wherein Rₓ is selected from H, halogen, C₁₋₆ alkyl or C₁₋₆ haloalkyl; alternatively, Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₄ cycloalkyl or (CH₂)ₙ-C₆₋₁₀ aryl;
wherein m is 0, 1, 2, 3, 4 or 5;
n is 0, 1, 2, 3, 4 or 5.

In another more specific embodiment, the present disclosure relates to a compound of formula (II), or a tautomer, stereoisomer, crystal form, pharmaceutically acceptable salt, hydrate or solvate thereof:
wherein
X is N or C(Rₓ); alternatively, X is C(Rₓ);
wherein Rₓ is selected from H, halogen, C₁₋₆ alkyl or C₁₋₆ haloalkyl; alternatively, Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl or (CH₂)ₙ-C₃₋₄ cycloalkyl; alternatively, R₃ is selected from (CH₂)ₘ-C₁₋₆ haloalkyl or (CH₂)ₙ-C₃₋₄ cycloalkyl;
wherein m is 0, 1, 2, 3, 4 or 5;
n is 0, 1, 2, 3, 4 or 5.

In another more specific embodiment, the present disclosure relates to a compound of formula (II), or a tautomer, stereoisomer, crystal form, pharmaceutically acceptable salt, hydrate or solvate thereof:
wherein
X is C(Rₓ);
wherein Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ haloalkyl or (CH₂)ₙ-C₃₋₄ cycloalkyl;
wherein m is 0, 1, 2 or 3;
n is 0, 1, 2 or 3.

In another more specific embodiment, the present disclosure relates to a compound of formula (III), or a tautomer, stereoisomer, crystal form, pharmaceutically acceptable salt, hydrate or solvate thereof: 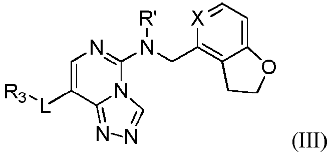 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
L is selected from -O-, -S-, -N(R")-, -C₁₋₄ alkylene- or -C₂₋₄ alkenylene-; alternatively, L is -O-;
wherein R" is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl; alternatively, R₃ is selected from (CH₂)ₘ-C₁₋₆ alkyl, (CH₂)ₘ-C₁₋₆ haloalkyl, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl;
wherein m is 0, 1, 2, 3, 4 or 5;
n is 0, 1, 2, 3, 4 or 5.

In another more specific embodiment, the present disclosure relates to a compound of formula (III), or a tautomer, stereoisomer, crystal form, pharmaceutically acceptable salt, hydrate or solvate thereof:
wherein
X is C(Rₓ);
wherein Rₓ is selected from H or halogen;
R' is selected from H, C₁₋₆ alkyl or C₁₋₆ haloalkyl;
L is -O-;
R₃ is selected from (CH₂)ₙ-C₃₋₇ cycloalkyl or (CH₂)ₙ-4- to 7-membered heterocyclyl only containing O as heteroatom;
wherein m is 0, 1, 2, 3, 4 or 5;
n is 0, 1, 2, 3, 4 or 5.

In another more specific embodiment, the present disclosure relates to a compound of formula (III), or a tautomer, stereoisomer, crystal form, pharmaceutically acceptable salt, hydrate or solvate thereof:
wherein
X is N or C(Rₓ); alternatively, X is C(Rₓ);
wherein Rₓ is selected from H, halogen, C₁₋₆ alkyl or C₁₋₆ haloalkyl; alternatively, wherein Rₓ is selected from H or halogen;
R' is selected from H, C₁₋₆ alkyl or C₁₋₆ haloalkyl;
L is O;
R₃ is selected from (CH₂)ₘ-C₁₋₆ alkyl, (CH₂)ₘ-C₁₋₆ haloalkyl, (CH₂)ₙ-C₃₋₇ cycloalkyl or (CH₂)ₙ-5- to 6-membered heteroaryl containing O as heteroatom;
wherein m is 0, 1, 2, 3, 4 or 5;
n is 0, 1, 2, 3, 4 or 5.

In another more specific embodiment, the present disclosure relates to a compound of formula (III), or a tautomer, stereoisomer, crystal form, pharmaceutically acceptable salt, hydrate or solvate thereof:
wherein
X is N or C(Rₓ); alternatively, X is C(Rₓ);
wherein Rₓ is selected from H, halogen, C₁₋₆ alkyl or C₁₋₆ haloalkyl; alternatively, wherein Rₓ is selected from H or halogen;
R' is selected from H, C₁₋₆ alkyl or C₁₋₆ haloalkyl;
L is -O-;
R₃ is selected from (CH₂)ₘ-C₁₋₆ alkyl, (CH₂)ₘ-C₁₋₆ haloalkyl or (CH₂)ₙ-C₃₋₇ cycloalkyl;
wherein m is 0, 1, 2, 3, 4 or 5;
n is 0, 1, 2, 3, 4 or 5.

In another more specific embodiment, the present disclosure relates to a compound of formula (IV), or a tautomer, stereoisomer, crystal form, pharmaceutically acceptable salt, hydrate or solvate thereof: 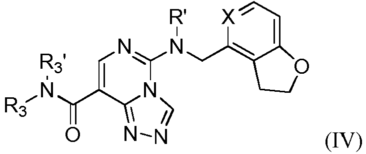 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
or, R₃, R₃' and the N atom to which they are attached together form a 4- to 7-membered heterocyclyl group; wherein m is 0, 1, 2, 3, 4 or 5;
n is 0, 1, 2, 3, 4 or 5.

In another more specific embodiment, the present disclosure relates to a compound of formula (IV), or a tautomer, stereoisomer, crystal form, pharmaceutically acceptable salt, hydrate or solvate thereof:
wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl or (CH₂)ₙ-C₃₋₇ cycloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
or, R₃, R₃' and the N atom to which they are attached together form a 4- to 7-membered heterocyclyl group; wherein m is 0, 1, 2, 3, 4 or 5;
n is 0, 1, 2, 3, 4 or 5.

In another more specific embodiment, the present disclosure relates to a compound of formula (V), or a tautomer, stereoisomer, crystal form, pharmaceutically acceptable salt, hydrate or solvate thereof: 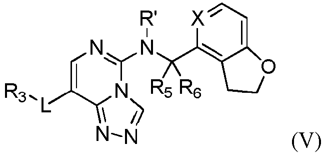 wherein
X is N or C(Rₓ); alternatively, X is C(Rₓ),
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
L is -C(O)-N(R)-, -C(O)-O-, -S(O)_{q}-N(R)- or -S(O)_{q}-O-;
wherein R is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
q is 0, 1 or 2;
R₃ is selected from (CR₇R₈)ₘ-C₁₋₆ alkyl, (CR₇R₈)ₘ-C₁₋₆ haloalkyl, (CR₇R₈)ₙ₋C₁₋₆ alkoxy, (CR₇R₈)ₙ₋C₁₋₆ haloalkoxy, (CR₇R₈)ₙ-NRₐR_{b}, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl;
wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substitued with a group selected from H, D, halogen, CN, OH, C₁₋₆ alkyl, C₁₋₆ haloalkyl, C₃₋₇ cycloalkyl, 4- to 7-membered heterocyclyl, C₆₋₁₀ aryl, 5- to 10-membered heteroaryl, (CR₇R₈)ₙ-C₁₋₆ alkoxy or -S(O)_{q}-C₁₋₆ alkyl;
wherein the alkyl, haloalkyl, alkoxy, haloalkoxy, cycloalkyl, heterocyclyl, aryl and heteroaryl are optionally substitued with one or more D, up to complete deuteration.

In another more specific embodiment, the present disclosure relates to a compound of formula (V), or a tautomer, stereoisomer, crystal form, pharmaceutically acceptable salt, hydrate or solvate thereof:
wherein
X is C(Rₓ),
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
L is -C(O)-N(R)-, -C(O)-O-, -S(O)_{q}-N(R)- or -S(O)_{q}-O-;
wherein R is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
q is 0, 1 or 2;
R₃ is selected from (CR₇R₈)ₘ-C₁₋₆ alkyl, (CR₇R₈)ₘ-C₁₋₆ haloalkyl, (CR₇R₈)ₙ₋C₁₋₆ alkoxy, (CR₇R₈)ₙ-NRₐR_{b}, (CR₇R₈)ₙ-C₃₋₇ cycloalkyl, (CR₇R₈)ₙ-C₆₋₁₀ aryl or (CR₇R₈)ₙ-5- to 10-membered heteroaryl;
wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with a group selected from H, D, halogen, C₁₋₆ alkyl, C₁₋₆ haloalkyl, C₁₋₆ alkoxy or C₃₋₇ cycloalkyl.

In another more specific embodiment, the present disclosure relates to a compound of formula (V), or a tautomer, stereoisomer, crystal form, pharmaceutically acceptable salt, hydrate or solvate thereof:
wherein
X is C(Rₓ),
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
L is -C(O)-N(R)- or -C(O)-O-;
wherein R is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl, (CR₇R₈)ₙ-C₁₋₆ alkoxy, (CR₇R₈)ₙ-NRₐR_{b} or (CR₇R₈)ₙ-C₃₋₄ cycloalkyl;
wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₇ and R₈ are independently selected from H or D;
R₅ and R₆ are independently selected from H or D;
wherein R₃ is optionally substituted with a group selected from H, D, C₁₋₆ alkyl, C₁₋₆ haloalkyl or C₁₋₆ alkoxy.

In another more specific embodiment, the present disclosure relates to a compound of formula (V), or a tautomer, stereoisomer, crystal form, pharmaceutically acceptable salt, hydrate or solvate thereof:
wherein
X is C(Rₓ),
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
L is -C(O)-N(R)-;
wherein R is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ haloalkyl or (CR₇R₈)ₙ-C₃₋₄ cycloalkyl;
wherein m is 0, 1, 2, 3, 4 or 5;
n is 0, 1, 2, 3, 4 or 5;
R₇ and R₈ are independently selected from H or D;
R₅ and R₆ are independently selected from H or D.

In another more specific embodiment, the present disclosure relates to a compound of formula (VI), or a tautomer, stereoisomer, crystal form, pharmaceutically acceptable salt, hydrate or solvate thereof: 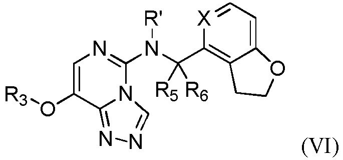 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration; or R₇ and R₈ on the same carbon atom are combined to form =O;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl;
wherein R_{c} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
R_{d} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
wherein the alkyl, haloalkyl, alkoxy, cycloalkyl, heterocyclyl, aryl and heteroaryl are optionally substitued with substituent groups selected from H, D, CN, OH, =O, halogen, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl, or substitued with one or more D, up to complete deuteration.

In another more specific embodiment, the present disclosure relates to a compound of formula (VI), or a tautomer, stereoisomer, crystal form, pharmaceutically acceptable salt, hydrate or solvate thereof:
wherein
R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, -C(O)-C₁₋₆ alkyl, -C(O)-C₃₋₇ cycloalkyl or -O-C₆₋₁₀ aryl;
wherein R_{c} is selected from H, C₁₋₆ alkyl, C₁₋₆ haloalkyl or -C(O)-C₃₋₇ cycloalkyl;
R_{d} is selected from H, C₁₋₆ alkyl, C₁₋₆ haloalkyl or -C(O)-C₃₋₇ cycloalkyl;
wherein the alkyl, haloalkyl, alkoxy, cycloalkyl, heterocyclyl, aryl and heteroaryl are optionally substitued with substituent groups selected from H, D, CN, =O, halogen, C₁₋₆ alkyl, C₁₋₆ haloalkyl, C₁₋₆ alkoxy or -C(O)-C₁₋₆ alkyl, or substitued with one or more D, up to complete deuteration.

In another more specific embodiment, the present disclosure relates to a compound of formula (VI), or a tautomer, stereoisomer, crystal form, pharmaceutically acceptable salt, hydrate or solvate thereof:
wherein
X is C(Rₓ);
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl, (CR₇R₈)ₙ-C₃₋₇ cycloalkyl or (CR₇R₈)ₙ-4- to 7-membered heterocyclyl only containing O as heteroatom;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with a group selected from H, D, C₁₋₆ alkyl, C₁₋₆ haloalkyl or C₁₋₆ alkoxy.

In another more specific embodiment, the present disclosure relates to a compound of formula (VI), or a tautomer, stereoisomer, crystal form, pharmaceutically acceptable salt, hydrate or solvate thereof:
wherein
X is C(Rₓ);
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl or (CR₇R₈)ₙ-4- to 7-membered heterocyclyl only containing O as heteroatom;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with a group selected from H, D, C₁₋₆ alkyl, C₁₋₆ haloalkyl or C₁₋₆ alkoxy.

In another more specific embodiment, the present disclosure relates to a compound of formula (VII), or a tautomer, stereoisomer, crystal form, pharmaceutically acceptable salt, hydrate or solvate thereof: 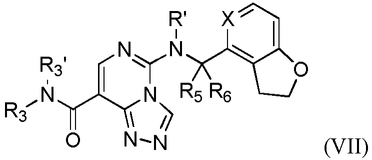 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R₃ is selected from (CR₇R₈)ₘ-C₁₋₆ alkyl, (CR₇R₈)ₘ-C₁₋₆ haloalkyl, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl;
R₃' is selected from H, (CR₇R₈)ₘ-C₁₋₆ alkyl, (CR₇R₈)ₘ-C₁₋₆ haloalkyl or (CR₇R₈)ₙ-C₃₋₇ cycloalkyl;
or, R₃, R₃' and the N atom to which they are attached together form a 4- to 7-membered heterocyclyl group; wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration; or R₇ and R₈ on the same carbon atom are combined to form =O;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with one or more groups selected from H, D, halogen, C₁₋₆ alkyl, C₁₋₆ haloalkyl or C₁₋₆ alkoxy.

In another more specific embodiment, the present disclosure relates to a compound of formula (VII), or a tautomer, stereoisomer, crystal form, pharmaceutically acceptable salt, hydrate or solvate thereof:
wherein
X is C(Rₓ),
wherein Rₓ is selected from H or halogen;
R' is selected from H, C₁₋₆ alkyl or C₁₋₆ haloalkyl;
R₃ is selected from (CR₇R₈)ₘ-C₁₋₆ alkyl, (CR₇R₈)ₘ-C₁₋₆ haloalkyl or (CR₇R₈)ₙ-C₃₋₇ cycloalkyl;
R₃' is selected from H, (CR₇R₈)ₘ-C₁₋₆ alkyl, (CR₇R₈)ₘ-C₁₋₆ haloalkyl or (CR₇R₈)ₙ-C₃₋₇ cycloalkyl;
or, R₃, R₃' and the N atom to which they are attached together form a 4- to 7-membered heterocyclyl group; m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with one or more groups selected from H, D, halogen, C₁₋₆ alkyl, C₁₋₆ haloalkyl or C₁₋₆ alkoxy.

In another more specific embodiment, the present disclosure relates to the following compounds, or a tautomer, stereoisomer, crystal form, pharmaceutically acceptable salt, hydrate or solvate thereof:

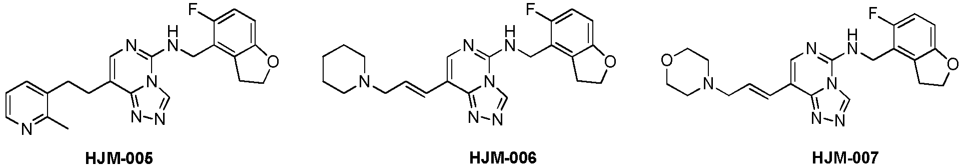

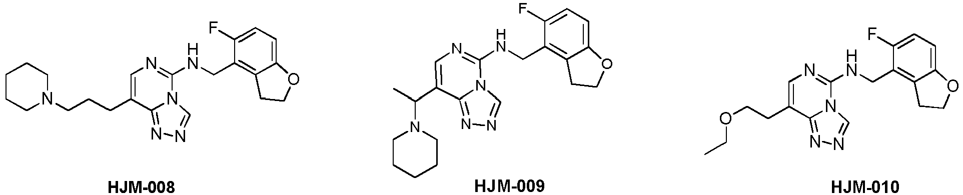

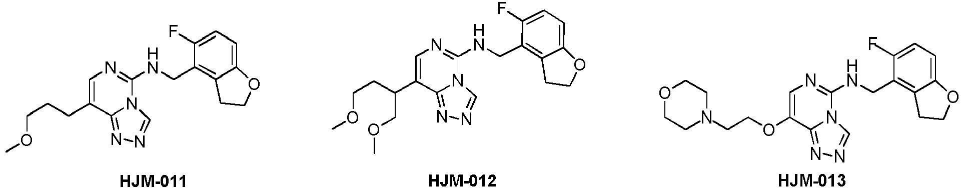

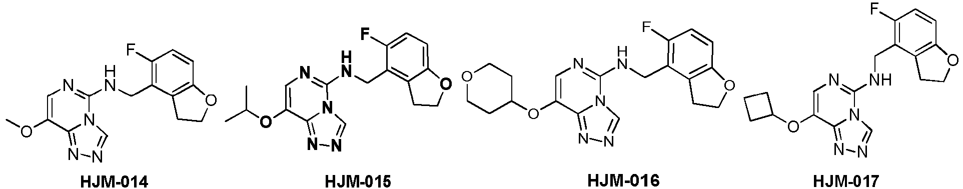

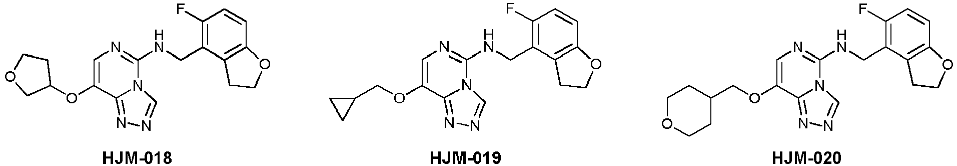

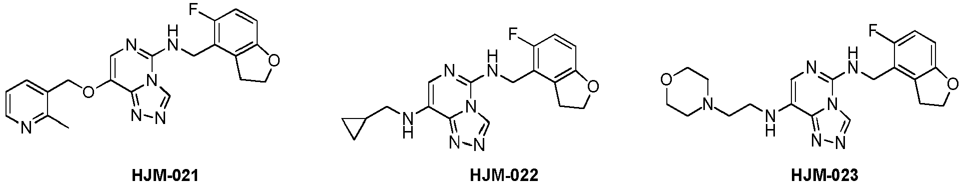

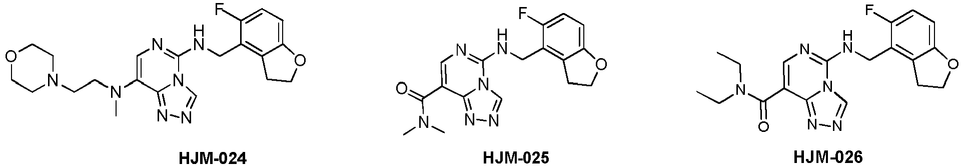

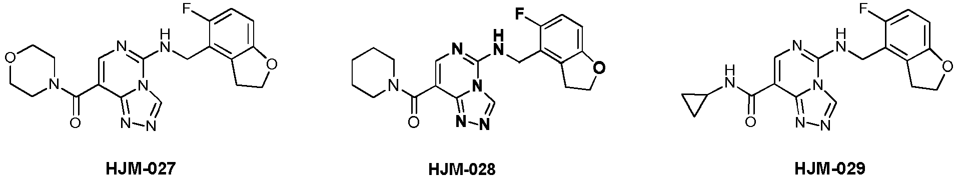

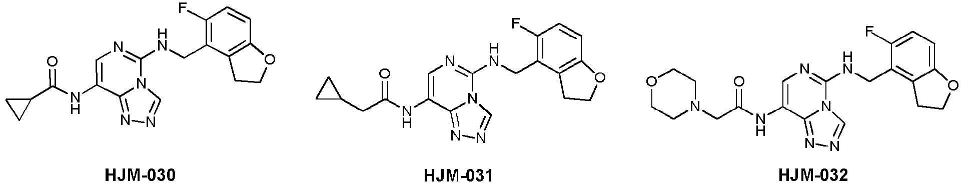

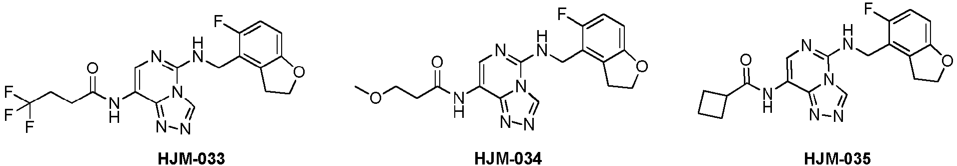

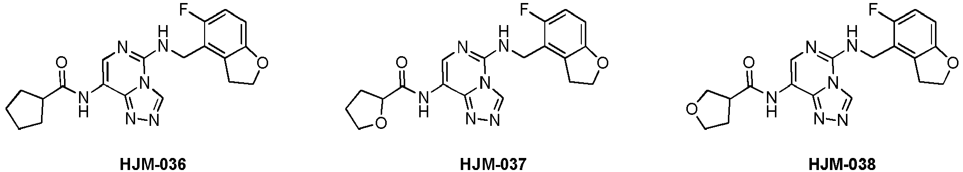

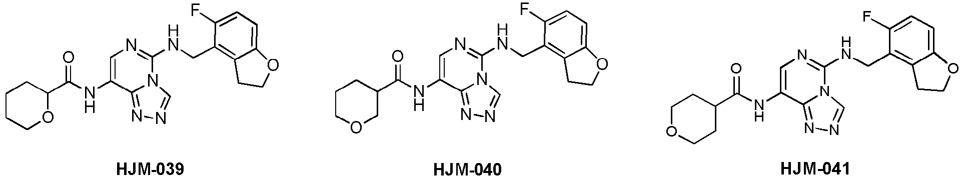

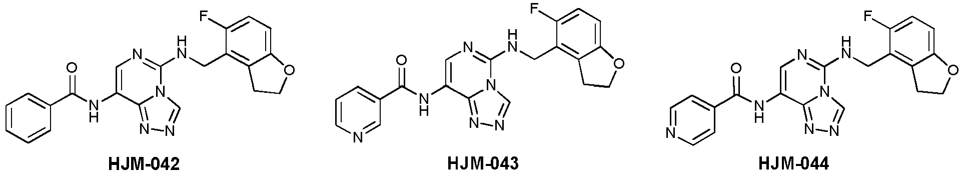

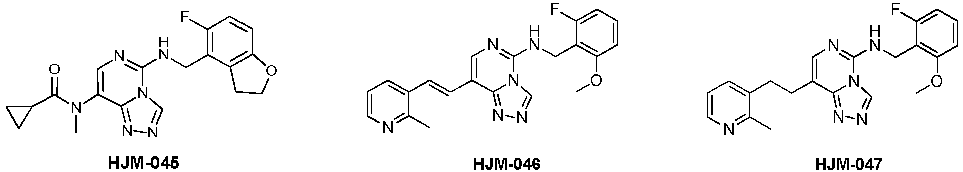

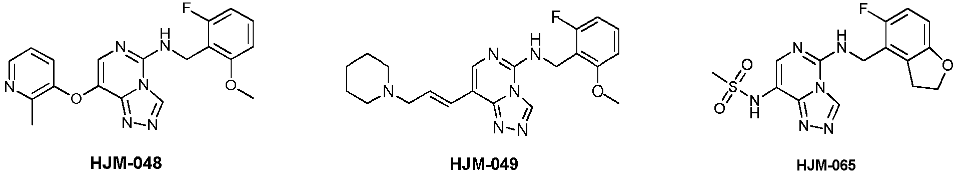

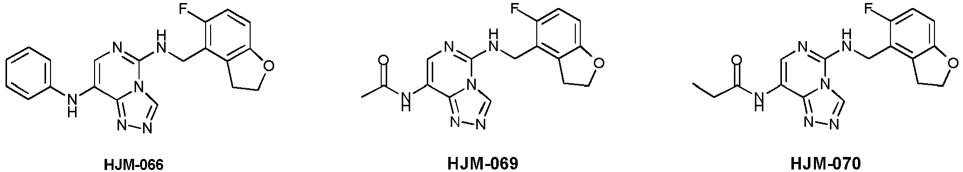

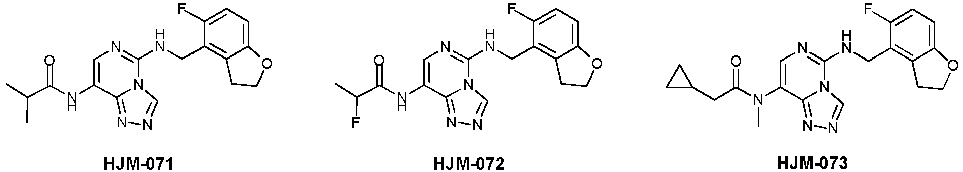

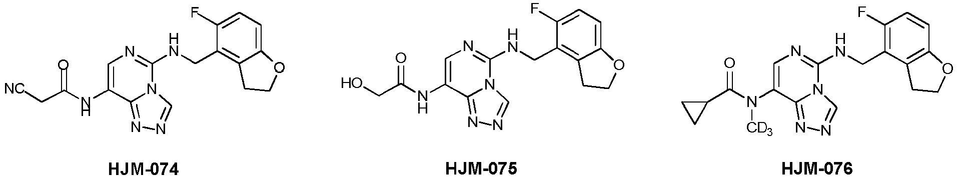

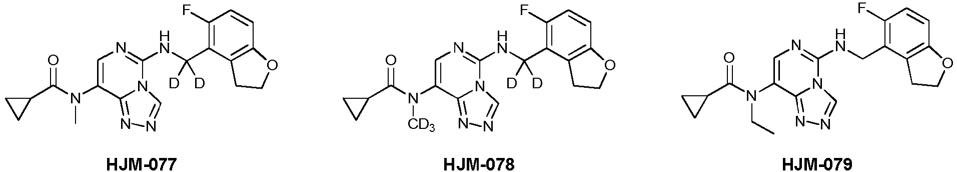

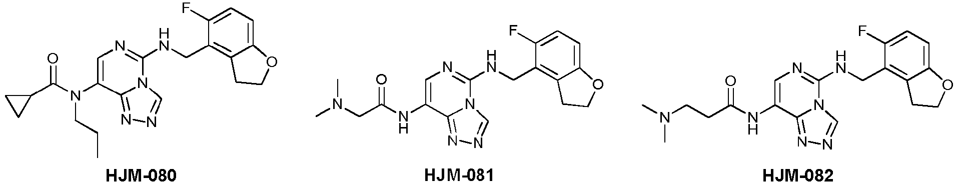

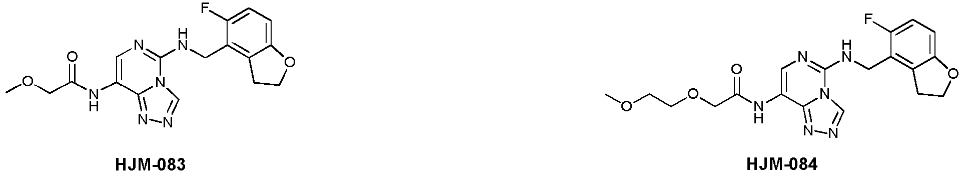

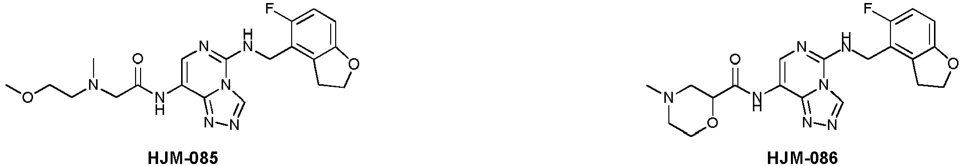

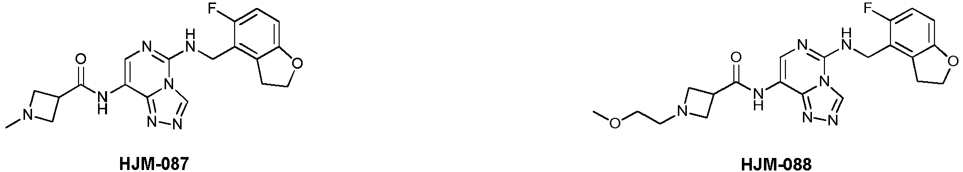

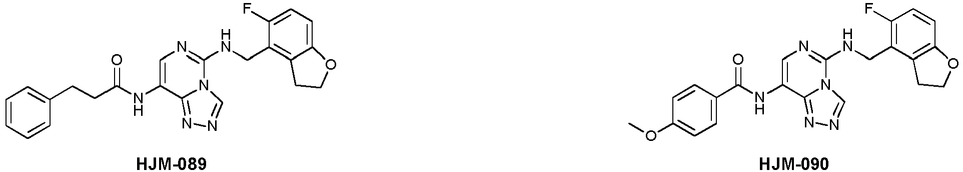

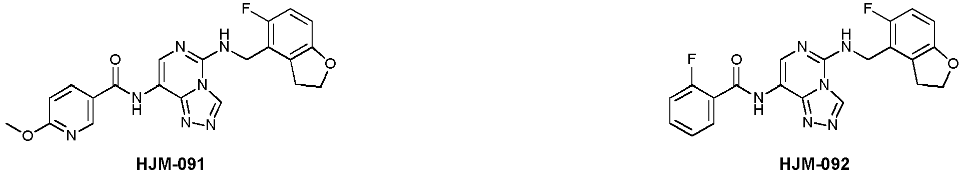

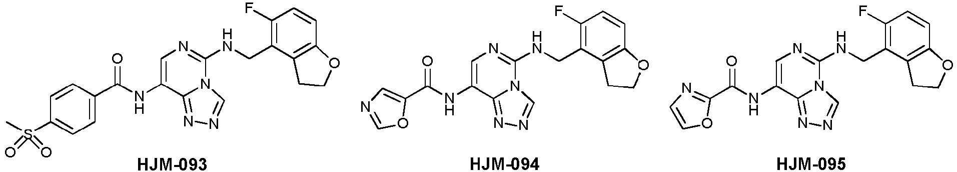

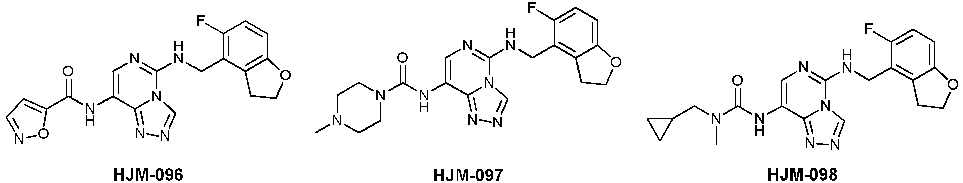

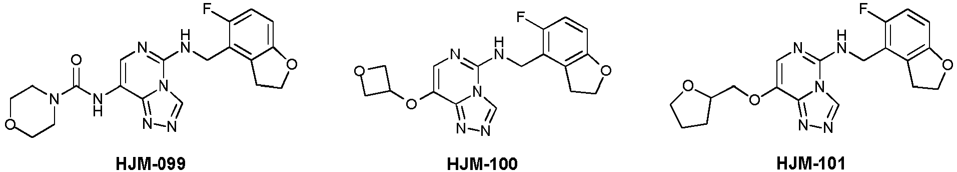

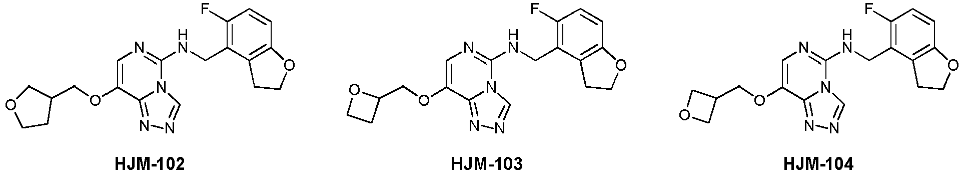

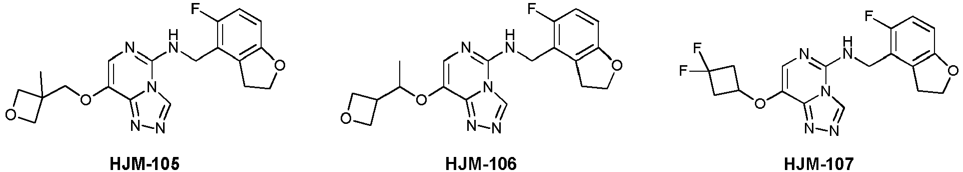

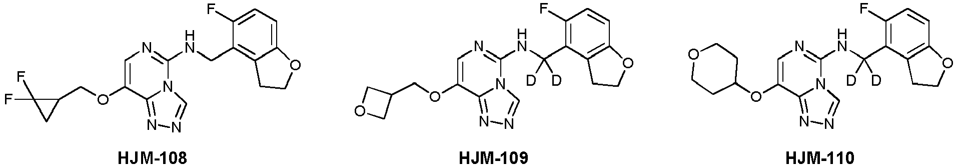

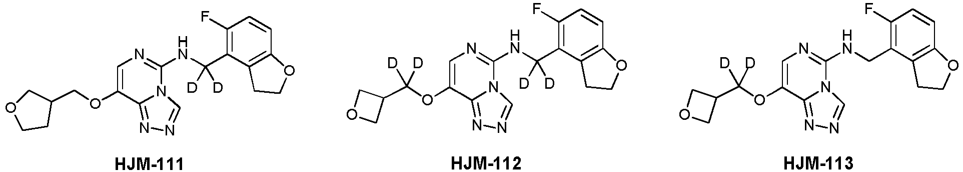

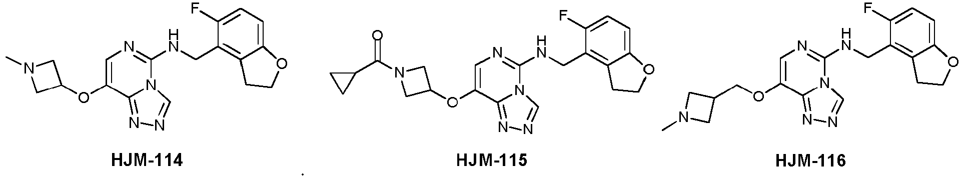

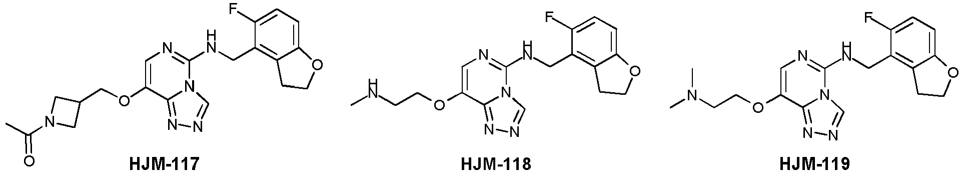

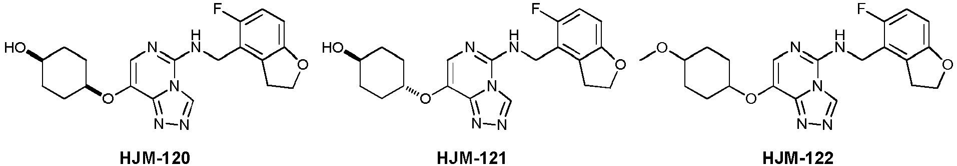

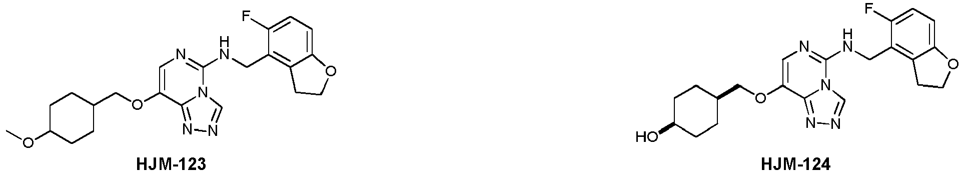

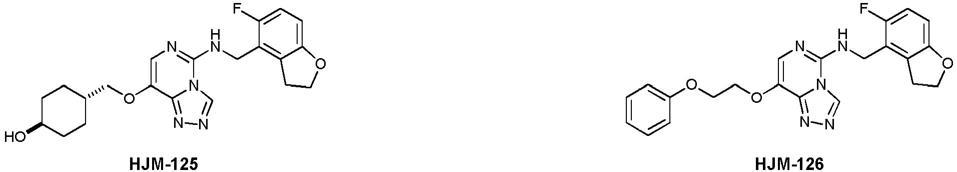

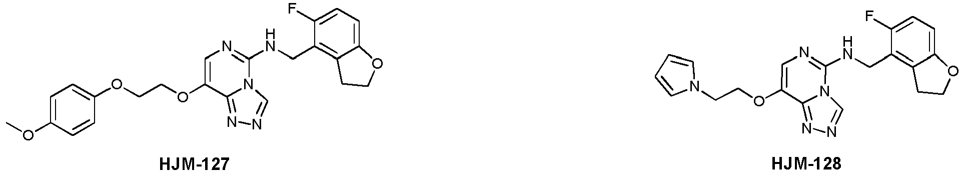

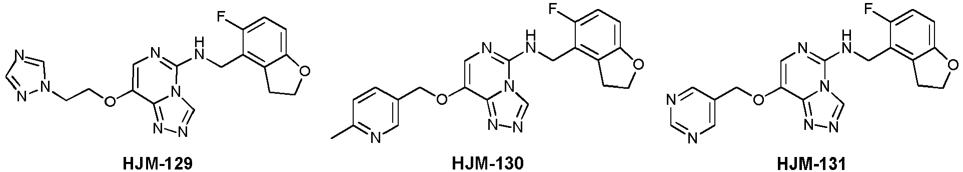

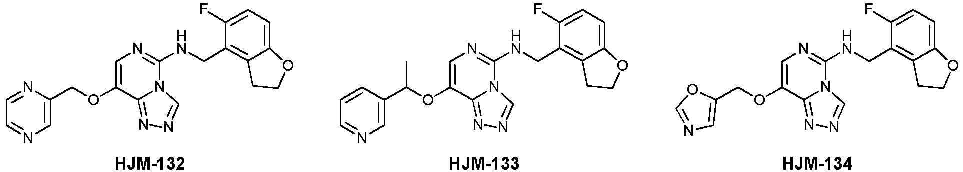

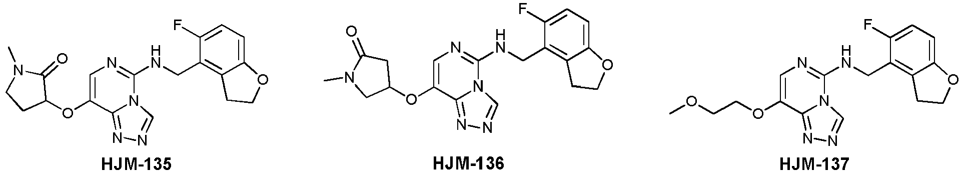

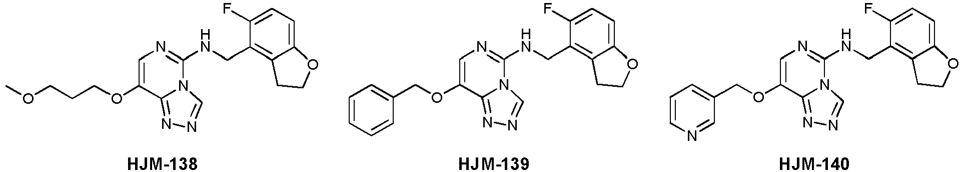

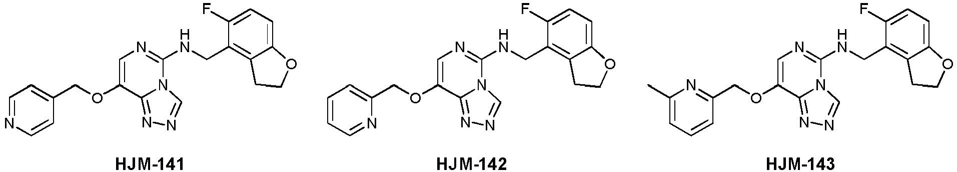

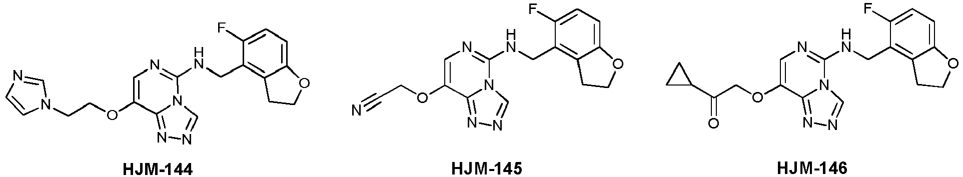

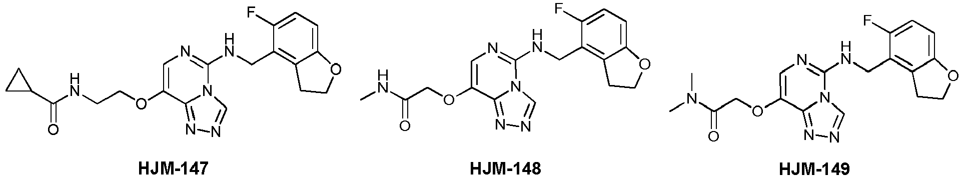

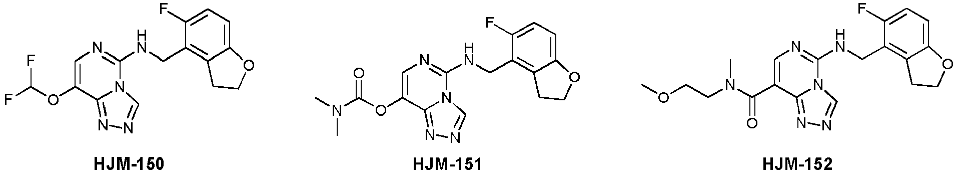

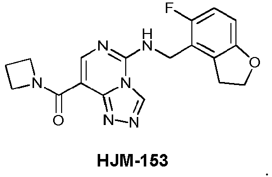

The compounds disclosed herein may include one or more asymmetric centers, and thus may exist in a variety of stereoisomeric forms, for example, enantiomers and/or diastereomers. For example, the compounds disclosed herein may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

"Tautomer" refers to an isomer in which one functional group in a compound changes its structure into another functional group, wherein the compound and the isomer can quickly convert between each other, thus being in dynamic equilibrium; this two isomers are called tautomers.

It will be understood by those skilled in the art that the organic compounds can form complexes with solvents in which they are reacted or from which they are precipitated or crystallized. These complexes are known as "solvates." Where the solvent is water, the complex is known as "hydrate." The present disclosure encompasses all solvates of the compounds disclosed herein.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula R·x H₂O, wherein R is the compound, and x is a number greater than 0. Given compounds can form more than one type of hydrates, including, for example, monohydrates (x is 1), lower hydrates (x is a number greater than 0 and smaller than 1, for example, hemihydrates (R·0.5 H₂O)) and polyhydrates (x is a number greater than 1, for example, dihydrates (R·2 H₂O) and hexahydrates (R·6 H₂O)).

Compounds disclosed herein may be in an amorphous or crystalline form (crystal form or polymorph). Furthermore, the compounds disclosed herein may exist in one or more crystalline forms. Therefore, the present disclosure includes all amorphous or crystalline forms of the compounds disclosed herein within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes, which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds disclosed herein that comprise the above isotopes and/or other isotopes of other atoms, and pharmaceutically acceptable salts of said compounds all are within the scope of the present disclosure. Certain isotope-labeled compounds disclosed herein, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are alternative, because they are easy to prepare and detect. Furthermore, replaced by heavier isotopes, such as deuterium, which is ²H, may provide therapeutic benefits due to the higher metabolic stability, such as prolonging the half-life *in vivo* or decreasing the dosage requirements, and thus may be alternative in some cases. Isotope-labeled compounds of formula (I) of the present disclosure can be prepared generally by using readily available isotope-labeled reagents to replace non-isotope-labeled reagents in the following schemes and/or the procedures disclosed in the examples and preparation examples.

### Pharmaceutical composition, formulation, and kit

In another aspect, the disclosure provides a pharmaceutical composition comprising a compound disclosed herein (also referred to as the "active ingredient") and a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other materials) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

The pharmaceutical composition provided by the present disclosure may be administered by a variety of routes including, but not limited to, oral administration, parenteral administration, inhalation administration, topical administration, rectal administration, nasal administration, oral mucosal administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc.,* or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present disclosure may be further delivered using a variety of dosing methods. For example, in certain embodiments, the pharmaceutical composition may be given as a bolus, e.g., in order to raise the concentration of the compound in the blood to an effective level rapidly. The placement of the bolus dose depends on the desired systemic levels of the active ingredient, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and yet alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavours and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavouring agent such as peppermint, methyl salicylate, or orange flavouring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration may be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds disclosed herein can also be administered in sustained release forms or from sustained release drug delivery systems. A description of representative sustained release materials may be found in Remington's Pharmaceutical Sciences.

The present disclosure also relates to the pharmaceutically acceptable formulations of a compound disclosed herein. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as Captisol. See, *e.g.,* U.S. 5,376,645. In certain embodiments, the formulation comprises hexapropyl-β-cyclodextrin (e.g., 10-50% in water).

### Indication

Examples of EED- and/or PRC2-mediated diseases or disorders to be treated by the compounds of the present disclosure include, but are not limited to, diffuse Large B-Cell Lymphoma (DLBCL), follicular lymphoma, other lymphomas, leukemia, multiple myeloma, mesothelioma, gastric cancer, malignant rhabdomyoma, liver cancer, prostate cancer, breast cancer, bile duct and gallbladder cancer, bladder cancer, brain tumors (including neuroblastoma, schwannoma, glioma, glioblastoma and astrocytoma), cervical cancer, colon cancer, melanoma, endometrial cancer, esophageal cancer, head and neck cancer, lung cancer, nasopharyngeal cancer, ovarian cancer, pancreatic cancer, renal cell cancer, rectal cancer, thyroid cancer, parathyroid tumor, uterine tumor and soft tissue sarcomas such as rhabdomyosarcoma (RMS), Kaposi's sarcoma, synovial sarcoma, osteosarcoma and Ewing's sarcoma.

In addition, the compounds of the present disclosure can also be used for tumor immunization.

### Drug combination

The compound of the present disclosure can be used in combination with one or more other active ingredients in pharmaceutical compositions or methods to treat the diseases and conditions described herein. Other additional active ingredients include other therapeutic agents or medicines that mitigate adverse effects of the therapeutic agent on the intended disease target. The combination can be used to increase efficacy, improve other disease symptoms, reduce one or more negative effects, or reduce the required dosage of the compound of the present disclosure. The additional active ingredient may be formulated into a pharmaceutical composition separate from the compound of the present disclosure or may be included in a single pharmaceutical composition with the compound of the present disclosure. The additional active ingredient may be administered simultaneously with, before, or after the administration of the compound of the present disclosure.

Common chemotherapeutic agents considered for combination therapy include anastrozole, bicalutamide, bleomycin sulfate, busulfan, busulfan injection, capecitabine, N4-pentoxycarbonyl-5-deoxy-5-fluorocytidine, carboplatin, carmustine, chlorambucil, cisplatin, cladribine, cyclophosphamide, cytarabine, cytosine cytarabine, cytarabine liposome injection, dacarbazine, actinomycin (actinomycin D), daunorubicin hydrochloride, daunorubicin citrate liposome injection, dexamethasone, Docetaxel, doxorubicin hydrochloride, etoposide, fludarabine phosphate, 5-fluorouracil, flutamide, tezacitabine, Gemcitabine (difluorodeoxycytidine), hydroxyurea, idarubicin, ifosfamide, irinotecan, L-asparaginase, calcium leucovorin, melphalan, 6-mercaptopurine, methotrexate, mitoxantrone, mylotarg, paclitaxel, nab-paclitaxel, Phoenix (Yttrium90/MX-DTPA), pentostatin, carmustine implants with polybenzamide 20 as carrier, tamoxifen citrate, teniposide, 6-thioguanine, thiatepa, tirapazamine, topotecan hydrochloride for injection, vinblastine, vincristine and vinorelbine.

In addition, the compounds of the present disclosure can be used in combination with immune checkpoint inhibitors.

The present disclosure provides use of a first therapeutic agent and optionally a second therapeutic agent in a method of treating a disease or condition mediated by EED and/or PRC2, comprising administering to a patient in need thereof a therapeutically effective amount of a first therapeutic agent and optionally a second therapeutic agent, wherein the first therapeutic agent is an EED inhibitor, and the second therapeutic agent is another type of therapeutic agent, wherein the disease or disorder is selected from diffuse large B-cell lymphoma (DLBCL), follicular lymphoma, other lymphomas, leukemia, multiple myeloma, gastric cancer, malignant rhabdomyoma and liver cancer.

### Detailed description

The present disclosure is further described below in combination with specific examples, but the present disclosure is not limited to these examples.

### Examples

### Preparation of compound of formula (I)

The present disclosure also provides the preparation method of a compound as shown in formula (I) and an intermediate thereof, the method comprising:

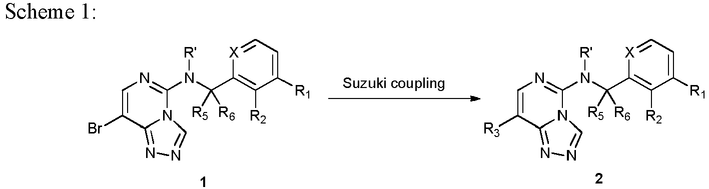

The target product **2** was afforded by Suzuki coupling reaction of bromide **1** with various boric acids with R₃ groups or their equivalents under the action of palladium catalyst.

In the Scheme, the definition of each group was the same as above.

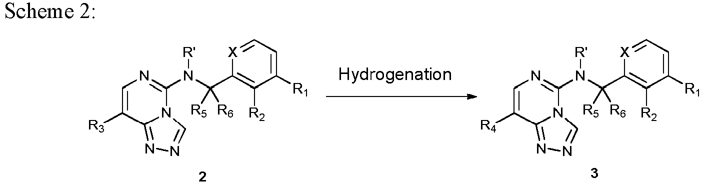

Compound **1** or compound **2** afforded from scheme 1 was dissolved in a solvent, such as but not limited to methanol, ethanol or tetrahydrofuran. A metal catalyst such as but not limited to 10% palladium/carbon, Pd(OH)₂ or Al₂O₃ was added. Hydrogen gas was introduced. The mixture was reacted at room temperature or under heating condition to afford the target product **3** in which the double bond was reduced.

In the Scheme, R₄ was the group in which the double bond in the R₃ group was reduced, and the definitions of other groups were the same as above.

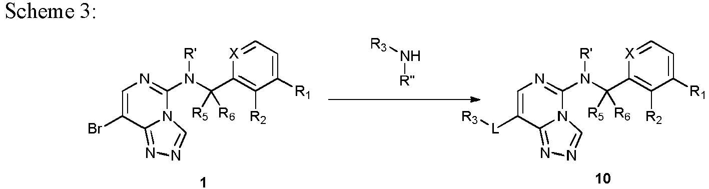

The bromide **1** was directly substituted by the amine R₃R"NH to afford target product **10.**

In the Scheme, L was N(R"), and the definitions of other groups were the same as above.

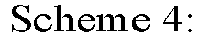

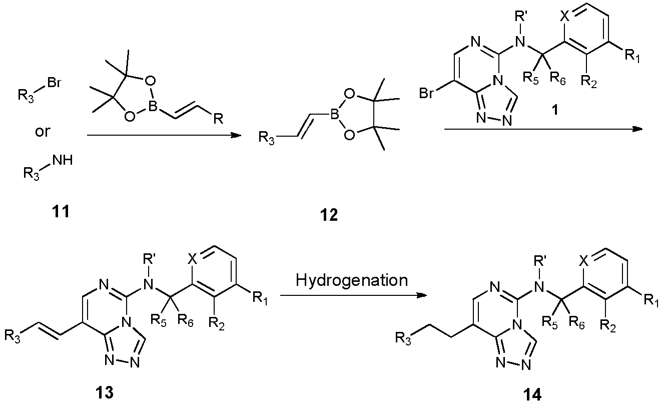

Firstly, halide or amine **11** was reacted with substituted or unsubstituted pinacol vinylboronate to afford borate ester **12;** then, borate ester **12** was subjected to Suzuki reaction with bromide 1 to afford alkene **13;** and finally, alkene 13 was hydrogenated to afford the target product **14** in which the double bond was reduced.

In the Scheme, the definition of each group was the same as above.

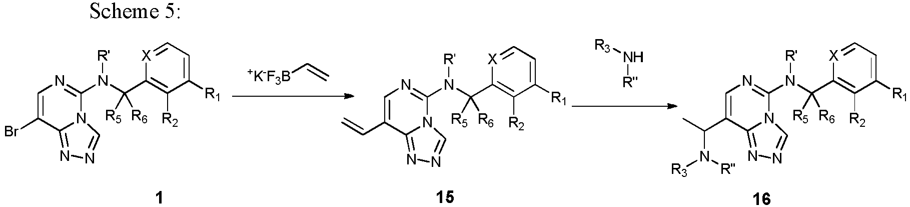

Firstly, bromide **1** was reacted with potassium vinyl trifluoroborate to afford alkene compound **15;** and then alkene **15** was subjected to addition reaction with amine R₃R"NH to afford target product **16.**

In the Scheme, the definition of each group was the same as above.

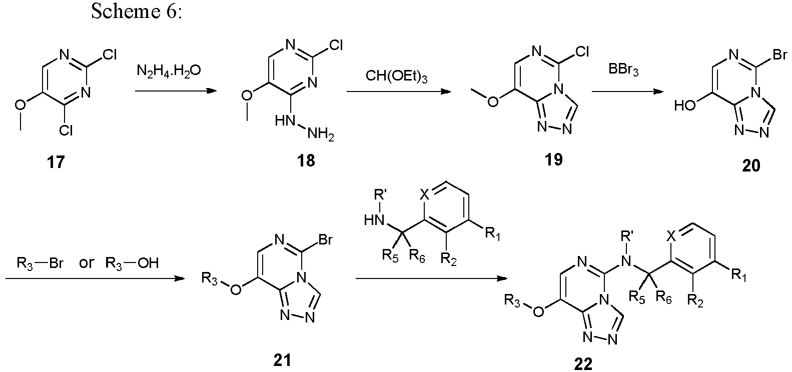

Firstly, 2,4-dichloro-5-methoxypyrimidine **17** was reacted with hydrazine hydrate to afford compound **18,** and compound **18** was converted into triazole product **19** using triethyl orthoformate; then, boron tribromide was demethylated to afford compound **20,** and compound **20** was subjected to substitution reaction with bromide R₃Br or subjected to Mitusunobu reaction with alcohol R₃OH to afford compound **21;** and finally, triazole product **21** was substituted by amine to afford product **22.**

In the Scheme, the definition of each group was the same as above.

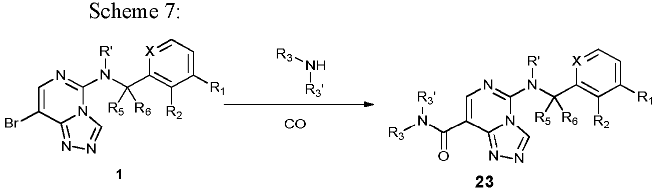

Bromide **1** was subjected to carbonyl insertion reaction with amine R₃R₃'NH in the presence of carbon monoxide to afford the target product **23.**

In the Scheme, the definition of each group was the same as above.

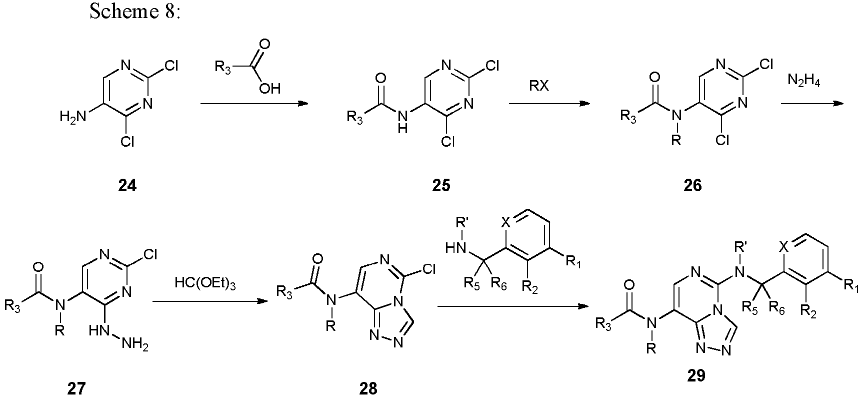

5-Amino-2,4-dichloropyrimidine **24** was condensed with carboxylic acid R₃COOH in the presence of phosphorus oxychloride to afford compound **25,** and compound **25** was subjected to substitution reaction with halide RX to afford substituted amide **26** (this step was not necessary); then, the amide compound **26** was treated with hydrazine hydrate to afford compound **27;** compound **27** was then converted into triazole product **28** using triethyl orthoformate; and finally, triazole product **28** was substituted by amine to afford target product**29.**

In the Scheme, the definition of each group was the same as above.

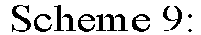

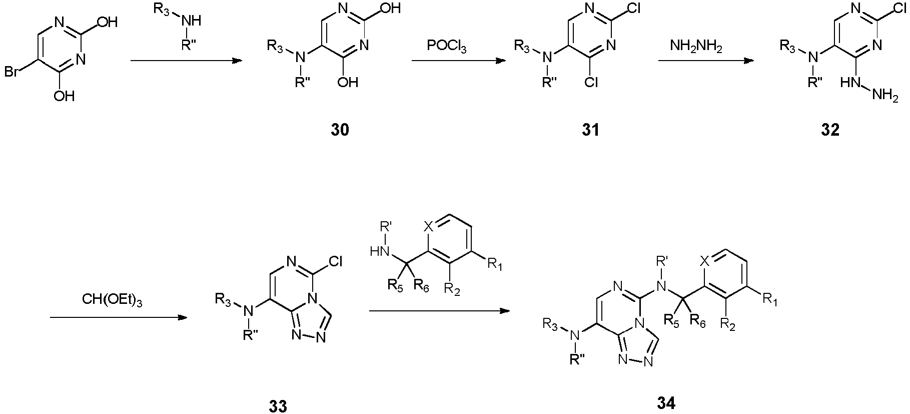

5-Bromouracil was reacted with amine R₃R"NH to afford amino compound **30,** and the amino compound **30** was treated with phosphorus oxychloride to afford compound **31;** then, compound **31** was treated with hydrazine hydrate to afford compound **32,** and compound **32** was converted into triazole product **33** using triethyl orthoformate; and finally, the triazole product **33** was substituted by amine to afford the target product **34.**

In the Scheme, the definition of each group was the same as above.

All raw materials in Schemes 1 to 9 can be purchased from reagent companies or prepared according to conventional literature.

### Examples of compound preparation

### Preparation example 1: synthesis of compound HJM-001 (for reference)

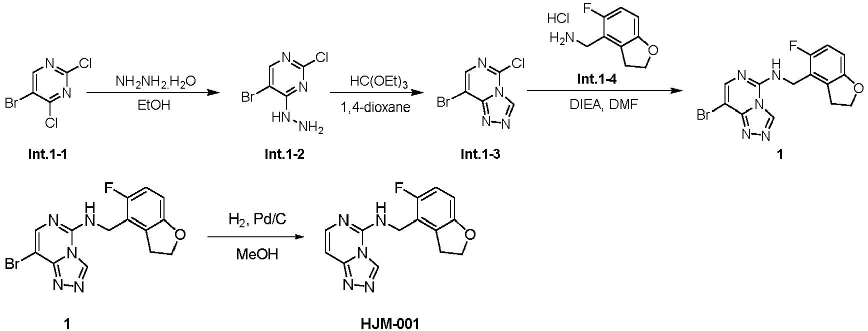

### Step 1:

At room temperature, 5-bromo-2,4-dichloropyrimidine **Int.1-1** (115 g, 504.66 mmol, 64.61 mL) was dissolved in EtOH (800 mL), then hydrazine hydrate (purity: 85%, 51.50 g, 874.45 mmol, 50 mL) was added, and the mixture was reacted with stirring at room temperature for 10 h. The mixture was filterd and the filter cake was washed with ethanol (3×800 mL) for three times. The resulting solid was dried in vacuum to afford compound 5-bromo-2-chloro-4-hydrazinylpyrimidine **Int.1-2** (230 g, crude) as a white solid. The crude product was directly used in the next step.

### Step 2:

At room temperature, 5-bromo-2-chloro-4-hydrazinylpyrimidine **Int.1-2** (124 g, 554.91 mmol) was dissolved in dioxane (1 L) solvent, triethyl orthoformate (131.58 g, 887.86 mmol, 147.68 mL) was added, and the mixture was reacted with stirring at 75 °C for 2 h. Raw materials were completely reacted. The mixture was cooled to room temperature, and filtered. The filtrate was concentrated to afford a crude product. The crude product was purified by silica gel column chromatography (petroleum ether:ethyl acetate (V/V) =5:1-1:1) to afford compound 8-bromo-5-chloro-[1,2,4]triazolo[4,3-c]pyrimidine **Int.1-3** (43 g, yield: 33.2%).

¹H NMR (400 MHz, DMSO-*d*₆) δ 9.68 (s, 1H), 8.24 (s, 1H).

### Step 3:

At room temperature, 8-bromo-5-chloro-[1,2,4]triazolo[4,3-c]pyrimidine **Int.1-3** (2.00 g, 8.57 mmol) and (5-fluoro-2,3-dihydrobenzofuran-4-yl)methylamine **Int.1-4** hydrochloride (1.92 g, 9.42 mmol) were added successively to *N,N*-dimethylformamide (15 mL), then *N,N*-diisopropylethylamine (3.32 g, 25.70 mmol, 4.48 mL) was added, and the mixture was reacted with stirring at 85 °C for 0.5 h. The mixture was cooled to room temperature, diluted with water (80 mL), and extracted with ethyl acetate (2×50 mL) for two times. The organic phases were combined. The organic phase was washed twice with water (2×50 mL) and once with saturated brine (50 mL), dried with anhydrous sodium sulfate, and filtered. The filtrate was concentrated to afford a crude product. The crude product was slurried with petroleum ether and ethyl acetate (20:1, 60 mL), and filtered to afford compound 8-bromo-N-((5-fluoro-2,3-dihydrobenzofuran-4-yl)methyl)-[1,2,4]triazolo[4,3-c]pyrimidine-5-amine **1** (2.50 g, yield: 80.1%).

¹H NMR (400 MHz, DMSO-*d*₆) δ 9.46 (s, 1H), 8.73 (br s, 1H), 7.84 (s, 1H), 6.92 (d, *J =* 10.0 Hz, 1H), 6.69 (dd, *J=* 3.9, 8.6 Hz, 1H), 4.64 (s, 2H), 4.52 (t, *J* = 8.8 Hz, 2H), 3.26 (t, *J* = 8.7 Hz, 2H).

### Step 4:

8-Bromo-N-((5-fluoro-2,3-dihydrobenzofuran-4-yl)methyl)-[1,2,4]triazolo[4,3-c]pyrimidine-5-amine **1** (50 mg, 0.14 mmol) was dissolved in 20 mL of methanol, and the atmosphere was replaced with N₂. 10 mg of 10% Pd/C was added quickly, and the atmosphere was replaced with H₂. The mixture was reacted at room temperature under a hydrogen balloon for 2 days. The mixture was filtered, and the filter cake was washed twice with methanol. The filtrates were combined, concentrated, and purified by preparative HPLC to afford **HJM-001** (13 mg, yield: 33.3 %) as a yellow solid.

¹H NMR (400 MHz, DMSO-*d*₆) δ 9.40 (s, 1H), 8.62 (s, 1H), 7.68 (t, *J =* 6.8 Hz, 1H), 7.04 - 6.81 (m, 2H), 6.71 (dd, *J* = 8.8, 4.0 Hz, 1H), 4.68 (d, *J =* 4.0 Hz, 2H), 4.54 (t, *J* = 8.8 Hz, 2H), 3.28 (t, *J* = 8.8 Hz, 2H). LCMS [M+H]⁺ = 286.2.

### Preparation example 2: synthesis of compound HJM-002 (for reference)

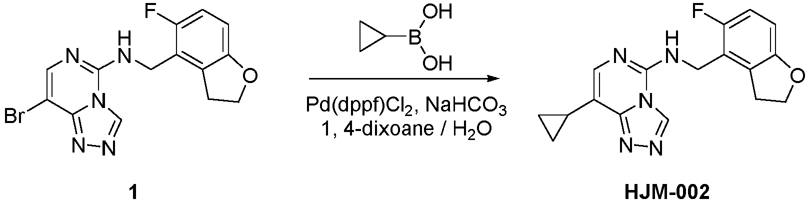

Under Ar protection, 8-bromo-N-((5-fluoro-2,3-dihydrobenzofuran-4-yl)methyl)-[1,2,4]triazolo[4,3-c]pyrimidine-5-amine **1** (300 mg, 0.83 mmol), cyclopropylboronic acid (710 mg, 8.26 mmol) and NaHCO₃ (264 mg, 2.49 mmol) were dissolved in 18 mL of a mixture of 1,4-dioxane and water (5:1), 1,1'-bis(diphenylphosphino)ferrocene-palladium(II)dichloride dichloromethane complex (59 mg, 0.08 mmol) was added at room temperature, and the mixture was heated to 100 °C and reacted for 16 h. TLC showed that the reaction was completed. The mixture was stopped from heating, naturally cooled to room temperature, and concentrated under reduced pressure. The residue was purified by preparative HPLC to afford **HJM-002** (3.0 mg, yield: 1.1 %) as a white solid.

¹H NMR (400 MHz, CD₃OD) δ 9.24 (s, 1H), 7.44 (d, *J* = 0.8 Hz, 1H), 6.87 - 6.80 (m, 1H), 6.66 - 6.58 (m, 1H), 4.72 (s, 2H), 4.59 - 4.51 (m, 2H), 3.35 - 3.32 (m, 2H), 2.12 - 2.02 (m, 1H), 1.01 - 0.94 (m, 2H), 0.87 - 0.81 (m, 2H). LCMS [M+H]⁺ = 326.3.

### Preparation example 3: synthesis of compound HJM-003 (for reference)

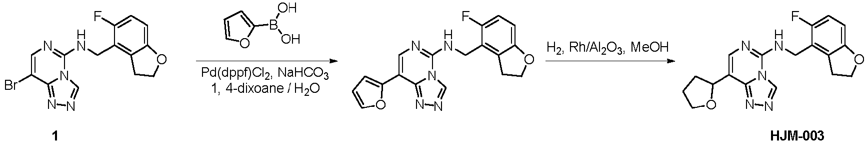

### Step 1:

Under Ar protection, 8-bromo-N-((5-fluoro-2,3-dihydrobenzofuran-4-yl)methyl)-[1,2,4]triazolo[4,3-c]pyrimidine-5-amine 1 (400 mg, 1.09 mmol), furan-2-boric acid (123 mg, 1.09 mmol) and NaHCO₃ (369 mg, 4.39 mmol) were dissolved in a mixture (13 mL) of 1,4-dioxane and water (10:3), 1,1'-bis(diphenylphosphino)ferrocene-palladium(II)dichloride dichloromethane complex (86 mg, 0.11 mmol) was added at room temperature, and the mixture was heated to 105 °C and reacted for 2 h. TLC showed that the reaction was completed. The mixture was stopped from heating, naturally cooled to room temperature, and concentrated under reduced pressure. The residue was purified by silica gel column chromatography (DCM:MeOH=20: 1) to afford *N*-((5-fluoro-2,3-dihydrobenzofuran-4-yl)methyl)-8-(furan-2-yl)-[1,2,4]triazolo[4,3-c]pyrimidin-5-amine (150 mg, yield: 39.0 %) as a yellow solid, LCMS [M+H]⁺ = 352.1.

### Step 2:

Compound *N*-((5-fluoro-2,3-dihydrobenzofuran-4-yl)methyl)-8-(furan-2-yl)-[1,2,4]triazolo[4,3-c]pyrimidin-5-amine (150 mg, 0.42 mmol) was dissolved in methanol (10 mL), and the atmosphere was replaced with N₂. 30 mg of Rh/Al₂O₃ was added quickly, and the atmosphere was replaced with H₂ for three times. The mixture was heated to 40 °C and reacted under a hydrogen balloon for 3 days. The mixture was filtered, and the filter cake was washed twice with methanol. The filtrates were combined, concentrated, and purified by preparative HPLC to afford **HJM-003** (12 mg, yield: 8.0 %) as a yellow solid.

¹H NMR (400 MHz, CD₃OD) δ 9.32 (s, 1H), 7.89 (s, 1H), 8.87-8.82 (m, 1H), 6.66-6.62 (m, 1H), 5.16-5.14 (m, 1H), 4.78 (s, 2H), 4.57 *(t, J=* 8.8 Hz, 2H), 4.15-4.14 (m, 1H), 3.94-3.92 (m, 1H), 3.37-3.35 (m, 2H), 2.42-2.41 (m, 1H), 2.10-2.04 (m, 3H). LCMS [M+H]⁺ = 356.1.

### Preparation example 4: synthesis of compound HJM-004 (for reference)

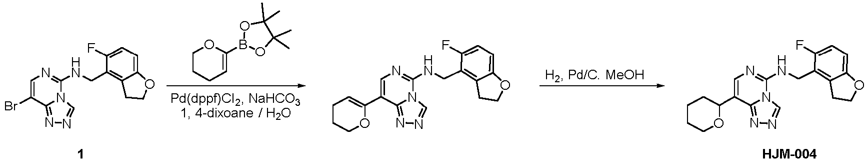

Compound N-((5-fluoro-2,3-dihydrobenzofuran-4-yl)methyl)-8-(tetrahydro-2H-pyran-2-yl)-[1,2,4]triazolo[4,3-c]pyrimidin-5-amine **HJM-004** (9 mg, yield: 9.9 %) was prepared as a white solid using 3,4-dihydro-2H-pyran-6-boronic acid pinacol ester as starting material according to the method similar to that for preparation example **3.**

¹H NMR (400 MHz, DMSO-*d*₆) δ 9.41 (s, 1H), 8.57 (d, *J* = 4.4 H_{Z},1H), 7.62 (s,1H), 6.96-6.91 (m, 1H), 6.71-6.68 (m, 1H), 4.66-4.63 (m, 3H), 4.55-4.51 (m, 2H), 4.04 (d, *J=* 12.0 H_{Z}, 1H), 3.60-3.56 (m, 1H), 3.30-3.26 (m, 2H), 2.03-2.01 (m, 1H), 1.88-1.87 (m, 1H), 1.69-1.57 (m, 4H). LCMS [M+H]⁺ = 370.2.

### Preparation example 5: synthesis of compound HJM-005

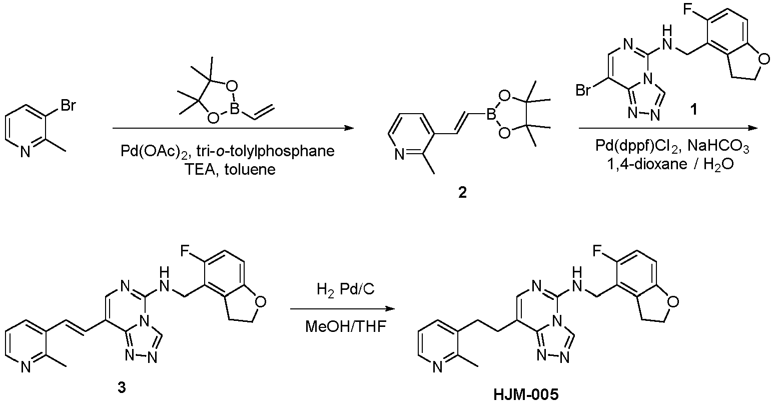

### Step 1:

Under N₂ protection, 3-bromo-2-methylpyridine (1.0 g, 5.84 mmol), pinacol vinylboronate (2 mL, 11.7 mmol) and triethylamine (2.4 mL, 17.52 mmol) were dissolved in toluene (20 mL), and palladium acetate (129 mg, 0.58 mmol) and tris(o-methylphenyl)phosphine (176 mg, 0.58 mmol) were added at room temperature. The atmosphere was replaced three times with N₂, and the mixture was heated to 100 °C and reacted for 18 h. TLC showed that the reaction was completed. The mixture was stopped from heating, naturally cooled to room temperature, and concentrated under reduced pressure. The residue was purified by silica gel column chromatography (PE:EA = 5:1) to afford brownish yellow liquid **2** (500 mg, yield: 35.0 %), LCMS [M+H]⁺ = 246.4.

### Step 2:

Under N₂ protection, 8-bromo-N-((5-fluoro-2,3-dihydrobenzofuran-4-yl)methyl)-[1,2,4]triazolo[4,3-c]pyrimidine-5-amine **1** (200 mg, 0.55 mmol), pinacol borate **2** (405 mg, 1.65 mmol) and NaHCO₃ (292 mg, 2.75 mmol) were dissolved in a mixture (8 mL) of 1,4-dioxane and water (3:1), 1,1'-bis(diphenylphosphino)ferrocene-palladium(II)dichloride dichloromethane complex (36 mg, 0.05 mmol) was added at room temperature, and the mixture was heated to 100 °C and reacted for 2 h. TLC showed that the reaction was completed. The mixture was stopped from heating, naturally cooled to room temperature, and concentrated under reduced pressure. The residue was purified by silica gel column chromatography (DCM:MeOH = 20:1) to afford **3** (150 mg, yield: 90.1%) as a white solid, LCMS [M+H]⁺ = 403.4.

### Step 3:

Compound **3** (150 mg, 0.372 mmol) was dissolved in a mixture of tetrahydrofuran (30 mL) and methanol (30 mL), and the atmosphere was replaced with N₂. 30.0 mg of 10% Pd/C was added quickly, the atmosphere was replaced with H₂, and the mixture was stirred at room temperature under a hydrogen balloon for 2 h. The mixture was filtered, and the filter cake was washed twice with methanol. The filtrates were combined, concentrated, and purified by preparative HPLC to afford **HJM-005** (29.0 mg, yield: 19.2 %) as a white solid.

¹H NMR (400 MHz, DMSO-*d*₆) δ 9.44 (s, 1H), 8.66 (dd, *J* = 5.6, 1.2 Hz, 1H), 8.56 (s, 1H), 8.29 (d, *J =* 8.0 Hz, 1H), 7.80 (d, *J* = 7.6, 6.0 Hz, 1H), 7.50 (s, 1H), 7.01 - 6.88 (m, 1H), 6.70 (dd, *J* = 8.8, 4.0 Hz, 1H), 4.65 (s, 2H), 4.54 (t, *J =* 8.8 Hz, 2H), 3.31 - 3.16 (m, 4H), 3.11 - 3.00 (m, 2H), 2.77 (s, 3H). LCMS [M+H]⁺ = 405.2.

### Preparation example 6: synthesis of compound HJM-006

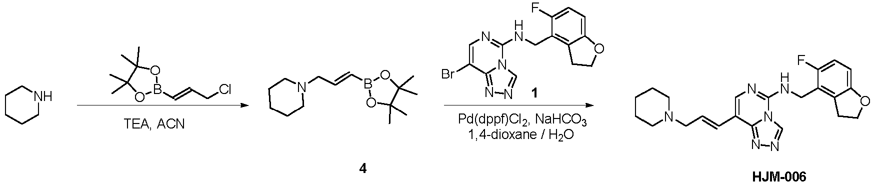

### Step 1:

Under N₂ protection, piperidine (0.18 mL, 1.98 mmol) and triethylamine (0.27 mL, 1.98 mmol) were dissolved in acetonitrile (3 mL), 3-chloropropenyl-1-boric acid pinacol ester (200 mg, 0.99 mmol) was added at room temperature, the atmosphere was replaced three times with N₂, and the mixture was stirred at room temperature overnight. TLC showed that the reaction was completed. The mixture was concentrated under reduced pressure to afford crude product **4** (400 mg, quantitative yield), which was directly used in the next step without purification. LCMS [M+H]⁺ = 252.3.

### Step 2:

Under N₂ protection, pinacol borate **4** (205 mg, crude), 8-bromo-N-((5-fluoro-2,3-dihydrobenzofuran-4-yl)methyl)-[1,2,4]triazolo[4,3-c]pyrimidine-5-amine **1** (150 mg, 0.41 mmol) and NaHCO₃ (131 mg, 1.24 mmol) were dissolved in a mixture (8 mL) of 1,4-dioxane and water, 1,1'-bis(diphenylphosphino)ferrocene-palladium(II)dichloride dichloromethane complex (30 mg, 0.04 mmol) was added at room temperature, and the mixture was heated to 100 °C and reacted for 2 h. TLC showed that the reaction was completed. The mixture was stopped from heating, naturally cooled to room temperature, and concentrated under reduced pressure. The residue was purified by preparative HPLC to afford **HJM-006** (70 mg, yield: 41.9 %) as a brown viscous solid.

¹H NMR (400 MHz, DMSO-*d*₆) δ 9.53 (s, 1H), 8.98 (s, 1H), 7.80 (s, 1H), 7.13 - 7.02 (m, 1H), 6.98 - 6.91 (m, 1H), 6.85 (d*, J =* 15.6 Hz, 1H), 6.71 (dd, *J* = 8.8, 4.0 Hz, 1H), 4.73 (s, 2H), 4.55 *(t, J=* 8.8 Hz, 2H), 3.99 - 3.87 (m, 2H), 3.46 (d, *J=* 11.6 Hz, 2H), 3.29 (t, *J* = 8.8 Hz, 2H), 2.97 - 2.82 (m, 2H), 1.92 - 1.78 (m, 2H), 1.77 - 1.57 (m, 3H), 1.48 - 1.30 (m, 1H). LCMS [M+H]⁺ = 409.6.

### Preparation example 7: synthesis of compound HJM-007

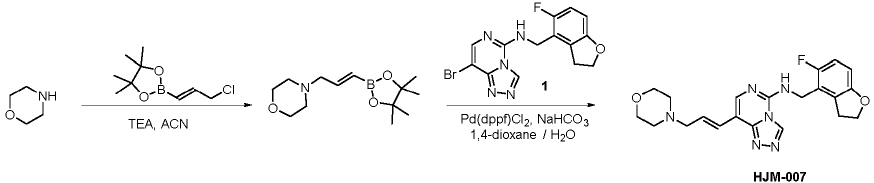

**HJM-007** (30 mg, yield: 26.3 %) was prepared as a white solid using morpholine as starting material according to the method similar to that for preparation example 6.

¹H NMR (400 MHz, DMSO-*d*₆) δ 9.44 (s, 1H), 8.66 (t, *J* = 5.2 Hz, 1H), 7.68 (s, 1H), 7.15 - 7.00 (m, 1H), 6.99 - 6.90 (m, 1H), 6.70 (dd, *J =* 8.8, 4.0 Hz, 1H), 6.57 (d, *J =* 15.6 Hz, 1H), 4.69 (d, *J* = 4.8 Hz, 2H), 4.53 (t, *J =* 8.8 Hz, 2H), 3.63 - 3.56 (m, 4H), 3.26 (t, *J =* 8.8 Hz, 2H), 3.15 (d, *J =* 6.4 Hz, 2H), 2.43 (s, 4H). LCMS [M+H]⁺ = 411.2.

### Preparation example 8: synthesis of compound HJM-008

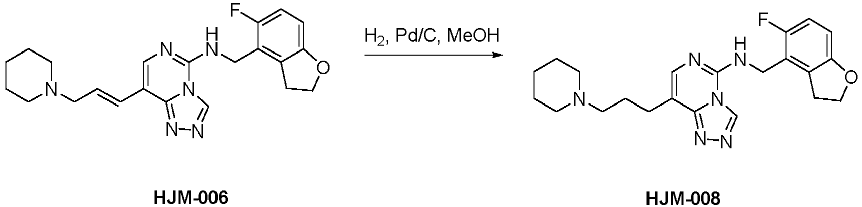

Compound **HJM-006** (60 mg, 0.15 mmol) was dissolved in methanol (6 mL), and the atmosphere was replaced with N₂. 10.0 mg of 10% Pd/C was added quickly, the atmosphere was replaced with H₂, and the mixture was stirred at room temperature under a hydrogen balloon for 2 h. The mixture was filtered, and the filter cake was washed twice with methanol. The filtrates were combined, concentrated, and purified by preparative HPLC to afford **HJM-008** (4.2 mg, yield: 7.0 %) as a white solid.

¹H NMR (400 MHz, CD₃OD) δ 9.27 (s, 1H), 7.61 (s, 1H), 6.84 (t, *J =* 9.6 Hz, 1H), 6.63 (dd, *J* = 8.8, 4.0 Hz, 1H), 4.74 (s, 2H), 4.57 (t, *J* = 8.8 Hz, 2H), 3.36 (t, *J =* 8.8 Hz, 2H), 3.33 - 3.31 (m, 4H), 3.15 - 3.09 (m, 2H), 2.88 (t, *J* = 7.2 Hz, 2H), 2.23 - 2.11 (m, 2H), 1.95 - 1.51 (m, 6H). LCMS [M+H]⁺ = 411.4.

### Preparation example 9: synthesis of compound HJM-009

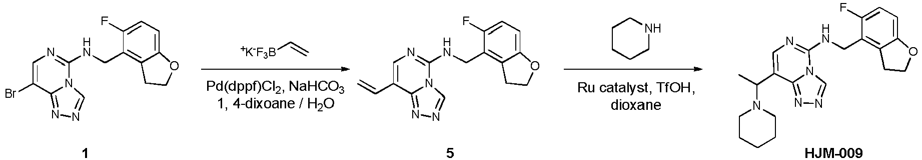

### Step 1:

Under N₂ protection, 8-bromo-N-((5-fluoro-2,3-dihydrobenzofuran-4-yl)methyl)-[1,2,4]triazolo[4,3-c]pyrimidine-5-amine **1** (200 mg, 0.55 mmol), potassium vinyl trifluoroborate (148 mg, 1.10) and NaHCO₃ (175 mg, 1.65 mmol) were dissolved in a mixture (12 mL) of 1,4-dioxane and water (3:1), 1,1'-bis(diphenylphosphino)ferrocene-palladium(II)dichloride dichloromethane complex (37 mg, 0.05 mmol) was added at room temperature, and the mixture was heated to 100 °C and reacted for 2 h. TLC showed that the reaction was completed. The mixture was stopped from heating, naturally cooled to room temperature, and concentrated under reduced pressure. The residue was purified by silica gel column chromatography (DCM:MeOH = 50:1) to afford **5** (150 mg, yield: 87.7%) as a yellow solid, LCMS [M+H]⁺ = 312.3.

### Step 2:

Under Ar protection, compound **5** (50 mg, 0.16mmol) and piperidine (0.03 mL, 0.32 mmol) were dissolved in 1,4-dioxane (3 mL). Bis-(2-methylallyl)cyclooctyl-1,5-diene ruthenium (3 mg, 0.01 mmol), 1,5-bis(diphenylphosphino)pentane (4 mg, 0.01 mmol) and trifluoromethanesulfonic acid (0.01 mL) were added at room temperature, and the mixture was heated to 100 °C and reacted for 16 h. TLC showed that the reaction was completed. The mixture was stopped from heating, naturally cooled to room temperature, and concentrated under reduced pressure. The residue was purified by preparative HPLC to afford **HJM-009** (6.0 mg, yield: 9.5%) as a white solid.

¹H NMR (400 MHz, DMSO-*d*₆) δ 9.39 (s, 1H), 8.49 (t, *J =* 4.8 Hz, 1H), 7.57 (s, 1H), 7.03 - 6.85 (m, 1H), 6.76 - 6.64 (m, 1H), 4.65 (d, *J* = 4.8 Hz, 2H), 4.55 (t, *J =* 8.8 Hz, 2H), 4.08 - 4.00 (m, 1H), 3.34 - 3.26 (m, 6H), 1.52 - 1.45 (m, 7H), 1.37 - 1.27 (m, 2H). LCMS [M+H]⁺ = 397.4.

### Preparation example 10: synthesis of compound HJM-010

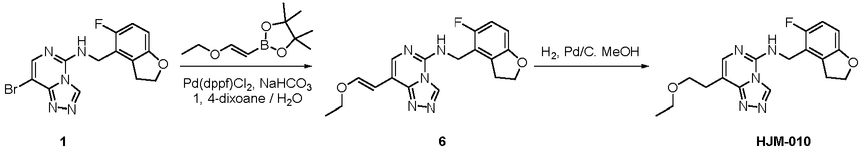

### Step 1:

Under N₂ protection, 8-bromo-N-((5-fluoro-2,3-dihydrobenzofuran-4-yl)methyl)-[1,2,4]triazolo[4,3-c]pyrimidine-5-amine **1** (200 mg, 0.55 mmol), (*E*)-1-ethoxyvinyl-2-boronic acid pinacol ester (109 mg, 0.55 mmol) and NaHCO₃ (140 mg, 1.667 mmol) were dissolved in a mixture (10 mL) of 1,4-dioxane and water (4:1), 1,1'-bis(diphenylphosphino)ferrocene-palladium(II)dichloride dichloromethane complex (60 mg, 0.081 mmol) was added at room temperature, and the mixture was heated to 100 °C and reacted for 8 h. TLC showed that the reaction was completed. The mixture was stopped from heating, and naturally cooled to room temperature. Water and ethyl acetate were added, and the mixture was layered. The aqueous phase was extracted with ethyl acetate (2×20 mL). The organic phases were combined, and then washed successively with water (2×20 mL) and saturated brine. The organic layer was dried with Na₂SO₄, filtered, and concentrated. The residue was purified by silica gel column chromatography (DCM:MeOH=100:1) to afford **6** (50 mg, yield: 24.9 %) as a yellow solid, LCMS [M+H]⁺ = 356.1.

### Step 2:

Compound **6** (50 mg, 0.14 mmol) was dissolved in methanol (10 mL), and the atmosphere was replaced with N₂. 50.0 mg of 10% Pd/C was added quickly, the atmosphere was replaced with H₂, and the mixture was stirred at room temperature under a hydrogen balloon for 6 h. The mixture was filtered, and the filter cake was washed twice with methanol. The filtrates were combined, concentrated, and purified by preparative HPLC to afford **HJM-010** (7.0 mg, yield: 14.1 %) as a yellow solid.

¹H NMR (400 MHz, CD₃OD) δ 9.27 (s, 1H), 7.71 (s, 1H), 6.85-6.83 (d, *J* = 9.6 Hz, 1H), 6.66-6.63 (m, 1H), 4.76 (s, 2H), 4.59-4.55 (m, 2H), 3.78-3.75 (m, 2H), 3.53-3.48 (m, 2H), 3.37-3.35 (m, 2H), 3.06-3.03 (m, 2H), 1.13-1.12 (m, 3H). LCMS [M+H]⁺ = 358.4.

### Preparation example 11: synthesis of compound HJM-011

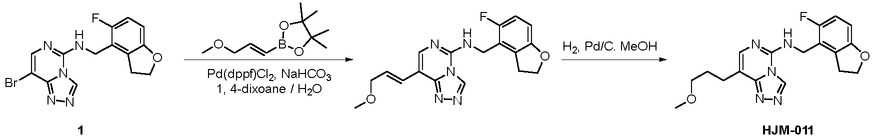

**HJM-011** (90 mg, yield: 64.3 %) was prepared as a yellow solid using trans-3-methoxy-1-propenylboronic acid pinacol ester as starting material according to the method similar to that for preparation example **10.**

¹H NMR (400 MHz, CD₃OD) δ 9.23 (s, 1H), 7.53 (s, 1H), 6.84-6.81 (d, *J* = 9.2 Hz,1H), 6.64-6.61 (m,1H), 4.73 (s, 2H), 4.58-4.54 (m, 2H), 3.46-3.43 (m, 2H), 3.35-3.30 (m, 5H), 2.87-2.84 (m, 2H), 2.00-1.97 (m, 2H). LCMS: [M+H]⁺ = 358.4.

### Preparation example 12: synthesis of compound HJM-012

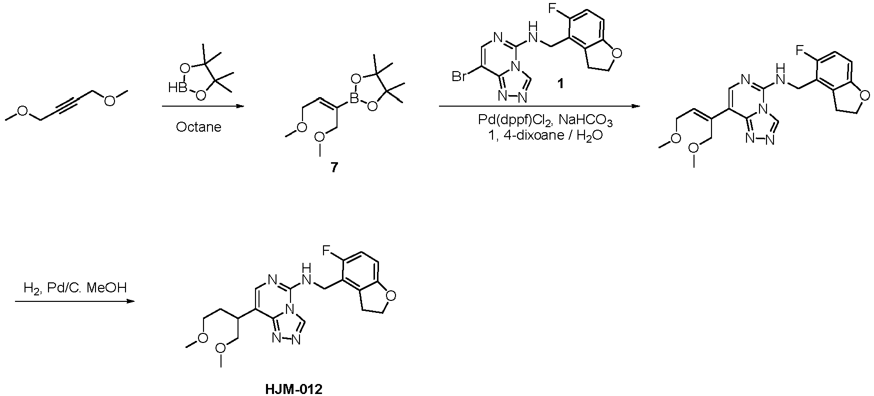

### Step 1:

Under N₂ protection, 1,4-dimethoxy-2-butyne (500 mg, 4.38 mmol) and triethylamine (0.27 mL, 1.98 mmol) were dissolved in n-octane (5 mL). 4-Dimethylaminobenzoic acid (36 mg, 1.06 mmol) and pinacol borane (2.8 g, 21.90 mmol) were added at room temperature, and the mixture was heated to 100 °C and reacted for 12 h. TLC showed that the reaction was completed. The mixture was stopped from heating, naturally cooled to room temperature, and concentrated under reduced pressure to afford crude product **7** (quantitative yield) as a black oil, which was directly used in the next step without purification.

### Steps 2-3:

**HJM-012** (35 mg, yield: 22.9 %) was prepared as a yellow solid using intermediate **7** as starting material according to the method similar to that for preparation example **10.**

¹H NMR (400 MHz, CD₃OD) δ 9.23 (s, 1H), 7.55 (s, 1H), 6.86-6.82 (m, 1H), 6.65-6.62 (m, 1H), 4.73 (s, 2H), 4.56 (t, *J* = 8.8 Hz, 2H), 3.87-3.65 (m, 2H), 3.37-3.33 (m, 3H), 3.31-3.30 (m, 5H), 3.23 (s, 3H), 2.11-2.08 (m, 2H). LCMS [M+H]⁺ = 402.2.

### Preparation example 13: synthesis of compound HJM-013

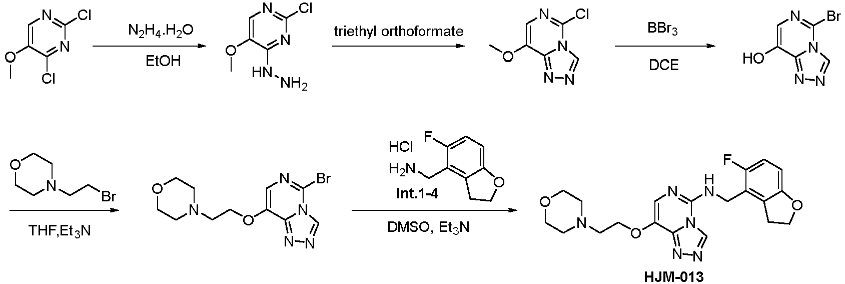

### Step 1:

2,4-Dichloro-5-methoxypyrimidine (15 g, 83.8 mmol) was dissolved in 200 mL of ethanol, hydrazine hydrate (5.42 g, 92.18 mmol) was slowly added at room temperature, and the mixture was stirred at room temperature for 2 h. TLC showed that the reaction was completed. The mixture was allowed to stand, and filtered. The filter cake was dried in vacuum to afford compound 2-chloro-5-methoxy-4-hydrazino-pyrimidine (10.5 g, yield: 72.0 %) as a solid, LCMS [M+H]⁺ = 175.3.

### Step 2:

Under N₂ protection, 2-chloro-5-methoxy-4-hydrazino-pyrimidine (10.5 g, 60.14 mmol) was dissolved in 100 mL of triethyl orthoformate, and the mixture was heated to 150 °C and stirred for 2 h. TLC showed that the reaction was completed. The mixture was stopped from heating, naturally cooled to room temperature, and filtered. The filter cake was dried in vacuum to afford compound 5-chloro-8-methoxy-[1,2,4]triazolo[4,3-c]pyrimidine (7 g, yield: 63.6 %) as a solid, LCMS [M+H]⁺ = 185.3.

### Step 3:

Under N₂ protection, 5-chloro-8-methoxy-[1,2,4]triazolo[4,3-c]pyrimidine (184 mg, 1.0 mmol) was dissolved in 10 mL of 1,2-dichloroethane, boron tribromide (10 mL, 10 mmol) was slowly added under an ice-water bath, and the mixture was heated to 85 °C and stirred overnight. TLC showed that the reaction was completed. The mixture was stopped from heating, and naturally cooled to room temperature. 1M Na₂CO₃ solution was added under an ice-water bath to quench the reaction, and the mixture was stirred at room temperature for 1 h. The mixture was acidified with saturated NH₄Cl solution and acetic acid to a pH of 6-7, and layered. The aqueous phase was extracted with ethyl acetate (2×20 mL). The organic phases were combined, washed with saturated brine (2×20 mL), dried with Na₂SO₄, and filtered. The filtrate was concentrated to afford 5-bromo-[1,2,4]triazolo[4,3-c]pyrimidine-8-ol (100 mg, yield: 47.0 %), LCMS [M+H]⁺ = 215.2. (Note: 5-chloro-[1,2,4]triazolo[4,3-c]pyrimidine-8-ol was afforded sometimes)

### Step 4:

Under N₂ protection, 5-bromo-[1,2,4]triazolo[4,3-c]pyrimidine-8-ol (100 mg, 0.47 mmol) was dissolved in 5 mL of tetrahydrofuran. Triethylamine (119 mg, 1.176 mmol) and 4-(2-bromoethyl)morpholine (228 mg, 1.176 mmol) were added successively, and the mixture was heated to 75 °C and stirred overnight. TLC showed that the reaction was completed. The mixture was stopped from heating, naturally cooled to room temperature, and concentrated under reduced pressure. The residue was purified by preparative TLC to afford compound 4-(2-((5-bromo-[1,2,4]triazolo[4,3-c]pyrimidine-8-yl)oxy)ethyl)morpholine (70 mg, yield: 46.1 %) as a solid, LCMS [M+H]⁺ = 328.0.

### Step 5:

Under N₂ protection, compound 4-(2-((5-bromo-[1,2,4]triazolo[4,3-c]pyrimidine-8-yl)oxy)ethyl)morpholine (70 mg, 0.213 mmol) was dissolved in 3 mL of dimethyl sulfoxide. 5-Fluoro-2,3-dihydrobenzofuran-4-methylamine **Int.1-4** hydrochloride (86 mg, 0.427 mmol) and triethylamine (86 mg, 0.853 mmol) were added successively, and the mixture was heated to 75 °C and stirred overnight. The next day, TLC showed that the reaction was completed. The mixture was stopped from heating, naturally cooled to room temperature, and concentrated under reduced pressure. The residue was purified by preparative TLC to afford **HJM-013** (20 mg, yield: 22.6 %) as a white solid.

¹H NMR (400 MHz, CD₃OD) δ 9.28 (s, 1H), 7.49 (s, 1H), 6.87 - 6.79 (m, 1H), 6.63 (dd, *J* = 8.4, 3.6 Hz, 1H), 4.71 (s, 2H), 4.60 - 4.51 (m, 4H), 4.13-3.96 (m, 4H), 3.72 - 3.68 (m, 2H), 3.38 - 3.33 (m, 6H).

¹H NMR (400 MHz, DMSO-*d*₆) δ 9.45 (s, 1H), 8.38 (t, *J =* 4.8 Hz, 1H), 7.47 (s, 1H), 6.94 (t, *J =* 9.6 Hz, 1H), 6.70 (dd, *J =* 8.4, 3.6 Hz, 1H), 4.62 (t, *J =* 4.8 Hz, 2H), 4.56 - 4.52 (m, 4H), 4.37-3.95 (m, 4H), 3.62 (t, *J =* 4.4 Hz, 4H), 3.29 (t, *J* = 8.4 Hz, 4H). LCMS [M+H]⁺ = 415.2.

### Preparation example 14: synthesis of compound HJM-014

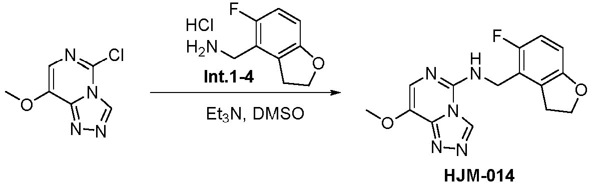

Under N₂ protection, 5-fluoro-2,3-dihydrobenzofuran-4-methylamine **Int.1-4** hydrochloride (131 mg, 0.65 mmol) and triethylamine (0.15 mL, 1.08 mmol) were dissolved in 3 mL of dimethyl sulfoxide, 5-chloro-8-methoxy-[1,2,4]triazolo[4,3-c]pyrimidine **1** (100 mg, 0.54 mmol) was added at room temperature, and the mixture was stirred at room temperature for 16 h. TLC showed that the reaction was completed. The mixture was concentrated under reduced pressure, and purified by preparative HPLC to afford **HJM-014** (63 mg, yield: 36.8 %) as a white solid.

¹H NMR (400 MHz, DMSO-*d*₆) δ 9.39 (s, 1H), 8.11 (t, *J* = 5.2 Hz, 1H),7.28 (s, 1H), 6.99- 6.87 (m, 1H), 6.69 (dd, *J =* 8.8, 4.0 Hz, 1H), 4.61 (d, *J =* 4.8 Hz, 2H), 4.54 (t, *J* = 8.8 Hz, 2H), 3.28 (t, *J* = 8.8 Hz, 2H), 2.56 - 2.43 (m, 3H). LCMS [M+H]⁺ = 316.1.

### Preparation example 15: synthesis of compound HJM-015

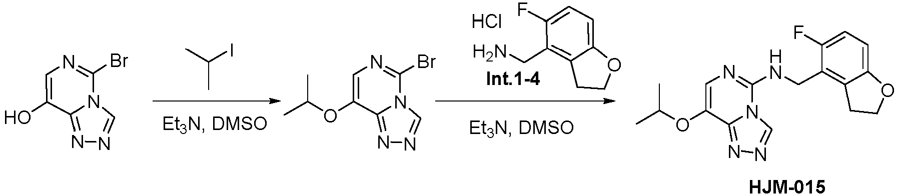

### Step 1:

Under N₂ protection, 5-bromo-[1,2,4]triazolo[4,3-c]pyrimidin-8-ol (100 mg, 0.47 mmol) was dissolved in dimethyl sulfoxide (3 mL). Triethylamine (94 mg, 0.93 mmol) and isopropyl iodide (158 mg, 0.93 mmol) were added successively, and the mixture was heated to 75 °C and stirred overnight. TLC showed that the reaction was completed. The mixture was stopped from heating, and naturally cooled to room temperature. The mixture was poured into water to quench the reaction, and the aqueous phase was extracted with ethyl acetate (2×20 mL). The organic phases were combined, washed with saturated brine (2×5 mL), dried with Na₂SO₄, filtered, and concentrated. The residue was purified by silica gel column chromatography (PE:EA = 2:1) to afford compound 5-bromo-8-isopropyl-[1,2,4]triazolo[4,3-c]pyrimidine (30 mg, yield: 25.2 %) as a white solid, LCMS [M+H]⁺ = 257.1.

### Step 2:

Under N₂ protection, 5-fluoro-2,3-dihydrobenzofuran-4-methylamine **Int.1-4** hydrochloride (48 mg, 0.23 mmol) and triethylamine (23 mg, 0.23 mmol) were dissolved in dimethyl sulfoxide (2 mL), 5-bromo-8-isopropyl-[1,2,4]triazolo[4,3-c]pyrimidine (30 mg, 0.12 mmol) was added at room temperature, and the mixture was heated to 75 °C and stirred for 2 h. TLC showed that the reaction was completed. The mixture was stopped from heating, naturally cooled to room temperature, and concentrated under reduced pressure. The residue was purified by preparative HPLC to afford **HJM-015** (5 mg, yield: 12.5 %) as a white solid.

¹H NMR (400 MHz, DMSO-*d*₆) δ 9.39 (s, 1H), 8.16 (t, *J* = 4.8 Hz, 1H), 7.31 (s, 1H), 7.01-6.87 (m, 1H), 6.70 (dd, *J* = 8.6, 3.9 Hz, 1H), 4.83-4.77 (m, 1H), 4.60 (d, *J=* 4.8 Hz, 2H), 4.54 (t, *J* = 8.8 Hz, 2H), 3.28 (t, *J=* 8.7 Hz, 2H), 1.29 (s, 3H), 1.27 (s, 3H). LCMS [M+H]⁺ = 344.1.

### Preparation example 16: synthesis of compound HJM-016

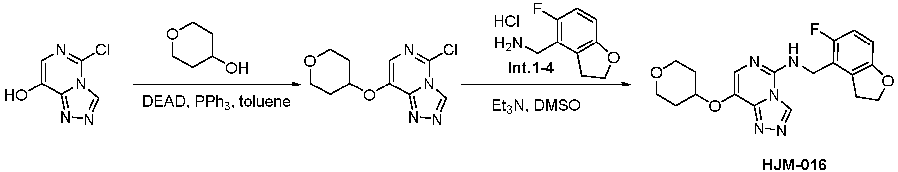

### Step 1:

Under N₂ protection, 5-chloro-[1,2,4]triazolo[4,3-c]pyrimidin-8-ol (50 mg, 0.29 mmol) and tetrahydropyran-4-ol (44 mg, 0.44 mmol) were dissolved in anhydrous toluene (5 mL). Diethyl azodicarboxylate DEAD (102 mg, 0.59 mmol) and triphenyl phosphine (154 mg, 0.59 mmol) were added successively, and the mixture was heated to 110 °C and stirred overnight. The next day, TLC showed that the reaction was completed. The mixture was stopped from heating, naturally cooled to room temperature, and concentrated under reduced pressure. The residue was purified by preparative TLC to afford compound 5-chloro-8-(tetrahydro-2H-pyran-4-yl)-[1,2,4]triazolo[4,3-c]pyrimidine (30 mg, yield: 41.1 %) as a yellow solid, LCMS [M+H]⁺ = 255.1.

### Step 2:

Under N₂ protection, compound 5-fluoro-2,3-dihydrobenzofuran-4-methylamine **Int.1-4** hydrochloride (48 mg, 0.236 mmol) and triethylamine (0.05 mL, 0.36 mmol) were dissolved in dimethyl sulfoxide (5 mL), compound 5-chloro-8-(tetrahydro-2H-pyran-4-yl)[1,2,4]triazolo[4,3-c]pyrimidine (30 mg, 0.12 mmol) was slowly added, and the mixture was heated to 50 °C and stirred for 2 h. TLC showed that the reaction was completed. The mixture was stopped from heating, naturally cooled to room temperature, and concentrated under reduced pressure. The residue was purified by preparative HPLC to afford **HJM-016** (3.3 mg, yield: 7.3 %) as a white solid.

¹H NMR (400 MHz, DMSO-*d*₆) δ 9.43 (s, 1H), 8.31 (s, 1H),7.40 (s, 1H), 6.98 -6.89 (m, 1H), 6.70 (dd, *J=* 8.8, 4.0 Hz, 1H), 4.78-4.69 (m, 1H), 4.61 (d, *J* = 4.4 Hz, 2H), 4.54 (t, *J* = 8.8 Hz, 2H), 3.92-3.83 (m, 2H), 3.45 - 3.40 (m, 2H), 3.32 - 3.25 (m, 2H), 2.05- 1.90 (m, 2H), 1.71-1.56 (m, 2H). LCMS [M+H]⁺ = 386.3.

### Preparation example 17: synthesis of compound HJM-017

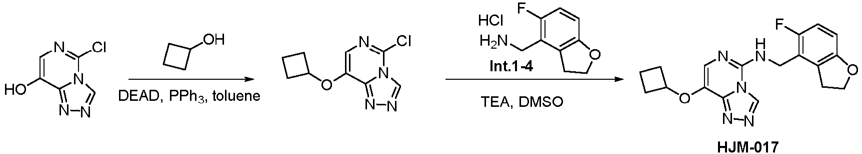

**HJM-017** (32 mg) was prepared as a white solid using cyclobutanol as starting material according to the method similar to that for preparation example 16.

¹H NMR (400 MHz, DMSO-*d*₆) δ 9.39 (s, 1H), 8.17 - 8.09 (m, 1H), 7.16 (s, 1H), 6.98 - 6.89 (m, 1H), 6.69 (dd, *J =* 8.8, 4.0 Hz, 1H), 4.82 (q, *J =* 7.2 Hz, 1H), 4.62 - 4.49 (m, 4H), 3.27 (t, *J* = 8.8 Hz, 2H), 2.47 - 2.36 (m, 2H), 2.18 - 2.03 (m, 2H), 1.84-1.75 (m, 1H), 1.66-1.59 (m, 1H). LCMS [M+H]⁺ = 356.2.

### Preparation example 18: synthesis of compound HJM-018

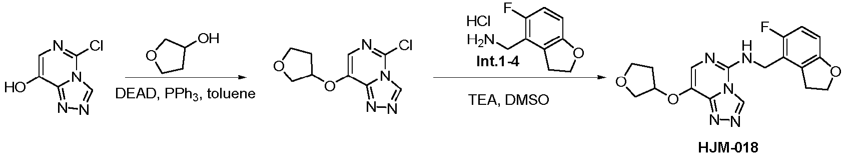

**HJM-018** (39 mg) was prepared as a white solid using 3-hydroxymethyl tetrahydrofuran as starting material according to the method similar to that for preparation example 16.

¹H NMR (400 MHz, DMSO-*d*₆) δ 9.40 (s, 1H), 8.19 (t, *J =* 5.2 Hz, 1H), 7.31 (s, 1H), 6.94 (t, *J =* 9.6 Hz, 1H), 6.70 (dd, *J =* 8.4, 3.6 Hz, 1H), 5.28 - 5.22 (m, 1H), 4.60 (d, *J =* 4.8 Hz, 2H), 4.54 (t, *J* = 8.8 Hz, 2H), 3.94 - 3.81 (m, 3H), 3.76 (td, *J* = 8.0, 4.4 Hz, 1H), 3.28 (t, *J* = 8.4 Hz, 2H), 2.22 - 1.96 (m, 2H). LCMS [M+H]⁺ = 372.1.

### Preparation example 19: synthesis of compound HJM-019

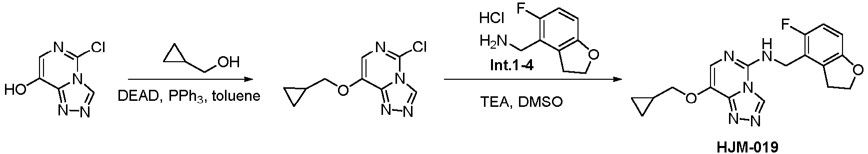

**HJM-019** (21 mg) was prepared as a white solid using hydroxymethylcyclopropane as starting material according to the method similar to that for preparation example 16.

¹H NMR (400 MHz, DMSO-*d*₆) δ 9.39 (s, 1H), 8.14 (t, *J* = 5.2 Hz, 1H), 7.28 (s, 1H), 6.93 (t, *J* = 9.6 Hz, 1H), 6.69 (dd, *J* = 8.4, 4.0 Hz, 1H), 4.60 (d*, J =* 4.8 Hz, 2H), 4.53 (t*, J =* 8.8 Hz, 2H), 3.97 (d, *J =* 7.2 Hz, 2H), 3.27 (t, *J* = 8.8 Hz, 2H), 1.33 - 1.23 (m, 1H), 0.63 - 0.54 (m, 2H), 0.35-0.31 (m, 2H). LCMS [M+H]⁺ = 356.1.

### Preparation example 20: synthesis of compound HJM-020

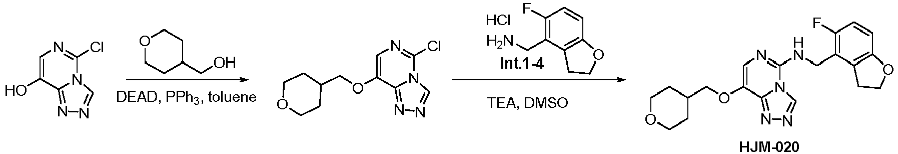

**HJM-020** (23 mg) was prepared as a white solid using 4-hydroxymethyltetrahydropyran as starting material according to the method similar to that for preparation example 16.

¹H NMR (400 MHz, DMSO-*d*₆): δ 9.38 (s, 1H), 8.13 (t, *J* = 5.2 Hz, 1H), 7.29 (s, 1H), 6.93 (t, *J* = 8.8 Hz, 1H), 6.70 - 6.67 (m, 1H), 4.60 (d, *J=* 5.2 Hz, 2H), 4.53 (t, *J* = 8.8 Hz, 2H), 3.99 (d, *J* = 6.4 Hz, 2H), 3.89 (dd, *J=* 3.2, 11.2 Hz, 2H), 3.31-3.25 (m, 4H), 2.00-1.97 (m, 1H), 1.71-1.68 (m, 2H), 1.38-1.28 (m, 2H). LCMS [M+H]⁺ = 400.0.

### Preparation example 21: synthesis of compound HJM-021

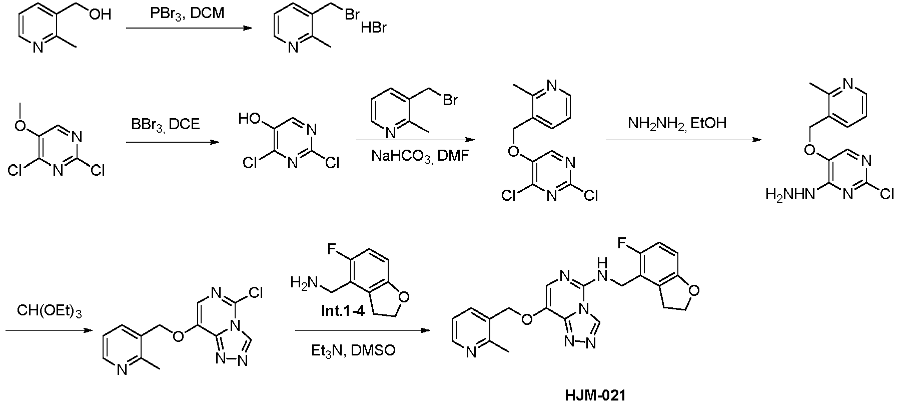

### Step 1:

Compound 3-hydroxymethyl-2-methylpyridine (1.6 g, 13.0 mmol) was dissolved in dichloromethane (20 mL), phosphorus tribromide (1.5 mL, 15.6 mmol) was slowly added under an ice-water bath and N₂ protection, and the mixture was stirred at room temperature overnight. TLC showed that the reaction was completed. The mixture was concentrated under reduced pressure to afford 3-bromomethyl-2-methylpyridine hydrobromide (3.5 g, yield: 99.0 %) as a yellow solid, LCMS [M+H]⁺ = 186.1.

### Step 2:

2,4-Dichloro-5-methoxypyrimidine (5.0 g, 27.9 mmol) was dissolved in 1,2-dichloroethane (75 mL), boron tribromide (13.5 mL, 140 mmol) was slowly added under an ice-water bath and N₂ protection, and the mixture was heated to 80 °C and stirred overnight. TLC showed that the reaction was completed. The mixture was stopped from heating, and naturally cooled to room temperature. 1M NaOH solution was added under an ice-water bath to quench the reaction, and the mixture was stirred at room temperature for 1 h. The mixture was acidified with saturated NH₄Cl solution and acetic acid to a pH of 6-7. The mixture was layered, and the aqueous phase was extracted with ethyl acetate (2×20 mL). The organic phases were combined, washed with saturated brine (2×20 mL), dried with Na₂SO₄, filtered, and concentrated. The residue was purified by silica gel column chromatography (PE:EA=5:1) to afford compound 2,4-dichloropyrimidine-5-ol (4.0 g, yield: 86.9 %) as a solid, LCMS [M+H]⁺ = 162.8.

### Step 3:

Under N₂ protection, compound 2,4-dichloropyrimidine-5-ol (1.8 g, 10.9 mmol) was dissolved in N,N-dimethylformamide (20 mL), 3-bromomethyl-2-methylpyridine hydrobromide (1.5 mL, 13.1 mmol) and NaHCO₃ (1.1 g, 13.1 mmol) were added successively, and the mixture was stirred at room temperature overnight. TLC showed that the reaction was completed. Water was added to quench the reaction, and the aqueous phase was extracted with ethyl acetate (2×20 mL). The organic phases were combined, washed with saturated brine (2×20 mL), dried with Na₂SO₄, and filtered. The filtrate was concentrated to afford 2,4-dichloro-5-((2-methylpyridin-3-yl)methoxy)pyrimidine (1.75 g, yield: 59.4 %) as a yellow solid, LCMS: [M+H]⁺ = 269.9.

### Step 4:

Compound 2,4-dichloro-5-((2-methylpyridin-3-yl)methoxy)pyrimidine (1.75 g, 6.48 mmol) was dissolved in ethanol (30 mL), 85% hydrazine hydrate (457.4 mg, 7.78 mmol) was slowly added at room temperature, and the mixture was stirred at room temperature overnight. TLC showed that the reaction was completed. The mixture was concentrated under reduced pressure to afford crude compound 2-chloro-4-hydrazinyl-5-((2-methylpyridin-3-yl)methoxy)pyrimidine (2.0 g, yield: 99.0 %), LCMS [M+H]⁺ = 266.2.

### Step 5:

Under N₂ protection, the crude compound 2-chloro-4-hydrazinyl-5-((2-methylpyridin-3-yl)methoxy)pyrimidine (2.0 g, 7.51 mmol) was dissolved in 15 mL of triethyl orthoformate, and the mixture was heated to 150 °C and stirred overnight. TLC showed that the reaction was completed. The mixture was stopped from heating, naturally cooled to room temperature, and concentrated under reduced pressure. The residue was purified by silica gel column chromatography to afford 5-chloro-8-((2-methylpyridin-3-yl)methoxy)-[1,2,4]triazolo[4,3-c]pyrimidine (600 mg, yield: 33.7 %) as a yellow solid, LCMS [M+H]⁺ = 276.2.

### Step 6:

Under N₂ protection, compound 5-chloro-8-((2-methylpyridin-3-yl)methoxy)-[1,2,4]triazolo[4,3-c]pyrimidine (150 mg, 0.545 mmol) was dissolved in dimethyl sulfoxide (5 mL), 5-fluoro-2,3-dihydrobenzofuran-4-methylamine **Int.1-4** (273 mg, 1.636 mmol) and triethylamine (276 mg, 2.727 mmol) were added successively, and the mixture was heated to 75 °C and stirred overnight. TLC showed that the reaction was completed. The mixture was stopped from heating, and naturally cooled to room temperature. Water and ethyl acetate were added, and the mixture was layered. The aqueous phase was extracted with ethyl acetate (2×20 mL). The organic phases were combined, and then washed successively with water (2×20 mL) and saturated brine. The organic layer was dried with Na₂SO₄, filtered, and concentrated. The residue was purified by silica gel column chromatography (DCM:MeOH = 20:1) to afford **HJM-021** (160 mg, yield: 72.2 %) as a white solid.

¹H NMR (400 MHz, DMSO-*d*₆) δ 9.45 (s, 1H), 8.75 (d, *J =* 5.2 Hz, 1H), 8.47 (d, *J* = 7.6 Hz, 1H), 8.34 (s, 1H), 7.89 - 7.78 (m, 1H), 7.50 (s, 1H), 6.94 (t, *J =* 9.6 Hz, 1H), 6.70 (dd, *J* = 8.4, 3.2 Hz, 1H), 5.45 (s, 2H), 4.61 (d, *J* = 3.2 Hz, 2H), 4.54 (t, *J =* 8.4 Hz, 2H), 3.28 (t, *J* = 8.4 Hz, 2H), 2.76 (s, 3H). LCMS [M+H]⁺ = 407.3.

### Preparation example 22: synthesis of compound HJM-022

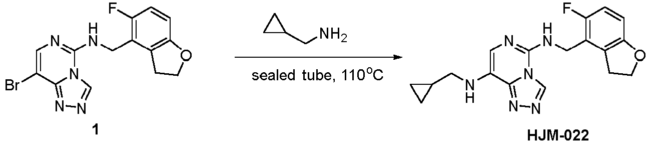

Under N₂ protection, 8-bromo-N-((5-fluoro-2,3-dihydrobenzofuran-4-yl)methyl)-[1,2,4]triazolo[4,3-c]pyrimidine-5-amine 1 (83 mg, 0.23 mmol) and cyclopropylmethylamine (3 mL) were placed in a sealed tube, and the mixture was heated to 110 °C and stirred for 1.5 h. TLC showed that the reaction was completed. The mixture was stopped from heating, naturally cooled to room temperature, and concentrated under reduced pressure. The residue was purified by preparative HPLC to afford **HJM-022** (16 mg, yield: 20 %) as a white solid.

¹H NMR (400 MHz, DMSO-*d*₆) δ 9.32 (s, 1H), 7.66 (t, *J* = 5.6 Hz, 1H), 6.92 (t, *J* = 9.2 Hz, 1H), 6.83 (s, 1H), 6.69 (q, *J=* 4.0 Hz, 1H), 5.14 (t, *J* = 5.6 Hz, 1H), 4.56 - 4.50 (m, 4H), 3.27 (t, *J* = 8.8 Hz, 2H), 3.00 (t, *J =* 6.4 Hz, 2H), 1.15 - 1.11 (m, 1H), 0.48 - 0.44 (m, 2H), 0.25 - 0.21 (m, 2H). LCMS [M+H]⁺ = 355.4.

### Preparation example 23: synthesis of compound HJM-023

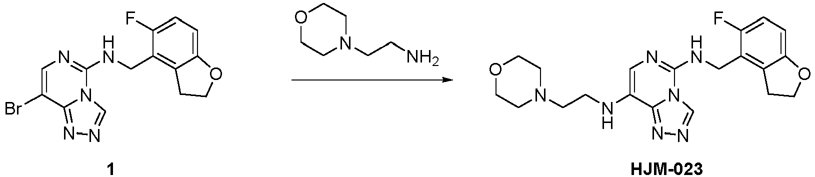

**HJM-023** (105 mg, yield: 46.5 %) was prepared as a solid using N-(2-aminoethyl)morpholine as starting material according to the method similar to that for preparation example 22.

¹H NMR (400 MHz, CD₃OD) δ 9.28 (s, 1H), 8.50 (s, 1H), 7.07 (s, 1H), 6.95-6.78 (m, 1H), 6.68-6.65 (m, 1H), 4.65 (s, 2H), 4.60 (t, *J* = 8.7 Hz, 2H), 3.94-3.81 (m, 4H), 3.52 (t, *J =* 6.2 Hz, 2H), 3.38-3.29 (m, 4H), 3.09 (t, *J* = 5.7 Hz, 2H), 3.03-2.97 (m, 4H). LCMS [M+H]⁺ = 414.2.

### Preparation example 24: synthesis of compound HJM-024

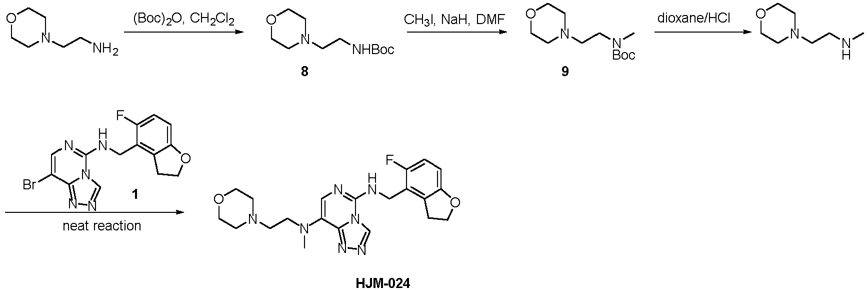

### Step 1:

*N*-(2-aminoethyl)morpholine (2 g, 15.38 mmol) was dissolved in dichloromethane (30 mL), di-tert-butyl dicarbonate (6.7 g, 30.77 mmol) was slowly added at room temperature, and the mixture was stirred at room temperature for 2 h. TLC showed that the reaction was completed. The mixture was concentrated under reduced pressure, and purified by silica gel column chromatography (PE:EA=3.3:1) to afford compound 8 (2.4 g, yield: 67.0%) as a solid, LCMS [M+H]⁺ = 231.2.

### Step 2:

Under N₂ protection, compound **8** (2.4 g, 10.43 mmol) was dissolved in anhydrous *N*,*N-*dimethylformamide (20 mL), 60% sodium hydride (626 mg, 15.65 mmol) was slowly added under an ice-water bath, and the mixture was stirred in the ice-water bath for 30 min. Iodomethane (1.3 mL, 20.86 mmol) was then added dropwise, and the mixture was kept at 0 °C and further stirred for 2 h. TLC showed that the reaction was completed. The mixture was poured into water to quench the reaction, and the aqueous phase was extracted with ethyl acetate (20 mL×2). The organic phases were combined, washed with saturated brine (5 mL×2), dried with Na₂SO₄, filtered, and concentrated. The residue was purified by silica gel column chromatography (PE:EA = 3.3:1) to afford compound 9 (1.5 g, yield: 59.1 %) as a solid, LCMS [M+H]⁺ = 245.2.

### Step 3:

Compound **9** (1.5 g, 15.38 mmol) was dissolved in a solution of hydrochloric acid in 1,4-dioxane (2 M, 4 mL), and the mixture was stirred at room temperature for 2 h. TLC showed that the reaction was completed. The mixture was concentrated, and the resulting crude was washed with ethyl acetate to afford solid compound *N*-methyl-2-morpholinylethylamine (500 mg, yield: 56.5 %), which was directly used in the next step without purification.

### Step 4:

**HJM-024** (16 mg, yield: 13.7 %) was prepared as a solid using *N*-methyl-2-morpholinylethylamine as starting material according to the method similar to that for preparation example **22.**

¹H NMR (400 MHz, CD₃OD) δ 9.28 (s, 1H), 7.11 (s, 1H), 6.88-6.75 (m, 1H), 6.62 (dd, *J =* 8.4, 3.6 Hz, 1H), 4.69 (s, 2H), 4.56 (t, *J =* 8.8 Hz, 2H), 3.70 (t, *J* = 6.8 Hz, 2H), 3.55-3.47 (m, 4H), 3.35 (t, *J =* 8.8 Hz, 2H), 2.95 (s, 3H), 2.59 (t, *J =* 6.8 Hz, 2H), 2.50-2.40 (m, 4H). LCMS [M+H]⁺ = 428.4.

### Preparation example 25: synthesis of compound HJM-025

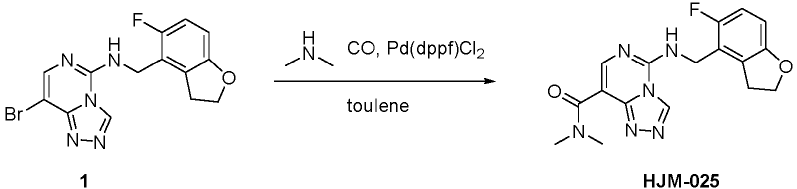

Under N₂ protection, a solution of 8-bromo-N-((5-fluoro-2,3-dihydrobenzofuran-4-yl)methyl)-[1,2,4]triazolo[4,3-c]pyrimidine-5-amine 1 (150 mg, 0.41 mmol) and dimethylamine in tetrahydrofuran (1M, 4.0 mL) was dissolved in anhydrous toluene (10 mL). 1,1'-Bis(diphenylphosphino)ferrocene-palladium(II)dichloride dichloromethane complex (60 mg, 0.08 mmol) was added at room temperature, the atmosphere was replaced three times with CO, and the mixture was heated to 110 °C under a CO balloon overnight (2 MP). TLC showed that the reaction was completed. The mixture was stopped from heating, naturally cooled to room temperature, and purified by preparative HPLC to afford **HJM-025** (7 mg, yield: 4.8 %) as a white solid.

¹H NMR (400 MHz, DMSO-*d*₆) δ 9.42 (s, 1H), 8.85 (s, 1H), 7.74 (s, 1H), 6.97-6.93 (m, 1H), 6.72-6.69 (m, 1H), 4.70 (s, 2H), 4.54 (t, *J =* 8.8 Hz, 2H), 3.29-3.27 (m, 2H), 2.99-2.90 (m, 6H). LCMS [M+H]⁺ = 357.2.

### Preparation example 26: synthesis of compound HJM-026

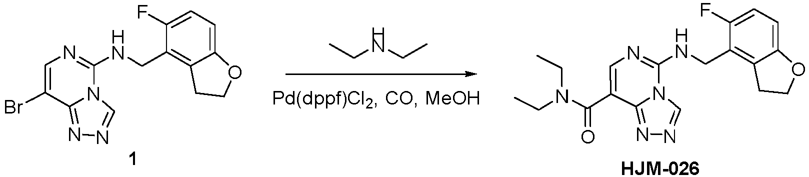

**HJM-026** (6.5 mg) was prepared as a white solid using diethylamine as starting material according to the method similar to that for preparation example **25.**

¹H NMR (400 MHz, CD₃OD) δ 9.29 (s, 1H), 7.79 (s, 1H), 6.85 (t, *J =* 9.2 Hz, 1H), 6.64 (dd, *J* = 8.8, 4.0 Hz, 1H), 4.80 (s, 2H), 4.57 (t, *J* = 8.8 Hz, 2H), 3.64-3.56 (m, 2H), 3.37 (t, *J* = 8.4 Hz, 4H), 1.36 - 1.30 (m, 3H), 1.29-1.11 (m, 3H). LCMS [M+H]⁺ = 385.1.

### Preparation example 27: synthesis of compound HJM-027

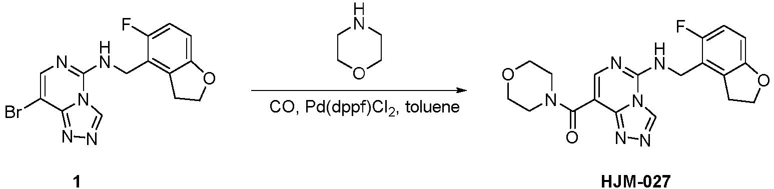

**HJM-027** (62 mg, yield: 18.3 %) was prepared as a yellow solid using morpholine as starting material according to the method similar to that for preparation example **25.**

¹H NMR (400 MHz, DMSO-*d*₆) δ 9.36 (s, 1H), 8.89-8.88 (m, 1H), 7.78 (s, 1H), 6.98-6.91 (m, 1H), 6.71-6.68 (m, 1H), 4.69 (s, 2H), 4.53 (t, *J =* 8.8 Hz, 2H), 3.60-3.59 (m, 4H), 3.50-3.46 (m, 2H), 3.29-3.27 (m, 2H), 2.67-2.66 (m, 2H). LCMS [M+H]⁺ = 399.2.

### Preparation example 28: synthesis of compound HJM-028

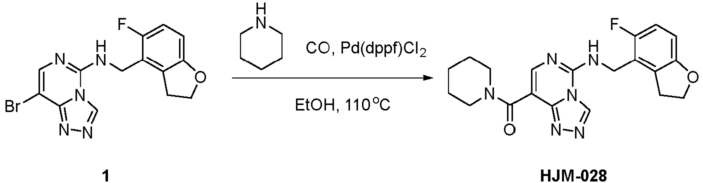

**HJM-028** (9 mg, yield: 4.3 %) was prepared as a white solid using piperidine as starting material according to the method similar to that for preparation example **25.**

¹H NMR (400 MHz, CD₃OD) δ 9.33 (s, 1H), 7.85 (s, 1H), 6.84-6.82 (m, 1H), 6.65-6.61 (m, 1H), 4.80 (s, 2H), 4.57 (t,*J* = 8.8 Hz, 2H), 3.76-3.78 (m, 2H), 3.39-3.34 (m, 4H), 1.69-1.58 (m, 6H). LCMS [M+H]⁺ = 397.4.

### Preparation example 29: synthesis of compound HJM-029

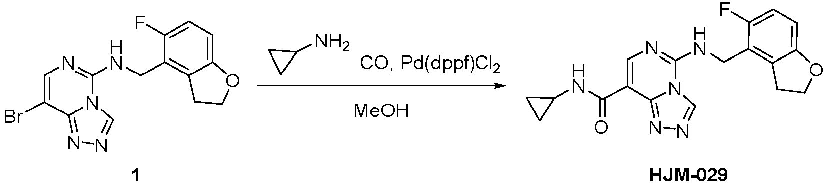

**HJM-029** (16 mg) was prepared as a white solid using cyclopropylamine as starting material according to the method similar to that for preparation example **25.**

¹H NMR (400 MHz, DMSO-*d*₆) δ 9.49 (s, 1H), 9.20 (t, *J =* 5.2 Hz, 1H), 8.76 (d, *J =* 4.0 Hz, 1H), 8.30 (s, 1H), 6.95 (dd, *J =* 10.2, 8.8 Hz, 1H), 6.71 (dd, *J =* 8.8, 4.0 Hz, 1H), 4.75 (d, *J =* 5.2 Hz, 2H), 4.54 (t, *J* = 8.8 Hz, 2H), 3.29 (t, *J =* 8.8 Hz, 2H), 2.94-2.87 (m, 1H), 0.82-77 (m, 2H), 0.62 - 0.53 (m, 2H). LCMS [M+H]⁺ = 369.1.

### Preparation example 30: synthesis of compound HJM-030

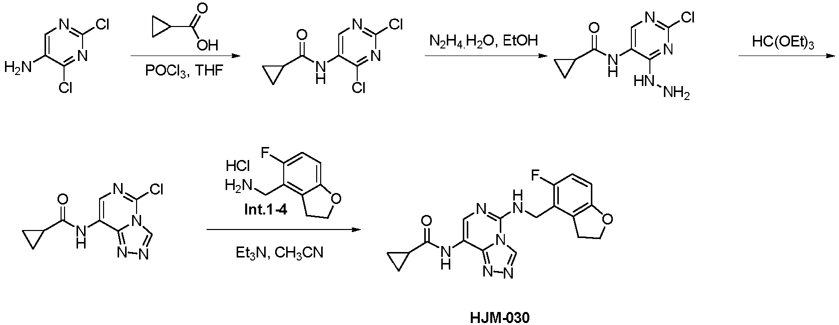

### Step 1:

5-Amino-2,4-dichloropyrimidine (1.0 g, 6.13 mmol) and cyclopropanecarboxylic acid (1.5 mL, 18.4 mmol) were dissolved in anhydrous tetrahydrofuran (20 mL), phosphorus oxychloride (0.2 mL, 2.18 mmol) was slowly added under N₂ protection, and the mixture was heated to 70 °C and stirred overnight. TLC showed that the reaction was completed. The mixture was stopped from heating, and naturally cooled to room temperature. Saturated NaHCO₃ solution was added to quench the reaction, and the aqueous phase was extracted with ethyl acetate (2×20 mL). The organic phases were combined, washed with saturated brine (2×20 mL), dried with Na₂SO₄, filtered, and concentrated. The residue was purified by silica gel column chromatography (PE:EA = 5:1) to afford compound *N*-(2,4-dichloropyrimidin-5-yl)cyclopropanecarboxamide (1.2 g, yield: 84.7 %) as a white solid, LCMS [M+H]⁺ = 232.1.

### Step 2:

Compound *N*-(2,4-dichloropyrimidin-5-yl)cyclopropanecarboxamide (600 mg, 2.60 mmol) was dissolved in ethanol (20 mL), hydrazine hydrate (0.18 mL, 3.12 mmol) was slowly added at room temperature, and the mixture was stirred at room temperature overnight. TLC showed that the reaction was completed. The mixture was concentrated under reduced pressure to afford crude compound *N*-(2-chloro-4-hydrazinopyrimidin-5-yl)cyclopropanecarboxamide (590 mg, quantitative yield), LCMS [M+H]⁺ = 228.2.

### Step 3:

Under N₂ protection, compound *N*-(2-chloro-4-hydrazinopyrimidin-5-yl)cyclopropanecarboxamide (590 mg, 2.60 mmol) was dissolved in 20 mL of triethyl orthoformate, and the mixture was heated to 150 °C and stirred for 3 h. TLC showed that the reaction was completed. The mixture was stopped from heating, naturally cooled to room temperature, and concentrated under reduced pressure. The residue was purified by silica gel column chromatography (PE:EA=5:1) to afford compound *N*-(5-chloro-[1,2,4]triazolo[4,3-c]pyrimidin-8-yl)cyclopropanecarboxamide (120 mg, yield: 19.2 %), LCMS [M+H]⁺ = 238.1.

### Step 4:

Under N₂ protection, 5-fluoro-2,3-dihydrobenzofuran-4-methylamine **Int.1-4** hydrochloride (153 mg, 0.75 mmol) and triethylamine (0.2 mL, 1.51 mmol) were dissolved in acetonitrile (5 mL), compound *N-*(5-chloro-[1,2,4]triazolo[4,3-c]pyrimidin-8-yl)cyclopropanecarboxamide (120 mg, 0.51 mmol) was added, and the mixture was heated to 50 °C and stirred for 2 h. TLC showed that the reaction was completed. The mixture was stopped from heating, naturally cooled to room temperature, and concentrated under reduced pressure. The residue was purified by preparative HPLC to afford **HJM-030** (50 mg, yield: 26.7 %) as a white solid.

¹H NMR (400 MHz, DMSO-*d*₆) δ 10.19 (s, 1H), 9.43 (s, 1H), 8.41 (t, *J* = 4.4 Hz, 1H), 8.08 (s, 1H), 7.02 - 6.85 (m, 1H), 6.70 (dd, *J =* 8.8, 4.0 Hz, 1H), 4.65 (d, *J =* 4.4 Hz, 2H), 4.54 (t, *J =* 8.8 Hz, 2H), 3.29 (t, *J =* 8.8 Hz, 2H), 2.17 - 2.06 (m, 1H), 0.79 (d, *J =* 6.0 Hz, 4H). LCMS [M+H]⁺ = 369.1.

### Preparation example 31: synthesis of compound HJM-031

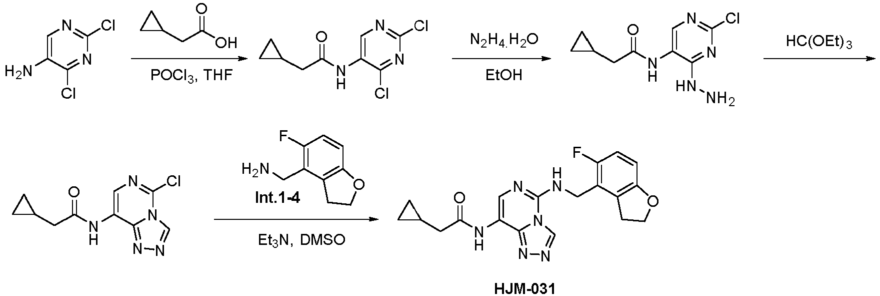

**HJM-031** (9 mg, yield: 7.6 %) was prepared as a white solid using cyclopropane acetic acid as starting material according to the method similar to that for preparation example **30.**

¹H NMR (400 MHz, DMSO-*d*₆) δ 10.19 (s, 1H), 9.43 (s, 1H), 8.41 (t, *J =* 4.4 Hz, 1H), 8.08 (s,1H), 7.02-6.85 (m, 1H), 6.70 (dd, *J =* 8.8, 4.0 Hz, 1H), 4.65 (d, *J =* 4.4 Hz, 2H), 4.54 (t, *J=* 8.8 Hz, 2H), 3.29 (t, *J* = 8.8 Hz, 2H), 2.17-2.06 (m, 1H), 0.79 (d, *J* = 6.0 Hz, 4H). LCMS [M+H]⁺ = 383.2.

### Preparation example 32: synthesis of compound HJM-032

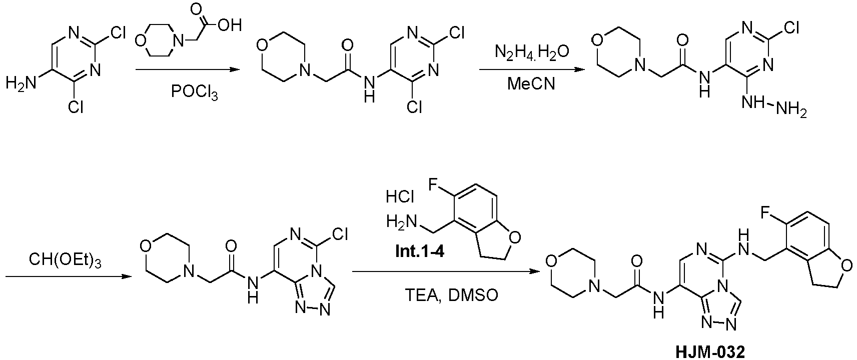

**HJM-032** (10 mg, yield: 5.8 %) was prepared as a white solid using 2-morpholinoacetic acid as starting material according to the method similar to that for preparation example **30.**

¹H NMR (400 MHz, CD₃OD) δ 9.29 (s, 1H), 8.08 (s, 1H), 6.89-6.75 (m, 1H), 6.65-6.52 (m, 1H), 4.76 (s, 2H), 4.57 (t, *J =* 6.8 Hz, 2H), 3.99-3.86 (m, 6H), 3.35 (t, *J =* 8.8 Hz, 2H), 3.22-3.17 (m, 4H). LCMS [M+H]⁺ = 428.1.

### Preparation example 33: synthesis of compound HJM-033

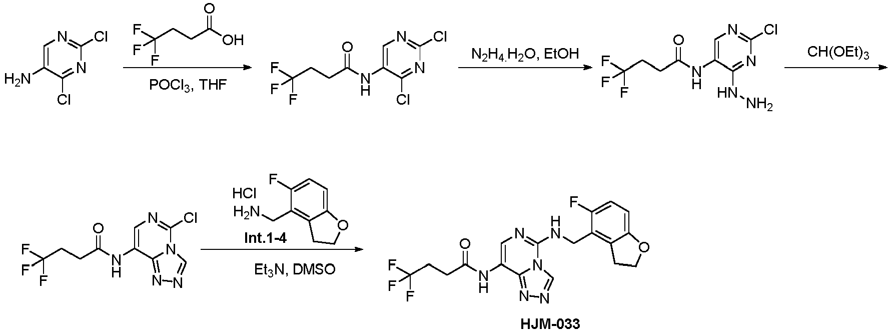

**HJM-033** (20 mg, yield: 23.1 %) was prepared as a white solid using 4,4,4-trifluorobutyric acid as starting material according to the method similar to that for preparation example **30.**

¹H NMR (400 MHz, DMSO-*d*₆) δ 10.1 (s, 1H), 9.42 (s, 1H), 8.43 (s, 1H), 8.11 (s, 1H), 6.94-6.92 (m, 1H), 6.71-6.89 (m, 1H), 4.66-4.64 (m, 2H), 4.56-4.52 (m, 2H), 3.33-3.22 (m, 2H), 2.76-2.72(m, 2H), 2.58-2.57 (m, 2H). LCMS [M+H]⁺ = 425.3.

### Preparation example 34: synthesis of compound HJM-034

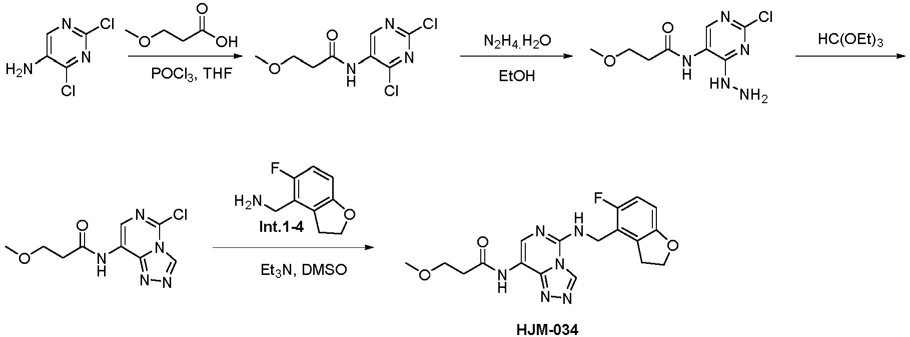

**HJM-034** (37 mg, yield: 50 %) was prepared as a white solid using 3-methoxypropionic acid as starting material according to the method similar to that for preparation example **30.**

¹H NMR (400 MHz, DMSO-*d*₆) δ 9.93 (s, 1H), 9.42 (s, 1H), 8.41 (t, *J =* 4.8 Hz, 1H), 8.13 (s, 1H), 6.94 (t, *J =* 9.2 Hz, 1H), 6.71-6.68 (m, 1H), 4.64 (d, *J* = 4.8 Hz, 2H), 4.53 (t, *J =* 8.8 Hz, 2H), 3.61 (t, *J* = 6.4 Hz, 2H), 3.31-3.27 (m, 2H), 3.26 (s, 3H), 2.67 (t, *J* = 6.4 Hz, 2H). LCMS [M+H]⁺ = 387.1.

### Preparation example 35: synthesis of compound HJM-035

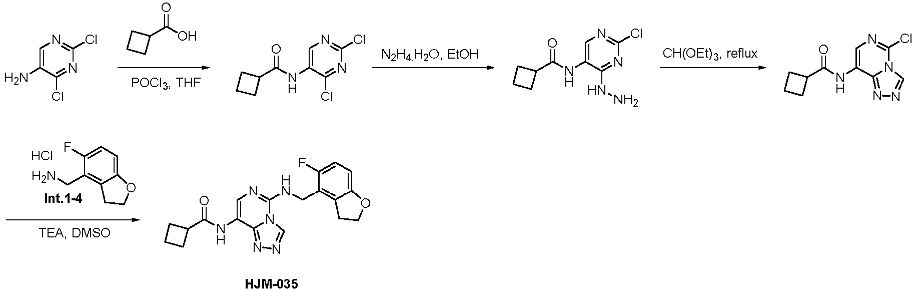

**HJM-035** (13 mg) was prepared as a white solid using cyclobutanecarboxylic acid as starting material according to the method similar to that for preparation example **30.**

¹H NMR (400 MHz, DMSO-*d*₆) δ 9.67 (s, 1H), 9.41 (s, 1H), 8.40 (t, *J =* 5.2 Hz, 1H), 8.07 (s, 1H), 6.94 (t, *J =* 9.6 Hz, 1H), 6.70 (dd, *J =* 8.8, 4.0 Hz, 1H), 4.65 (d, *J =* 4.8 Hz, 2H), 4.54 (t, *J* = 8.8 Hz, 2H), 3.56-3.25 (m, 3H), 2.27-2.17 (m, 2H), 2.16-2.06 (m, 2H), 1.98-1.89 (m, 1H), 1.86-1.77 (m, 1H). LCMS [M+H]⁺ = 383.2.

### Preparation example 36: synthesis of compound HJM-036

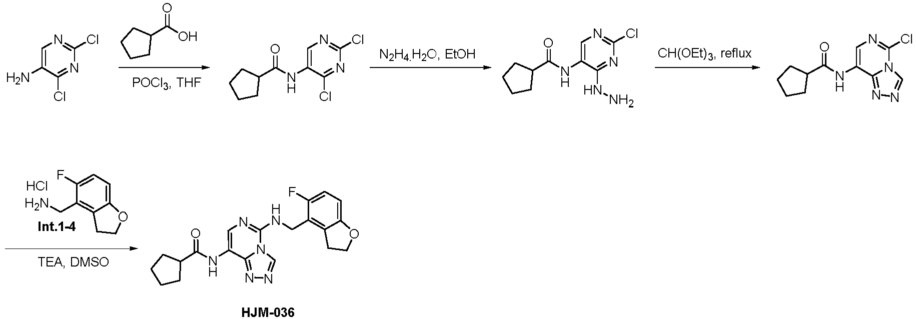

**HJM-036** (40 mg) was prepared as a white solid using cyclopentanecarboxylic acid as starting material according to the method similar to that for preparation example **30.**

¹H NMR (400 MHz, DMSO-*d*₆) δ 9.80 (s, 1H), 9.42 (s, 1H), 8.41 (t, *J =* 5.2 Hz, 1H), 8.06 (s, 1H), 6.94 (t, *J =* 9.2 Hz, 1H), 6.70 (dd, *J =* 8.4, 3.6 Hz, 1H), 4.65 (d, *J* = 4.8 Hz, 2H), 4.54 (t, *J* = 8.8 Hz, 2H), 3.29 (t, *J* = 8.8 Hz, 2H), 3.05-2.97 (m, 1H), 1.91-1.79 (m, 2H), 1.79-1.60 (m, 4H), 1.60-1.49 (m, 2H). LCMS [M+H]⁺ = 397.1.

### Preparation example 37: synthesis of compound HJM-037

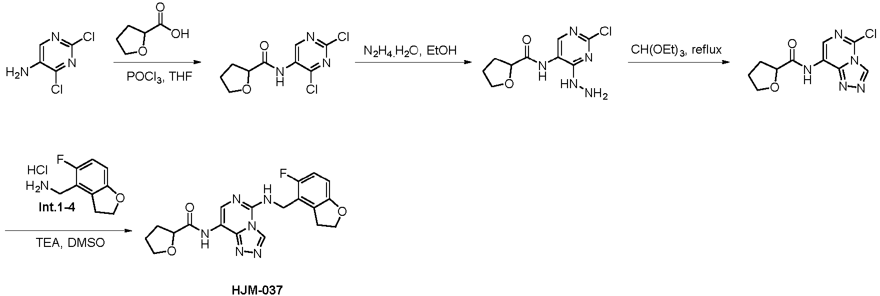

**HJM-037** (46 mg, yield: 42.7 %) was prepared as a white solid using 2-tetrahydrofuroic acid as starting material according to the method similar to that for preparation example **30.**

¹H NMR (400 MHz, DMSO-*d*₆) δ 9.42 (s, 1H), 9.33 (s, 1H), 8.49-8.48 (m, 1H), 8.07 (s, 1H), 6.95-6.91 (m, 1H), 6.71-6.68 (m, 1H), 4.65 (d, *J* = 4.4 Hz, 2H), 4.56-4.48 (m, 3H), 4.02-3.86 (m, 2H), 3.31-3.26 (m, 2H), 2.23-2.18 (m, 2H), 1.92-1.87 (m, 2H). LCMS [M+H]⁺ = 399.4.

### Preparation example 38: synthesis of compound HJM-038

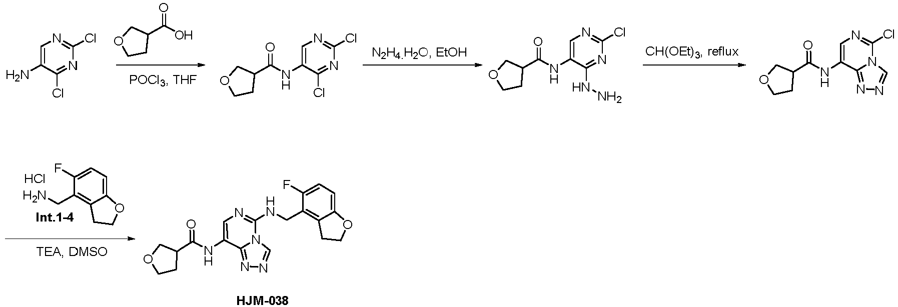

**HJM-038** (45 mg) was prepared as a white solid using tetrahydrofuran-3-carboxylic acid as starting material according to the method similar to that for preparation example **30.**

¹H NMR (400 MHz, DMSO-*d*₆) δ 10.01 (s, 1H), 9.42 (s, 1H), 8.42 (t, *J* = 5.2 Hz, 1H), 8.08 (s, 1H), 6.94 (dd, *J =* 10.4, 8.8 Hz, 1H), 6.70 (dd, *J =* 8.8, 4.0 Hz, 1H), 4.65 (d, *J =* 4.8 Hz, 2H), 4.54 (t, *J =* 8.8 Hz, 2H), 3.94 (t, *J* = 8.0 Hz, 1H), 3.83 - 3.65 (m, 3H), 341-3.35 (m, 1H), 3.30-.27 (m, 2H), 2.12-2.06 (m, 2H). LCMS [M+H]⁺ = 399.4.

### Preparation example 39: synthesis of compound HJM-039

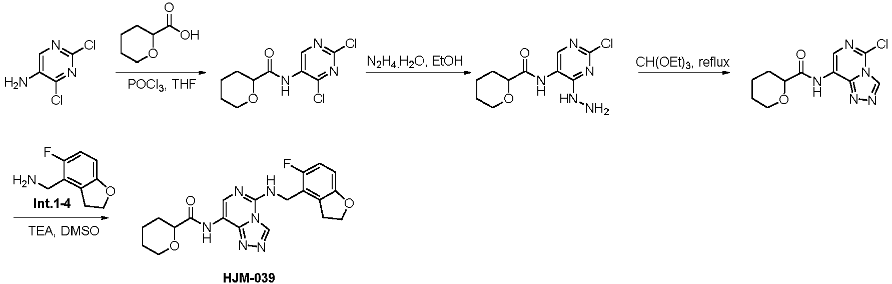

**HJM-039** (33 mg, yield: 44.5 %) was prepared as a white solid using tetrahydropyran-2-carboxylic acid as starting material according to the method similar to that for preparation example **30.**

¹H NMR (400 MHz, CD₃OD) δ 9.26 (s, 1H), 8.17 (s, 1H), 6.86-6.81 (m, 1H), 6.64-6.61 (m, 1H), 4.87 (s, 2H), 4.56 (t, *J =* 8.8Hz, 2H), 4.17-4.01 (m, 2H), 3.61-3.60 (m, 1H), 3.34 (t, *J* = 8.8 Hz, 2H), 2.15-1.98 (m, 2H), 1.67-1.60 (m, 4H). LCMS [M+H]⁺ = 413.2.

### Preparation example 40: synthesis of compound HJM-040

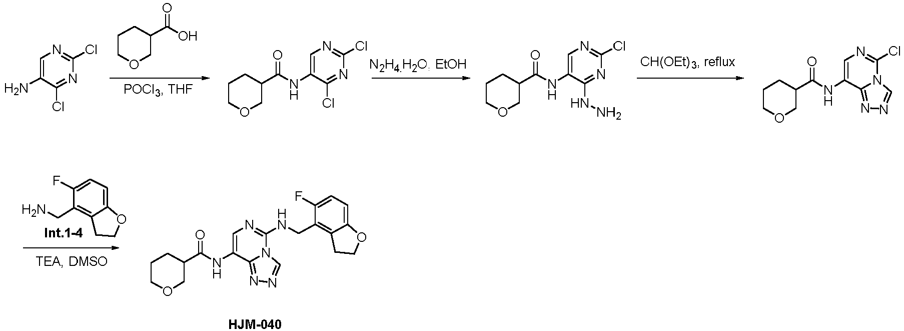

**HJM-040** (69 mg, yield: 47.8 %) was prepared as a white solid using tetrahydropyran-3-carboxylic acid as starting material according to the method similar to that for preparation example **30.**

¹H NMR (400 MHz, DMSO-*d*₆) δ 9.93 (s, 1H), 9.42 (s, 1H), 8.45-8.43 (m, 1H), 8.03 (s, 1H), 6.96-6.91 (m, 1H), 6.71-6.68 (m, 1H), 4.65-4.64 (m, 2H), 4.53 (t, *J =* 8.8 Hz, 2H), 3.96-3.78 (m, 2H), 3.41 (t, *J =* 6.8 Hz, 1H), 3.32-3.26 (m, 3H), 3.26-3.25 (m, 1H), 1.94-1.93 (m, 1H), 1.68-1.61 (m, 3H). LCMS [M+H]⁺ = 413.4.

### Preparation example 41: synthesis of compound HJM-041

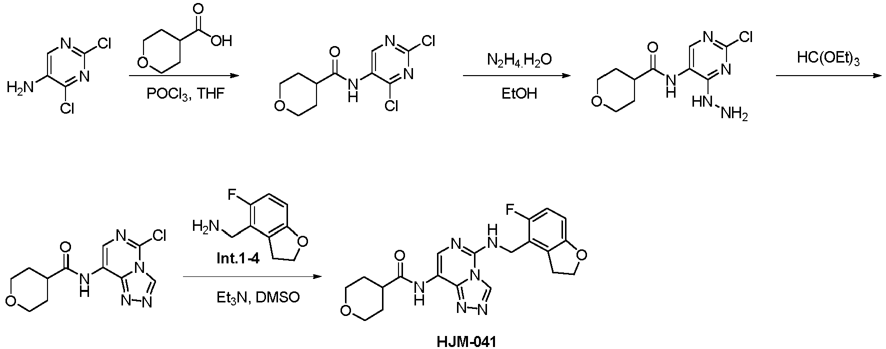

**HJM-041** (25 mg, yield: 28.0 %) was prepared as a white solid using tetrahydropyran-4-carboxylic acid as starting material according to the method similar to that for preparation example **30.**

¹H NMR (400 MHz, DMSO-*d*₆) δ 9.86 (s, 1H), 9.42 (s, 1H), 8.42 (t, *J =* 4.8 Hz, 1H), 8.06 (s, 1H), 6.94 (t, *J =* 9.2 Hz, 1H), 6.71-6.68 (m, 1H), 4.64 (d, *J* = 4.8 Hz, 2H), 4.53 (t, *J =* 8.8 Hz, 2H), 3.89 (d, *J =* 11.2 Hz, 2H), 3.37-3.34 (m, 2H), 3.28-3.26 (m, 2H), 2.84-2.82 (m, 1H), 1.72-1.63 (m, 4H). LCMS [M+H]⁺ = 413.2.

### Preparation example 42: synthesis of compound HJM-042

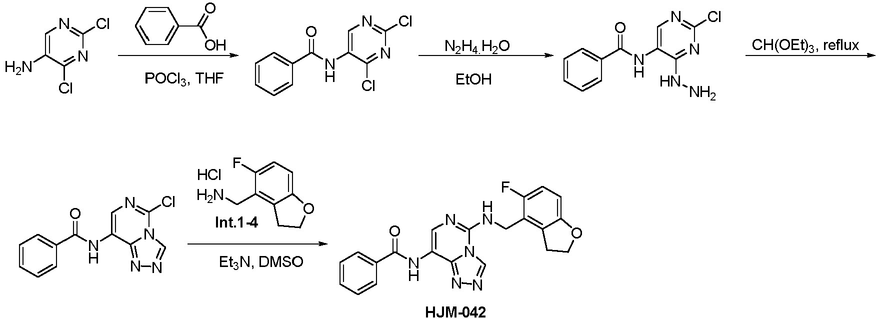

**HJM-042** (15 mg, yield: 25.8 %) was prepared as a blue solid using benzoic acid as starting material according to the method similar to that for preparation example **30.**

¹H NMR (400 MHz, CD₃OD) δ 9.31 (s, 1H), 8.12 (s, 1H), 8.06-8.04 (m, 2H), 7.63-7.55 (m, 3H), 6.89 (t, *J =* 8.8 Hz, 1H), 6.66-6.64 (m, 1H), 4.82-4.80 (m, 2H), 4.61 (t, *J* = 8.4 Hz, 2H), 3.41 (t, *J =* 8.4 Hz, 2H). LCMS [M+H]⁺ = 405.1.

### Preparation example 43: synthesis of compound HJM-043

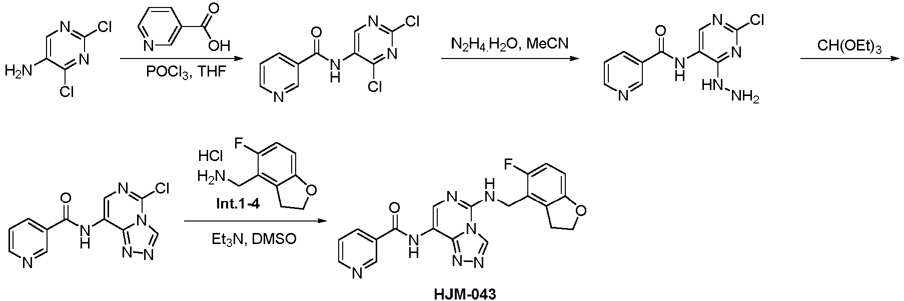

**HJM-043** (87 mg) was prepared as a white solid using nicotinic acid as starting material according to the method similar to that for preparation example **30.**

¹H NMR (400 MHz, DMSO-*d*₆) δ 10.48 (s, 1H), 9.48 (s, 1H), 9.16 (s, 1H), 8.79 (dd, *J* = 4.8, 1.6 Hz, 1H), 8.64 (t, *J =* 5.2 Hz, 1H), 8.36 (d, *J =* 8.0 Hz, 1H), 7.90 (s, 1H), 7.60 (dd, *J =* 8.0, 4.8 Hz, 1H), 7.03-6.89 (m, 1H), 6.75-6.70 (m, 1H), 4.70 (d, *J* = 4.8 Hz, 2H), 4.56 (t, *J* = 8.8 Hz, 2H), 3.32 (t, *J* = 8.8 Hz, 2H). LCMS [M+H]⁺ = 406.1.

### Preparation example 44: synthesis of compound HJM-044

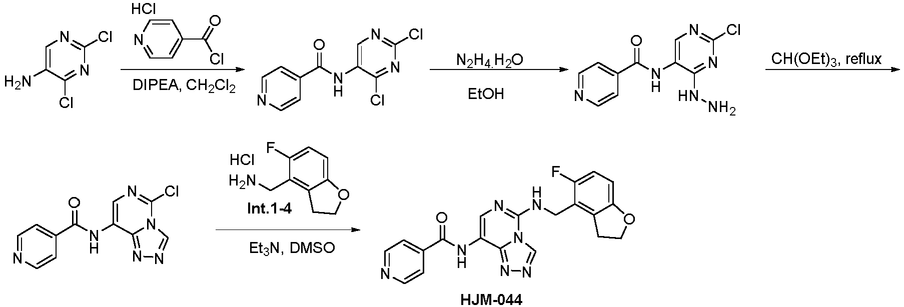

**HJM-044** (7.0 mg) was prepared as a white solid using isonicotinic acid chloride as starting material according to the method similar to that for preparation example **30.**

¹H NMR (400 MHz, DMSO-*d*₆) δ 10.54 (s, 1H), 9.46 (s, 1H), 8.81-8.80 (m, 2H), 8.64 (t, *J =* 5.2 Hz, 1H), 7.91-7.90 (m, 2H), 7.88 (s, 1H), 6.98 (t, *J =* 8.8 Hz, 1H), 6.73-6.68 (m, 1H), 4.70(d, *J* = 4.8 Hz, 2H), 4.55 (t, *J =* 8.8 Hz, 2H), 3.41-3.29 (m, 2H). LCMS [M+H]⁺ = 406.1.

### Preparation example 45: synthesis of compound HJM-045

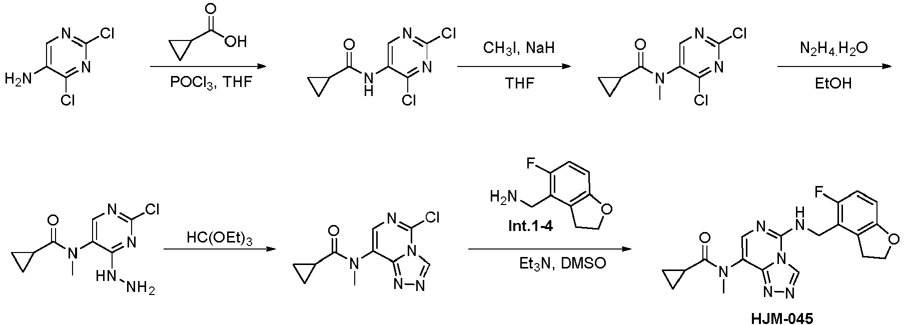

### Steps 1-2:

Under N₂ protection, compound *N*-(2,4-dichloropyrimidin-5-yl)cyclopropanecarboxamide (See Step 1 of preparation example **30,** 700 mg, 3.0 mmol) was dissolved in anhydrous tetrahydrofuran (10 mL), 60% sodium hydride (145 mg, 3.6 mmol) was slowly added under an ice-water bath, and the mixture was stirred in an ice-water bath for 16 min. Iodomethane (1.28 g, 9.0 mmol) was then slowly added dropwise. After the dropwise addition, the ice-water bath was removed, and the mixture was stirred at room temperature for 2 h. TLC showed that the reaction was completed. The mixture was concentrated under reduced pressure, and purified by silica gel column chromatography (PE:EA = 2:1) to afford compound *N*-(2,4-dichloropyrimidin-5-yl)-*N*-methylcyclopropanecarboxamide (350 mg, yield: 47 %) as a white solid, LCMS [M+H]⁺ = 246.1.

### Steps 3-5:

**HJM-045** (12 mg, yield: 26 %) was prepared as a white solid using compound *N*-(2,4-dichloropyrimidin-5-yl)-*N*-methylcyclopropanecarboxamide as starting material according to the method similar to that for preparation example **30.**

¹H NMR (400 MHz, DMSO-*d*₆) δ 9.47 (s, 1H), 8.81 (s, 1H), 7.73 (s, 1H), 6.95 (t, *J =* 9.2 Hz, 1H), 6.72-6.69 (m, 1H), 4.67 (s, 2H), 4.55 (t, *J =* 8.8 Hz, 2H), 3.32 (t, *J =* 8.8 Hz, 2H), 3.17(s, 3H), 1.54-1.50 (m, 1H), 0.82-70 (m, 2H), 0.65-0.47 (m, 2H). LCMS [M+H]⁺ = 383.2.

### Preparation example 46: synthesis of compound HJM-046

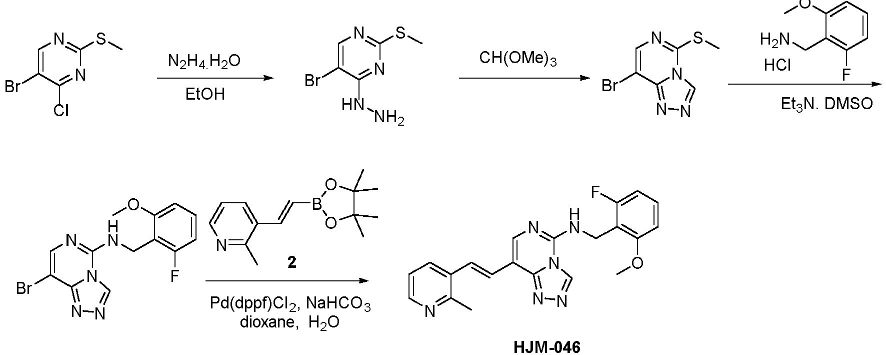

### Step 1:

2-Methylthio-4-chloro-5-bromopyrimidine (33.0 g, 0.138 mol) was dissolved in ethanol (350 mL), 85% hydrazine hydrate (13.8 g, 0.235 mol) was slowly added at room temperature, and the mixture was stirred at room temperature for 4 h. TLC showed that the reaction was completed. The mixture was concentrated under reduced pressure to afford crude compound 2-methylthio-4-hydrazinyl-5-bromopyrimidine (32.0 g, yield: 99.0 %), which was directly used in the next step without purification.

¹H NMR (400 MHz, DMSO-*d*₆) δ 8.07 (s, 1H), 2.45 (s, 3H). LCMS [M+CH₃CN]⁺ = 275.0.

### Step 2:

Under N₂ protection, the crude compound 2-methylthio-4-hydrazinyl-5-bromopyrimidine (32.0 g, 0.138 mol) was dissolved in 150 mL of triethyl orthoformate, and the mixture was heated to 150 °C and stirred for 3 h. TLC showed that the reaction was completed. The mixture was stopped from heating, and naturally cooled to room temperature. The mixture was concentrated under reduced pressure, slurried with ethyl acetate, and filtered to afford 8-bromo-5-(methylthio)-[1,2,4]triazolo[4,3-c]pyrimidine (27.0 g, yield: 80.0 %) as a grayish white solid.

¹H NMR (400 MHz, DMSO-*d*₆) δ 9.58 (s, 1H), 8.19 (s, 1H), 2.75 (s, 3H). LCMS [M+H]⁺ = 245.1.

### Step 3:

Under N₂ protection, compound 8-bromo-5-(methylthio)-[1,2,4]triazolo[4,3-c]pyrimidine (1.0 g, 4.08 mmol) was dissolved in dimethyl sulfoxide (10 mL), 2-fluoro-6-methoxybenzylamine hydrochloride (3.9 g, 20.40 mmol) and triethylamine (4.1g, 40.80 mmol) were added successively, and the mixture was heated to 70 °C and stirred overnight. TLC showed that the reaction was completed. The mixture was stopped from heating, and naturally cooled to room temperature. Water and ethyl acetate were added, and the mixture was layered. The aqueous phase was extracted with ethyl acetate (2×50 mL). The organic phases were combined, and then washed successively with water (2×20 mL) and saturated brine. The organic layer was dried with Na₂SO₄, filtered, and concentrated. The residue was purified by silica gel column chromatography (DCM:MeOH = 20:1) to afford 8-bromo-*N*-(2-fluoro-6-methoxybenzyl)-[1,2,4]triazolo[4,3-c]pyrimidin-5-amine (550 mg, yield: 38.0 %) as a white solid.

¹H NMR (400 MHz, DMSO-*d*₆) δ 9.50 (s, 1H), 8.49 (s, 1H), 7.86 (s, 1H), 7.38 - 7.40 (m, 1H), 6.93 (d, *J =* 7.2 Hz, 1H), 6.86 (t, *J* = 8.8 Hz, 1H), 4.66 (s, 2H), 3.83 (s, 3H). LCMS [M+H]⁺ = 352.1.

### Step 4:

Under N₂ protection, 8-bromo-*N*-(2-fluoro-6-methoxybenzyl)-[1,2,4]triazolo[4,3-c]pyrimidin-5-amine (200 mg, 0.57 mmol), pinacol borate **2** (418 mg, 1.71 mmol) and NaHCO₃ (181 mg, 1.71 mmol) were dissolved in a mixture (8 mL) of 1,4-dioxane and water (3:1), and 1,1'-bis(diphenylphosphino)ferrocene-palladium(II)dichloride dichloromethane complex (37 mg, 0.05 mmol) was added at room temperature. The atmosphere was replaced three times with N₂, and the mixture was heated to 100 °C and reacted for 2 h. TLC showed that the reaction was completed. The mixture was stopped from heating, naturally cooled to room temperature, and concentrated under reduced pressure. The residue was purified by silica gel column chromatography (DCM:MeOH = 12:1) to afford **HJM-046** (200 mg, yield: 90.1%) as a yellow solid.

¹H NMR (400 MHz, DMSO-*d*₆) δ 9.52 (s, 1H), 8.52 (t, *J =* 4.4 Hz, 1H), 8.33 (d, *J =* 4.4 Hz, 1H), 8.25 (d, *J =* 16.0 Hz, 1H), 8.06 (d, *J =* 8.0 Hz, 1H), 7.89 (s, 1H), 7.40 (dd, *J* = 16.0, 8.0 Hz, 1H), 7.30-7.25 (m, 2H), 6.95 (d, *J =* 8.0 Hz, 1H), 6.88 (t, *J =* 8.8 Hz, 1H), 4.73 (d, *J =* 4.0 Hz, 2H), 3.84 (s, 3H), 2.61 (s, 3H). LCMS [M+H]⁺ = 391.4.

### Preparation example 47: synthesis of compound HJM-047

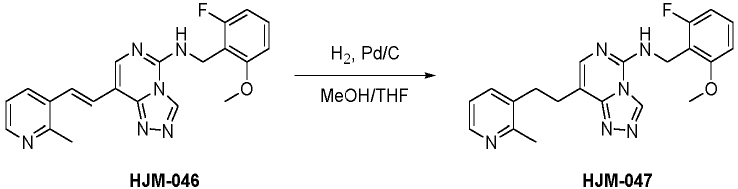

Compound **HJM-046** (150 mg, 0.38 mmol) was dissolved in a mixture of tetrahydrofuran (7 mL) and methanol (7 mL), and the atmosphere was replaced with N₂. 30.0 mg of 10% Pd/C was added quickly, the atmosphere was replaced with H₂, and the mixture was stirred at room temperature under a hydrogen balloon for 2 h. The mixture was filtered, and the filter cake was washed twice with methanol. The filtrates were combined, concentrated, and purified by preparative HPLC to afford **HJM-047** (65 mg, yield: 43.0 %) as a white solid.

¹H NMR (400 MHz, DMSO-*d*₆) δ 9.43 (s, 1H), 8.28 (dd, *J =* 4.8, 1.6 Hz, 1H), 8.11 (t, *J =* 4.4 Hz, 1H), 7.54 - 7.48 (m, 1H), 7.44 (s, 1H), 7.38 (dd, *J =* 15.2, 8.4 Hz, 1H), 7.13 (dd, *J =* 7.6, 4.8 Hz, 1H), 6.93 (d, *J =* 8.4 Hz, 1H), 6.86 (t, *J =* 8.8 Hz, 1H), 4.64 (d, *J* = 3.6 Hz, 2H), 3.82 (s, 3H), 3.08 - 2.93 (m, 4H), 2.49 (s, 3H). LCMS [M+H]⁺ = 393.6.

### Preparation example 48: synthesis of compound HJM-048

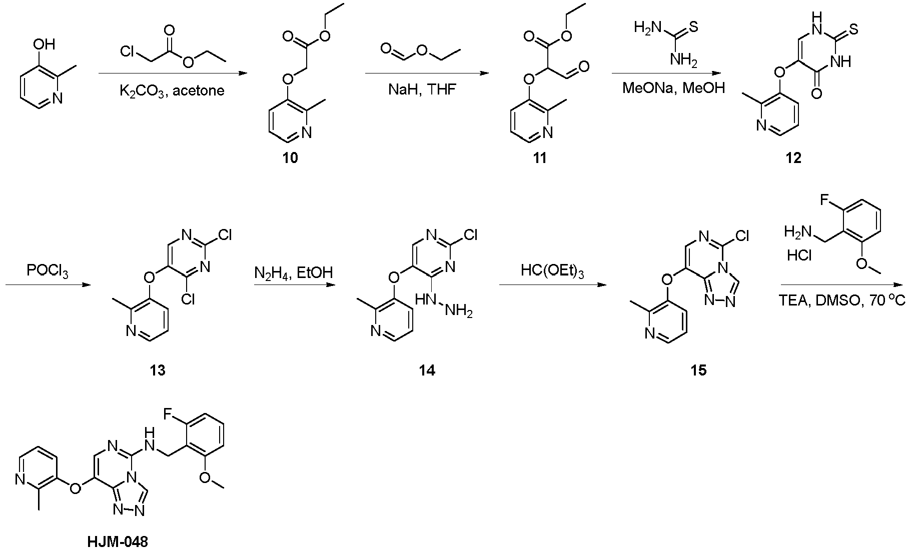

### Step 1:

3-Hydroxy-2-methylpyridine (4.7 g, 0.043 mol) and K₂CO₃ (8.9 g, 0.065 mol) were dissolved in anhydrous acetone (100 mL), ethyl chloroacetate (6.9 mL, 0.065 mol) was slowly added under N₂ protection, and the mixture was heated to 80 °C and stirred for 16 h. TLC showed that the reaction was completed. The mixture was stopped from heating, naturally cooled to room temperature, and filtered. The filtrate was concentrated under reduced pressure, and purified by silica gel column chromatography (PE:EA=20:1) to afford compound **10** (3.0 g, yield: 36.4 %) as a brown liquid, LCMS [M+H]⁺ = 196.2.

### Step 2:

Compound **10** (3.0 g, 0.015 mol) was dissolved in anhydrous tetrahydrofuran (50 mL), NaH (0.92 g, 0.023 mol, w/w 60%) was added under an ice-water bath and N₂ protection, and the mixture was stirred at room temperature for 1 h. The mixture was then cooled to 0 °C, and ethyl formate (1.8 mL, 0.023 mol) was slowly added dropwise. After the dropwise addition, the mixture was stirred at room temperature overnight. TLC showed that the reaction was completed. The mixture was poured into ice water to quench the reaction, and the aqueous phase was extracted with ethyl acetate (2×100 mL). The organic phases were combined, washed with saturated brine (2×20 mL), dried with Na₂SO₄, and filtered. The filtrate was concentrated to afford crude compound **11** (3.3 g, quantitative yield), which was directly used in the next step without purification, LCMS [M+H]⁺ = 224.3.

### Step 3:

Compound **11** (3.3 g, 15 mol) and thiourea (2.2 g, 30 mol) were dissolved in methanol (200 mL), sodium methoxide (1.6 g, 30 mmol) was slowly added under N₂ protection, and the mixture was heated to 80 °C and stirred for 16 h. TLC showed that the reaction was completed. The mixture was stopped from heating, and naturally cooled to room temperature. The mixture was poured into ice water to quench the reaction, and the aqueous phase was extracted with ethyl acetate (2×100 mL). The organic phases were combined, washed with saturated brine (2×20 mL), dried with Na₂SO₄, filtered, and concentrated. The residue was purified by silica gel column chromatography (DCM:MeOH = 20:1) to afford compound **12** (1.2 g, yield: 34.3%) as a yellow solid, LCMS [M+H]⁺ = 236.1.

### Step 4:

Under N₂ protection, compound **12** (700 mg, 2.98 mmol) was dissolved in phosphorus oxychloride (10 mL), and the mixture was heated to 90 °C and stirred for 48 h. TLC showed that the reaction was completed. The mixture was stopped from heating, naturally cooled to room temperature, and concentrated under reduced pressure to remove most of the solvent. The residue was adjusted to weak alkalinity with saturated NaHCO₃ solution, and the aqueous phase was extracted with ethyl acetate (2×50 mL). The organic phases were combined, washed with saturated brine (2×20 mL), dried with Na₂SO₄, filtered, and concentrated. The residue was purified by silica gel column chromatography (DCM:MeOH = 100:1) to afford compound **13** (350 mg, yield: 46.1 %) as a white solid, LCMS [M+H]⁺ = 256.1.

### Step 5:

Compound **13** (350 mg, 1.39 mmol) was dissolved in ethanol (5 mL), hydrazine hydrate (0.1 mL, 1.67 mmol) was slowly added at room temperature, and the mixture was stirred at room temperature for 2 h. TLC showed that the reaction was completed. The mixture was concentrated under reduced pressure to afford crude compound **14** (350 mg, quantitative yield), LCMS [M+H]⁺ = 252.1.

### Step 6:

Under N₂ protection, compound **14** (300 mg, 1.20 mmol) was dissolved in 30 mL of triethyl orthoformate, and the mixture was heated to 150 °C and reacted for 4 h. TLC showed that the reaction was completed. The mixture was stopped from heating, naturally cooled to room temperature, and concentrated under reduced pressure. The residue was purified by silica gel column chromatography (DCM:MeOH=50: 1) to afford **15** (150 mg, yield: 48.1 %) as a yellow solid, LCMS [M+H]⁺ = 262.3.

### Step 7:

Under N₂ protection, 2-fluoro-6-methoxybenzylamine hydrochloride (330 mg, 1.72 mmol) and triethylamine (0.40 mL, 2.85 mmol) were dissolved in dimethyl sulfoxide (5 mL), compound **15** (150 mg, 0.57 mmol) was added, and the mixture was heated to 80 °C and stirred for 2 h. TLC showed that the reaction was completed. The mixture was stopped from heating, naturally cooled to room temperature, and poured into ice water. Solid was precipitated, and filtered. The filter cake was purified by preparative HPLC to afford **HJM-048** (85 mg, yield: 39.3 %) as a white solid.

¹H NMR (400 MHz, DMSO-*d*₆) δ 9.48 (s, 1H), 8.32-8.16 (s, 1H), 7.69 (s, 1H), 7.45-7.32 (m, 2H), 7.24 (dd, *J =* 8.4, 4.8 Hz, 1H), 6.96 (d, *J =* 8.4 Hz, 1H), 6.89 (t, *J =* 8.8 Hz, 1H), 4.67 (d, *J =* 3.2 Hz, 2H), 3.85 (s, 3H), 2.63 (s, 3H). LCMS [M+H]⁺ = 381.2.

### Preparation example 49: synthesis of compound HJM-049

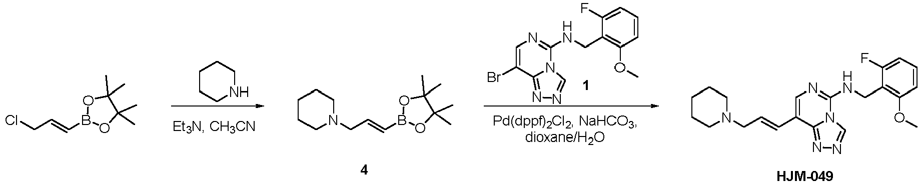

### Step 1:

Under N₂ protection, piperidine (0.1 mL, 0.99 mmol) and triethylamine (0.2 mL, 1.49 mmol) were dissolved in acetonitrile (3 mL), and 3-chloropropenyl-1-boric acid pinacol ester (100 mg, 0.50 mmol) was added at room temperature. The atmosphere was replaced three times with N₂, and the mixture was stirred at room temperature overnight. TLC showed that the reaction was completed. The mixture was concentrated under reduced pressure to afford crude compound **2** (120 mg, quantitative yield), which was directly used in the next step without purification. LCMS [M+H]⁺ = 252.3.

### Step 2:

Under N₂ protection, pinacol borate **4** (100 mg, 0.40 mmol), 8-bromo-*N*-((2-fluoro-6-methoxybenzyl)-[1,2,4]triazolo[4,3-c]pyrimidin-5-amine **1** (140 mg, 0.40 mmol) and NaHCO₃ (127 mg, 1.20 mmol) were dissolved in a mixture (8 mL) of 1,4-dioxane and water (3:1), 1,1'-bis(diphenylphosphino)ferrocene-palladium(II)dichloride dichloromethane complex (30 mg, 0.04 mmol) was added at room temperature, and the mixture was heated to 100 °C and reacted for 2 h. TLC showed that the reaction was completed. The mixture was stopped from heating, naturally cooled to room temperature, and concentrated under reduced pressure. The residue was purified by preparative HPLC to afford **HJM-049** (12 mg, yield: 7.6 %) as a white solid.

¹H NMR (400 MHz, DMSO-*d*₆) δ 9.48 (s, 1H), 8.39 (t, *J=* 4.4 Hz, 1H), 7.70 (s, 1H), 7.39 (dd, *J*= 15.6, 8.4 Hz, 1H), 7.13 - 7.02 (m, 1H), 6.93 (d, *J* = 8.4 Hz, 1H), 6.86 (t, *J =* 8.4 Hz, 1H), 6.58 (d, *J =* 15.6 Hz, 1H), 4.69 (d, *J =* 3.6 Hz, 2H), 3.83 (s, 3H), 3.21 (d, *J =* 6.8 Hz, 2H), 2.52 - 2.49 (m, 4H), 1.59 - 1.51 (m, 4H), 1.46 - 1.35 (m, 2H). LCMS [M+H]⁺ = 397.4.

### Preparation example 50: synthesis of compound HJM-050 (for reference)

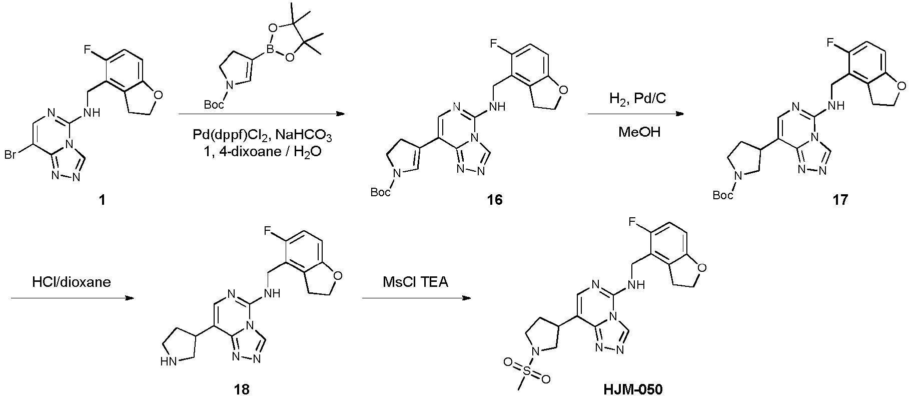

### Step 1:

Under N₂ protection, 8-bromo-N-((5-fluoro-2,3-dihydrobenzofuran-4-yl)methyl)-[1,2,4]triazolo[4,3-c]pyrimidine-5-amine **1** (200 mg, 0.55 mmol), *N*-tert-butoxycarbonyl-2,3-dihydro-1H-pyrrole-4-boronic acid pinacol ester (325 mg, 1.10 mmol) and NaHCO₃ (175 mg, 1.65 mmol) were dissolved in a mixture (12 mL) of 1,4-dioxane and water (3:1), 1,1'-bis(diphenylphosphino)ferrocene-palladium(II)dichloride dichloromethane complex (36 mg, 0.05 mmol) was added at room temperature, and the mixture was heated to 100 °C and reacted for 2 h. TLC showed that the reaction was completed. The mixture was stopped from heating, naturally cooled to room temperature, and concentrated under reduced pressure. The residue was purified by silica gel column chromatography (DCM:MeOH=20:1) to afford 16 (200 mg, yield: 80.6 %) as a brown solid, LCMS [M+H]⁺ = 453.1.

### Step 2:

Compound **16** (200 mg, 0.44 mmol) was dissolved in methanol (20 mL), and the atmosphere was replaced with N₂. 20 mg of 10% Pd/C was added quickly, the atmosphere was replaced with H₂ for three times, and the mixture was stirred at room temperature under a hydrogen balloon for 2 h. The mixture was filtered, and the filter cake was washed twice with methanol. The filtrates were combined and concentrated to afford crude product compound **17** (200 mg, quantitative yield), which was directly used in the next step without purification. LCMS [M+H]⁺ = 455.2.

### Step 3:

Compound **17** (200 mg, 0.44 mmol) was dissolved in ethanol (5 mL), and the mixture was slowly added to a solution of hydrochloric acid in 1,4-dioxane (4 M, 5 mL) at room temperature. The mixture was stirred at room temperature for 4 h. TLC showed that the reaction was completed. The mixture was concentrated to afford crude compound **18** (150 mg, quantitative yield), which was directly used in the next step without purification. LCMS [M+H]⁺ = 355.4.

### Step 4:

Under N₂ protection, compound **18** (150 mg, 0.42 mmol) and triethylamine (0.18 mL, 1.27 mmol) were dissolved in dichloromethane (5 mL), methanesulfonyl chloride (0.06 mL, 0.85 mmol) was slowly added dropwise at room temperature, and the mixture was stirred at room temperature for 30 min. TLC showed that the reaction was completed. The mixture was poured into water to quench the reaction, and the aqueous phase was extracted with dichloromethane (2×50 mL). The organic phases were combined, washed with saturated brine (2×5 mL), dried with Na₂SO₄, and filtered. The filtrate was concentrated and purified by preparative HPLC to afford **HJM-050** (39 mg, yield: 21.5 %) as a white solid.

¹H NMR (400 MHz, DMSO-*d*₆) δ 9.41 (s, 1H), 8.53 (t, *J =* 5.2 Hz, 1H), 7.57 (s, 1H), 7.03 - 6.87 (m, 1H), 6.70 (dd, *J =* 8.4, 4.0 Hz, 1H), 4.65 (d, *J* = 4.8 Hz, 2H), 4.54 (t, *J* = 8.8 Hz, 2H), 3.76 - 3.67 (m, 1H), 3.67 - 3.57 (m, 1H), 3.56 - 3.44 (m, 2H), 3.42 - 3.36 (m, 1H), 3.30 - 3.26 (m. 2H), 2.98 (s, 3H), 2.39 - 2.21 (m, 2H). LCMS [M+H]⁺ = 433.3.

### Preparation example 51: synthesis of compound HJM-051 (for reference)

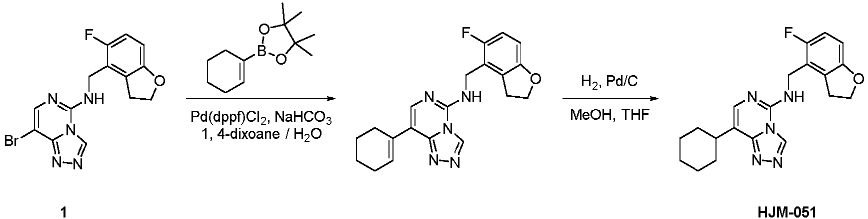

**HJM-051** (9 mg, yield: 9.9 %) was prepared as a white solid using cyclohexene-1-boronic acid pinacol ester as starting material according to the method similar to that for preparation example **3.**

¹H NMR (400 MHz, DMSO-*d*₆) δ 9.38 (s, 1H), 8.37 (s, 1H), 7.44 (s, 1H), 6.94 (t, *J =* 9.2 Hz, 1H), 6.70 (d, *J =* 5.2 Hz, 1H), 4.63 (s, 2H), 4.54 (t, *J* = 8.4 Hz, 2H), 3.28 (t, *J =* 8.4 Hz, 2H), 2.92 - 2.75 (m, 1H), 1.95-1.85 (m, 2H), 1.85-1.76 (m, 2H), 1.74 - 1.61 (m, 3H), 1.48 - 1.17 (m, 3H). LCMS [M+H]⁺ = 368.4.

### Preparation example 52: synthesis of compound HJM-052 (for reference)

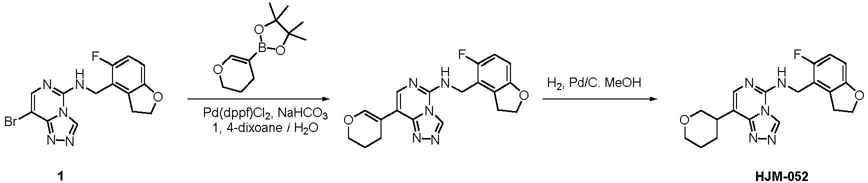

**HJM-052** (10 mg, yield: 14.1%) was prepared as a yellow solid using 2-(3,4-dihydro-2H-pyran-5-yl)-4,4,5,5-tetramethyl-1,3,2-dioxaborolane as starting material according to the method similar to that for preparation example **3.**

¹H NMR (400MHz, CD₃OD) δ 9.24 (s, 1H), 7.63 (s, 1H), 6.84 (t, *J* = 9.2 Hz, 1H), 6.63 (dd, *J* = 8.4, 4.0 Hz, 1H), 4.74 (s, 2H), 4.56 (t, *J =* 8.4 Hz, 2H), 4.10-4.06 (m, 1H), 3.96-3.93 (m, 1H), 3.73-3.66 (m, 1H), 3.60-3.54 (m, 1H), 3.38-3.35 (m, 1H), 3.34-3.31 (m, 2H), 2.08 -2.02 (m, 2H), 1.83-1.72 (m, 2H). LCMS [M+H]⁺ = 370.3.

### Preparation example 53: synthesis of compound HJM-053 (for reference)

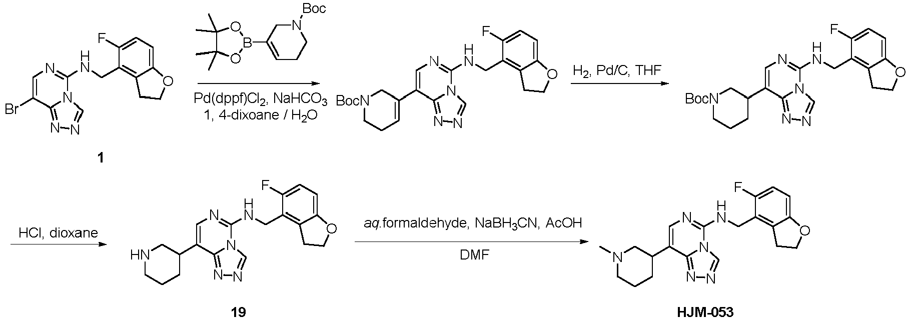

### Steps 1-3:

Compound **19** (200 mg) was prepared as a white solid using *N*-tert-butoxycarbonyl-1,2,5,6-tetrahydropyridine-3-boronic acid pinacol ester as starting material according to the method similar to that for preparation example **50.**

### Step 4:

Under N₂ protection, compound **19** (40 mg, 0.38 mmol) was dissolved in *N*,*N*-dimethylformamide (3 mL), 37% formaldehyde aqueous solution (0.7 mL) and a drop of glacial acetic acid were slowly added at room temperature, and the mixture was stirred at 0 °C for 30 min. Sodium cyanobohydride (27 mg, 0.42 mmol) was added, and the mixture was further stirred at 0 °C for 30 min. TLC showed that the reaction was completed. The rection mixture was poured into saturated NaHCO₃ solution to quench the reaction, and the aqueous phase was extracted with ethyl acetate (50 mL×2). The organic phases were combined, washed with saturated brine (2×5 mL), dried with Na₂SO₄, and filtered. The filtrate was concentrated and purified by preparative HPLC to afford **HJM-053** (33 mg, yield: 22.76 %) as a white solid.

¹H NMR (400 MHz, DMSO-*d*₆) δ 9.64 (s, 1H), 9.43 (s, 1H), 8.63 (s, 1H), 7.55 (s, 1H), 6.99 - 6.86 (m, 1H), 6.70 (dd, *J =* 8.4, 3.6 Hz, 1H), 4.65 (s, 2H), 4.54 (t, *J =* 8.8 Hz, 2H), 3.62 (d, *J =* 11.2 Hz, 1H), 3.50 (d, *J =* 11.2 Hz, 1H), 3.43 - 3.24 (m, 4H), 3.04 - 2.92 (m, 1H), 2.83 (d, *J =* 4.4 Hz, 3H), 2.06 - 1.73 (m, 4H). LCMS [M+H]⁺ = 383.3.

### Preparation example 54: synthesis of compound HJM-054 (for reference)

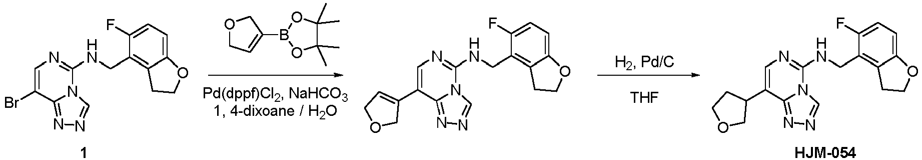

**HJM-054** (7 mg, yield: 8.7 %) was prepared as a white solid using 2-(2,5-dihydrofuran-3-yl)-4,4,5,5-tetramethyl-1,3,2-dioxaborolane as starting material according to the method similar to that for preparation example **3.**

¹H NMR (400 MHz, CD₃OD) δ 9.28 (s, 1H), 7.68 (s, 1H), 6.88 - 6.79 (m, 1H), 6.63 (dd, *J =* 8.8, 4.0 Hz, 1H), 4.75 (s, 2H), 4.57 (t, *J =* 8.8 Hz, 2H), 4.18 - 4.12 (m, 1H), 4.11 - 4.05 (m, 1H), 3.98 - 3.88 (m, 2H), 3.76 - 3.69 (m, 1H), 3.38 - 3.32 (m, 2H), 2.41 - 2.35 (m, 1H), 2.32 - 2.23 (m, 1H). LCMS [M+H]⁺ = 356.3.

### Preparation example 55: synthesis of compound HJM-055 (for reference)

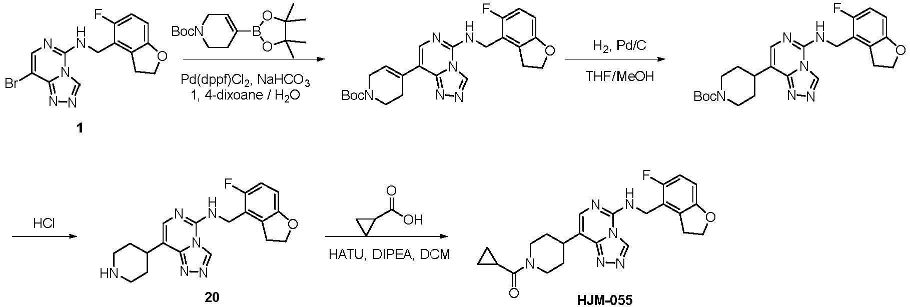

### Steps 1-3:

Compound **20** (200 mg) was prepared as a white solid using *N*-tert-butoxycarbonyl-1,2,5,6-tetrahydropyridine-4-boronic acid pinacol ester as starting material according to the method similar to that for preparation example **50.**

### Step 4:

Under N₂ protection, compound **20** (100 mg, 0.25 mmol) and cyclopropanecarboxylic acid (43 mg, 0.50 mmol) were dissolved in dichloromethane (4 mL), HATU (141 mg, 0.37 mmol) and *N*,*N-*diisopropylethylamine (160 mg, 1.24 mmol) were slowly added at room temperature, and the mixture was stirred at room temperature for 2 h. TLC showed that the reaction was completed. The mixture was concentrated under reduced pressure, and purified by preparative HPLC to afford **HJM-055** (18 mg, yield: 15.25 %) as a white solid.

¹H NMR (400 MHz, DMSO-*d*₆) δ 9.39 (s, 1H), 8.41 (t, *J =* 5.2 Hz, 1H), 7.48 (s, 1H), 7.00 - 6.86 (m, 1H), 6.69 (dd, *J =* 8.4, 4.0 Hz, 1H), 4.64 (d, *J =* 4.8 Hz, 2H), 4.54 (t, *J =* 8.8 Hz, 4H), 4.40 (d, *J* = 12.0 Hz, 1H), 3.29 (t, *J* = 8.8 Hz, 2H), 3.16 - 3.05 (m, 1H), 2.71-2.65 (m, 1H), 2.07 - 1.94 (m, 2H), 1.91 - 1.85 (m, 2H), 1.75 - 1.68 (m, 1H), 0.76 - 0.69 (m, 4H). LCMS [M+H]⁺ = 437.2.

### Preparation example 56: synthesis of compound HJM-056 (for reference)

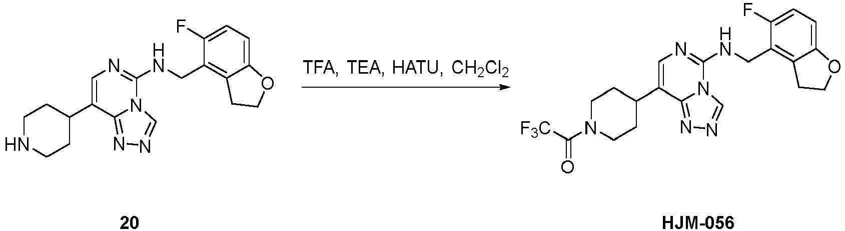

**HJM-056** (3.1 mg, yield: 2.5 %) was prepared as a white solid using trifluoroacetic acid as starting material according to the method similar to that for preparation example **55.**

¹H NMR (400 MHz, DMSO-*d*₆) δ 9.39 (s, 1H), 8.47 (s, 1H), 7.50 (s, 1H), 6.99 - 6.87 (m, 1H), 6.69 (dd, *J =* 8.8, 4.0 Hz, 1H), 4.64 (d, *J =* 4.8 Hz, 2H), 4.53 (t, *J =* 8.8 Hz, 2H), 4.44 (d, *J* = 13.2 Hz, 1H), 3.98 (d, *J* = 13.2 Hz, 1H), 3.46 - 3.42 (m, 1H), 3.30 - 3.26 (m, 2H), 3.23 - 3.16 (m, 1H), 3.03 (t, *J =* 12.0 Hz, 1H), 2.09 - 1.86 (m, 4H). LCMS [M+H]⁺ = 465.2.

### Preparation example 57: synthesis of compound HJM-057 (for reference)

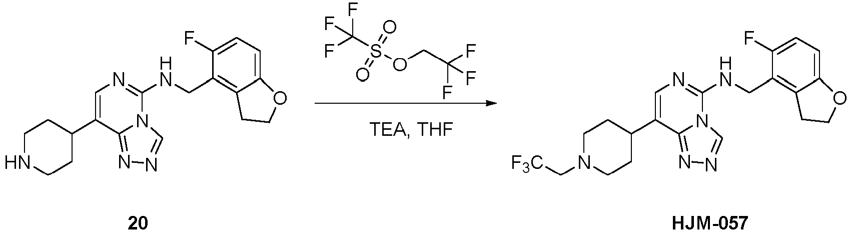

Under N₂ protection, compound **20** (100 mg, 0.25 mmol) and triethylamine (0.11 mL, 0.81 mmol) were dissolved in tetrahydrofuran (5 mL), trifluoroethyl trifluoromethanesulfonate (0.08 mL, 0.54 mmol) was slowly added at room temperature, and the mixture was stirred at room temperature for 16 h. TLC showed that the reaction was completed. The mixture was concentrated under reduced pressure, and purified by preparative HPLC to afford **HJM-057** (28 mg, yield: 23.1 %) as a white solid.

¹H NMR (400 MHz, DMSO-*d*₆) δ 9.39 (s, 1H), 8.45 (s, 1H), 7.47 (s, 1H), 6.97 - 6.88 (m, 1H), 6.69 (dd, *J =* 8.8, 4.0 Hz, 1H), 4.63 (d, *J =* 4.4 Hz, 2H), 4.53 (t, *J =* 8.8 Hz, 2H), 3.28 (t, *J =* 8.8 Hz, 2H), 3.19 (dd, *J =* 20.4, 10.0 Hz, 2H), 3.02 (d, *J =* 11.2 Hz, 2H), 2.88 - 2.77 (m, 1H), 2.50 - 2.42 (m, 2H), 2.00 - 1.79 (m, 4H). LCMS [M+H]⁺ = 451.2.

### Preparation example 58: synthesis of compound HJM-058 (for reference)

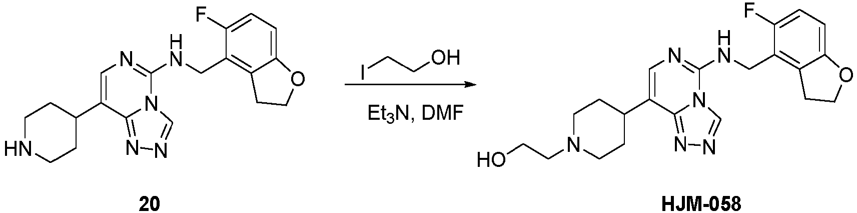

Under N₂ protection, compound **20** (100 mg, 0.25 mmol) and triethylamine (125 mg, 1.24 mmol) were dissolved in *N*,*N-*dimethylformamide (4 mL), 2-iodoethanol (0.08 mL, 0.54 mmol) was slowly added at room temperature, and the mixture was stirred at room temperature for 16 h. TLC showed that the reaction was completed. The mixture was concentrated under reduced pressure, and purified by preparative HPLC to afford **HJM-058** (9 mg, yield: 8.8 %) as a white solid.

¹H NMR (400 MHz, DMSO-*d*₆) δ 9.40 (s, 1H), 8.47 (s, 1H), 8.26 (s, 1H), 7.45 (s, 1H), 6.93 (t, *J =* 9.2 Hz, 1H), 6.70 - 6.67 (m, 1H), 4.64 (s, 2H), 4.53 (t, *J* = 8.4 Hz, 2H), 3.60 (s, 2H), 3.28 (t, *J* = 8.4 Hz, 2H), 3.21 - 3.15 (m, 2H), 2.94 - 2.87 (m, 1H), 2.69 - 2.65 (m, 2H), 2.45 (d, *J =* 10.0 Hz, 2H), 2.17 - 1.83 (m, 4H). LCMS [M+H]⁺ = 413.3.

### Preparation example 59: synthesis of compound HJM-059 (for reference)

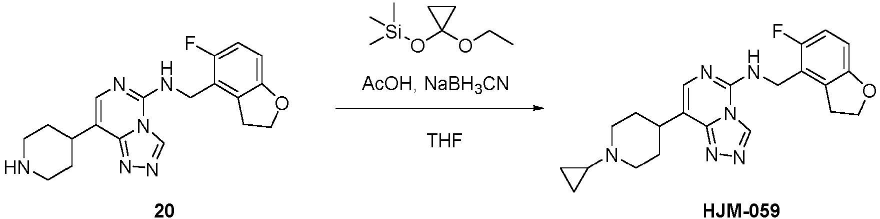

Under N₂ protection, compound **20** (100 mg, 0.25 mmol) and (1-ethoxycyclopropyloxy)trimethylsilane (0.27 mL, 1.36 mmol) were dissolved in tetrahydrofuran (10 mL), glacial acetic acid (1 mL) and sodium cyanobohydride (85 mg, 1.36 mmol) were slowly added at room temperature, and the mixture was stirred at 70 °C for 16 h. TLC showed that the reaction was completed. The mixture was concentrated under reduced pressure, and purified by preparative HPLC to afford **HJM-059** (3.1 mg, yield: 2.8 %) as a yellow solid.

¹H NMR (400 MHz, DMSO-*d*₆) δ 9.38 (s, 1H), 8.39 (t, *J =* 4.8 Hz, 1H), 7.44 (s, 1H), 6.97 - 6.89 (m, 1H), 6.69 (dd, *J =* 8.8, 4.0 Hz, 1H), 4.63 (d, *J =* 4.8 Hz, 2H), 4.53 (t, *J =* 8.8 Hz, 2H), 3.28 (t, *J* = 8.8 Hz, 2H), 3.05 (d, *J =* 11.6 Hz, 2H), 2.89 - 2.78 (m, 1H), 2.35 - 2.23 (m, 2H), 1.90 - 1.76 (m, 4H), 1.68 - 1.62 (m, 1H), 0.47 - 0.40 (m, 2H), 0.34 - 0.28 (m, 2H). LCMS [M+H]⁺ = 409.2.

### Preparation example 60: synthesis of compound HJM-060 (for reference)

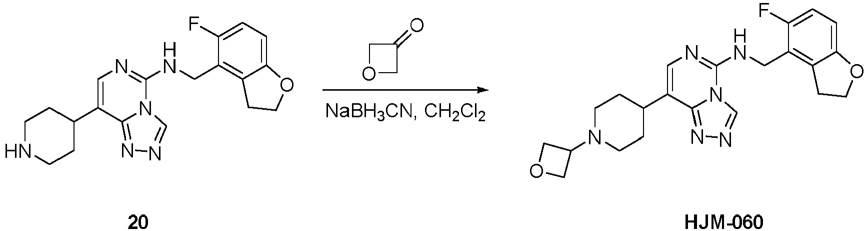

Under N₂ protection, compound **20** (100 mg, 0.25 mmol) and oxetan-3-one (0.08 mL, 1.36 mmol) were dissolved in dichloromethane (5 mL), sodium cyanobohydride (85 mg, 1.36 mmol) was slowly added at room temperature, and the mixture was stirred at room temperature for 16 h. TLC showed that the reaction was completed. The mixture was concentrated under reduced pressure, and purified by preparative HPLC to afford **HJM-060** (11 mg, yield: 9.6 %) as a yellow solid.

¹H NMR (400 MHz, DMSO-*d*₆) δ 9.39 (s, 1H), 8.42 (s, 1H), 7.47 (s, 1H), 6.93 (t, *J =* 9.2 Hz, 1H), 6.69 (dd, *J =* 8.8, 4.0 Hz, 1H), 4.64 (d, *J =* 4.0 Hz, 2H), 4.58 - 4.51 (m, 4H), 4.45 (t, *J =* 6.0 Hz, 2H), 3.44 - 3.40 (m, 1H), 3.28 (t, *J =* 8.8 Hz, 2H), 2.91 - 2.74 (m, 3H), 1.99 - 1.78 (m, 6H). LCMS [M+H]⁺ = 425.2.

### Preparation example 61: synthesis of compound HJM-061 (for reference)

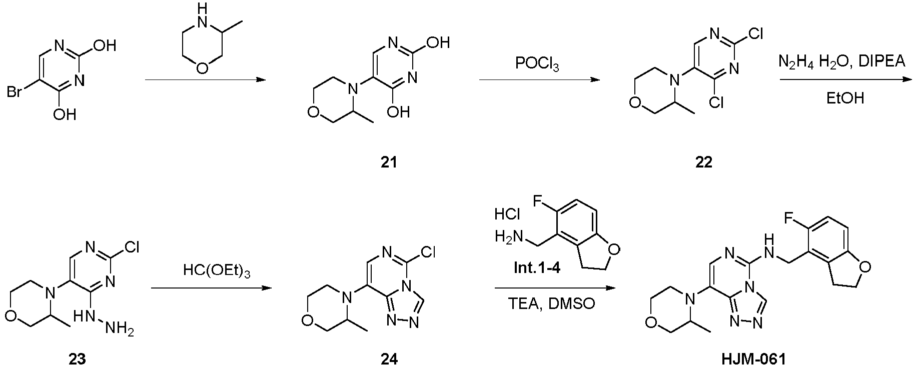

### Step 1:

Under N₂ protection, a solution of 5-bromo-uracil (2.0 g, 10.5 mmol) and 3-methylmorpholine (5.32 g, 52.5 mmol) was heated to 135 °C and stirred for 2 h. TLC showed that the reaction was completed. The mixture was stopped from heating, and naturally cooled to room temperature. The mixture was concentrated under reduced pressure, and the solid was washed twice with dichloromethane to afford **21** (2.0 g, yield: 90 %) as a white solid, LCMS [M+H]⁺ = 212.2.

### Step 2:

Under N₂ protection, compound **21** (2.0 g, 9.48 mmol) was dissolved in phosphorus oxychloride (12 mL), and the mixture was heated to 110 °C and stirred for 96 h. TLC showed that the reaction was completed. The mixture was stopped from heating, naturally cooled to room temperature, and concentrated under reduced pressure to remove most of the solvent. The residue was adjusted to weak alkalinity with saturated NaHCO₃ solution, and the aqueous phase was extracted with ethyl acetate (2×50 mL). The organic phases were combined, washed with saturated brine (2×20 mL), dried with Na₂SO₄, filtered, and concentrated. The residue was purified by silica gel column chromatography (PE:EA=2:1) to afford **22** (800 mg, yield: 34 %) as a white solid, LCMS [M+H]⁺ = 248.1.

### Step 3:

Compound **22** (200 mg, 0.81 mmol) and *N*,*N*-diisopropylethylamine (209 mg, 1.62 mmol) were dissolved in ethanol (3 mL), 85% hydrazine hydrate (58 mg, 0.98 mmol) was slowly added at room temperature, and the mixture was stirred at room temperature for 16 h. TLC showed that the reaction was completed. The mixture was concentrated under reduced pressure, and purified by reversed silica gel column chromatography (CH₃CN/H₂O=30/70) to afford **23** (100 mg, yield: 50 %) as a white solid, LCMS [M+H]⁺ = 244.1.

### Step 4:

Under N₂ protection, compound **23** (100 mg, 0.41 mmol) was dissolved in 9 mL of triethyl orthoformate, and the mixture was heated to 150 °C and stirred for 4 h. TLC showed that the reaction was completed. The mixture was stopped from heating, naturally cooled to room temperature, and concentrated under reduced pressure. The residue was purified by silica gel column chromatography (PE:EA=5:4) to afford **24** (100 mg, yield: 96 %) as a white solid, LCMS [M+H]⁺ = 254.1.

### Step 5:

Under N₂ protection, 5-fluoro-2,3-dihydrobenzofuran-4-methylamine **Int.1-4** hydrochloride (120 mg, 0.59 mmol) and triethylamine (79 mg, 0.78 mmol) were dissolved in dimethyl sulfoxide (3 mL), compound **24** (100 mg, 0.39 mmol) was added, and the mixture was stirred at room temperature for 4 h. TLC showed that the reaction was completed. The mixture was stopped from heating, and naturally cooled to room temperature. Water and ethyl acetate were added, and the mixture was layered. The aqueous phase was extracted with ethyl acetate (3×5 mL). The organic phases were combined, and then washed successively with water and saturated brine. The organic layer was dried with Na₂SO₄, and filtered. The filtrate was concentrated and purified by preparative HPLC to afford **HJM-061** (50 mg, yield: 33.0 %) as a white solid.

¹H NMR (400 MHz, DMSO-*d*₆) δ 9.39 (s, 1H), 8.13 (t, *J =* 4.8 Hz, 1H), 7.126 (s, 1H), 6.94 (t, *J =* 8.8 Hz, 1H), 6.67-6.68 (m, 1H), 4.61 (d, *J =* 4.8 Hz, 2H), 4.54 (t, *J =* 8.8 Hz, 2H), 4.31-4.27 (m, 1H), 3.84-3.79 (m, 2H), 3.71-3.65 (m, 1H), 3.52-3.48 (m, 1H), 3.31(d, *J* = 4.4 Hz, 2H), 3.17-3.06 (m, 2H), 0.85 (d, *J =* 6.4 Hz, 3H). LCMS [M+H]⁺ = 385.2.

### Preparation example 62: synthesis of compound HJM-062 (for reference)

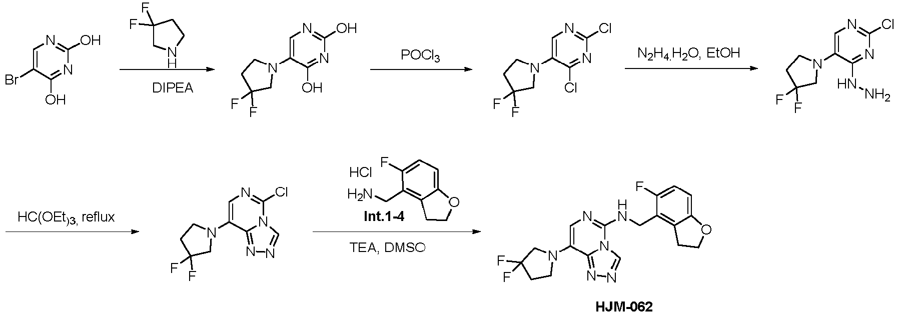

**HJM-062** (100 mg, yield: 46.0 %) was prepared as a white solid using 3,3-difluoropyrrolidine as starting material according to the method similar to that for preparation example 61.

¹H NMR (400 MHz, DMSO-*d*₆) δ 9.39 (s, 1H), 8.00 (t, *J =* 5.2 Hz, 1H), 6.92 (t, *J =* 9.2 Hz, 2H), 6.70-6.67 (m, 1H), 4.59 (d, *J =* 4.8 Hz, 2H), 4.53 (t, *J =* 8.4 Hz, 2H), 3.86 (t, *J* = 13.2 Hz, 2H), 3.64 (t, *J =* 7.2 Hz, 2H), 3.27 (t, *J* = 8.8 Hz, 2H), 2.54(d, *J* = 7.2 Hz, 2H). LCMS [M+H]⁺ = 391.1.

### Preparation example 63: synthesis of compound HJM-063 (for reference)

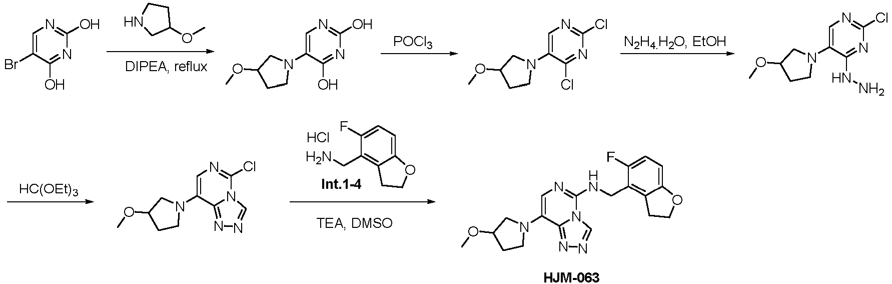

**HJM-063** (10 mg, yield: 4.0 %) was prepared as a white solid using 3-methoxypyrrolidine as starting material according to the method similar to that for preparation example **61.**

¹H NMR (400 MHz, DMSO-*d*₆) δ 9.36 (s, 1H), 7.79 (t, *J =* 5.2 Hz, 1H), 6.92 (t, *J =* 8.8 Hz, 1H), 6.77 (s, 1H), 6.70-6.67 (m, 1H), 4.58-4.50 (m, 4H), 4.08-4.05 (m, 1H), 3.64-3.60 (m, 1H), 3.54-3.43 (m, 3H), 3.29-3.25 (m, 5H), 2.11-2.07 (m, 1H), 1.99-1.97 (m, 1H). LCMS [M+H]⁺ = 385.1.

### Preparation example 64: synthesis of compound HJM-064 (for reference)

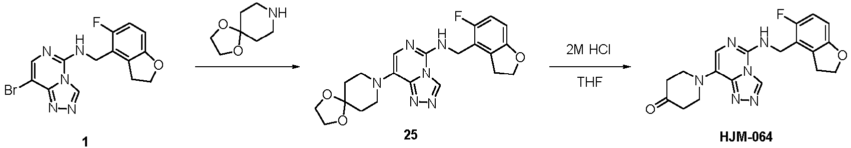

### Step 1:

Compound **25** (200 mg) was prepared as a white solid using 4-piperidone ethylene glycol acetal as starting material according to the method similar to that for preparation example **22.** LCMS: [M+H]⁺ = 427.1.

### Step 2:

Compound **25** (100 mg, 0.23 mmol) was dissolved in tetrahydrofuran (2 mL), 2M HCl aqueous solution (0.5 mL) was added, and the mixture was stirred at room temperature overnight. The mixture was concentrated to afford a crude product. The crude product was purified by preparative HPLC to afford compound **HJM-064** (20 mg, yield: 22.3%) as a white solid.

¹H NMR (400 MHz, DMSO-*d*₆) δ 9.41 (s, 1H), 8.11 (t, *J =* 5.2 Hz, 1H), 7.18 (s, 1H), 6.93 (t, *J =* 9.6 Hz, 1H), 6.69 (dd, *J* = 8.4, 3.6 Hz, 1H), 4.61 (d, *J =* 4.8 Hz, 2H), 4.54 (t, *J =* 8.8 Hz, 2H), 3.65 (t, *J =* 6.0 Hz, 4H), 3.29 (t, *J* = 8.8 Hz, 2H), 2.55-2.48 (m, 4H). LCMS [M+H]⁺ = 383.3.

### Preparation example 65: synthesis of compound HJM-065

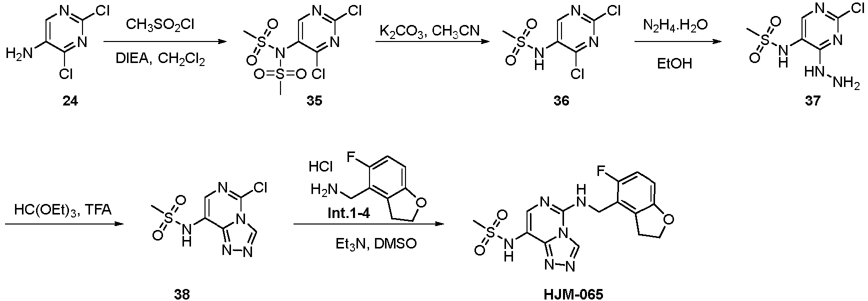

### Step 1:

5-Amino-2,4-dichloropyrimidine **24** (944 mg, 5.75 mmol) and *N*,*N*-diisopropylethylamine (2.96 g, 23 mmol) were dissolved in dichloromethane (25 mL), methanesulfonyl chloride (2.62 g, 23 mmol) was added dropwise, and the mixture was stirred at room temperature overnight. The mixture was concentrated to afford a crude product. The crude product was purified by silica gel column chromatography (petroleum ether/ethyl acetate, ethyl acetate: 0-20%) to afford compound **35** (455 mg, yield: 24.8%) as a white solid.

¹H NMR (400 MHz, CDCl₃) δ 8.54 (s, 1H), 3.51 (s, 6H). LCMS [M+H]⁺ = 320.1.

### Step 2:

Compound **35** (455 mg, 1.42 mmol) was dissolved in acetonitrile (30 mL), potassium carbonate (1.95 g, 14.2 mmol) was added, and the mixture was stirred at room temperature overnight. The mixture was filtered, and the filtrate was concentrated to afford crude compound **36** (310 mg, yield: 91%) as a white solid.

¹H NMR (400 MHz, DMSO-*d*₆) δ 8.35 (s, 1H), 2.67 (s, 3H). LCMS [M-H]⁻ = 239.9.

### Step 3:

Compound **36** (260 mg, 1.07 mmol) was dissolved in ethanol (5 mL), hydrazine hydrate (76 mg, 1.29 mmol) was added, and the mixture was stirred at room temperature for 2 h. The mixture was concentrated to afford a crude product. The crude product was purified by silica gel column chromatography (dichloromethane/methanol, methanol: 0-10%) to afford compound **37** (190 mg, yield: 75%) as a brown oil. LCMS [M+H]⁺ = 238.1.

### Step 4:

Compound **37** (190 mg, 0.86 mmol) was dissolved in triethyl orthoformate (8 mL), trifluoroacetic acid (1 mL) was added, and the mixture was stirred at 120 °C for 2 h. The mixture was cooled, and concentrated to afford a crude product. The crude product was purified by preparative TLC to afford compound **38** (12 mg, yield: 5.6%) as a white solid. LCMS [M+H]⁺ = 248.1.

### Step 5:

Compound **38** (12 mg, 0.048 mmol), (5-fluoro-2,3-dihydrobenzofuran-4-yl)methylamine **Int.1-4** hydrochloride (12 mg, 0.057 mmol) and triethylamine (10 mg, 0.096 mmol) were dissolved in dimethyl sulfoxide (3 mL), and the mixture was stirred at room temperature for 1 h. The mixture was purified by preparative HPLC to afford compound **HJM-065** (2 mg, yield: 11.1%) as a white solid.

¹H NMR (400 MHz, CD₃OD) δ 9.28 (s, 1H), 7.72 (s, 1H), 6.85 (t, *J =* 9.5 Hz, 1H), 6.65 (dd, *J* = 8.6, 3.8 Hz, 1H), 4.77 (s, 2H), 4.58 (t, *J =* 8.7 Hz, 2H), 3.36 (t, *J =* 8.7 Hz, 1H), 3.09 (s, 1H). LCMS [M+H]⁺ = 379.2.

### Preparation example 66: synthesis of compound HJM-066

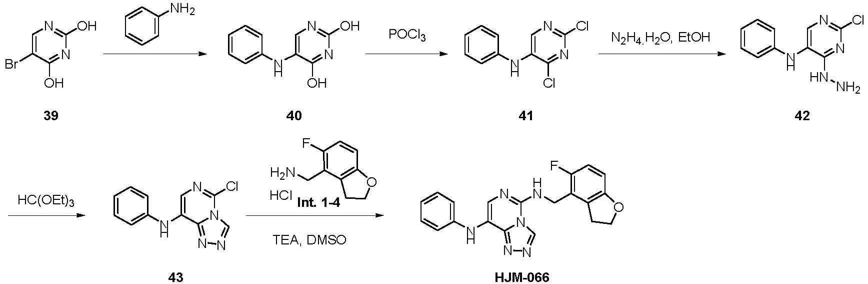

### Step 1:

Compound 2,4-dihydroxy-5-bromopyrimidine **39** (5 g, 26.2 mmol) was added to aniline (50 mL, 547.6 mmol), and the mixture was heated at 135 °C and stirred overnight. The mixture was cooled, and filtered to afford a solid. The solid was washed with 1 N HCl, and dried in vacuum to afford compound **40** (2 g, yield: 37.6%) as a white solid. LCMS [M+H]⁺ = 204.2.

### Step 2:

Compound **40** (1.0 g, 4.92 mmol) was added to POCl₃ (10 mL), and the mixture was heated at 100 °C and stirred overnight. The mixture was cooled, and concentrated to afford a crude product. The crude product was purified by silica gel column chromatography (petroleum ether/ethyl acetate, ethyl acetate: 0-50%) to afford compound **41** (500 mg, yield: 42.3%) as a yellow solid. LCMS [M+H]⁺ = 240.1.

### Step 3:

Compound **41** (200 mg, 0.83 mmol) was dissolved in ethanol (5 mL), 85% hydrazine hydrate (51.8 mg, 0.88 mmol) was added, and the mixture was stirred at room temperature for 6 h. The mixture was concentrated to afford a crude product. The crude product was purified by reversed-phase column chromatography (acetonitrile:water = 20:80) to afford compound **42** (150 mg, yield: 76.4%) as a white solid. LCMS [M+H]⁺ = 236.2.

### Step 4:

Compound **42** (100 mg, 0.42 mmol) was added to triethyl orthoformate (2 mL), and the mixture was heated at 120 °C and stirred for 3 h. The mixture was cooled, and concentrated to afford a crude product. The crude product was purified by preparative TLC to afford compound **43** (70 mg, yield: 67.1%) as a white solid. LCMS [M+H]⁺ = 246.3.

### Step 5:

Compound **43** (70 mg, 0.28 mmol), **Int. 1-4** hydrochloride (87 mg, 0.43 mmol) and triethylamine (62 mg, 0.6 mmol) were dissolved in dimethyl sulfoxide (2 mL), and the mixture was stirred at room temperature for 4 h. The mixture was purified by preparative HPLC to afford compound HJM-066 (9 mg, yield: 8.4%) as a white solid.

¹H NMR (400 MHz, CD₃OD) δ 9.27 (s, 1H), 7.65 (s, 1H), 7.23 - 7.14 (m, 2H), 6.96 - 6.88 (m, 2H), 6.90 - 6.76 (m, 2H), 6.64 (dd, *J =* 8.7, 3.9 Hz, 1H), 4.74 (s, 2H), 4.57 (t, *J* = 8.7 Hz, 2H), 3.35 (t, *J =* 8.7 Hz, 2H). LCMS [M+H]⁺ = 377.1.

### Preparation example 67: synthesis of compound HJM-067 (for reference)

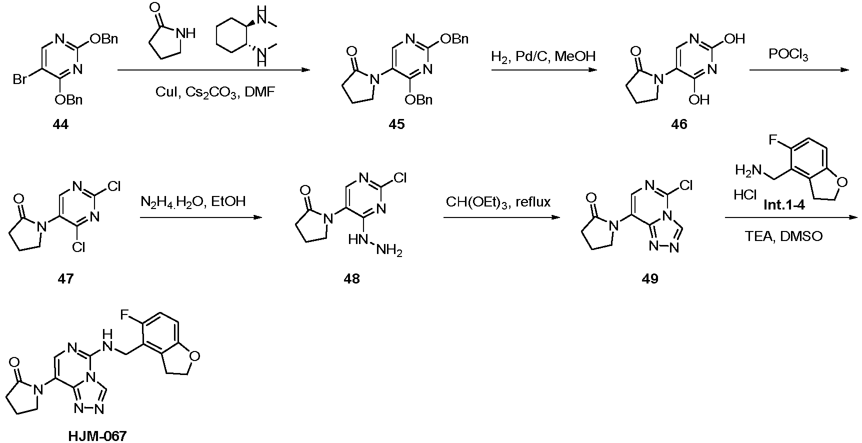

### Step 1:

Compound 2,4-dibenzyloxy-5-bromopyrimidine **44** (6.0 g, 16.16 mmol) and pyrrolidone (1.5 g, 17.78 mmol) were dissolved in *N*,*N*-dimethylformamide (100 mL), cesium carbonate (10.5 g, 32.32 mmol), (1R,2R)-*N*1,*N*2-dimethylcyclohexyl-1,2-diamine (690 mg, 4.84 mmol) and cuprous iodide (4.0 g, 21.01 mmol) were added successively, and the mixture was heated under nitrogen atmosphere at 85 °C for 2 h. The mixture was cooled, and concentrated to afford a residue. The residue was purified by silica gel column chromatography (petroleum ether/ethyl acetate, ethyl acetate: 0-30%) to afford compound **45** (3.0 g, yield: 49.4 %) as a white solid. LCMS [M+H]⁺ = 376.3.

### Step 2:

Compound **45** (3.0 g, 8.0 mmol) was dissolved in methanol (50 mL), and 5% Pd/C (500 mg) was added under nitrogen protection. The atmosphere was replaced three times with hydrogen gas, and the mixture was stirred at room temperature under a hydrogen balloon for 16 h. The mixture was filtered, and the filter cake was washed with methanol for three times. The filtrate was concentrated to afford compound **46** (1.2 g, yield: 76.9 %) as a yellow solid. LCMS [M+H]⁺ = 196.2.

### Step 3:

Compound **46** (1.2 g, 6.24 mmol) was added to POCl₃ (10 mL), and the mixture was heated at 100 °C overnight. The mixture was cooled, and concentrated to afford a crude product. The crude product was purified by silica gel column chromatography (petroleum ether/ethyl acetate, ethyl acetate: 0-30%) to afford compound **47** (700 mg, yield: 48.3%) as a yellow solid. LCMS [M+H]⁺ = 232.1.

### Step 4:

Compound **47** (700 mg, 3.02 mmol) and 85% hydrazine hydrate (213 mg, 3.62 mmol) were added to ethanol (5 mL), and the mixture was stirred at room temperature for 2 h. The reaction mixture was concentrated to afford crude compound **48** (400 mg, yield: 58.2%) as a yellow solid. LCMS [M+H]⁺ = 228.1.

### Step 5:

Compound **48** (400 mg, 1.76 mmol) was added to triethyl orthoformate (10 mL), and the mixture was heated at 120 °C and refluxed with stirring for 2 h. The mixture was cooled, and concentrated to afford a crude product. The crude product was purified by preparative HPLC to afford compound **49** (40 mg, yield: 9.6%) as a yellow solid. LCMS [M+H]⁺ = 238.1.

### Step 6:

Compound **49** (40 mg, 0.17 mmol), **Int. 1-4** hydrochloride (34 mg, 0.17 mmol) and triethylamine (51 mg, 0.51 mmol) were dissolved in dimethyl sulfoxide (3 mL), and the mixture was stirred at room temperature for 2 h. The mixture was purified by preparative HPLC to afford compound **HJM-067** (25 mg, yield: 40.0 %) as a white solid.

¹H NMR (400 MHz, CD₃OD) δ 9.32 (s, 1H), 7.87 (s, 1H), 6.86 (t, *J =* 9.4 Hz, 1H), 6.65 (dd, *J* = 8.7, 3.9 Hz, 1H), 4.80 (s, 2H), 4.58 (t, *J* = 8.7 Hz, 2H), 3.94 (t, *J =* 7.1 Hz, 2H), 3.37 (t, *J* = 8.7 Hz, 2H), 2.62 (t, *J* = 8.1 Hz, 2H), 2.30 (p, *J* = 7.6 Hz, 2H). LCMS [M+H]⁺ = 369.4.

### Preparation example 68: synthesis of compound HJM-068 (for reference)

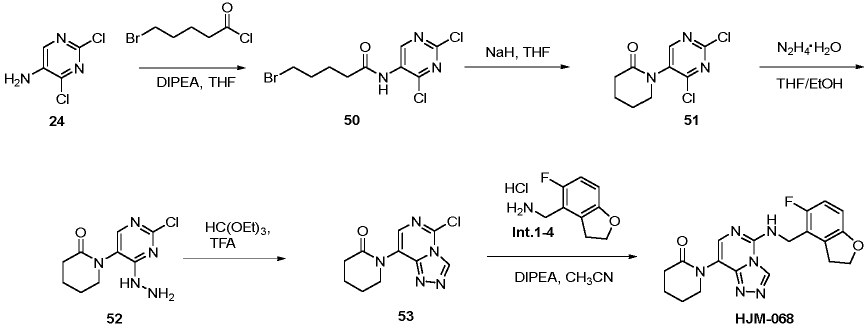

### Step 1:

5-Amino-2,4-dichloropyrimidine **24** (1.64 g, 10 mmol) and *N*,*N*-diisopropylethylamine (5 mL, 30 mmol) were dissolved in tetrahydrofuran (25 mL), 5-bromovaleryl chloride (2.4 mL, 18 mmol) was added dropwise at 0 °C, and the mixture was warmed to room temperature and stirred overnight. The mixture was concentrated to afford a crude product. The crude product was purified by silica gel column chromatography (petroleum ether/ethyl acetate, ethyl acetate: 0-50%) to afford compound **50** (290 mg, yield: 8.9%) as a white solid. LCMS [M+H]⁺ = 324.2.

### Step 2:

Compound **50** (290 mg, 0.88 mmol) was dissolved in anhydrous tetrahydrofuran (12 mL), and sodium hydride (75 mg, 1.87 mmol, purity: 60%) was added at 0 °C. After the addition, the mixture was warmed to room temperature and stirred overnight. The mixture was cooled to 0 °C, quenched with saturated aqueous ammonium chloride, and extracted with ethyl acetate (25 mL). The organic phases were combined, washed with saturated brine, dried with anhydrous sodium sulfate, and filtered. The filtrate was rotary evaporated to dryness to afford a crude product. The crude product was purified by silica gel column chromatography (petroleum ether/ethyl acetate, ethyl acetate: 30-70%) to afford compound **51** (135 mg, yield: 62%) as a white solid. LCMS [M+H]⁺ = 246.2.

### Step 3:

85% Hydrazine hydrate (47 mg, 0.82 mmol) was dissolved in a mixture of tetrahydrofuran (2.5 mL) and ethanol (0.5 mL), compound **51** (135 mg, 0.55 mmol) was added at 0 °C, and the mixture was stirred at 0 °C for 1 h. The reaction was stopped, and ethyl acetate and water were added. The mixture was extracted, and layered. The organic layers were combined, washed with saturated brine, dried with anhydrous sodium sulfate, and filtered. The filtrate was concentrated to afford crude compound **52** (60 mg, yield: 45%) as a white solid, which was directly used in the next step.

### Step 4:

Compound **52** (60 mg, 0.25 mmol) was suspended in triethyl orthoformate (2.5 mL), a catalytic amount of trifluoroacetic acid (12 uL) was added, and the mixture was heated to 80 °C and stirred for 1.5 h. The mixture was cooled and concentrated to afford a residue. The residue was purified by silica gel column chromatography (ethyl acetate/methanol, methanol: 0-5%) to afford compound **53** (30 mg, yield: 48%) as an off-white solid. LCMS [M+H]⁺ = 252.2.

### Step 5:

Compound **53** (30 mg, 0.12 mmol), **Int. 1-4** hydrochloride (30 mg, 0.15 mmol) and *N*,*N-*diisopropylethylamine (0.064 mL, 0.38 mmol) were dissolved in acetonitrile (4 mL), and the mixture was stirred at room temperature for 16 h. The mixture was purified by preparative HPLC to afford compound **HJM-068** (17 mg, yield: 37%) as a white solid.

¹H NMR (500 MHz, DMSO-*d*₆) δ 9.91 (s, 1H), 9.61 (s, 1H), 7.97 (s, 1H), 6.93 (t, *J =* 9.4 Hz, 1H), 6.69 (dd, *J =* 9.4, 3.7 Hz, 1H), 4.69 (s, 2H), 4.54 (t, *J* = 8.6 Hz, 2H), 3.62 (t, *J* = 5.5 Hz, 2H), 3.35 (t, *J =* 8.7 Hz, 2H), 2.44 (t, *J =* 6.2 Hz, 2H), 1.96 - 1.83 (m, 4H). LCMS [M+H]⁺ = 383.3.

### Preparation example 69: synthesis of compound HJM-069

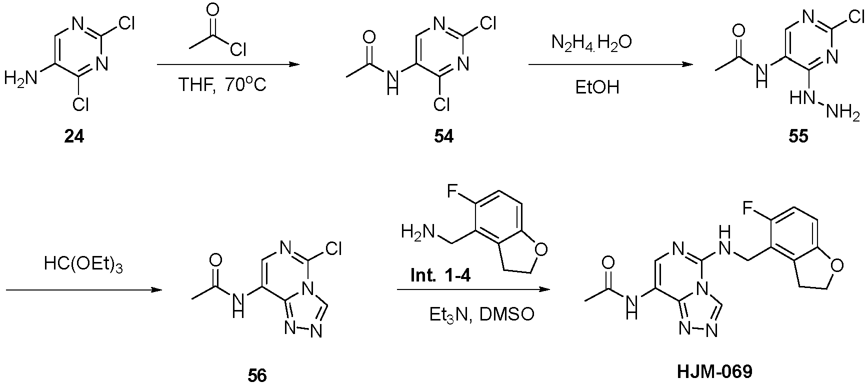

### Step 1:

5-Amino-2,4-dichloropyrimidine **24** (300 mg, 1.83 mmol) and acetyl chloride (287 mg, 3.66 mmol) were dissolved in tetrahydrofuran (10 mL), and the mixture was heated at 70 °C for 4 h. The mixture was cooled, diluted with water, and extracted twice with ethyl acetate (2×30 mL). The organic phases were combined, washed once with saturated brine, dried with anhydrous sodium sulfate, and filtered. The filtrate was concentrated to afford crude compound **54** (220 mg, yield: 58.4 %) as a yellow oil. LCMS [M+H]⁺ = 206.0.

### Step 2:

Compound **54** (220 mg, 1.07 mmol) was dissolved in ethanol (2 mL), a solution of 85% hydrazine hydrate (188.2 mg, 3.20 mmol) in ethanol (2 mL) was added at room temperature, and the mixture was reacted at room temperature for 1 h. The reaction mixture was diluted with water, and extracted twice with ethyl acetate. The organic phases were combined, washed once with saturated brine, dried with anhydrous sodium sulfate, and filtered. The filtrate was concentrated to afford crude compound **55** (160 mg, yield: 74.2%) as a yellow oil. LCMS [M+H]⁺ = 202.1.

### Step 3:

Compound **55** (160 mg, 0.79 mmol) was added to triethyl orthoformate (5 mL), and the mixture was heated at 120 °C for 2 h. The mixture was cooled, and concentrated to afford crude compound **56,** which was directly used in the next step.

### Step 4:

Above compound **56, Int.1-4** (132 mg, 0.79 mmol) and triethylamine (240 mg, 2.38 mmol) were dissolved in dimethyl sulfoxide (3 mL), and the mixture was stirred at room temperature for 1 h. The mixture was diluted with water, and extracted twice with ethyl acetate. The organic phases were combined, dried with anhydrous sodium sulfate, and filtered. The filtrate was concentrated to afford a crude product. The crude product was slurried with methanol. The resulting solid was filtered, and dried to afford compound **HJM-069** (40 mg, two-step yield: 14.8 %) as a white solid.

¹H NMR (400 MHz, DMSO-*d*₆) δ 9.91 (s, 1H), 9.41 (s, 1H), 8.39 (t, *J =* 4.9 Hz, 1H), 8.06 (s, 1H), 6.97 - 6.91 (m, 1H), 6.70 (dd, *J =* 8.7, 3.9 Hz, 1H), 4.65 (d, *J =* 5.0 Hz, 2H), 4.54 (t, *J =* 8.7 Hz, 2H), 3.28 (t, *J =* 8.7 Hz, 2H), 2.11 (s, 3H). LCMS [M+H]⁺ = 343.2.

### Preparation example 70: synthesis of compound HJM-070

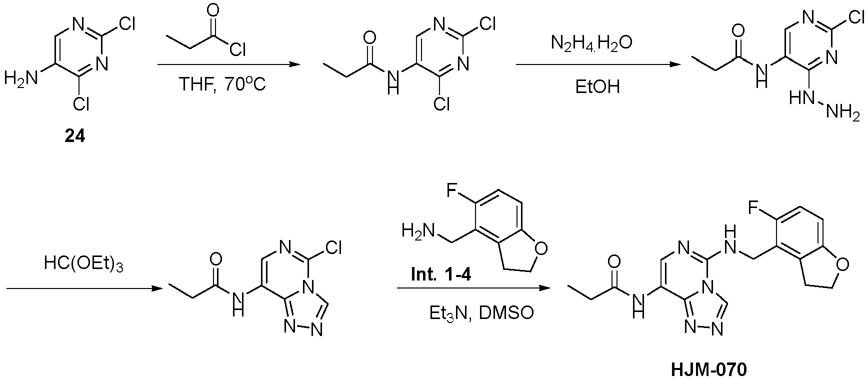

**HJM-070** (78 mg) was prepared as a white solid using propionyl chloride as starting material according to the method similar to that for preparation example 69.

¹H NMR (400 MHz, DMSO-*d*₆) δ 9.82 (s, 1H), 9.41 (s, 1H), 8.38 (t, *J =* 5.2 Hz, 1H), 8.08 (s, 1H), 6.94 (t, *J =* 9.5 Hz, 1H), 6.70 (dd, *J =* 8.6, 3.9 Hz, 1H), 4.65 (d, *J =* 4.9 Hz, 2H), 4.54 (t, *J* = 8.7 Hz, 2H), 3.28 (t, *J =* 8.7 Hz, 2H), 2.43 (q, *J =* 7.6 Hz, 2H), 1.08 (t, *J* = 7.5 Hz, 3H). LCMS [M+H]⁺ = 357.2.

### Preparation example 71: synthesis of compound HJM-071

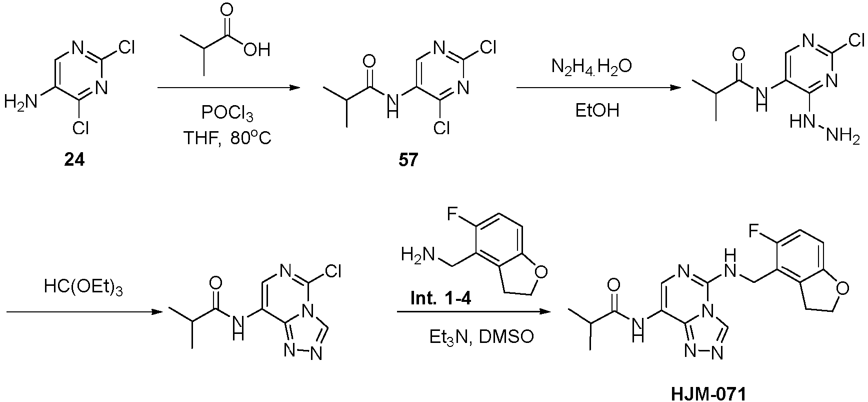

### Step 1:

5-Amino-2,4-dichloropyrimidine **24** (600 mg, 3.66 mmol) and isobutyric acid (322 mg, 3.66 mmol) were dissolved in tetrahydrofuran (10 mL), phosphorus oxychloride (0.3 mL) was added dropwise, and the mixture was heated at 80 °C for 2 h. The mixture was cooled, and concentrated to afford a crude product. The crude product was purified by silica gel column chromatography (petroleum ether/ethyl acetate, ethyl acetate: 0-20%) to afford compound **57** (500 mg, yield: 58.4%) as a yellow solid. LCMS [M+H]⁺ = 234.0.

In the following steps, **HJM-071** (60 mg) was prepared as a white solid according to the method similar to that for preparation example **69.**

¹H NMR (400 MHz, CD₃OD) δ 9.26 (s, 1H), 8.04 (s, 1H), 6.89 - 6.80 (m, 1H), 6.64 (dd, *J =* 8.6, 3.9 Hz, 1H), 4.76 (s, 2H), 4.57 (t, *J =* 8.7 Hz, 2H), 3.35 (t, *J =* 8.7 Hz, 2H), 2.76 (p, *J =* 6.9 Hz, 1H), 1.26 (d, *J* = 6.8 Hz, 6H). LCMS [M+H]⁺ = 371.2.

### Preparation example 72: synthesis of compound HJM-072

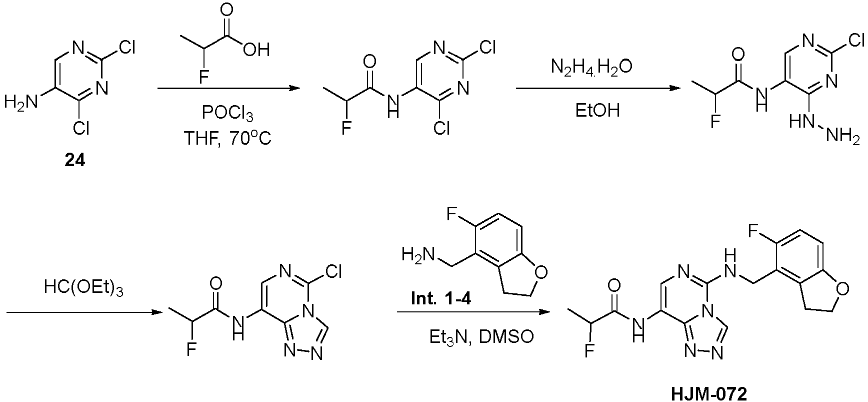

**HJM-072** (31 mg) was prepared as a white solid using 2-fluoropropionic acid as starting material according to the method similar to that for preparation example **71.**

¹H NMR (400 MHz, CD₃OD) δ 9.28 (s, 1H), 7.99 (s, 1H), 6.85 (t, *J =* 9.4 Hz, 1H), 6.64 (dd, *J* = 8.7, 3.9 Hz, 1H), 5.25 (dq, *J =* 48.8, 6.8 Hz, 1H), 4.77 (s, 2H), 4.57 (t, *J* = 8.7 Hz, 2H), 3.36 (t, *J =* 8.8 Hz, 2H), 1.67 (dd, *J* = 24.3, 6.8 Hz, 3H). LCMS [M+H]⁺ = 375.1.

### Preparation example 73: synthesis of compound HJM-073

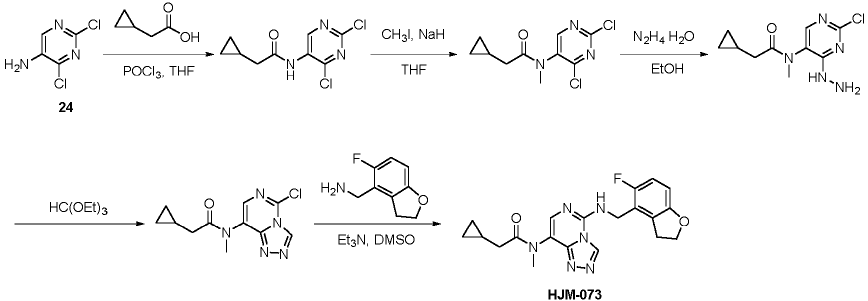

**HJM-073** (9 mg) was prepared as a white solid using 2-cyclopropylacetic acid as starting material according to the method similar to that for preparation example **45.**

¹H NMR (400 MHz, CD₃OD) δ 9.30 (s, 1H), 7.76 (s, 1H), 6.89 - 6.83 (m, 1H), 6.65 (dd, *J =* 8.7, 3.9 Hz, 1H), 4.79 (d, *J =* 6.8 Hz, 2H), 4.58 (t, *J =* 8.7 Hz, 2H), 3.38 (t, *J =* 8.6 Hz, 2H), 3.29 (s, 3H), 2.27 - 2.15 (m, 1H), 2.07 - 1.97 (m, 1H), 0.99 - 0.90 (m, 1H), 0.46 - 0.36 (m, 2H), -0.03 (d, *J =* 5.0 Hz, 2H). LCMS [M+H]⁺ = 397.5.

### Preparation example 74: synthesis of compound HJM-074

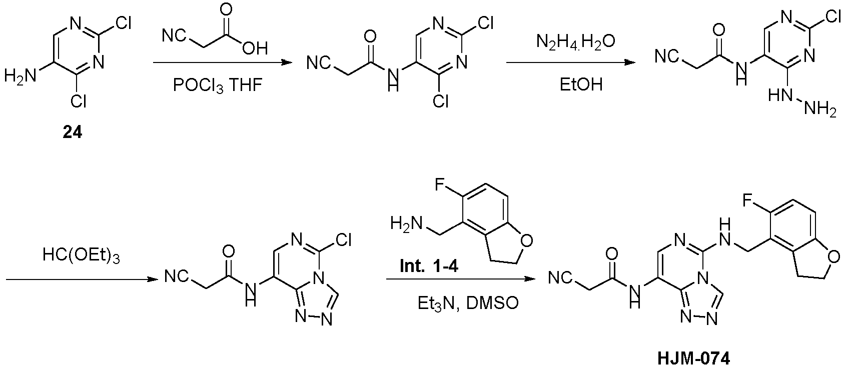

**HJM-074** (31 mg) was prepared as a white solid using cyanoacetic acid as starting material according to the method similar to that for preparation example **71.**

¹H NMR (400 MHz, DMSO-*d*₆) δ 10.43 (s, 1H), 9.44 (s, 1H), 8.52 (t, *J* = 5.1 Hz, 1H), 8.12 (s, 1H), 6.94 (dd, *J =* 10.3, 8.7 Hz, 1H), 6.70 (dd, *J =* 8.7, 3.9 Hz, 1H), 4.66 (d, *J =* 4.5 Hz, 2H), 4.54 (t, *J* = 8.7 Hz, 2H), 4.03 (s, 2H), 3.29 (t, *J =* 8.7 Hz, 2H). LCMS [M+H]⁺ = 368.0.

### Preparation example 75: synthesis of compound HJM-075

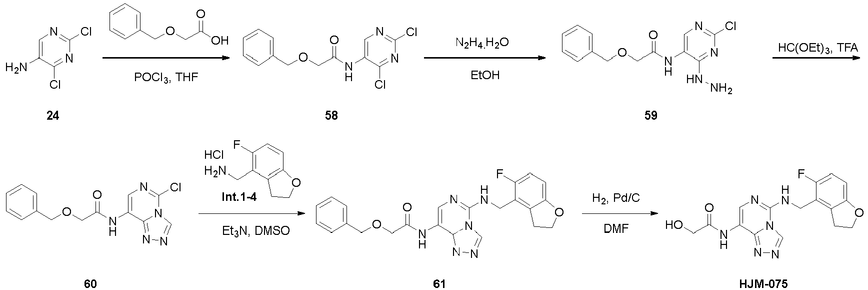

### Step 1:

5-Amino-2,4-dichloropyrimidine **24** (800 mg, 4.88 mmol) and 2-benzyloxyacetic acid (890 mg, 5.36 mmol) were dissolved in tetrahydrofuran (10 mL), phosphorus oxychloride (0.2 mL) was added dropwise, and the mixture was heated at 70 °C under nitrogen atmosphere for 16 h. The mixture was cooled, and concentrated to afford a crude product. The crude product was purified by silica gel column chromatography (petroleum ether/ethyl acetate, ethyl acetate: 0-20%) to afford compound **58** (820 mg, yield: 49%) as a white solid. LCMS [M+H]⁺ = 312.1.

### Step 2:

Compound **58** (800 mg, 2.56 mmol) was dissolved in ethanol (15 mL), 85% hydrazine hydrate (181.2 mg, 3.08 mmol) was added, and the mixture was reacted at room temperature for 1 h. The mixture was concentrated to afford crude compound **59,** which was directly used in the next step. LCMS [M+H]⁺ = 308.1.

### Step 3:

The above compound **59** was added to triethyl orthoformate (10 mL), 2 drops of trifluoroacetic acid were added dropwise, and the mixture was heated at 120 °C for 1 h. The mixture was cooled, and concentrated to afford a crude product. The crude product was purified by silica gel column chromatography (petroleum ether/ethyl acetate, ethyl acetate: 0-100%) to afford compound **60** (250 mg, two-step yield: 31%) as a yellow oil.

¹H NMR (400 MHz, DMSO-*d*₆) δ 10.25 (s, 1H), 9.59 (s, 1H), 8.61 (s, 1H), 7.45 - 7.30 (m, 5H), 4.66 (s, 2H), 4.33 (s, 2H). LCMS [M+H]⁺ = 318.1.

### Step 4:

Compound **60** (250 mg, 0.79 mmol), **Int.1-4** hydrochloride (174 mg, 0.87 mmol) and triethylamine (96 mg, 0.95 mmol) were dissolved in dimethyl sulfoxide (10 mL), and the mixture was stirred at room temperature for 2 h. The mixture was diluted with water, and extracted twice with ethyl acetate. The organic phases were combined, dried with anhydrous sodium sulfate, and filtered. The filtrate was concentrated to afford a crude product. The crude product was purified by silica gel column chromatography (dichloromethane/methanol, methanol: 0-5%) to afford compound **61** (301 mg, yield: 85%) as a white solid. LCMS [M+H]⁺ = 449.3.

### Step 5:

Compound **61** (100 mg, 0.22 mmol) was dissolved in *N*,*N*-dimethylformamide (6 mL), and 10% Pd/C (30 mg) was added under nitrogen atmosphere. The atmosphere was replaced three times with hydrogen gas, and the mixture was hydrogenated at ambient pressure at room temperature for 16 h. The mixture was filtered, and the filter cake was washed twice with methanol. The filtrates were combined, concentrated, and purified by preparative HPLC to afford compound **HJM-075** (33 mg, yield: 41%) as a white solid.

¹H NMR (400 MHz, DMSO-*d*₆) δ 9.43 (s, 1H), 9.34 (s, 1H), 8.45 (t, *J =* 5.0 Hz, 1H), 8.21 (s, 1H), 6.98 - 6.91 (m, 1H), 6.70 (dd, *J =* 8.7, 3.9 Hz, 1H), 6.03 (t, *J* = 5.7 Hz, 1H), 4.65 (d, *J =* 5.0 Hz, 2H), 4.54 (t, *J =* 8.7 Hz, 2H), 4.06 (d, *J =* 5.7 Hz, 2H), 3.28 (t, *J* = 8.8 Hz, 2H). LCMS [M+H]⁺ = 359.4.

### Preparation example 76: synthesis of compound HJM-076

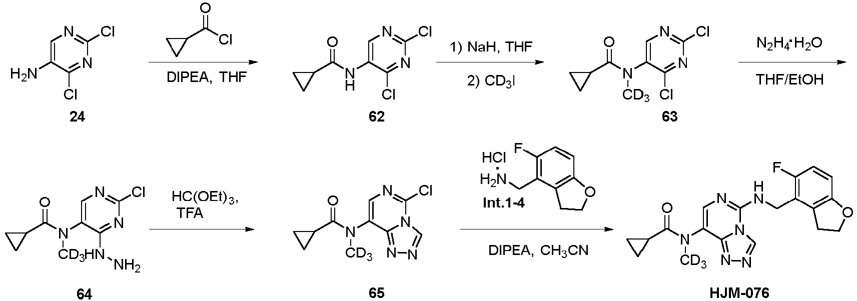

### Step 1:

5-Amino-2,4-dichloropyrimidine **24** (20.1 g, 0.12 mol) and *N*,*N-*diisopropylethylamine (28.3 g, 0.22 mol) were dissolved in tetrahydrofuran (450 mL), and cyclopropyl chloride (17.9 g, 0.17 mol) was added dropwise at 0 °C. After the addition, the mixture was warmed to room temperature and stirred for 6 h. The mixture was quenched with saturated aqueous ammonium chloride, and extracted with ethyl acetate (300 mL). The organic layer was washed with saturated brine, dried with anhydrous sodium sulfate, and filtered. The filtrate was concentrated to afford a residue. The residue was slurried with methyl tert-butyl ether, and filtered. The filter cake was dried to afford compound **62** (21 g, yield: 74%) as a white solid. LCMS [M+H]⁺ = 232.0.

### Step 2:

Compound **62** (2.55 g, 12.3 mmol) was dissolved in anhydrous tetrahydrofuran (35 mL), sodium hydride (575 mg, 1.42 mmol, purity: 60%) was added portionwise at 0 °C, and the mixture was reacted with stirring at 0-5°C for 1 h. Deuterated iodomethane (4.4 g, 30.3 mmol) was added dropwise, and the mixture was warmed to room temperature and stirred for 3 h. The mixture was cooled to 0 °C, quenched with saturated aqueous ammonium chloride, and extracted with ethyl acetate (50 mL). The organic layer was washed with saturated brine, dried with anhydrous sodium sulfate, and filtered. The filtrate was concentrated to afford a crude product. The crude product was purified by silica gel column chromatography (petroleum ether/ethyl acetate, ethyl acetate: 20%-50%) to afford compound **63** (2.6 g, yield: 85%) as a colorless oil. LCMS [M+H]⁺ = 249.0.

### Step 3:

Compound **63** (2.6 g, 10.5 mmol) was dissolved in a mixture of tetrahydrofuran (12 mL) and dioxane (3 mL), and 85% hydrazine hydrate (1.05 mL, 21.0 mmol) was added at 0 °C. After the addition, the mixture was warmed to room temperature and stirred for 1.5 h. Ethyl acetate and water were added to the reaction mixture, and the mixture was layered to afford an organic phase. The organic layer was washed with saturated brine, dried with anhydrous sodium sulfate, and filtered. The filtrate was concentrated to afford crude compound **64** (2.2 g, crude yield: 89%) as a white solid, which was directly used in the next step.

### Step 4:

Compound **64** (2.2 g, 8.98 mmol) was suspended in triethyl orthoformate (12 mL), a catalytic amount of trifluoroacetic acid (90 uL) was added, and the mixture was heated to 85 °C and stirred for 45 min. The mixture was cooled, and concentrated to afford a crude product. The crude product was purified by silica gel column chromatography (ethyl acetate/methanol, methanol: 0-5%) to afford compound 65 (750 mg, yield: 32%) as a yellow oil. LCMS [M+H]⁺ = 255.1.

### Step 5:

Compound **65** (370 mg, 1.45 mmol) and **Int.1-4** hydrochloride (320 mg, 1.57 mmol) were dissolved in acetonitrile (8 mL), *N*,*N*-diisopropylethylamine (0.58 mL, 3.5 mmol) was added, and the mixture was stirred at room temperature overnight. The mixture was concentrated to afford a crude product. The crude product was purified by preparative HPLC to afford compound **HJM-076** (64 mg, yield: 11.5%) as a white solid.

¹H NMR (500 MHz, DMSO-*d*₆) δ 9.57 (s, 1H), 8.93 (t, *J =* 5.0 Hz, 1H), 7.74 (s, 1H), 6.94 (t, *J =* 9.4 Hz, 1H), 6.70 (dd, *J* = 8.6, 3.8 Hz, 1H), 4.67 (s, 2H), 4.54 (t, *J =* 8.7 Hz, 2H), 3.33 (t, *J =* 8.8 Hz, 2H), 1.60 - 1.44 (m, 1H), 0.88 - 0.41 (m, 4H). LCMS [M+H]⁺ = 386.5.

### Preparation example 77: synthesis of compound HJM-077

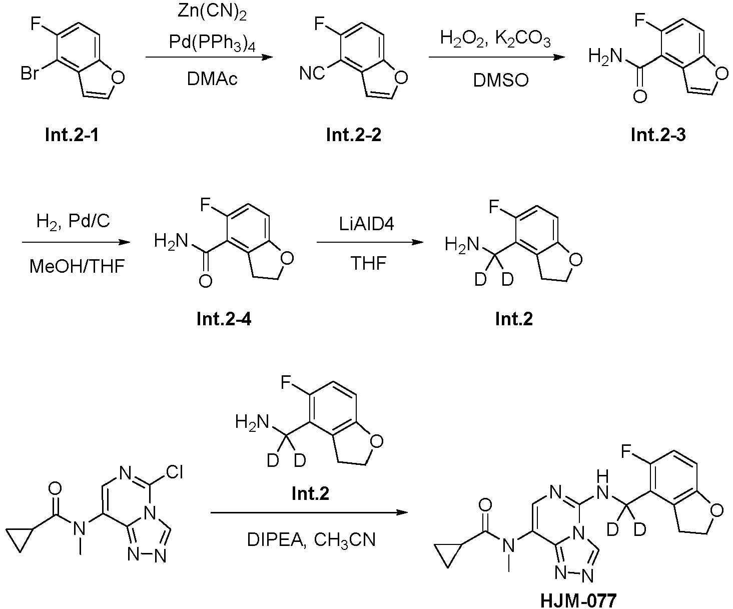

### Step 1:

Compound 4-bromo-5-fluorobenzofuran **Int.2-1** (60 g, 279 mmol) was dissolved in *N*,*N-*dimethylacetamide (600 mL), zinc cyanide (130.6 g, 1.116 mol) and Pd(PPh₃)₄ (32.23 g, 27.9 mmol) were added under nitrogen atmosphere, and the mixture was heated at 110 °C and stirred for 12 h. The mixture was cooled to room temperature, diluted with ethyl acetate (500 mL), and washed with saturated brine for 5 times (5×400 mL). The organic phases were combined, dried with anhydrous sodium sulfate, and filtered. The filtrate was concentrated to afford a crude product. The resulting crude product was purified by silica gel column chromatography (petroleum ether:ethyl acetate (V/V) =100:0-50:1) to afford compound 4-cyano-5-fluorobenzofuran **Int.2-2** (12 g, yield: 26.7%) as a white solid.

### Step 2:

Compound 4-cyano-5-fluorobenzofuran **Int.2-2** (12 g, 74.5 mmol) was dissolved in dimethyl sulfoxide (150 mL), and potassium carbonate (10.28 g, 74.5 mmol) was added. Hydrogen peroxide (30%, 42.2 g, 372.7 mmol) was added dropwise, and the mixture was stirred at room temperature under nitrogen atmosphere for 1 h. The reaction mixture was poured into ice water and stirred for 20 min. The mixture was filtered, and the solid was dried to afford compound 5-fluorobenzofuran-4-formamide **Int.2-3** (11 g, yield: 81.5%) as a white solid. LCMS [M+H]⁺ = 180.1.

### Step 3:

Under nitrogen protection, wet palladium on carbon (10% Pd/C, 6 g) was added to a mixture of 5-fluorobenzofuran-4-formamide **Int.2-3** (6.4 g, 35.7 mmol) in methanol (60 mL) and tetrahydrofuran (20 mL). The atmosphere was replaced three times with hydrogen gas, and the mixture was stirred at 40 °C under the pressure of hydrogen gas (50 psi) for 8 h. LCMS showed that the reaction was completed. The mixture was filtered through diatomite, and the filter cake was washed with methanol (10 mL) for three times. The filtrates were combined and concentrated under reduced pressure to afford compound 5-fluoro-2,3-dihydrobenzofuran-4-carboxamide **Int.2-4** (6.36 g, yield: 99.4%). LCMS [M+H]⁺ = 182.1.

### Step 4:

At 0 °C, under nitrogen protection, a solution of 5-fluoro-2,3-dihydrobenzofuran-4-carboxamide **Int.2-4** (5.0 g, 27.6 mmol) in anhydrous tetrahydrofuran (50 mL) was added dropwise to a solution of deuterated lithium aluminum hydride (2.1 g, 55.2 mmol) in anhydrous tetrahydrofuran (50 mL), and the mixture was heated to 100 °C and stirred for 1 h. LCMS showed that the reaction was completed. At 0 °C, water (2.1 mL) was added dropwise to the mixture to quench the reaction, and the mixture was stirred for 10 min. Then 15% sodium hydroxide solution (2.1 mL) was added dropwise to the mixture, and the mixture was stirred for 10 min. Finally, water (6.3 mL) was added dropwise to the mixture, and the mixture was stirred for 10 min. The mixture was filtered through diatomite, and the filter cake was washed with dichloromethane (50 mL) for three times. The filtrates were combined, and concentrated under reduced pressure to afford a crude product. The crude product was purified by reversed-phase medium pressure column to afford compound (5-fluoro-2,3-dihydrobenzofuran-4-yl)dideuteromethylamine **Int.2** (2.5 g, yield: 53.5%) as a brown oil.

¹H NMR (400 MHz, CDCl₃) δ 6.82 - 6.76 (m, 1H), 6.59 (dd, *J =* 8.6, 3.9 Hz, 1H), 4.59 (t, *J =* 8.7 Hz, 2H), 3.23 (t, *J* = 8.7 Hz, 2H). LCMS [M+H]⁺ = 170.1.

### Step 5:

Compound *N*-(5-chloro-[1,2,4]triazolo[4,3-c]pyrimidin-8-yl)-*N*-methylcyclopropanecarboxamide (see preparation example **45** for the synthesis procedure, 80 mg, 0.32 mmol) and **Int.2** (60 mg, 0.35 mmol) were dissolved in acetonitrile (4 mL), *N*,*N*-diisopropylethylamine (80 mg, 0.62 mmol) was added, and the mixture was stirred at room temperature overnight. The mixture was concentrated to afford a crude product. The crude product was purified by preparative HPLC to afford compound **HJM-077** (32 mg, yield: 26%) as a white solid.

¹H NMR (500 MHz, DMSO-*d*₆) δ 9.51 (s, 1H), 8.82 (s, 1H), 7.75 (s, 1H), 6.95 (dd, *J* = 10.2, 8.7 Hz, 1H), 6.71 (dd, *J =* 8.6, 3.8 Hz, 1H), 4.55 (t, *J =* 8.7 Hz, 2H), 3.32 (t, *J =* 8.8 Hz, 2H), 3.17 (s, 3H), 1.58 - 1.47 (m, 1H), 0.87 - 0.42 (m, 4H). LCMS [M+H]⁺ = 385.6.

### Preparation example 78: synthesis of compound HJM-078

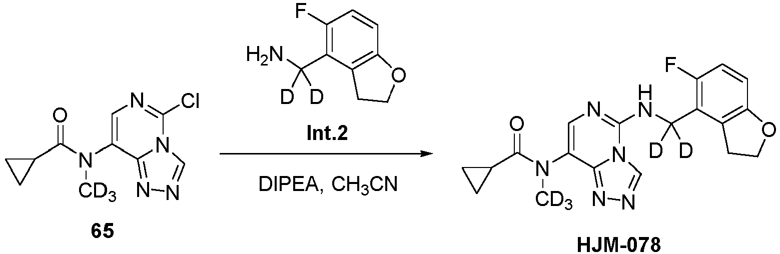

Compound **65** (80 mg, 0.32 mmol) and **Int.2** (58 mg, 0.34 mmol) were dissolved in acetonitrile (4 mL), *N*,*N*-diisopropylethylamine (80 mg, 0.62 mmol) was added, and the mixture was stirred at room temperature overnight. The mixture was concentrated to afford a crude product. The crude product was purified by preparative HPLC to afford compound **HJM-078** (18 mg, yield: 15%) as a white solid.

¹H NMR (500 MHz, DMSO-*d*₆) δ 9.75 (s, 1H), 9.27 (s, 1H), 7.85 (s, 1H), 6.96 - 6.91 (m, 1H), 6.70 (dd, *J =* 8.6, 3.9 Hz, 1H), 4.54 (t, *J =* 8.7 Hz, 2H), 3.34 (t, *J =* 8.7 Hz, 2H), 1.60 - 1.50 (m, 1H), 0.88 - 0.43 (m, 4H). LCMS [M+H]⁺ = 388.6.

### Preparation example 79: synthesis of compound HJM-079

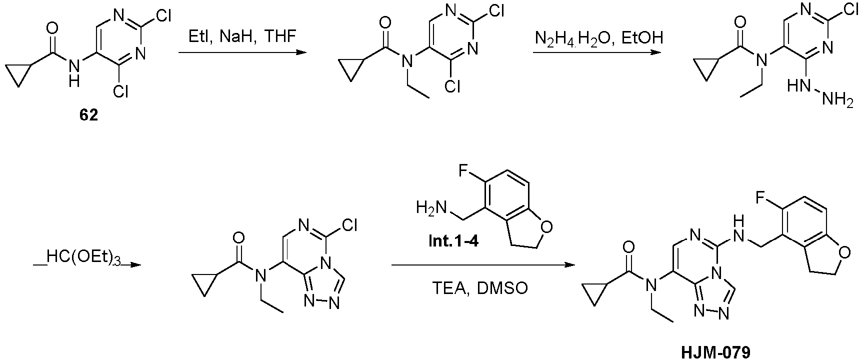

**HJM-079** (4 mg) was prepared as a white solid using iodoethane instead of iodomethane according to the method similar to that for preparation example **45.**

¹H NMR (400 MHz, CD₃OD) δ 9.32 (s, 1H), 7.75 (s, 1H), 6.86 (dd, *J* = 10.2, 8.7 Hz, 1H), 6.65 (dd, *J =* 8.7, 3.9 Hz, 1H), 4.79 (s, 2H), 4.59 (t, *J =* 8.7 Hz, 2H), 4.14 - 4.03 (m, 1H), 3.54 - 3.44 (m, 1H), 3.39 (t, *J =* 8.7 Hz, 2H), 1.56 - 1.48 (m, 1H), 1.15 (t, *J =* 7.1 Hz, 3H), 1.03 - 0.82 (m, 2H), 0.77 - 0.54 (m, 2H). LCMS [M+H]⁺ = 397.1.

### Preparation example 80: synthesis of compound HJM-080

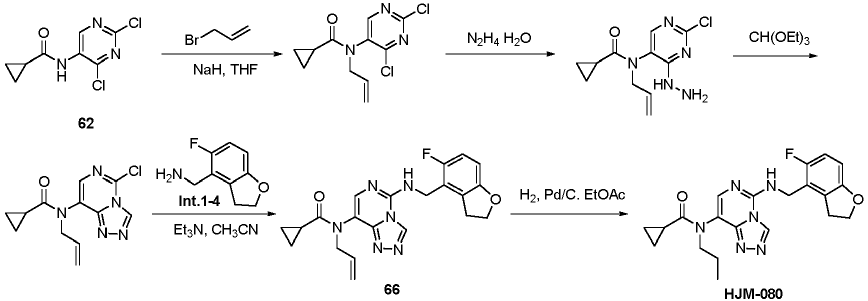

### Steps 1-4:

Compound **66** (60 mg) was prepared using allyl bromide instead of iodomethane according to the method similar to that for preparation example **45.** LCMS [M+H]⁺ = 409.1.

### Step 5:

Compound **66** (60 mg, 0.14 mmol) was dissolved in ethyl acetate (3 mL), and 10% Pd/C (10 mg) was added under nitrogen atmosphere. The atmosphere was replaced three times with hydrogen gas, and the mixture was hydrogenated at ambient pressure at room temperature for 16 h. The mixture was filtered, and the filter cake was washed twice with methanol. The filtrates were combined, concentrated, and purified by preparative HPLC to afford compound **HJM-080** (15 mg, yield: 26%) as a white solid.

¹H NMR (400 MHz, DMSO-*d*₆) δ 9.45 (s, 1H), 8.73 (s, 1H), 7.67 (s, 1H), 6.96 (dd, *J* = 10.2, 8.6 Hz, 1H), 6.72 (dd, *J* = 8.6, 3.8 Hz, 1H), 4.67 (s, 2H), 4.56 (t, *J* = 8.7 Hz, 2H), 3.81 (brs, 1H), 3.38 (brs, 1H), 3.33 (t, *J =* 8.6 Hz, 2H), 1.50 - 1.38 (m, 3H), 0.80 (t, *J =* 7.4 Hz, 3H), 0.80 - 0.68 (m, 2H), 0.66 - 0.42 (m, 2H). LCMS [M+H]⁺ = 411.2.

### Preparation example 81: synthesis of compound HJM-081

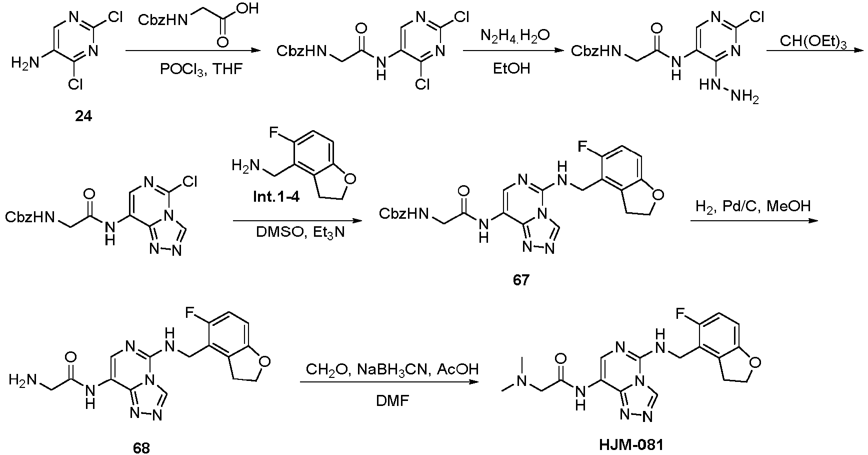

### Steps 1-4:

Compound **67** (700 mg) was prepared using benzyloxycarbonylglycine as starting material according to the method similar to that for preparation example **71.** LCMS [M+H]⁺ = 492.2.

### Step 5:

Compound **67** (700 mg, 1.425 mmol) was dissolved in methanol (20 mL), and 10% Pd/C (100 mg) was added under nitrogen atmosphere. The atmosphere was replaced three times with hydrogen gas, and the mixture was hydrogenated at ambient pressure at room temperature for 16 h. The mixture was filtered, and the filter cake was washed twice with methanol. The filtrates were combined, and concentrated to afford compound **68** (400 mg, yield: 78.6%). LCMS [M+H]⁺ = 358.1.

### Step 6:

Compound **68** (100 mg, 0.28 mmol) was dissolved in *N*,*N*-dimethylformamide (3 mL), and 37% formaldehyde aqueous solution (1 mL) was added. A drop of acetic acid was added dropwise, and the mixture was stirred at 0 °C for 10 min. Sodium cyanobohydride (53 mg, 0.84mmol) was added, and the mixture was stirred at 0 °C for 2 h. The mixture was quenched with saturated sodium bicarbonate solution, and extracted twice with ethyl acetate. The organic phases were combined, dried with anhydrous sodium sulfate, and filtered. The filtrate was concentrated to afford a crude product. The crude product was purified by preparative HPLC to afford compound **HJM-081** (22 mg, yield: 20.4 %) as a white solid.

¹H NMR (400 MHz, DMSO-*d*₆) δ 10.73 (s, 1H), 9.45 (s, 1H), 8.57 (t, *J* = 5.1 Hz, 1H), 8.11 (s, 1H), 7.01 - 6.90 (m, 1H), 6.71 (dd, *J =* 8.7, 3.9 Hz, 1H), 4.66 (d, *J =* 4.9 Hz, 2H), 4.54 (t, *J* = 8.7 Hz, 2H), 4.23 (s, 2H), 3.29 (t, *J* = 8.7 Hz, 2H), 2.88 (s, 6H). LCMS [M+H]⁺ = 386.2.

### Preparation example 82: synthesis of compound HJM-082

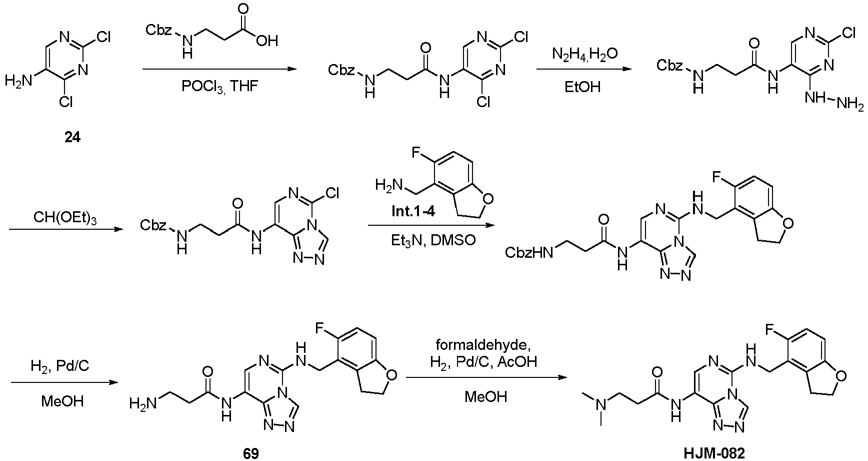

### Steps 1-5:

Compound **69** (80 mg) was prepared as a yellow solid using benzyloxycarbonylglycine as starting material according to the method similar to that for compound **68** in preparation example **81.** LCMS [M+H]⁺ = 372.1.

### Step 6:

Compound **69** (40 mg, 0.11 mmol) was dissolved in methanol (2 mL), and 37% formaldehyde aqueous solution (0.5 mL) was added. A drop of acetic acid was added dropwise, and 10% Pd/C (10 mg) was added under nitrogen atmosphere. The atmosphere was replaced three times with hydrogen gas, and the mixture was hydrogenated at ambient pressure at room temperature for 2 h. The mixture was filtered, and the filter cake was washed twice with methanol. The filtrates were combined, and concentrated to afford a crude product. The crude product was purified by preparative HPLC to afford compound **HJM-082** (2 mg, yield: 4.6%) as a white solid.

¹H NMR (400 MHz, CD₃OD) δ 9.28 (s, 1H), 7.96 (s, 1H), 6.84 (t, *J=* 9.4 Hz, 1H), 6.64 (dd, *J* = 8.6, 3.9 Hz, 1H), 4.76 (s, 2H), 4.57 (t, *J* = 8.7 Hz, 2H), 3.39 - 3.32 (m, 4H), 2.95 (t, *J* = 6.5 Hz, 2H), 2.83 (s, 6H). LCMS [M+H]⁺ = 400.1.

### Preparation example 83: synthesis of compound HJM-083

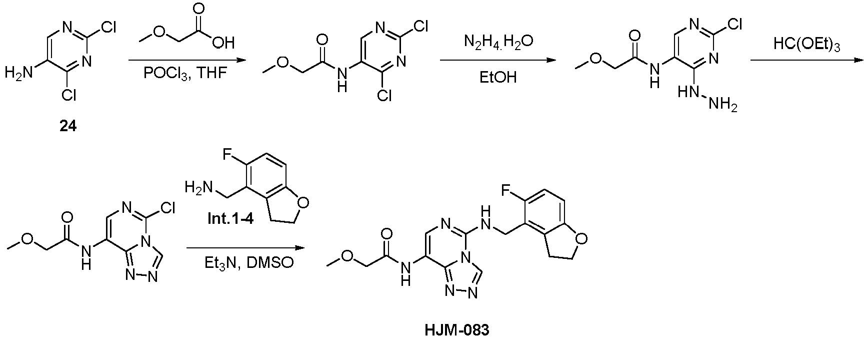

**HJM-083** (13 mg) was prepared as a white solid using 2-methoxyacetic acid as starting material according to the method similar to that for preparation example **71.**

¹H NMR (400 MHz, DMSO-*d*₆) δ 9.43 (s, 1H), 8.49 (t, *J =* 4.9 Hz, 1H), 8.07 (s, 1H), 6.94 (t, *J =* 9.5 Hz, 1H), 6.70 (dd, *J =* 8.7, 3.8 Hz, 1H), 4.65 (d, *J* = 4.9 Hz, 2H), 4.54 (t, *J =* 8.7 Hz, 2H), 4.08 (s, 2H), 3.42 (s, 3H), 3.29 (t, *J* = 8.7 Hz, 3H). LCMS [M+H]⁺ = 373.3.

### Preparation example 84: synthesis of compound HJM-084

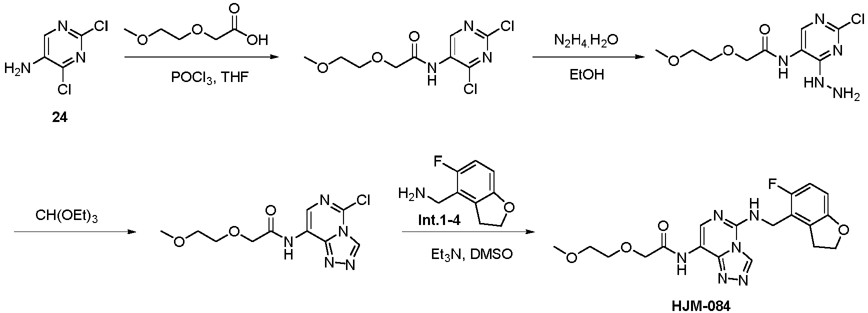

**HJM-084** (75 mg) was prepared as a white solid using 2-(2-methoxyethoxy)acetic acid as starting material according to the method similar to that for preparation example **71.**

¹H NMR (400 MHz, DMSO-*d*₆) δ 9.44 (s, 1H), 8.52 (t, *J =* 5.2 Hz, 1H), 8.13 (s, 1H), 6.94 (dd, *J =* 10.3, 8.7 Hz, 1H), 6.70 (dd, *J =* 8.7, 3.8 Hz, 1H), 4.65 (d, *J* = 4.6 Hz, 2H), 4.54 (t, *J =* 8.7 Hz, 2H), 4.16 (s, 2H), 3.75 - 3.67 (m, 2H), 3.57 - 3.50 (m, 2H), 3.29 (t, *J* = 8.8 Hz, 2H), 3.28 (s, 3H). LCMS [M+H]⁺ = 417.3.

### Preparation example 85: synthesis of compound HJM-085

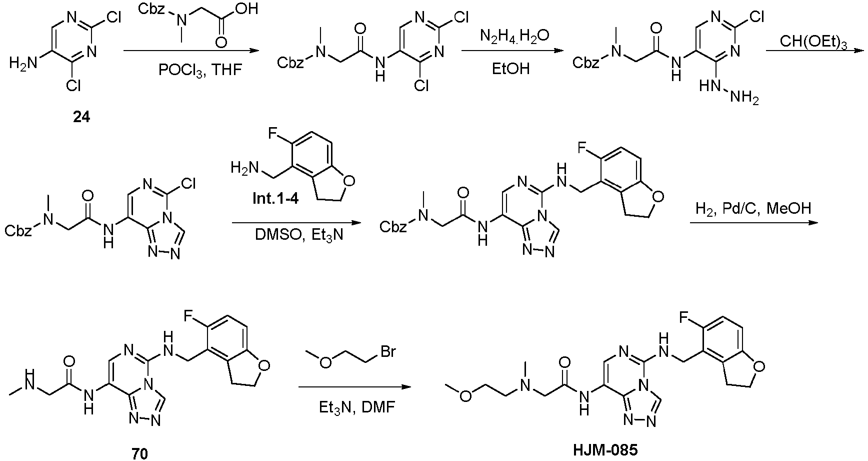

### Steps 1-5:

Compound **70** (240 mg) was prepared as a white solid using *N*-benzyloxycarbonyl-*N*-methylglycine as starting material according to the method similar to that for compound **68** in preparation example **81.** LCMS [M+H]⁺ = 372.0.

### Step 6:

Compound **70** (120 mg, 0.323 mmol), 1-bromo-2-methoxyethane (67 mg, 0.484 mmol) and triethylamine (65 mg, 0.646 mmol) were dissolved in *N*,*N*-dimethylformamide (3 mL), and the mixture was heated at 80 °C for 4 h. The mixture was cooled, and filtered. The filtrate was purified by preparative HPLC to afford compound **HJM-085** (85 mg, yield: 61.5%) as a white solid.

¹H NMR (400 MHz, CD₃OD) δ 9.30 (s, 1H), 8.04 (s, 1H), 6.85 (dd, *J* = 10.2, 8.7 Hz, 1H), 6.64 (dd, *J =* 8.7, 3.9 Hz, 1H), 4.77 (s, 2H), 4.57 (t, *J* = 8.7 Hz, 2H), 4.31 (brs, 2H), 3.78 (t, *J =* 4.9 Hz, 2H), 3.53 (brs, 2H), 3.42 (s, 3H), 3.35 (t, *J =* 8.7 Hz, 2H), 3.07 (s, 3H). LCMS [M+H]⁺ = 430.5.

### Preparation example 86: synthesis of compound HJM-086

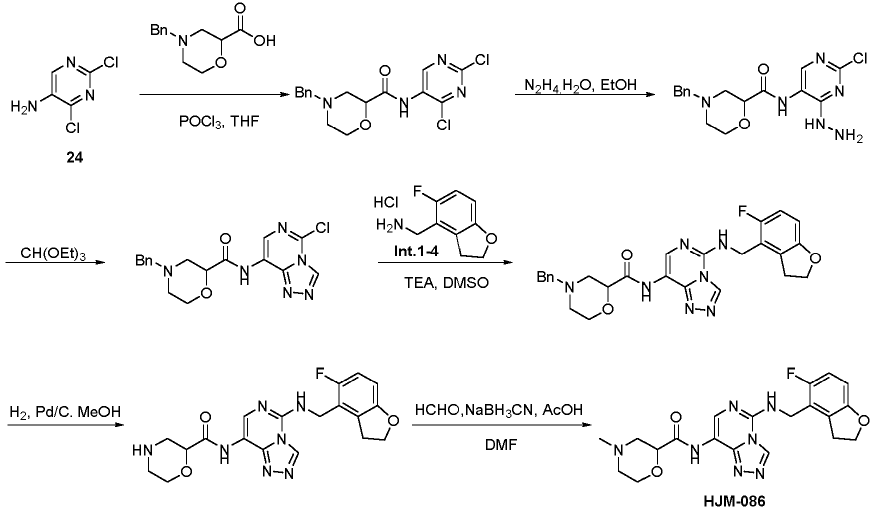

**HJM-086** (9 mg) was prepared as a white solid using 4-benzylmorpholine-2-carboxylic acid as starting material according to the method similar to that for preparation example **81.**

¹H NMR (400 MHz, CD₃OD) δ 9.27 (s, 1H), 8.13 (s, 1H), 6.85 (t, *J =* 9.9, 8.7 Hz, 1H), 6.64 (dd, *J* = 8.6, 3.8 Hz, 1H), 4.76 (s, 2H), 4.57 (t, *J =* 8.7 Hz, 2H), 4.26 (dd, *J =* 10.5, 2.8 Hz, 1H), 4.13 - 4.08 (m, 1H), 3.79 (td, *J =* 11.5, 2.6 Hz, 1H), 3.36 (t, *J* = 8.8 Hz, 2H), 3.20 - 3.14 (m, 1H), 2.78 - 2.72 (m, 1H), 2.36 (s, 3H), 2.26 (td, *J =* 11.6, 3.5 Hz, 1H), 2.23 - 2.15 (m, 1H). LCMS [M+H]⁺ = 428.1.

### Preparation example 87: synthesis of compound HJM-087

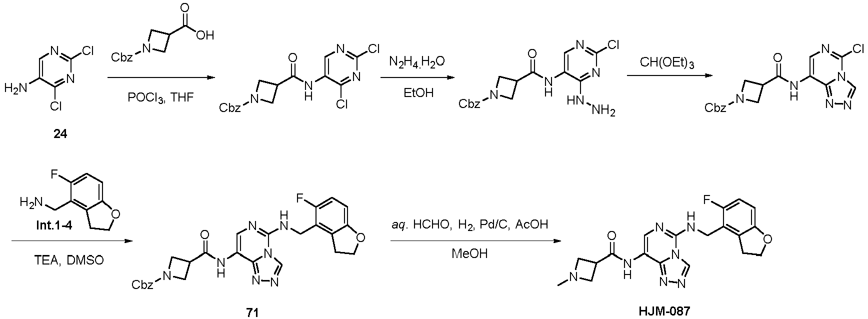

**HJM-087** (9.9 mg) was prepared as a white solid using 1-benzyloxycarbonylazetidine-3-carboxylic acid as starting material according to the method similar to that for preparation example **82.**

¹H NMR (400 MHz, CD₃OD) δ 9.29 (s, 1H), 8.00 (s, 1H), 6.85 (t, *J =* 9.5 Hz, 1H), 6.64 (dd, *J* = 8.8, 3.8 Hz, 1H), 4.77 (s, 2H), 4.57 (t, *J* = 8.7 Hz, 2H), 4.35 - 4.24 (m, 4H), 3.82 (p, *J =* 7.8 Hz, 1H), 3.35 (t, *J* = 9.0 Hz, 2H), 2.89 (s, 3H). LCMS [M+H]⁺ = 398.2..

### Preparation example 88: synthesis of compound HJM-088

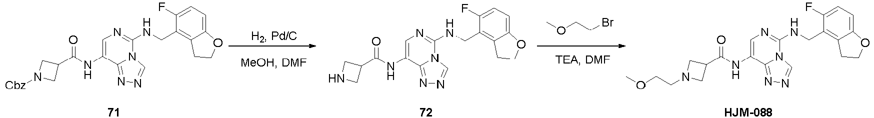

### Step 1:

Compound **71** (an intermediate in preparation example **87,** 100 mg, 0.19 mmol) was dissolved in a mixture of methanol (7 mL)/*N*,*N-*dimethylformamide (2 mL), and 10% Pd/C (20 mg) was added under nitrogen atmosphere. The atmosphere was replaced three times with hydrogen gas, and the mixture was hydrogenated at ambient pressure at room temperature for 16 h. The mixture was filtered, and the filter cake was washed twice with methanol. The filtrates were combined, and concentrated to afford crude compound **72** (50 mg, yield: 69%), which was directly used in the next step. LCMS [M+H]⁺ = 384.1.

### Step 2:

Compound **72** (45 mg, 0.11 mmol), 1-bromo-2-methoxyethane (17 mg, 0.11 mmol) and triethylamine (22 mg, 0.22 mmol) were dissolved in N,N-dimethylformamide (3 mL), and the mixture was heated at 70 °C and stirred for 3 h. The mixture was cooled and purified by preparative HPLC to afford compound **HJM-088** (6 mg, yield: 12%) as a white solid.

¹H NMR (400 MHz, CD₃OD) δ 9.30 (s, 1H), 7.99 (s, 1H), 6.85 (t, *J =* 9.5 Hz, 1H), 6.65 (dd, *J* = 8.7, 3.9 Hz, 1H), 4.77 (s, 2H), 4.57 (t, *J =* 8.7 Hz, 2H), 4.58 - 4.43 (m, 2H), 4.43 - 4.33 (m, 2H), 3.99 - 3.87 (m, 1H), 3.66 - 3.60 (m, 2H), 3.51 - 3.47 (m, 2H), 3.40 (s, 3H), 3.36 (t, *J =* 8.8 Hz, 2H). LCMS [M+H]⁺ = 442.2.

### Preparation example 89: synthesis of compound HJM-089

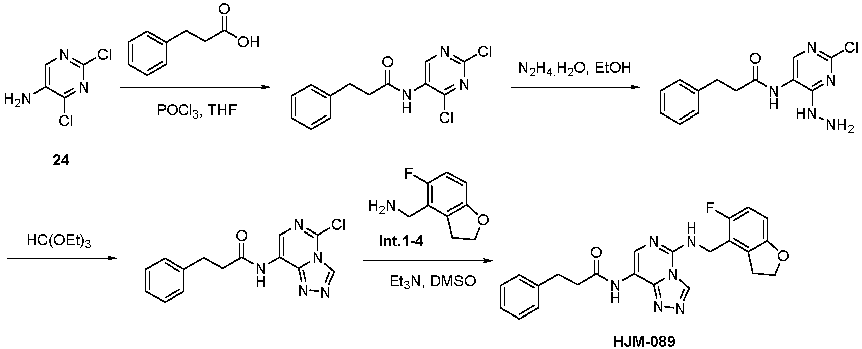

**HJM-089** (28 mg) was prepared as a white solid using phenylpropionic acid as starting material according to the method similar to that for preparation example **71.**

¹H NMR (400 MHz, DMSO-*d*₆) δ 9.95 (s, 1H), 9.41 (s, 1H), 8.40 (t, *J =* 5.0 Hz, 2H), 8.09 (s, 1H), 7.33 - 7.15 (m, 5H), 7.00 - 6.89 (m, 1H), 6.70 (dd, *J =* 8.7, 3.9 Hz, 1H), 4.65 (d, *J =* 4.9 Hz, 2H), 4.54 (t, *J =* 8.7 Hz, 2H), 3.28 (t, *J =* 8.7 Hz, 2H), 2.91 (t, *J =* 7.7 Hz, 2H), 2.74 (t, *J =* 7.8 Hz, 2H). LCMS [M+H]⁺ = 433.5.

### Preparation example 90: synthesis of compound HJM-090

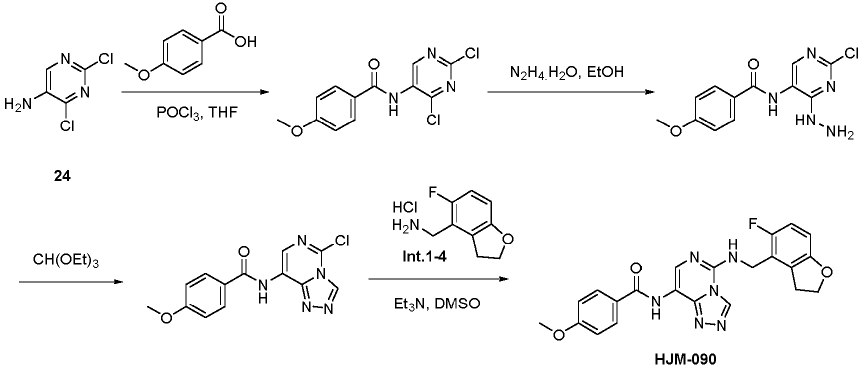

**HJM-090** (9.5 mg) was prepared as a white solid using 4-methoxybenzoic acid as starting material according to the method similar to that for preparation example **71.**

¹H NMR (400 MHz, DMSO-*d*₆) δ 9.98 (s, 1H), 9.45 (s, 1H), 8.56 (t, *J* = 5.2 Hz, 1H), 8.00 (d, *J =* 8.7 Hz, 2H), 7.82 (s, 1H), 7.07 (d, *J =* 8.8 Hz, 2H), 7.00 - 6.91 (m, 1H), 6.71 (dd, *J =* 8.5, 3.9 Hz, 1H), 4.69 (d, *J =* 4.9 Hz, 2H), 4.55 (t, *J* = 8.7 Hz, 2H), 3.85 (s, 3H), 2.54 (t, *J* = 8.7 Hz, 1H). LCMS [M+H]⁺ = 435.3.

### Preparation example 91: synthesis of compound HJM-091

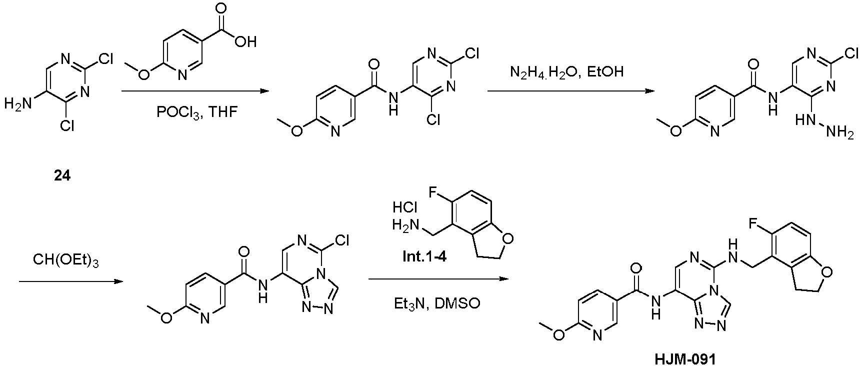

**HJM-091** (45 mg) was prepared as a white solid using 6-methoxynicotinic acid as starting material according to the method similar to that for preparation example **71.**

¹H NMR (400 MHz, DMSO-*d*₆) δ 10.24 (s, 1H), 9.47 (s, 1H), 8.83 (d, *J =* 2.6 Hz, 1H), 8.61 (t, *J =* 5.0 Hz, 1H), 8.27 (dd, *J* = 8.7, 2.5 Hz, 1H), 7.85 (s, 1H), 7.01 - 6.90 (m, 2H), 6.71 (dd, *J* = 8.6, 3.9 Hz, 1H), 4.69 (d, *J =* 5.1 Hz, 2H), 4.55 (t, *J =* 8.7 Hz, 2H), 3.95 (s, 3H), 3.30 (t, *J =* 8.7 Hz, 2H). LCMS [M+H]⁺ = 436.4.

### Preparation example 92: synthesis of compound HJM-092

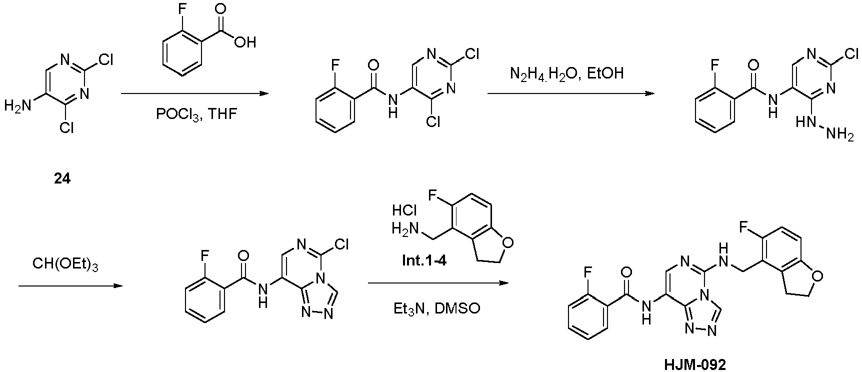

**HJM-092** (35 mg) was prepared as a white solid using 2-fluorobenzoic acid as starting material according to the method similar to that for preparation example **71.**

¹H NMR (400 MHz, DMSO-*d*₆) δ 10.10 (s, 1H), 9.45 (s, 1H), 8.56 (s, 1H), 8.10 (s, 1H), 7.82 - 7.75 (m, 1H), 7.69 - 7.57 (m, 1H), 7.46 - 7.29 (m, 2H), 6.95 (t, *J* = 9.4 Hz, 1H), 6.78 - 6.65 (m, 1H), 4.68 (s, 2H), 4.55 (t, *J* = 8.7 Hz, 2H), 3.30 (t, *J =* 8.7 Hz, 2H). LCMS [M+H]⁺ = 423.2.

### Preparation example 93: synthesis of compound HJM-093

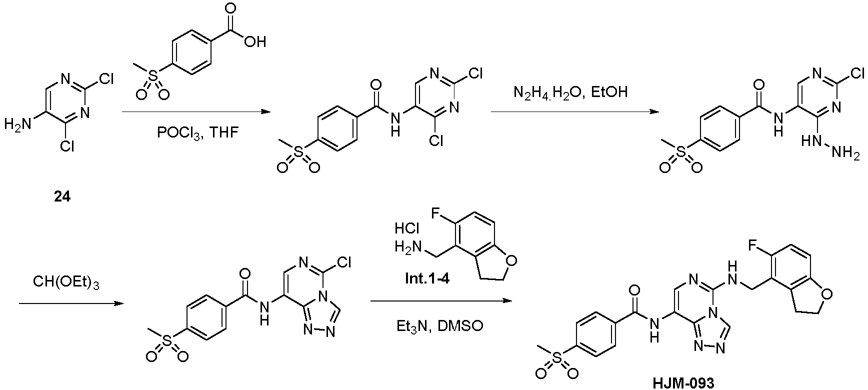

**HJM-093** (40 mg) was prepared as a white solid using 4-methanesulfonylbenzoic acid as starting material according to the method similar to that for preparation example **71.**

¹H NMR (400 MHz, DMSO-*d*₆) δ 10.51 (s, 1H), 9.47 (s, 1H), 8.65 (t, *J =* 5.0 Hz, 1H), 8.23 (d, *J =* 8.1 Hz, 2H), 8.11 (d, *J =* 8.2 Hz, 2H), 7.91 (s, 1H), 6.96 (t, *J =* 9.5 Hz, 1H), 6.72 (dd, *J* = 8.6, 3.9 Hz, 1H), 4.70 (d, *J =* 4.8 Hz, 2H), 4.56 (t, *J =* 8.7 Hz, 2H), 3.32 (t, *J =* 8.9 Hz, 2H), 3.31 (s, 3H). LCMS [M+H]⁺ = 483.4.

### Preparation example 94: synthesis of compound HJM-094

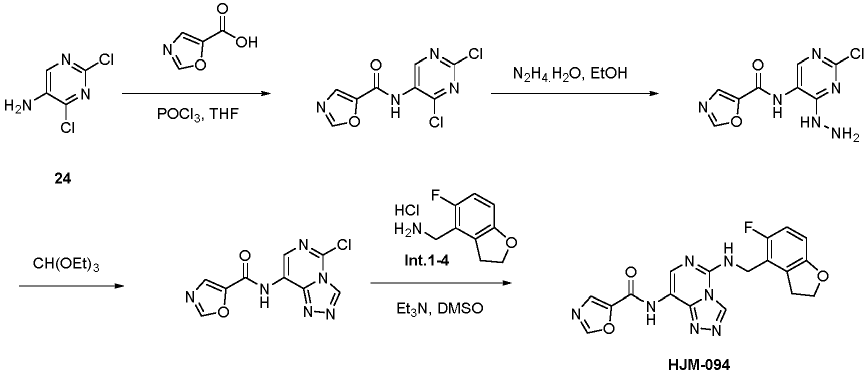

**HJM-094** (10 mg) was prepared as a white solid using oxazole-5-carboxylic acid as starting material according to the method similar to that for preparation example **71.**

¹H NMR (400 MHz, DMSO-*d*₆) δ 10.37 (s, 1H), 9.45 (s, 1H), 8.67 (s, 1H), 8.66 - 8.62 (m, 1H), 8.04 (s, 1H), 7.85 (s, 1H), 6.95 (t, *J* = 9.5 Hz, 1H), 6.71 (dd, *J =* 8.7, 3.9 Hz, 1H), 4.69 (d, *J =* 4.1 Hz, 2H), 4.55 (t, *J =* 8.7 Hz, 2H), 3.31 (t, *J =* 8.7 Hz, 2H). LCMS [M+H]⁺ = 396.3.

### Preparation example 95: synthesis of compound HJM-095

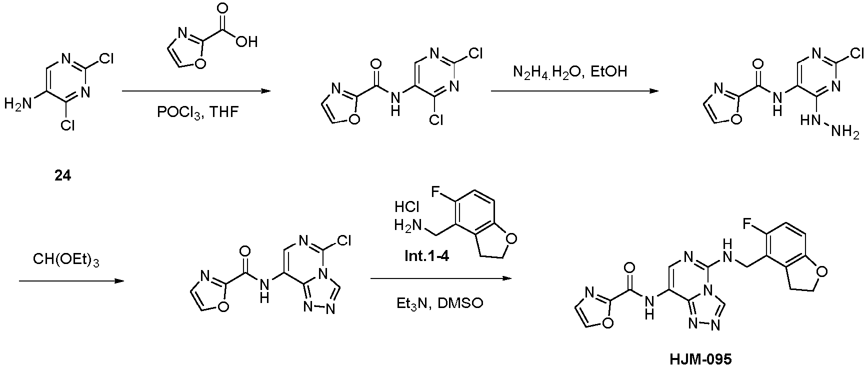

**HJM-095** (2.5 mg) was prepared as a white solid using oxazole-2-carboxylic acid as starting material according to the method similar to that for preparation example **71.**

¹H NMR (400 MHz, DMSO-*d*₆) δ 10.36 (s, 1H), 9.45 (s, 1H), 8.67 (t, *J* = 5.1 Hz, 1H), 8.44 (s, 1H), 7.91 (s, 1H), 7.58 (s, 1H), 6.95 (dd, *J =* 10.2, 8.7 Hz, 1H), 6.71 (dd, *J =* 8.6, 3.9 Hz, 1H), 4.69 (d, *J =* 4.9 Hz, 2H), 4.55 (t, *J =* 8.7 Hz, 2H), 3.31 (t, *J =* 8.8 Hz, 2H). LCMS [M+H]⁺ = 396.0.

### Preparation example 96: synthesis of compound HJM-096

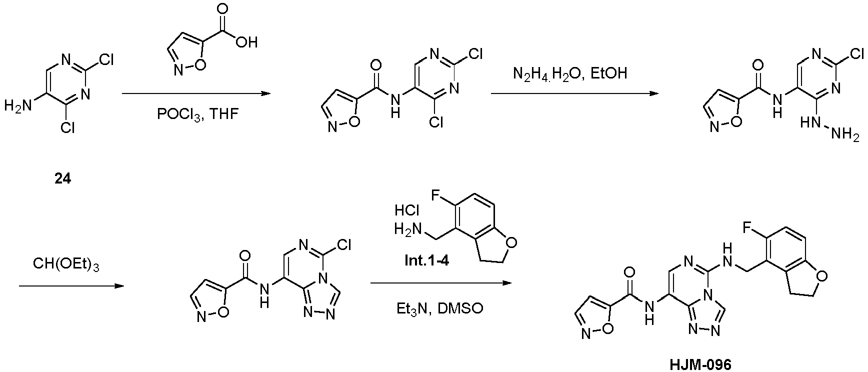

**HJM-096** (4 mg) was prepared as a white solid using isoxazole-5-carboxylic acid as starting material according to the method similar to that for preparation example **71.**

¹H NMR (400 MHz, DMSO-*d*₆) δ 10.61 (br s, 1H), 9.46 (s, 1H), 8.84 (s, 1H), 8.68 (br s, 1H), 7.85 (s, 1H), 7.32 (s, 1H), 6.95 (t, *J =* 9.4 Hz, 1H), 6.71 (dd, *J =* 8.7, 3.8 Hz, 1H), 4.69 (s, 2H), 4.55 (t, *J =* 8.7 Hz, 2H), 3.31 (t, *J* = 8.7 Hz, 2H). LCMS [M+H]⁺ = 396.2.

### Preparation example 97: synthesis of compound HJM-097

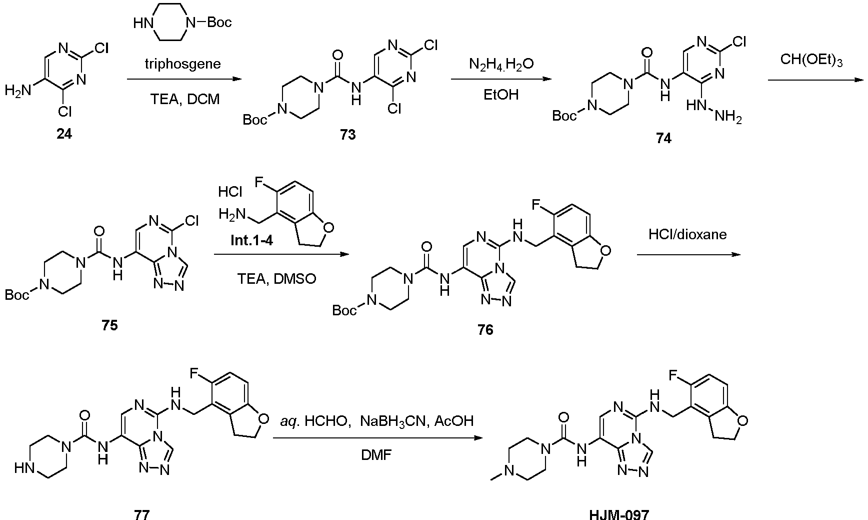

### Step 1:

At 0 °C, a solution of triphosgene (713 mg, 2.4 mmol) in dichloromethane (5 mL) was added dropwise to a solution of compound 5-amino-2,4-dichloropyrimidine **24** (656 mg, 4 mmol) and triethylamine (1.6 g, 16 mmol) in dichloromethane (10 mL). After the dropwise addition, the mixture was warmed to room temperature and stirred for 1 h. The mixture was then cooled to 0 °C, and a soluiton of tert-butyl piperazine-1-carboxylate (818 mg, 4.4 mmol) in dichloromethane (5 mL) was added dropwise. After the dropwise addition, the mixture was warmed to room temperature and stirred for 30 min. The mixture was concentrated to afford a crude product. The crude product was purified by silica gel column chromatography (petroleum ether/ethyl acetate, ethyl acetate: 0-100%) to afford compound **73** (1 g, yield: 66.5%) as a white solid. LCMS [M-tBu+H]⁺ = 320.0.

### Step 2:

Compound **73** (560 mg, 1.49 mmol) was dissolved in ethanol (8 mL), 85% hydrazine hydrate (131 mg, 2.23 mmol) was added at room temperature, and the mixture was reacted at room temperature for 2 h. The mixture was filtered and concentrated to afford crude compound **74** (280 mg, yield: 50.8%) as a white solid. LCMS [M+H]⁺ = 372.1.

### Step 3:

Compound **74** (280 mg, 1 mmol) was added to triethyl orthoformate (10 mL), and the mixture was heated at 120 °C for 1.5 h. The mixture was cooled, and concentrated to afford crude compound **75** (300 mg), which was directly used in the next step. LCMS [M+H]⁺ = 382.1.

### Step 4:

The above compound **75** (282 mg, 1 mmol), **Int.1-4** hydrochloride (203 mg, 1 mmol) and triethylamine (202 mg, 2 mmol) were dissolved in dimethyl sulfoxide (5 mL), and the mixture was stirred at room temperature for 2 h. The mixture was diluted with water, and extracted twice with ethyl acetate. The organic phases were combined, dried with anhydrous sodium sulfate, and filtered. The filtrate was concentrated to afford a crude product. The crude product was purified by preparative thin layer chromatography to afford compound **76** (300 mg, yield: 58.6%). LCMS [M+H]⁺ = 513.2.

### Step 5:

Compound **76** (300 mg, 0.59 mmol) was dissolved in 1,4-dioxane (5 mL), a solution of 4 M hydrochloric acid in 1,4-dioxane (1 mL) was added dropwise, and the mixture was stirred at room temperature for 30 min. The mixture was concentrated to afford crude compound **77** (220 mg, yield: 95.4%) as a yellow solid. LCMS [M+H]⁺ = 413.2.

### Step 6:

Compound **77** (90 mg, 0.22 mmol) was dissolved in *N*,*N*-dimethylformamide (4 mL), and 37% formaldehyde aqueous solution (0.5 mL) was added. A drop of acetic acid was added dropwise, and the mixture was stirred at 0 °C for 30 min. Sodium cyanobohydride (41 mg, 0.65 mmol) was added, and the mixture was stirred at room temperature for 4 h. The mixture was quenched with saturated sodium bicarbonate solution, and extracted twice with ethyl acetate. The organic phases were combined, dried with anhydrous sodium sulfate, and filtered. The filtrate was concentrated to afford a crude product. The crude product was purified by preparative HPLC to afford compound **HJM-097** (7 mg, yield: 11.8%) as a white solid.

¹H NMR (400 MHz, DMSO-*d*₆) δ 9.91 (br s, 1H), 9.43 (s, 1H), 8.68 (s, 1H), 8.50 (t, *J =* 5.2 Hz, 1H), 7.64 (s, 1H), 6.94 (t, *J =* 9.5 Hz, 1H), 6.71 (dd, *J =* 8.6, 3.8 Hz, 1H), 4.65 (d, *J* = 4.6 Hz, 2H), 4.54 (t, *J =* 8.7 Hz, 2H), 4.24 (d, *J =* 14.1 Hz, 2H), 3.48 (d, *J =* 12.1 Hz, 2H), 3.29 (t, *J =* 8.7 Hz, 2H), 3.17 (t, *J =* 12.8 Hz, 2H), 3.11 - 2.99 (m, 2H), 2.85 (s, 3H). LCMS [M+H]⁺ = 427.2.

### Preparation example 98: synthesis of compound HJM-098

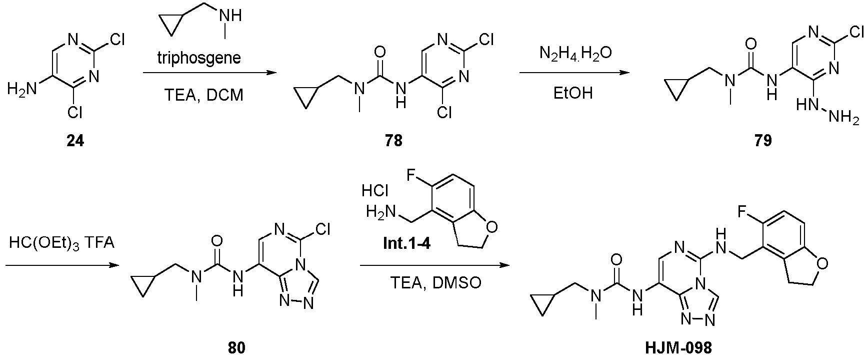

### Step 1:

At 0 °C, a solution of triphosgene (1.08 g, 3.65 mmol) in dichloromethane (5 mL) was added dropwise to a solution of compound 5-amino-2,4-dichloropyrimidine **24** (1 g, 6.09 mmol) and triethylamine (2.46 g, 24.36 mmol) in dichloromethane (30 mL). After the dropwise addition, the mixture was warmed to room temperature and stirred for 1 h. The mixture was then cooled to 0 °C, and a solution of 1-cyclopropyl-*N*-methylmethylamine (570 mg, 6.69 mmol) in dichloromethane (5 mL) was added dropwise. After the dropwise addition, the mixture was warmed to room temperature and stirred for 30 min. The mixture was concentrated to afford a crude product. The crude product was purified by silica gel column chromatography (petroleum ether/ethyl acetate, ethyl acetate: 0-50%) to afford compound **78** (620 mg, yield: 37.3%) as a colorless oil. ¹H NMR (400 MHz, CDCl₃) δ 9.49 (s, 1H), 6.84 (s, 1H), 3.30 (d, *J =* 6.7 Hz, 2H), 3.13 (s, 3H), 1.10 - 0.98 (m, 1H), 0.65 - 0.58 (m, 2H), 0.34 - 0.27 (m, 2H). LCMS [M+H]⁺ = 275.3.

### Step 2:

Compound **78** (191 mg, 0.697 mmol) was dissolved in ethanol (3 mL), 85% hydrazine hydrate (50 mg, 0.836 mmol) was added at room temperature, and the mixture was reacted at room temperature for 30 min. The mixture was concentrated to afford a crude product. The crude product was purified by preparative thin layer chromatography to afford compound **79** (175 mg, yield: 93%) as a white solid.

¹H NMR (400 MHz, CDCl₃) δ 8.06 (s, 1H), 7.17 (br s, 1H), 6.71 (br s, 1H), 4.05 (br s, 2H), 3.26 (d, *J =* 6.7 Hz, 2H), 3.09 (s, 3H), 1.07 - 0.95 (m, 1H), 0.62 - 0.55 (m, 2H), 0.31 - 0.24 (m, 2H). LCMS [M+H]⁺ = 271.1.

### Step 3:

Compound **79** (175 mg, 0.648 mmol) was added to triethyl orthoformate (5 mL), trifluoroacetic acid (0.5 mL) was added dropwise, and the mixture was heated at 100 °C for 1.5 h. The mixture was cooled, and concentrated to afford a crude product. The crude product was purified by preparative thin layer chromatography to afford compound **80** (44 mg, yield: 22.9%) as a white solid.

¹H NMR (400 MHz, CD₃OD) δ 9.40 (s, 1H), 8.41 (s, 1H), 3.37 (d, *J =* 6.9 Hz, 2H), 3.20 (s, 3H), 1.18 - 1.04 (m, 1H), 0.63 - 0.53 (m, 2H), 0.38 - 0.30 (m, 2H). LCMS [M+H]⁺ = 281.1.

### Step 4:

The above compound **80** (44 mg, 0.157 mmol), **Int.1-4** hydrochloride (38 mg, 0.188 mmol) and triethylamine (32 mg, 0.314 mmol) were dissolved in dimethyl sulfoxide (2 mL), and the mixture was stirred at 50 °C for 1 h. The mixture was cooled and purified by preparative HPLC to afford compound **HJM-098** (35 mg, yield: 54.6%) as a white solid.

¹H NMR (400 MHz, CD₃OD) δ 9.25 (s, 1H), 7.74 (s, 1H), 6.84 (t, *J* = 9.4 Hz, 1H), 6.63 (dd, *J* = 8.7, 3.9 Hz, 1H), 4.75 (s, 2H), 4.57 (t, *J =* 8.7 Hz, 2H), 3.36 (t, *J =* 8.7 Hz, 2H), 3.14 (d, *J =* 0.9 Hz, 3H), 1.17 - 1.04 (m, 1H), 0.59 - 0.50 (m, 2H), 0.34 - 0.24 (m, 2H). LCMS [M+H]⁺ = 412.1.

### Preparation example 99: synthesis of compound HJM-099

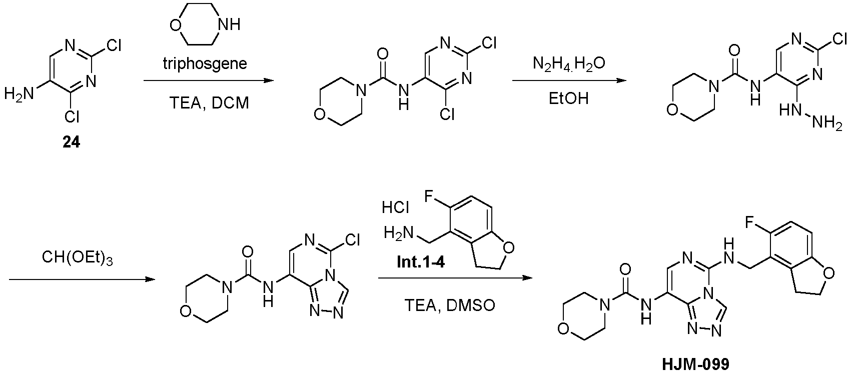

**HJM-099** (9 mg) was prepared as a white solid using morpholine as starting material according to the method similar to that for preparation example **98.**

¹H NMR (400 MHz, DMSO-*d*₆) δ 9.41 (s, 1H), 8.44 (t, *J =* 4.9 Hz, 1H), 8.37 (s, 1H), 7.63 (s, 1H), 6.94 (t, *J =* 9.4 Hz, 1H), 6.70 (dd, *J =* 8.7, 3.9 Hz, 1H), 4.65 (d, *J =* 4.9 Hz, 2H), 4.54 (t, *J* = 8.7 Hz, 2H), 3.64 - 3.60 (m, 4H), 3.46 - 3.40 (m, 4H), 3.29 (t, *J* = 8.7 Hz, 2H). LCMS [M+H] ⁺ = 414.1.

### Preparation example 100: synthesis of compound HJM-100

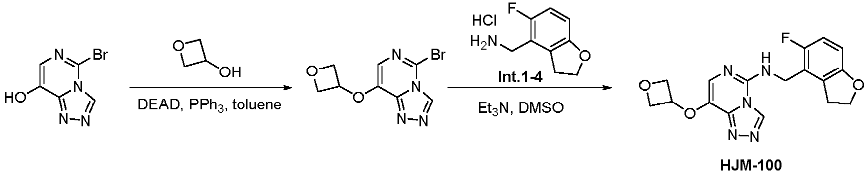

**HJM-100** (32 mg) was prepared as a white solid using 5-bromo-[1,2,4]triazolo[4,3-c]pyrimidine-8-ol (an intermediate in preparation example **13**) and 3-oxetanol as starting material according to the method similar to that for preparation example **16.**

¹H NMR (400 MHz, CD₃OD) δ 9.25 (s, 1H), 7.13 (s, 1H), 6.83 (t, *J =* 9.5 Hz, 1H), 6.63 (dd, *J* = 8.6, 3.9 Hz, 1H), 5.42 (p, *J =* 5.4 Hz, 1H), 5.01 (t, *J =* 6.8 Hz, 2H), 4.69 (s, 2H), 4.64 - 4.52 (m, 4H), 3.35 (t, *J =* 9.1 Hz, 2H). LCMS [M+H]⁺ = 358.4.

### Preparation example 101: synthesis of compound HJM-101

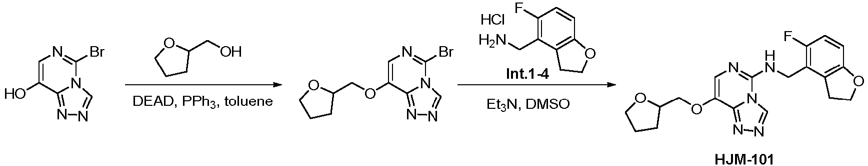

**HJM-101** (32 mg) was prepared as a white solid using tetrahydrofuran-2-methanol as starting material according to the method similar to that for preparation example **100.**

¹H NMR (400 MHz, DMSO-*d*₆) δ 9.39 (s, 1H), 8.13 (t, *J* = 5.1 Hz, 1H), 7.30 (s, 1H), 6.93 (dd, *J =* 10.3, 8.7 Hz, 1H), 6.69 (dd, *J =* 8.7, 3.9 Hz, 1H), 4.59 (d, *J =* 4.9 Hz, 2H), 4.53 (t, *J =* 8.7 Hz, 2H), 4.23 - 4.02 (m, 3H), 3.85 - 3.74 (m, 1H), 3.73 - 3.63 (m, 1H), 3.27 (t, *J =* 8.7 Hz, 2H), 2.06 - 1.94 (m, 1H), 1.94 - 1.77 (m, 2H), 1.75 - 1.63 (m, 1H). LCMS [M+H]⁺ = 386.0.

### Preparation example 102: synthesis of compound HJM-102

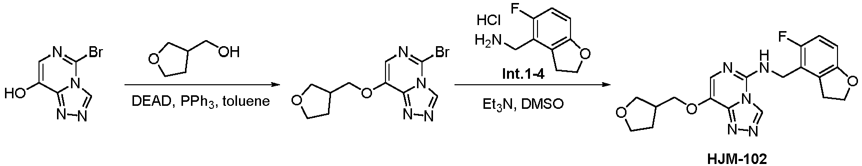

**HJM-102** (100 mg) was prepared as a white solid using tetrahydrofuran-3-methanol as starting material according to the method similar to that for preparation example **100.**

¹H NMR (400 MHz, DMSO-*d*₆) δ 9.41 (s, 1H), 8.19 (t, *J =* 5.1 Hz, 1H), 7.35 (s, 1H), 6.93 (dd, *J =* 10.3, 8.6 Hz, 1H), 6.69 (dd, *J =* 8.6, 3.9 Hz, 1H), 4.60 (d, *J =* 4.7 Hz, 2H), 4.53 (t, *J =* 8.7 Hz, 2H), 4.11 (dd, *J* = 9.5, 6.6 Hz, 1H), 4.03 (dd, *J* = 9.6, 7.9 Hz, 1H), 3.83 - 3.72 (m, 2H), 3.65 (td, *J =* 8.0, 6.8 Hz, 1H), 3.57 (dd, *J =* 8.6, 5.4 Hz, 1H), 3.28 (t, *J =* 8.7 Hz, 2H), 2.77 - 2.64 (m, 1H), 2.11 - 1.95 (m, 1H), 1.74 - 1.59 (m, 1H). LCMS [M+H]⁺ = 386.1.

### Preparation example 103: synthesis of compound HJM-103

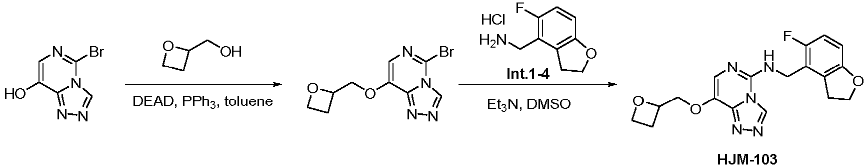

**HJM-103** (17 mg) was prepared as a white solid using oxetane-2-methanol as starting material according to the method similar to that for preparation example **100.**

¹H NMR (400 MHz, CD₃OD) δ 9.25 (s, 1H), 7.44 (s, 1H), 6.90 - 6.77 (m, 1H), 6.63 (dd, *J =* 8.7, 3.9 Hz, 1H), 5.25 - 5.15 (m, 1H), 4.75 - 4.65 (m, 4H), 4.57 (t, *J =* 8.7 Hz, 2H), 4.39 - 4.27 (m, 2H), 3.35 (t, *J* = 9.2 Hz, 2H), 2.86 - 2.75 (m, 2H). LCMS [M+H]⁺ = 372.1.

### Preparation example 104: synthesis of compound HJM-104

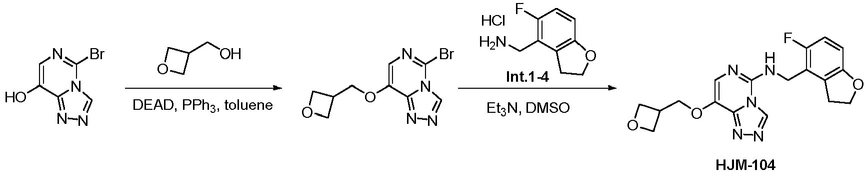

**HJM-104** (36 mg) was prepared as a white solid using oxetane-3-methanol as starting material according to the method similar to that for preparation example **100.**

¹H NMR (400 MHz, CD₃OD) δ 9.24 (s, 1H), 7.41 (s, 1H), 6.83 (dd, *J* = 10.2, 8.7 Hz, 1H), 6.62 (dd, *J =* 8.6, 3.9 Hz, 1H), 4.88 (dd, *J* = 7.9, 6.2 Hz, 2H), 4.70 (s, 2H), 4.63 (t, *J =* 6.1 Hz, 2H), 4.57 (t, *J* = 8.7 Hz, 2H), 4.41 (d, *J* = 6.8 Hz, 2H), 3.60 - 3.45 (m, 1H), 3.34 (t, *J =* 8.7 Hz, 2H). LCMS [M+H]⁺ = 372.0.

### Preparation example 105: synthesis of compound HJM-105

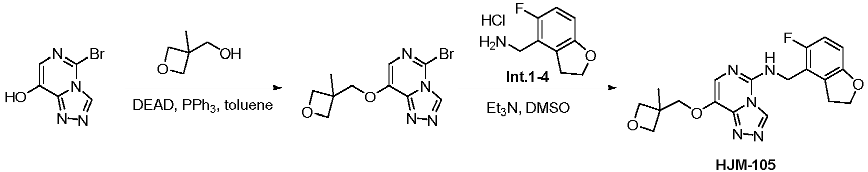

**HJM-105** (35 mg) was prepared as a white solid using 3-methyloxetane-3-methanol as starting material according to the method similar to that for preparation example **100.**

¹H NMR (400 MHz, CD₃OD) δ 9.26 (s, 1H), 7.42 (s, 1H), 6.82 (dd, *J* = 10.2, 8.7 Hz, 1H), 6.61 (dd, *J =* 8.7, 3.9 Hz, 1H), 4.73 - 4.68 (m, 4H), 4.56 (t, *J* = 8.7 Hz, 2H), 4.46 (d, *J =* 6.0 Hz, 2H), 4.26 (s, 2H), 3.34 (d, *J =* 8.7 Hz, 2H), 1.49 (s, 3H). LCMS [M+H]⁺ = 386.4..

### Preparation example 106: synthesis of compound HJM-106

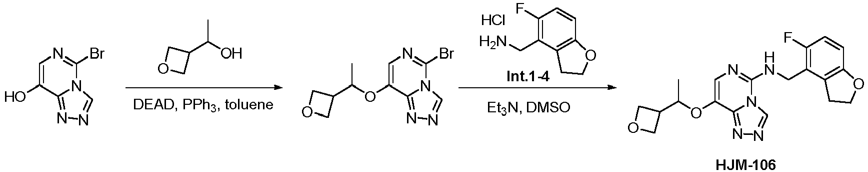

**HJM-106** (20 mg) was prepared as a white solid using 1-(oxetan-3-yl)ethanol as starting material according to the method similar to that for preparation example **100.**

¹H NMR (400 MHz, CD₃OD) δ 9.25 (s, 1H), 7.42 (s, 1H), 6.83 (t, *J =* 9.4 Hz, 1H), 6.62 (dd, *J* = 8.7, 3.9 Hz, 1H), 4.85 - 4.74 (m, 1H), 4.76 - 4.61 (m, 3H), 4.56 (t, *J =* 8.7 Hz, 2H), 4.01 - 3.82 (m, 2H), 3.83 - 3.71 (m, 1H), 3.34 (t, *J =* 8.7 Hz, 2H), 2.18 (h, *J =* 5.5 Hz, 1H), 1.39 (d, *J =* 6.4 Hz, 3H). LCMS [M+H]⁺ = 386.1.

### Preparation example 107: synthesis of compound HJM-107

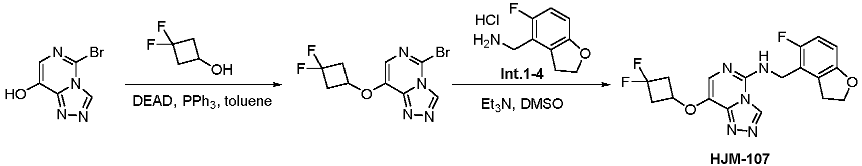

**HJM-107** (7 mg) was prepared as a white solid using 3,3-difluorocyclobutanol as starting material according to the method similar to that for preparation example **100.**

¹H NMR (400 MHz, CD₃OD) δ 9.25 (s, 1H), 7.28 (s, 1H), 6.83 (dd, *J* = 10.5, 8.4 Hz, 1H), 6.63 (dd, *J =* 8.7, 3.9 Hz, 1H), 4.96 - 4.88 (m, 1H), 4.70 (s, 2H), 4.57 (t, *J* = 8.7 Hz, 2H), 3.34 (t, *J =* 8.7 Hz, 2H), 3.19 - 3.07 (m, 2H), 2.92 - 2.78 (m, 2H). LCMS [M+H]⁻ = 392.5.

### Preparation example 108: synthesis of compound HJM-108

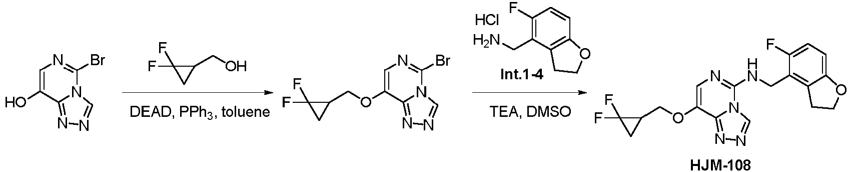

**HJM-108** (6 mg) was prepared as a white solid using 2,2-difluorocyclopropylmethanol as starting material according to the method similar to that for preparation example **100.**

¹H NMR (400 MHz, CD₃OD) δ 9.25 (s, 1H), 7.39 (s, 1H), 6.83 (dd, *J =* 10.2, 8.7 Hz, 1H), 6.61 (dd, *J =* 8.6, 3.9 Hz, 1H), 4.70 (s, 2H), 4.56 (t, *J =* 8.7 Hz, 2H), 4.37 - 4.27 (m, 1H), 4.18 (ddd, *J =* 10.6, 8.3, 1.9 Hz, 1H), 3.33 (t, *J* = 8.6 Hz, 2H), 2.29 - 2.13 (m, 1H), 1.70 - 1.57 (m, 1H), 1.46 - 1.33 (m, 1H). LCMS [M+H]⁺ = 392.5.

### Preparation example 109: synthesis of compound HJM-109

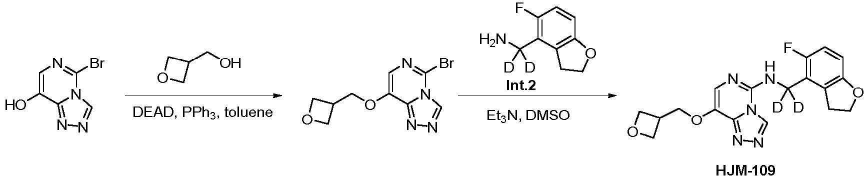

**HJM-109** (14 mg) was prepared as a white solid using deuterated compound **Int.2** as starting material according to the method similar to that for preparation example **104.**

¹H NMR (400 MHz, CD₃OD) δ 9.24 (s, 1H), 7.42 (s, 1H), 6.84 (dd, *J =* 10.2, 8.7 Hz, 1H), 6.63 (dd, *J =* 8.7, 3.9 Hz, 1H), 4.89 (dd, *J =* 7.9, 6.3 Hz, 2H), 4.63 (t, *J* = 6.1 Hz, 2H), 4.57 (t, *J* = 8.7 Hz, 2H), 4.41 (d, *J = 6.8* Hz, 2H), 3.60 - 3.47 (m, 1H), 3.35 (t, *J =* 8.8 Hz, 2H). LCMS [M+H]⁺ = 374.1.

### Preparation example 110: synthesis of compound HJM-110

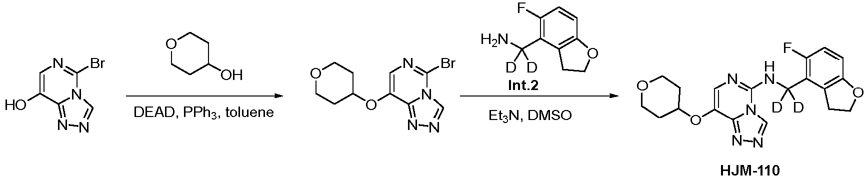

**HJM-110** (43 mg) was prepared as a white solid using deuterated compound **Int.2** as starting material according to the method similar to that for preparation example 16.

¹H NMR (400 MHz, CD₃OD) δ 9.25 (s, 1H), 7.43 (s, 1H), 6.83 (dd, *J =* 10.2, 8.7 Hz, 1H), 6.62 (dd, *J =* 8.6, 3.9 Hz, 1H), 4.69 (tt, *J =* 8.2, 3.9 Hz, 1H), 4.56 (t, *J =* 8.8 Hz, 2H), 4.00 (dt, *J =* 11.7, 4.5 Hz, 2H), 3.55 (ddd, *J* = 11.8, 8.7, 3.1 Hz, 2H), 3.34 (t, *J* = 8.7 Hz, 2H), 2.09 - 2.01 (m, 2H), 1.86 - 1.75 (m, 2H). LCMS [M+H]⁺ = 388.0.

### Preparation example 111: synthesis of compound HJM-111

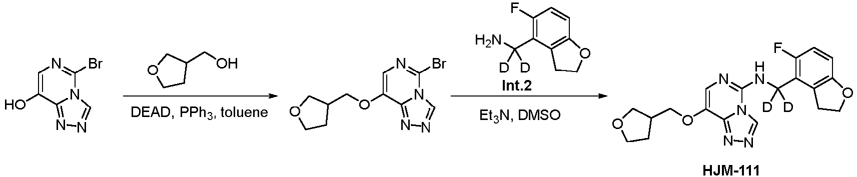

**HJM-111** (45 mg) was prepared as a white solid using deuterated compound **Int.2** as starting material according to the method similar to that for preparation example **102.**

¹H NMR (400 MHz, CD₃OD) δ 9.24 (s, 1H), 7.36 (s, 1H), 6.83 (dd, *J* = 10.2, 8.7 Hz, 1H), 6.62 (dd, *J =* 8.7, 3.9 Hz, 1H), 4.56 (t, *J =* 8.7 Hz, 2H), 4.15 (dd, *J =* 9.3, 6.4 Hz, 1H), 4.07 (dd, *J =* 9.3, 8.0 Hz, 1H), 3.97 - 3.87 (m, 2H), 3.82 - 3.72 (m, 2H), 3.34 (t, *J =* 8.7 Hz, 2H), 2.89 - 2.75 (m, 1H), 2.21 - 2.10 (m, 1H), 1.87 - 1.76 (m, 1H). LCMS [M+H]⁺ = 388.1.

### Preparation example 112: synthesis of compound HJM-112

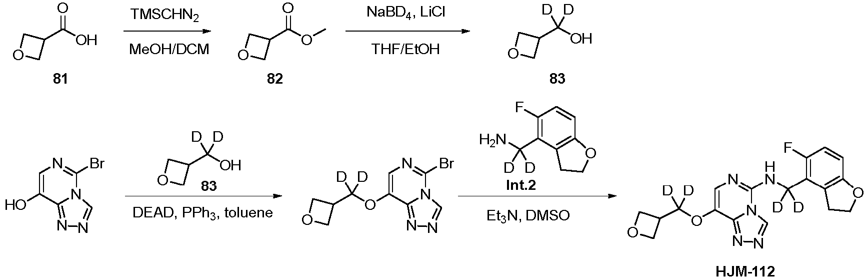

### Step 1:

At 0 °C, oxetane-3-carboxylic acid 81 (1.3 g, 12.73 mmol) was dissolved in a mixture of methanol (10 mL) and dichloromethane (10 mL), and trimethylsilyldiazomethane (2 M, 12.73 mL, 25.46 mmol) was slowly added. After the dropwise addition, the mixture was warmed to room temperature and stirred for 3 h. After the reaction was completed, acetic acid (0.05 mL) was added to the reaction mixture. The mixture was poured into water, and extracted with dichloromethane (2×30 mL). The organic phases were combined, and dried with anhydrous sodium sulfate. The mixture was filtered, and the filtrate was concentrated under reduced pressure to afford crude compound **82** (1.4 g, yield: 94.7%) as a colorless oil.

### Step 2:

Compound **82** (1.4 g, 12.06 mmol) was dissolved in ethanol (10 mL) and tetrahydrofuran (5 mL), and lithium chloride (51.11 mg, 1.21 mmol) was added. Sodium deuterated borohydride (912.30 mg, 24.11 mmol) was added portionwise at 0 °C, and the mixture was stirred at room temperature for 10 h. The mixture was cooled to 0 °C, and then deuterium oxide (1 mL) was added. The mixture was stirred for 10 min, and then dried by adding anhydrous sodium sulfate. The mixture was filtered, and concentrated. The resulting crude product was purified by silica gel column chromatography (petroleum ether/ethyl acetate, ethyl acetate: 50%-100%) to afford deuterated compound **83** (0.4 g, yield: 36.8%) as a yellowish oil.

¹H NMR (400 MHz, DMSO-*d*₆) δ 4.58 (dd, *J =* 7.8, 5.9 Hz, 1H), 4.29 (t, *J =* 5.9 Hz, 1H), 3.03 - 2.93 (m, 1H).

In the following steps, **HJM-112** (9 mg) was prepared as a white solid according to the method similar to that for preparation example **109.**

¹H NMR (400 MHz, CD₃OD) δ 9.24 (s, 1H), 7.41 (s, 1H), 6.83 (dd, *J =* 10.2, 8.7 Hz, 1H), 6.63 (dd, *J =* 8.6, 3.9 Hz, 1H), 4.88 (dd, *J =* 8.0, 6.3 Hz, 2H), 4.63 (t, *J =* 6.1 Hz, 2H), 4.57 (t, *J =* 8.7 Hz, 2H), 3.52 (p, *J* = 6.9 Hz, 1H), 3.35 (t, *J =* 8.8 Hz, 2H). LCMS [M+H]⁺ = 376.3.

### Preparation example 113: synthesis of compound HJM-113

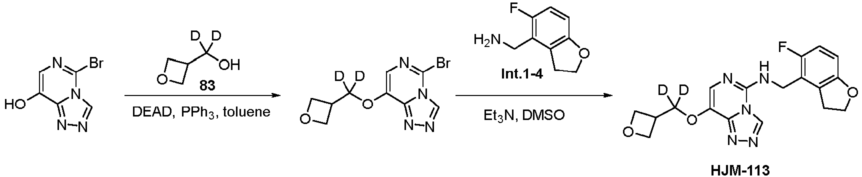

HJM-113 (10 mg) was prepared as a white solid using **Int.1-4** as starting material according to the method similar to that for preparation example 112.

¹H NMR (400 MHz, CD₃OD) δ 9.24 (s, 1H), 7.42 (s, 1H), 6.87 - 6.80 (m, 1H), 6.63 (dd, *J =* 8.7, 3.9 Hz, 1H), 4.89 (dd, *J* = 8.0, 6.3 Hz, 2H), 4.71 (s, 2H), 4.63 (t, *J* = 6.1 Hz, 2H), 4.57 (t, *J* = 8.7 Hz, 2H), 3.52 (p, *J* = 7.0 Hz, 1H), 3.35 (t, *J* = 9.0 Hz, 2H). LCMS [M+H]⁺ = 374.2.

### Preparation example 114: synthesis of compound HJM-114

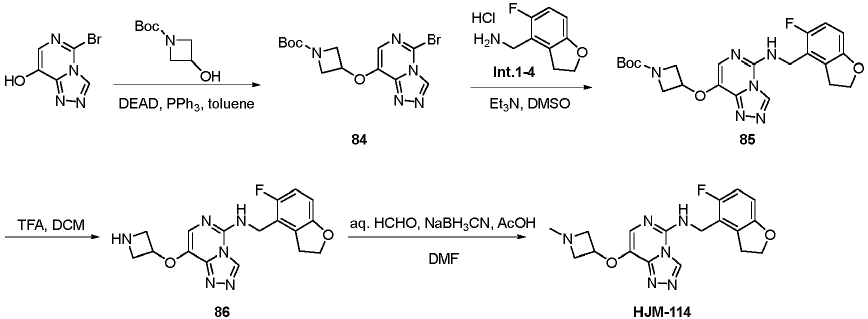

### Step 1:

5-bromo-[1,2,4]triazolo[4,3-c]pyrimidine-8-ol (150 mg, 0.697 mmol), tert-butyl 3-hydroxyazetidine-1-carboxylate (181 mg, 1.05 mmol) and triphenyl phosphine (274 mg, 1.05 mmol) were added to toluene (10 mL), and the mixture was heated to 100 °C under nitrogen protection. Diethyl azodicarboxylate (180 mg, 1.05 mmol) was added dropwise. After the dropwise addition, the mixture was heated at 100 °C for 2 h. The mixture was cooled, and concentrated to afford a crude product. The crude compound **84** was directly used in the next step. LCMS [M+H]⁺ = 370.3.

### Step 2:

The above compound **84, Int.1-4** hydrochloride (164 mg, 0.81 mmol) and triethylamine (163 mg, 1.62 mmol) were dissolved in dimethyl sulfoxide (5 mL), and the mixture was stirred at room temperature for 2 h. The reaction mixture was poured into water and stirred for 10 min. The precipitated solid was filtered, and dried to afford crude compound **85** (200 mg, yield: 81.3 %) as a yellow solid. LCMS [M+H]⁺ = 457.1.

### Step 3:

Compound **85** (200 mg, 0.438 mmol) was dissolved in dichloromethane (4 mL), and trifluoroacetic acid (1 mL) was added dropwise. After the dropwise addition, the mixture was stirred at room temperature for 2 h. The mixture was then concentrated to afford crude compound **86** (150 mg, yield: 72.8%).

### Step 4:

Compound **86** (40 mg, 0.112 mmol) was dissolved in *N,N*-dimethylformamide (3 mL), and 37% formaldehyde aqueous solution (0.7 mL) was added. A drop of acetic acid was added dropwise, and the mixture was stirred at 0 °C for 10 min. Sodium cyanobohydride (15 mg, 0.224 mmol) was added, and the mixture was stirred at 0 °C for 2 h. The reaction was quenched with saturated aqueous sodium bicarbonate solution, and the mixture was extracted twice with ethyl acetate. The organic phases were combined, dried with anhydrous sodium sulfate, and filtered. The filtrate was concentrated to afford a crude product. The crude product was purified by preparative HPLC to afford compound **HJM-114** hydrochloride (6 mg, yield: 14.4%) as a white solid.

¹H NMR (400 MHz, CD₃OD) δ 9.29 (s, 1H), 7.38 (s, 1H), 6.83 (t, *J =* 9.4 Hz, 1H), 6.63 (dd, *J =* 8.6, 3.9 Hz, 1H), 5.23 (br s, 1H), 4.70 (s, 2H), 4.57 (t, *J =* 8.6 Hz, 2H), 4.55 - 4.20 (m, 4H), 3.35 (t, *J* = 8.7 Hz, 2H), 3.04 (s, 3H). LCMS [M+H]⁺ = 371.1.

### Preparation example 115: synthesis of compound HJM-115

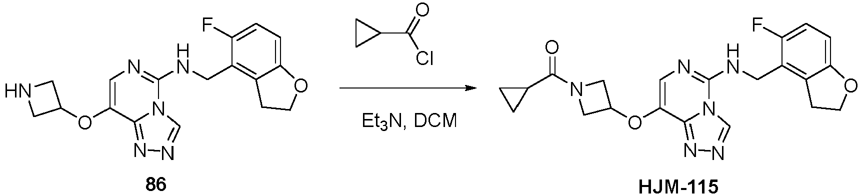

The crude compound 86 (75 mg, 0.21 mmol) and triethylamine (0.5 mL) were dissolved in dichloromethane (3 mL), cyclopropionyl chloride (44 mg, 0.42 mmol) was added dropwise, and the mixture was stirred at room temperature for 2 h. The mixture was concentrated to afford a crude product. The crude product was purified by preparative HPLC to afford compound **HJM-115** (31 mg, yield: 34.7 %) as a white solid.

¹H NMR (400 MHz, CD₃OD) δ 9.30 (s, 1H), 7.36 (s, 1H), 6.84 (t, *J =* 9.4 Hz, 1H), 6.63 (dd, *J =* 8.7, 3.8 Hz, 1H), 5.26 (tt, *J =* 6.7, 3.7 Hz, 1H), 4.76 (dd, *J =* 9.4, 6.5 Hz, 1H), 4.71 (s, 2H), 4.57 (t, *J* = 8.7 Hz, 2H), 4.47 (dd, *J =* 10.1, 3.6 Hz, 1H), 4.40 (dd, *J =* 11.1, 6.4 Hz, 1H), 4.09 (dd, *J =* 11.1, 3.7 Hz, 1H), 3.36 (t, *J =* 8.7 Hz, 2H), 1.66 - 1.56 (m, 1H), 0.93 - 0.79 (m, 4H). LCMS [M+H]⁺ = 425.4.

### Preparation example 116: synthesis of compound HJM-116

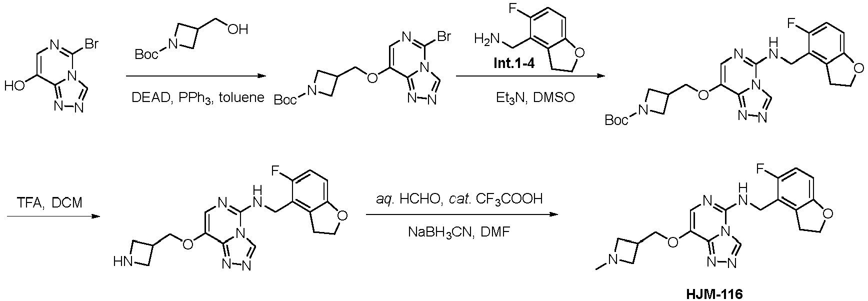

**HJM-116** (10 mg) was prepared as a white solid using tert-butyl 3-hydroxymethylazetidine-1-carboxylate as starting material according to the method similar to that for preparation example **114.**

¹H NMR (400 MHz, CD₃OD) δ 9.26 (s, 1H), 7.37 (s, 1H), 6.82 (dd, *J =* 10.2, 8.7 Hz, 1H), 6.61 (dd, *J =* 8.6, 3.8 Hz, 1H), 5.49 (s, 2H), 4.69 (s, 2H), 4.56 (t, *J* = 8.7 Hz, 2H), 4.27 (d, *J* = 6.3 Hz, 2H), 3.57 (t, *J =* 8.0 Hz, 2H), 3.34 (t, *J =* 8.1 Hz, 2H), 3.06 - 2.95 (m, 1H). LCMS [M+H]⁺ = 385.5.

### Preparation example 117: synthesis of compound HJM-117

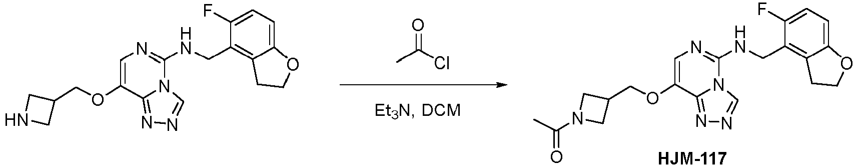

**HJM-117** (15 mg) was prepared as a white solid using acetyl chloride as starting material according to the method similar to that for preparation example **115.**

¹H NMR (400 MHz, DMSO-*d*₆) δ 9.40 (s, 1H), 7.93 (br s, 1H), 7.35 (s, 1H), 6.93 (t, *J* = 9.0 Hz, 1H), 6.76 - 6.65 (m, 1H), 4.60 (s, 2H), 4.53 (t, *J =* 8.2 Hz, 2H), 4.29 (d, *J =* 5.9 Hz, 2H), 4.24 (t, *J =* 8.2 Hz, 2H), 4.00 - 3.90 (m, 2H), 3.28 (t, *J =* 8.4 Hz, 2H), 3.08 - 2.96 (m, 1H), 1.75 (s, 3H). LCMS [M+H]⁺ = 413.4.

### Preparation example 118: synthesis of compound HJM-118

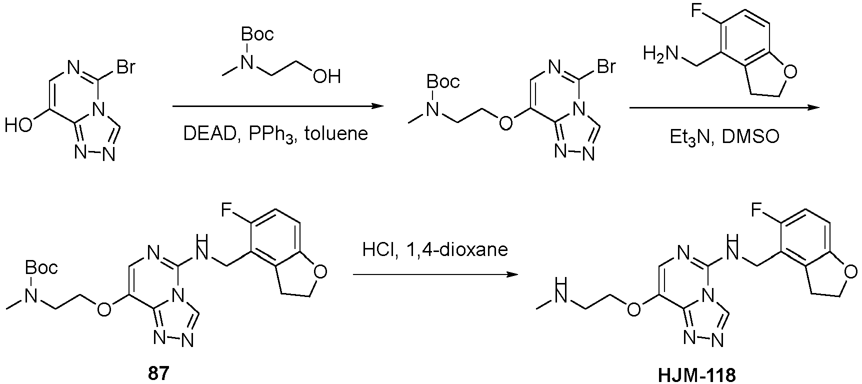

### Steps 1-2:

Compound **87** (400 mg) was prepared using tert-butyl N-methyl-N-(2-hydroxyethyl)carbamate as starting material according to the method similar to that for preparation example **100.** LCMS [M+H]⁺ = 459.2.

### Step 3:

Compound **87** (400 mg, 0.87mmol) was dissolved in 1,4-dioxane (5 mL), a solution of 4 M hydrochloric acid in 1,4-dioxane (1 mL) was added dropwise, and the mixture was stirred at room temperature for 2 h. The mixture was concentrated to afford a crude product. The crude product was purified by preparative HPLC to afford compound **HJM-118** (72 mg, yield: 24.0%) as a white solid.

¹H NMR (400 MHz, CD₃OD) δ 9.28 (s, 1H), 7.47 (s, 1H), 6.83 (dd, *J =* 10.2, 8.7 Hz, 1H), 6.63 (dd, *J =* 8.7, 3.9 Hz, 1H), 4.71 (s, 2H), 4.57 (t, *J =* 8.7 Hz, 2H), 4.45 - 4.40 (m, 2H), 3.54 - 3.48 (m, 2H), 3.36 (t, *J =* 8.7 Hz, 2H), 2.84 (s, 3H). LCMS [M+H]⁺ = 359.1.

### Preparation example 119: synthesis of compound HJM-119

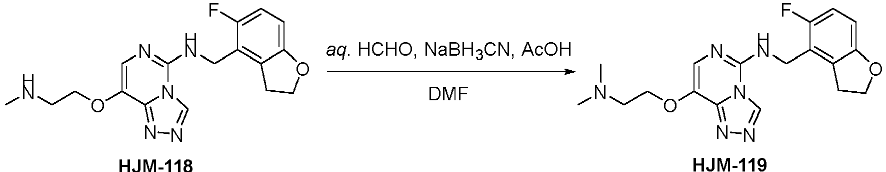

Compound **HJM-118** (150 mg, 0.419 mmol) was dissolved in *N,N*-dimethylformamide (5 mL), and 37% formaldehyde aqueous solution (0.7 mL) was added. A drop of acetic acid was added dropwise, and the mixture was stirred at 0 °C for 30 min. Sodium cyanobohydride (80 mg, 1.26 mmol) was added, and the mixture was stirred at 0 °C for 4 h. The mixture was quenched with saturated aqueous sodium bicarbonate solution, and extracted twice with ethyl acetate. The organic phases were combined, dried with anhydrous sodium sulfate, and filtered. The filtrate was concentrated to afford a crude product. The crude product was purified by preparative HPLC to afford compound **HJM-119** (7 mg, yield: 4.5%) as a white solid.

¹H NMR (400 MHz, CD₃OD) δ 9.28 (s, 1H), 7.48 (s, 1H), 6.87 - 6.80 (m, 1H), 6.63 (dd, *J =* 8.7, 3.9 Hz, 1H), 4.71 (s, 2H), 4.57 (t, *J =* 8.7 Hz, 2H), 4.54 - 4.47 (m, 2H), 3.68 - 3.63 (m, 2H), 3.36 (t, *J* = 8.7 Hz, 2H), 3.04 (s, 6H). LCMS [M+H]⁺ = 373.1.

### Preparation examples 120 and 121: synthesis of compounds HJM-120 and 121

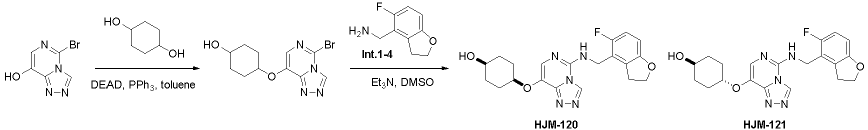

**HJM-120** (19 mg) and **HJM-121** (8 mg) were prepared as white solids using 1,4-cyclohexanediol as starting material according to the method similar to that for preparation example **100.** Cis-trans configuration was not confirmed.

**HJM-120:** ¹H NMR (400 MHz, DMSO-*d*₆) δ 9.39 (s, 1H), 8.18 (t, *J =* 5.1 Hz, 1H), 7.34 (s, 1H), 6.93 (dd, *J =* 10.0, 8.6 Hz, 2H), 6.69 (dd, *J =* 8.6, 3.9 Hz, 1H), 4.60 (d, *J =* 4.9 Hz, 2H), 4.53 (t, *J =* 8.7 Hz, 2H), 4.52 - 4.45 (m, 1H), 3.58 - 3.48 (m, 1H), 3.28 (t, *J =* 8.7 Hz, 2H), 2.05 - 1.95 (m, 2H), 1.88 - 1.79 (m, 2H), 1.51 - 1.39 (m, 2H), 1.31 - 1.20 (m, 2H). LCMS [M+H]⁺ = 400.5. HPLC_{tR} = 7.92 min. (SunFire C18 column 5 µm 4.6×150 mm, 25 °C, mobile phase: acetonitrile and water containing 0.03% of TFA, flow rate: 1.0 mL/min, method duration: 16 min)

**HJM-121:** ¹H NMR (400 MHz, DMSO-*d*₆) δ 9.39 (s, 1H), 8.17 (t, *J =* 4.9 Hz, 1H), 7.35 (s, 1H), 6.94 (dd, *J* = 9.9, 8.6 Hz, 2H), 6.70 (dd, *J* = 8.5, 3.9 Hz, 1H), 4.60 (d, *J* = 5.0 Hz, 3H), 4.54 (t, *J* = 8.7 Hz, 2H), 3.62 - 3.55 (m, 1H), 3.28 (t, *J =* 8.8 Hz, 2H), 1.90 - 1.79 (m, 2H), 1.69 - 1.48 (m, 6H). LCMS [M+H]⁺ = 400.5. HPLC_{tR} = 8.07 min. (SunFire C18 column 5 µm 4.6×150 mm, 25 °C, mobile phase: acetonitrile and water containing 0.03% of TFA, flow rate: 1.0 mL/min, method duration: 16 min)

### Preparation example 122: synthesis of compound HJM-122

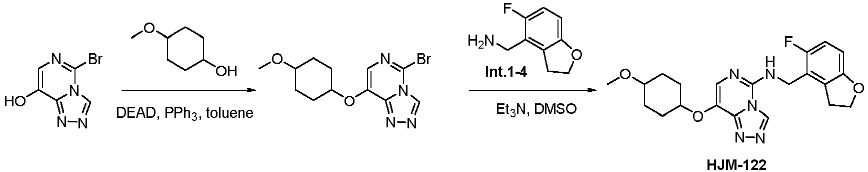

**HJM-122** (52 mg) was prepared as a white solid, a mixture of cis- and trans- configurations, using 4-methoxycyclohexanol as starting material according to the method similar to that for preparation example 100.

¹H NMR (400 MHz, CD₃OD) δ 9.31 (s, 0.65H), 9.30 (s, 0.35H), 7.60 (s, 0.65H), 7.57 (s, 0.35H), 6.88 - 6.79 (m, 1H), 6.63 (dd, *J =* 8.7, 3.9 Hz, 1H), 4.72 (s, 2H), 4.61 - 4.53 (m, 3H), 4.54 - 4.46 (m, 1H), 3.35 (t, *J =* 8.6 Hz, 2H), 3.35 (s, 3H), 2.17 - 1.37 (m, 8H). LCMS [M+H]⁺ = 414.3.

### Preparation example 123: synthesis of compound HJM-123

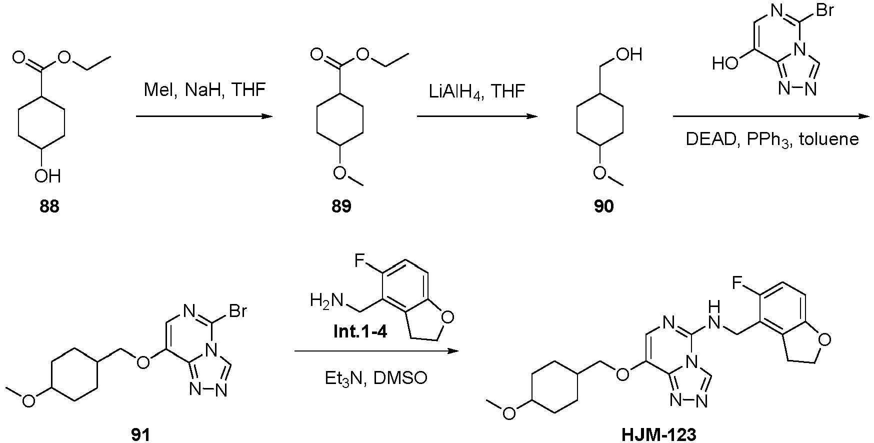

### Step 1:

Compound ethyl 4-hydroxycyclohexyl formate **88** (1.0 g, 5.81 mmol) was dissolved in anhydrous tetrahydrofuran (10 mL). Sodium hydride (60%, 348 mg, 8.71 mmol) was added at 0 °C under nitrogen protection, and the mixture was stirred for 10 min. Iodomethane (1.64 g, 11.61 mmol) was added dropwise, and the mixture was stirred at room temperature for 2 h. Water was added to quench the reaction, and the mixture was extracted with ethyl acetate. The organic phase was dried with anhydrous sodium sulfate, and filtered. The filtrate was concentrated to afford crude compound **89.**

### Step 2:

The above compound **89** was dissolved in anhydrous tetrahydrofuran (10 mL). Lithium aluminum hydride (440 mg, 11.61 mmol) was added portionwise at 0 °C, and the mixture was stirred at room temperature for 2 h. Ice was added to quench the reaction, and the mixture was further stirred for 30 min. The reaction mixture was filtered, and the filtrate was concentrated to afford crude compound **90** (240 mg, yield: 28.6%) as a colorless liquid.

### Step 3:

5-Bromo-[1,2,4]triazolo[4,3-c]pyrimidine-8-ol (215 mg, 1.00 mmol), compound **90** (240 mg, 1.60 mmol) and triphenyl phosphine (393 mg, 1.50 mmol) were added to toluene (10 mL), and the mixture was heated to 100 °C under nitrogen protection. Diethyl azodicarboxylate (208 mg, 1.20 mmol) was added dropwise. After the dropwise addition, the mixture was heated at 100 °C for 2 h. The mixture was cooled, and concentrated to afford crude compound **91,** which was directly used in the next step. LCMS [M+H]⁺ = 341.0.

### Step 4:

The above compound **91, Int.1-4** (134 mg, 0.80 mmol) and triethylamine (160 mg, 1.60 mmol) were dissolved in dimethyl sulfoxide (3 mL), and the mixture was stirred at room temperature for 2 h. The mixture was purified by preparative HPLC to afford compound **HJM-123** (11 mg, yield: 3.2 %) as a white solid.

¹H NMR (400 MHz, DMSO-*d*₆) δ 9.39 (s, 1H), 8.12 (t, *J =* 5.1 Hz, 1H), 7.28 (s, 1H), 6.93 (dd, *J* = 10.3, 8.7 Hz, 1H), 6.69 (dd, *J =* 8.6, 3.8 Hz, 1H), 4.59 (d, *J =* 4.9 Hz, 2H), 4.53 (t, *J =* 8.7 Hz, 2H), 3.94 (d, *J* = 6.4 Hz, 2H), 3.27 (t, *J =* 8.8 Hz, 2H), 3.14 - 3.02 (m, 1H), 2.08 - 2.00 (m, 2H), 1.92 - 1.84 (m, 2H), 1.78 - 1.68 (m, 1H), 1.20 - 1.02 (m, 4H). LCMS [M+H]⁺ = 428.2.

### Preparation examples 124 and 125: synthesis of compounds HJM-124 and 125

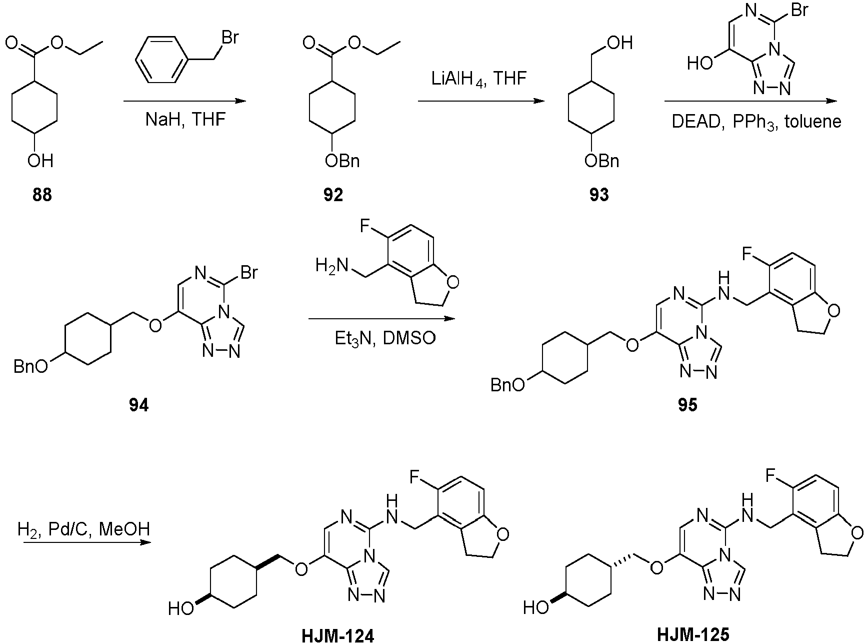

### Steps 1-4:

Compound **95** (30 mg) was prepared using benzyl bromide instead of iodomethane as starting material according to the method similar to that for preparation example 123. LCMS [M+H]⁺ = 504.3.

### Step 5:

Compound **95** (30 mg, 0.06 mmol) was dissolved in methanol (3 mL), and 10% Pd/C (20 mg) was added under nitrogen protection. The atmosphere was replaced three times with hydrogen gas, and the mixture was stirred at 40 °C under a hydrogen balloon for 4 h. The mixture was filtered, and the filter cake was washed with methanol for three times. The filtrates were combined, and concentrated to afford a crude product. The crude product was purified by preparative HPLC to afford compound **HJM-124** (1 mg) and **HJM-125** (2.8 mg), total yield: 15.3%. Both were yellow oil. Cis-trans configuration was not confirmed.

**HJM-124** : ¹H NMR (400 MHz, CD₃OD) δ 9.26 (s, 1H), 7.41 (s, 1H), 6.89 - 6.80 (m, 1H), 6.63 (dd, *J* = 8.7, 3.9 Hz, 1H), 4.70 (s, 2H), 4.57 (t, *J* = 8.7 Hz, 2H), 3.98 (d, *J* = 6.3 Hz, 2H), 3.59 - 3.50 (m, 1H), 3.35 (t, *J =* 8.8 Hz, 2H), 2.07 - 1.96 (m, 4H), 1.88 - 1.77 (m, 1H), 1.38 - 1.17 (m, 4H). LCMS [M+H]⁺ = 414.3. HPLC_{tR} = 7.967 min. (XBridge C18 column 5 µm 4.6×150 mm, 25 °C, mobile phase: acetonitrile and water containing 0.03% NH₃·H₂O, flow rate: 1.0 mL/min, method duration: 16 min)

**HJM-125 :** ¹H NMR (400 MHz, CD₃OD) δ 9.29 (s, 1H), 7.51 (d, *J* = 4.1 Hz, 1H), 6.84 (t,*J* = 9.5 Hz, 1H), 6.64 (dd, *J* = 8.6, 3.8 Hz, 1H), 4.71 (s, 2H), 4.57 (t, *J* = 8.7 Hz, 2H), 4.04 (d, *J* = 6.6 Hz, 2H), 3.98 - 3.93 (m, 1H), 3.36 (t, *J* = 8.8 Hz, 2H), 2.06 - 1.90 (m, 1H), 1.83 - 1.57 (m, 8H). LCMS [M+H]⁺ = 414.3. HPLC_{tR} = 8.190 min. (XBridge C18 column 5 µm 4.6×150 mm, 25 °C, mobile phase: acetonitrile and water containing 0.03% NH₃·H₂O, flow rate: 1.0 mL/min, method duration: 16 min)

### Preparation example 126: synthesis of compound HJM-126

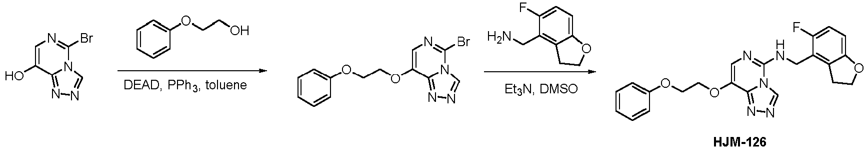

HJM-126 (38 mg) was prepared as a white solid using 2-phenoxyethanol as starting material according to the method similar to that for preparation example **100.**

¹H NMR (400 MHz, DMSO-*d*₆) δ 9.40 (s, 1H), 8.17 (t, *J =* 5.1 Hz, 1H), 7.36 (s, 1H), 7.33 - 7.27 (m, 2H), 7.01 - 6.90 (m, 4H), 6.69 (dd, *J* = 8.7, 3.9 Hz, 1H), 4.61 (d, *J* = 4.9 Hz, 2H), 4.53 (t, *J =* 8.7 Hz, 2H), 4.50 - 4.46 (m, 2H), 4.35 - 4.31 (m, 2H), 3.27 (t, *J =* 8.7 Hz, 2H). LCMS [M+H]⁺ = 422.1.

### Preparation example 127: synthesis of compound HJM-127

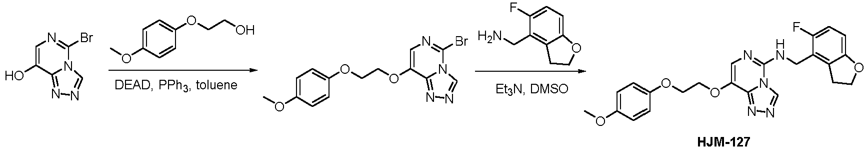

**HJM-127** (26 mg) was prepared as a white solid using 2-(4-methoxyphenoxy)ethanol as starting material according to the method similar to that for preparation example **100.**

¹H NMR (400 MHz, DMSO-*d*₆) δ 9.40 (s, 1H), 8.17 (t, *J =* 5.1 Hz, 1H), 7.36 (s, 1H), 6.97 - 6.84 (m, 5H), 6.69 (dd, *J* = 8.7, 3.9 Hz, 1H), 4.61 (d, *J* = 5.0 Hz, 2H), 4.53 (t, *J* = 8.7 Hz, 2H), 4.48 - 4.42 (m, 2H), 4.30 - 4.24 (m, 2H), 3.70 (s, 3H), 3.28 (t, *J =* 8.7 Hz, 2H). LCMS [M+H]⁺ = 452.3.

### Preparation example 128: synthesis of compound HJM-128

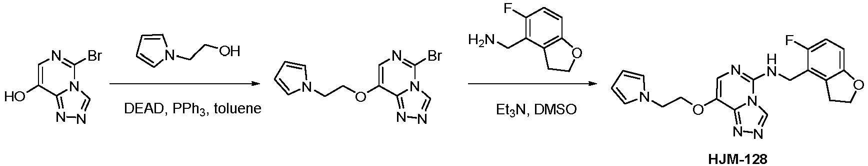

**HJM-128** (13 mg) was prepared as a white solid using 2-(pyrrol-1-yl)ethanol as starting material according to the method similar to that for preparation example **100.**

¹H NMR (400 MHz, CD₃OD) δ 9.23 (s, 1H), 7.21 (s, 1H), 6.86 - 6.80 (m, 3H), 6.62 (dd, *J =* 8.7, 3.8 Hz, 1H), 6.01 (t, *J =* 2.1 Hz, 2H), 4.67 (s, 2H), 4.56 (t, *J =* 8.7 Hz, 2H), 4.42 - 4.37 (m, 2H), 4.37 - 4.30 (m, 2H), 3.32 (t, *J =* 8.7 Hz, 2H). LCMS [M+H]⁺ = 395.5.

### Preparation example 129: synthesis of compound HJM-129

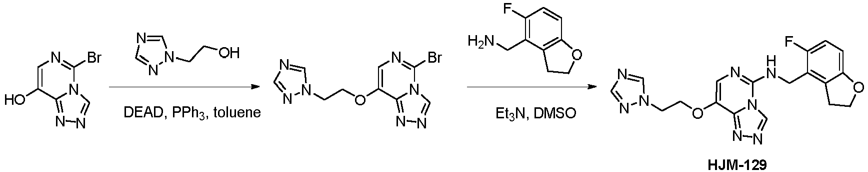

**HJM-129** (18 mg) was prepared as a white solid using 2-(1,2,4-triazol-1-yl)ethanol as starting material according to the method similar to that for preparation example **100.**

¹H NMR (400 MHz, DMSO-*d*₆) δ 9.39 (s, 1H), 8.62 (s, 1H), 8.21 (t, *J =* 5.2 Hz, 1H), 8.00 (s, 1H), 7.31 (s, 1H), 6.98 - 6.88 (m, 1H), 6.69 (dd, *J =* 8.7, 3.9 Hz, 1H), 4.63 - 4.58 (m, 4H), 4.56 - 4.50 (m, 4H), 3.27 (t, *J* = 8.7 Hz, 2H). LCMS [M+H]⁺ = 397.1.

### Preparation example 130: synthesis of compound HJM-130

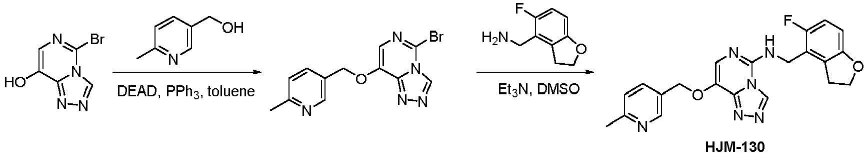

**HJM-130** (55 mg) was prepared as a white solid using (6-methylpyridin-3-yl)methanol as starting material according to the method similar to that for preparation example **100.**

¹H NMR (400 MHz, CD₃OD) δ 9.25 (s, 1H), 8.53 (d, *J =* 2.2 Hz, 1H), 7.91 (dd, *J* = 8.0, 2.3 Hz, 1H), 7.43 (s, 1H), 7.34 (d, *J =* 8.0 Hz, 1H), 6.87 - 6.79 (m, 1H), 6.62 (dd, *J =* 8.7, 3.8 Hz, 1H), 5.28 (s, 2H), 4.69 (s, 2H), 4.56 (t, *J =* 8.7 Hz, 2H), 3.33 (t, *J =* 8.5 Hz, 2H), 2.54 (s, 3H). LCMS [M+H]⁺ = 407.5.

### Preparation example 131: synthesis of compound HJM-131

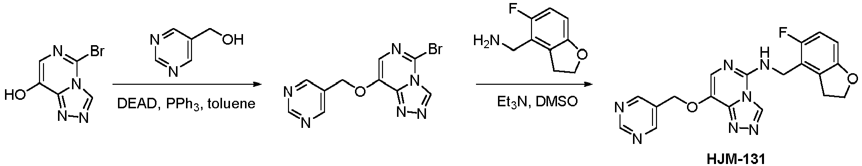

**HJM-131** (80 mg) was prepared as a white solid using pyrimidin-5-ylmethanol as starting material according to the method similar to that for preparation example **100.**

¹H NMR (400 MHz, DMSO-*d*₆) δ 9.43 (s, 1H), 9.19 (s, 1H), 8.93 (s, 2H), 8.26 (t, *J =* 5.1 Hz, 1H), 7.45 (s, 1H), 6.93 (dd, *J* = 10.3, 8.6 Hz, 1H), 6.69 (dd, *J* = 8.6, 3.9 Hz, 1H), 5.35 (s, 2H), 4.61 (d, *J* = 4.9 Hz, 2H), 4.53 (t, *J* = 8.7 Hz, 2H), 3.27 (t, *J* = 8.7 Hz, 2H). LCMS [M+H]⁺ = 394.2.

### Preparation example 132: synthesis of compound HJM-132

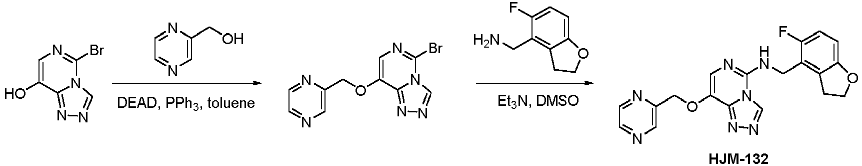

**HJM-132** (117 mg) was prepared as a white solid using pyrazin-2-ylmethanol as starting material according to the method similar to that for preparation example **100.**

¹H NMR (400 MHz, DMSO-*d*₆) δ 9.42 (s, 1H), 8.87 (d, *J =* 1.5 Hz, 1H), 8.69 - 8.64 (m, 2H), 8.22 (t, *J =* 5.0 Hz, 1H), 7.43 (s, 1H), 6.98 - 6.90 (m, 1H), 6.69 (dd, *J* = 8.7, 3.9 Hz, 1H), 5.42 (s, 2H), 4.60 (d, *J =* 5.0 Hz, 2H), 4.53 (t, *J* = 8.7 Hz, 2H), 3.27 (t, *J =* 8.8 Hz, 2H). LCMS [M+H]⁺ = 394.1.

### Preparation example 133: synthesis of compound HJM-133

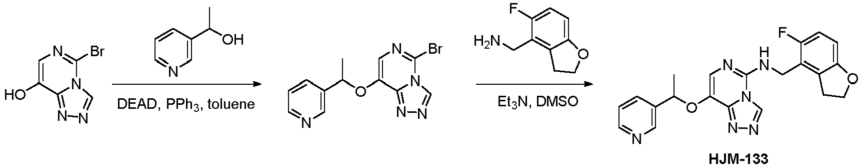

**HJM-133** (108 mg) was prepared as a white solid using 3-(1-hydroxyethyl)pyridine as starting material according to the method similar to that for preparation example **100.**

¹H NMR (400 MHz, DMSO-*d*₆) δ 9.38 (s, 1H), 8.63 (d, *J =* 2.3 Hz, 1H), 8.48 (dd, *J* = 4.8, 1.7 Hz, 1H), 8.18 (t, *J=* 5.0 Hz, 1H), 7.87 (dt, *J=* 7.9, 2.0 Hz, 1H), 7.38 (dd, *J =* 7.9, 4.8 Hz, 1H), 7.24 (s, 1H), 6.91 (dd, *J =* 10.2, 8.7 Hz, 1H), 6.68 (dd, *J =* 8.6, 3.9 Hz, 1H), 5.82 (q, *J =* 6.4 Hz, 1H), 4.55 (d, *J =* 5.0 Hz, 2H), 4.51 (t, *J =* 8.8 Hz, 2H), 3.22 (t, *J =* 8.7 Hz, 2H), 1.63 (d, *J =* 6.4 Hz, 3H). LCMS [M+H]⁺ = 407.4.

### Preparation example 134: synthesis of compound HJM-134

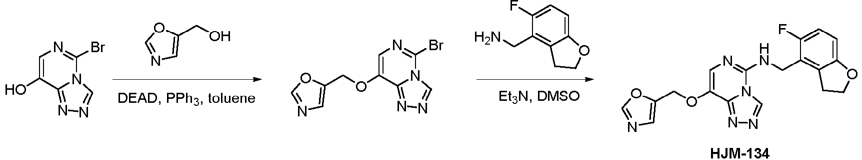

**HJM-134** (20 mg) was prepared as a white solid using oxazol-5-methanol as starting material according to the method similar to that for preparation example **100.**

¹H NMR (400 MHz, CD₃OD) δ 9.26 (s, 1H), 8.25 (s, 1H), 7.45 (s, 1H), 7.27 (s, 1H), 6.89 - 6.79 (m, 1H), 6.63 (dd, *J* = 8.7, 3.9 Hz, 1H), 5.35 (s, 2H), 4.70 (s, 2H), 4.57 (t, *J =* 8.7 Hz, 2H), 3.35 *(t, J=* 8.7 Hz, 2H). LCMS [M+H]⁺ = 383.2.

### Preparation example 135: synthesis of compound HJM-135

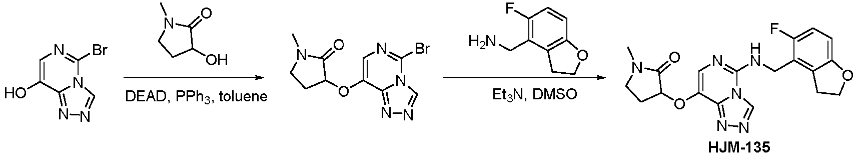

**HJM-135** (22 mg) was prepared as a white solid using 3-hydroxy-1-methylpyrrolidone as starting material according to the method similar to that for preparation example **100.**

¹H NMR (400 MHz, DMSO-*d*₆) δ 9.42 (s, 1H), 8.24 (br s, 1H), 7.43 (s, 1H), 6.93 (t, *J =* 9.3 Hz, 1H), 6.69 (dd, *J =* 8.4, 3.8 Hz, 1H), 5.18 (t, *J =* 6.9 Hz, 1H), 4.61 (s, 2H), 4.54 (t, *J =* 8.4 Hz, 2H), 3.47 - 3.38 (m, 1H), 3.32 - 3.23 (m, 3H), 2.79 (s, 3H), 2.58 - 2.50 (m, 1H), 2.11 - 1.94 (m, 1H). LCMS [M+H]⁺ = 399.1.

### Preparation example 136: synthesis of compound HJM-136

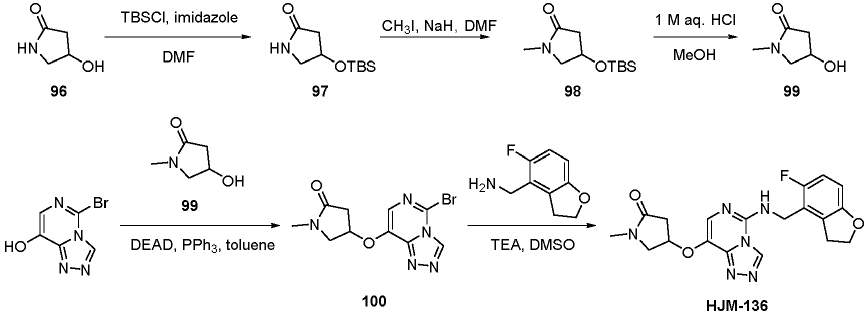

### Step 1:

Compound 4-hydroxypyrrolidone **96** (1.01 g, 10 mmol) and imidazole (1.02 g, 15 mmol) were dissolved in N,N-dimethylformamide (10 mL). Tert-butyl dimethylchlorosilane (1.8 g, 12 mmol) was added, and the mixture was stirred at room temperature under nitrogen protection overnight. The mixture was diluted with water, and extracted twice with ethyl acetate. The organic phases were combined, dried with anhydrous sodium sulfate, and filtered. The filtrate was concentrated to afford compound 97 (1.55 g, yield: 72.1%).

¹H NMR (400 MHz, CDCl₃) δ 6.33 (br s, 1H), 4.54 (tt, *J =* 6.7, 3.8 Hz, 1H), 3.58 (dd, *J =* 10.1, 6.1 Hz, 1H), 3.23 (dd, *J =* 10.0, 3.4 Hz, 1H), 2.53 (dd, *J =* 16.9, 6.8 Hz, 1H), 2.25 (dd, *J =* 16.9, 4.2 Hz, 1H), 0.87 (s, 9H), 0.06 (s, 6H).

### Step 2:

Compound **97** (1.55 g, 7.21 mmol) was dissolved in anhydrous *N,N*-dimethylformamide (10 mL). 60% sodium hydride (346 mg, 8.65 mmol) was added at 0 °C under nitrogen protection, and the mixture was stirred under an ice-water bath for 30 min. Iodomethane (0.9 mL, 14.42 mmol) was added, and the mixture was stirred at 0 °C for 2 h. The mixture was diluted with water, and extracted twice with ethyl acetate. The organic phases were combined, dried with anhydrous sodium sulfate, and filtered. The filtrate was concentrated to afford a crude product. The crude product was purified by silica gel column chromatography (petroleum ether/ethyl acetate, ethyl acetate: 0-30%) to afford compound **98** (1.33 g, yield: 80.6%).

¹H NMR (400 MHz, CDCl₃) δ 4.45 (tt, *J =* 7.0, 3.5 Hz, 1H), 3.57 (dd, *J =* 10.2, 6.2 Hz, 1H), 3.22 (dd, *J =* 10.2, 3.2 Hz, 1H), 2.84 (s, 1H), 2.60 (ddd, *J=* 16.9, 7.0, 0.9 Hz, 1H), 2.32 (dd, *J=* 17.0, 3.8 Hz, 1H), 0.87 (s, 9H), 0.06 (s, 6H).

### Step 3:

Compound **98** (1.33 g, 5.81 mmol) was dissolved in methanol (10 mL). 1 M HCl aqueous solution (5 mL, 5 mmol) was added, and the mixture was stirred at room temperature for 1 h. The mixture was diluted with saturated aqueous sodium bicarbonate solution, and concentrated to dryness to afford a residue. The residue was purified by silica gel column chromatography (dichloromethane/methanol, methanol: 0-10%) to afford compound 99 (500 mg, yield: 75.0%) as a colorless liquid.

¹H NMR (400 MHz, CDCl₃) δ 4.49 - 4.40 (m, 1H), 4.15 (d, *J =* 4.6 Hz, 1H), 3.61 (dd, *J* = 10.8, 5.7 Hz, 1H), 3.29 (dd, *J =* 10.9, 2.1 Hz, 1H), 2.82 (s, 3H), 2.65 - 2.58 (m, 1H), 2.32 (dd, *J =* 17.4, 2.4 Hz, 1H).

### Steps 4-5:

In the following steps, **HJM-136** (10 mg) was prepared as a white solid according to the method similar to that for preparation example **100.**

¹H NMR (400 MHz, CD₃OD) δ 9.27 (s, 1H), 7.43 (s, 1H), 6.88 - 6.79 (m, 1H), 6.63 (dd, *J =* 8.7, 3.9 Hz, 1H), 5.29 - 5.21 (m, 1H), 4.71 (s, 2H), 4.57 (t, *J =* 8.7 Hz, 2H), 3.89 (dd, *J =* 11.8, 5.6 Hz, 1H), 3.70 (dd, *J =* 11.7, 1.5 Hz, 1H), 3.36 (t, *J =* 8.7 Hz, 2H), 2.90 (s, 3H), 2.88 (dd, *J =* 17.6, 6.7 Hz, 1H), 2.63 (d, *J =* 17.9 Hz, 1H). LCMS [M+H]⁺ = 399.1.

### Preparation example 137: synthesis of compound HJM-137

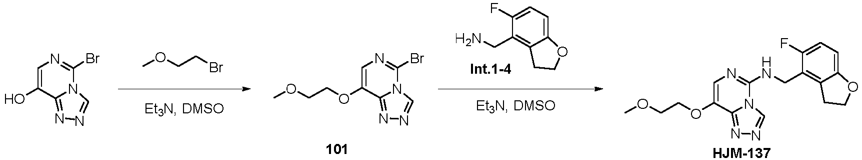

### Step 1:

5-Bromo-[1,2,4]triazolo[4,3-c]pyrimidine-8-ol (300 mg, 1.40 mmol), 1-bromo-2-methoxyethane (290 mg, 2.09 mmol) and triethylamine (423 mg, 4.19 mmol) were dissolved in dimethyl sulfoxide (5 mL), and the mixture was heated at 80 °C under nitrogen protection for 2 h. The mixture was cooled and lyophilized to afford a residue. The residue was purified by preparative thin layer chromatography (petroleum ether/ethyl acetate = 1:1) to afford compound **101** (80 mg, yield: 21.0%) as a yellow solid. LCMS [M+H]⁺ = 272.9.

### Step 2:

Compounds **101** (40 mg, 0.15 mmol), **Int.1-4** (36 mg, 0.22 mmol) and triethylamine (44 mg, 0.44 mmol) were dissolved in dimethyl sulfoxide (2 mL), and the mixture was stirred at room temperature for 1 h. The mixture was filtered, and the filtrates were combined, and purified by preparative HPLC to afford compound **HJM-137** (3 mg, yield: 5.7%) as a white solid.

¹H NMR (400 MHz, CD₃OD) δ 9.24 (s, 1H), 7.38 (s, 1H), 6.83 (t, *J =* 9.4 Hz, 1H), 6.62 (dd, *J* = 9.2, 3.8 Hz, 1H), 4.70 (s, 2H), 4.56 (t, *J =* 8.4 Hz, 2H), 4.30 (t, *J =* 4.6 Hz, 2H), 3.79 (t, *J =* 4.8 Hz, 2H), 3.42 (s, 3H), 3.34 (t, *J =* 8.6 Hz, 2H). LCMS [M+H]⁺ =360.0.

### Preparation example 138: synthesis of compound HJM-138

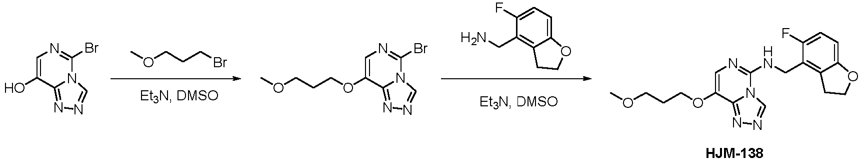

**HJM-138** (10 mg) was prepared as a white solid using 1-bromo-3-methoxypropane as starting material according to the method similar to that for preparation example **137.**

¹H NMR (400 MHz, CD₃OD) δ 9.24 (s, 1H), 7.34 (s, 1H), 6.83 (dd, *J* = 10.2, 8.6 Hz, 1H), 6.62 (dd, *J =* 8.7, 3.9 Hz, 1H), 4.69 (s, 2H), 4.56 (t, *J =* 8.7 Hz, 2H), 4.23 (t, *J =* 6.3 Hz, 2H), 3.62 (t, *J =* 6.2 Hz, 2H), 3.35 (s, 3H), 3.34 (t, *J =* 8.3 Hz, 2H), 2.09 (p, *J =* 6.2 Hz, 2H). LCMS [M+H]⁺ =374.5.

### Preparation example 139: synthesis of compound HJM-139

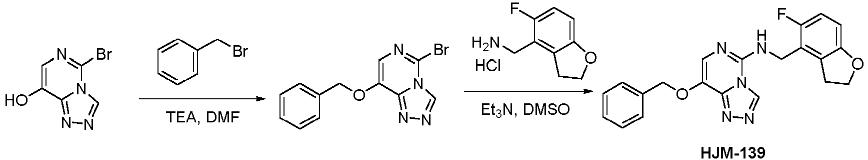

**HJM-139** (95 mg) was prepared as a white solid using benzyl bromide as starting material according to the method similar to that for preparation example **137.**

¹H NMR (400 MHz, DMSO-*d*₆) δ 9.41 (s, 1H), 7.47 (d, *J =* 7.4 Hz, 2H), 7.40 (t, *J =* 7.3 Hz, 2H), 7.37 - 7.32 (m, 2H), 6.93 (t, *J=* 9.4 Hz, 1H), 6.69 (dd, *J =* 8.6, 3.9 Hz, 1H), 6.04 (br s, 1H), 5.24 (s, 2H), 4.59 (s, 2H), 4.53 (t, *J =* 8.7 Hz, 2H), 3.26 (t, *J* = 8.7 Hz, 2H). LCMS [M+H]⁺ =392.1.

### Preparation example 140: synthesis of compound HJM-140

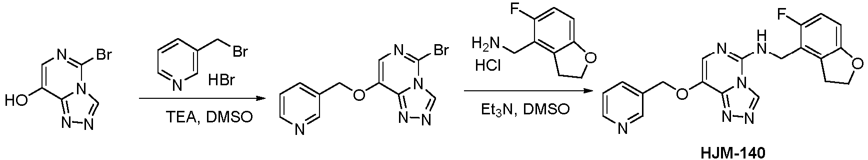

**HJM-140** (3 mg) was prepared as a white solid using 3-bromomethylpyridine hydrobromide as starting material according to the method similar to that for preparation example **137.**

¹H NMR (400 MHz, CD₃OD) δ 9.26 (s, 1H), 8.69 (s, 1H), 8.52 (d, *J* = 4.9 Hz, 1H), 8.05 (d, *J* = 8.0 Hz, 1H), 7.49 (dd, *J* = 7.9, 4.9 Hz, 1H), 7.45 (s, 1H), 6.88 - 6.80 (m, 1H), 6.63 (dd, *J* = 8.6, 3.8 Hz, 1H), 5.34 (s, 2H), 4.70 (s, 2H), 4.57 (t, *J =* 8.7 Hz, 2H), 3.34 (t, *J* = 8.7 Hz, 2H). LCMS [M+H]⁺ = 393.1.

### Preparation example 141: synthesis of compound HJM-141

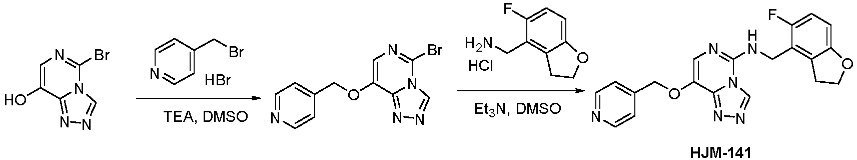

**HJM-141** (15 mg) was prepared as a white solid using 4-bromomethylpyridine hydrobromide as starting material according to the method similar to that for preparation example 137.

¹H NMR (400 MHz, CD₃OD) δ 9.27 (s, 1H), 8.59 - 8.51 (m, 2H), 7.65 - 7.58 (m, 2H), 7.42 (s, 1H), 6.88 - 6.79 (m, 1H), 6.63 (dd, *J* = 8.7, 3.9 Hz, 1H), 5.36 (s, 2H), 4.70 (s, 2H), 4.56 (t, *J =* 8.7 Hz, 2H), 3.35 (t, *J =* 8.7 Hz, 2H). LCMS [M+H]⁺ = 393.1.

### Preparation example 142: synthesis of compound HJM-142

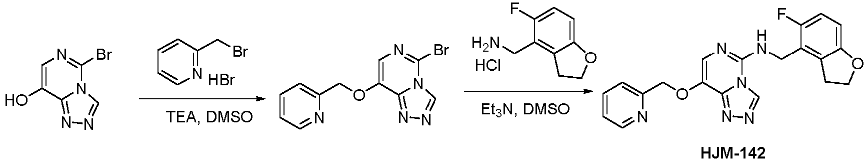

**HJM-142** (10 mg) was prepared as a white solid using 2-bromomethylpyridine hydrobromide as starting material according to the method similar to that for preparation example 137.

¹H NMR (400 MHz, CD₃OD) δ 9.26 (s, 1H), 8.55 (d, *J* = 4.5 Hz, 1H), 7.90 (td, *J* = 7.7, 1.8 Hz, 1H), 7.77 (d, *J* = 7.9 Hz, 1H), 7.43 - 7.36 (m, 2H), 6.88 - 6.78 (m, 1H), 6.63 (dd, *J* = 8.6, 3.9 Hz, 1H), 5.35 (s, 2H), 4.69 (s, 2H), 4.56 (t, *J* = 8.7 Hz, 2H), 3.34 (t, *J =* 8.7 Hz, 2H). LCMS [M+H]⁺ = 393.0.

### Preparation example 143: synthesis of compound HJM-143

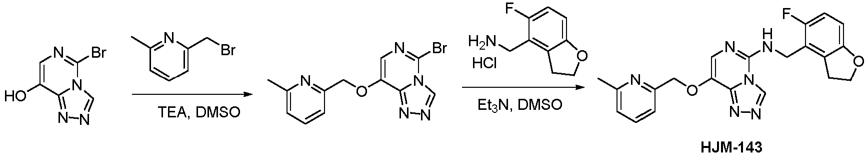

**HJM-143** (11 mg) was prepared as a white solid using 2-bromomethyl-6-methylpyridine as starting material according to the method similar to that for preparation example **137.**

¹H NMR (400 MHz, CD₃OD) δ 9.34 (s, 1H), 8.49 (t, *J =* 7.9 Hz, 1H), 8.03 (d, *J* = 7.9 Hz, 1H), 7.90 (d, *J =* 8.0 Hz, 1H), 7.64 (s, 1H), 6.84 (dd, *J =* 10.2, 8.7 Hz, 1H), 6.63 (dd, *J =* 8.7, 3.9 Hz, 1H), 5.59 (s, 2H), 4.73 (s, 2H), 4.58 (t, *J =* 8.7 Hz, 2H), 3.37 (t, *J =* 8.7 Hz, 2H), 2.88 (s, 3H). LCMS [M+H]⁺ = 407.5.

### Preparation example 144: synthesis of compound HJM-144

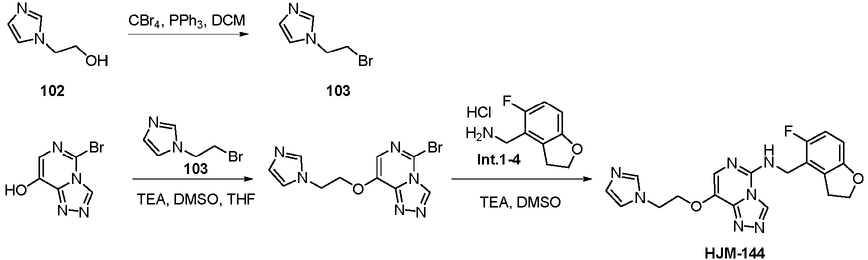

### Step 1:

Compound 1-(2-hydroxyethyl) imidazole **102** (4.08 g, 36.42 mmol) and triphenyl phosphine (10.5 g, 40.06 mmol) were dissolved in dichloromethane (100 mL). Carbon tetrabromide (13.25 g, 40.06 mmol) was added at 0 °C under nitrogen protection in batches. After the addition, the mixture was stirred at room temperature for 3 h. The mixture was concentrated to afford a crude product. The crude product was purified by silica gel column chromatography (petroleum ether/ethyl acetate, ethyl acetate: 0-50%) to afford compound 103 (2.5 g, yield: 39.2%) as a colorless liquid.

¹H NMR (400 MHz, DMSO-*d*₆) δ 7.68 (s, 1H), 7.24 (s, 1H), 6.90 (s, 1H), 4.38 (t, *J =* 6.1 Hz, 2H), 3.81 (t, *J =* 6.1 Hz, 2H).

### Steps 2-3:

In the following steps, **HJM-144** (3 mg) was prepared as a white solid according to the method similar to that for preparation example **137.**

¹H NMR (400 MHz, CD₃OD) δ 9.28 (s, 1H), 9.19 (s, 1H), 7.82 (s, 1H), 7.59 (s, 1H), 7.40 (s, 1H), 6.83 (t, *J =* 9.4 Hz, 1H), 6.62 (dd, *J =* 8.7, 3.8 Hz, 1H), 4.75 (t, *J =* 4.7 Hz, 2H), 4.69 (s, 2H), 4.59 - 4.52 (m, 4H), 3.35 (t, *J =* 8.7 Hz, 2H). LCMS [M+H]⁺ = 396.0.

### Preparation example 145: synthesis of compound HJM-145

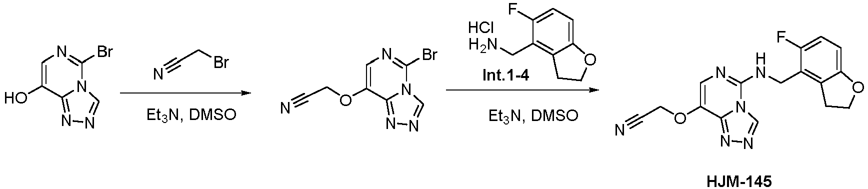

**HJM-145** (9 mg) was prepared as a white solid using bromoacetonitrile as starting material according to the method similar to that for preparation example **137.**

¹H NMR (400 MHz, DMSO-*d*₆) δ 9.44 (s, 1H), 8.39 (t, *J* = 5.4 Hz, 1H), 7.51 (s, 1H), 6.94 (t, *J =* 9.5 Hz, 1H), 6.70 (dd, *J* = 8.7, 3.8 Hz, 1H), 5.32 (s, 2H), 4.63 (d, *J =* 5.0 Hz, 2H), 4.54 (t, *J =* 8.7 Hz, 2H), 3.29 (t, *J =* 8.7 Hz, 2H). LCMS [M+H]⁺ = 341.1.

### Preparation example 146: synthesis of compound HJM-146

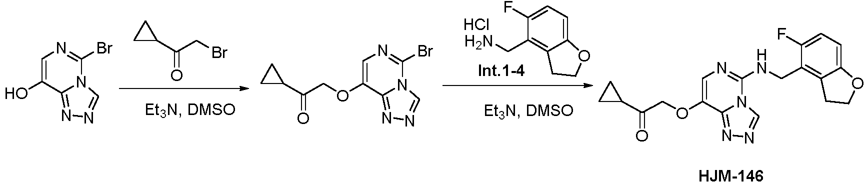

**HJM-146** (37 mg) was prepared as a white solid using 2-bromo-1-cyclopropyl ethanone as starting material according to the method similar to that for preparation example **137.**

¹H NMR (400 MHz, DMSO-*d*₆) δ 9.42 (s, 1H), 8.21 (t, *J =* 5.0 Hz, 1H), 7.26 (s, 1H), 6.98 - 6.90 (m, 1H), 6.69 (dd, *J* = 8.6, 3.9 Hz, 1H), 5.12 (s, 2H), 4.59 (d, *J =* 4.8 Hz, 2H), 4.53 (t, *J* = 8.7 Hz, 2H), 3.27 (t, *J =* 8.8 Hz, 2H), 2.27 - 2.20 (m, 1H), 1.02 - 0.87 (m, 4H). LCMS [M+H]⁺ = 384.2.

### Preparation example 147: synthesis of compound HJM-147

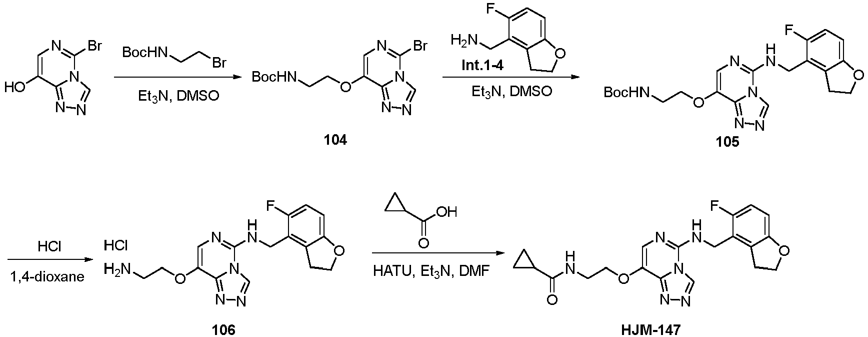

### Step 1:

5-Bromo-[1,2,4]triazolo[4,3-c]pyrimidine-8-ol (400 mg, 1.86 mmol), tert-butyl 2-bromoethylcarbamate (500 mg, 2.23 mmol) and triethylamine (564 mg, 5.58 mmol) were dissolved in dimethyl sulfoxide (3 mL), and the mixture was heated at 80 °C under nitrogen protection for 3 h. The reaction mixture containing compound **104** was cooled, and directly used in the next step. LCMS [M+H]⁺ = 358.0.

### Step 2:

The above reaction mixture containing compound 104 was added to a solution of **Int.1-4** (310 mg, 1.86 mmol) and triethylamine (44 mg, 0.44 mmol) in dimethyl sulfoxide (2 mL), and the mixture was heated at 80 °C for 2 h. The mixture was cooled, and purified by reversed-phase column chromatography to afford compound **105** (105 mg, yield: 12.7 %) as a yellow oil. LCMS [M+H]⁺ = 445.1.

### Step 3:

Compound 105 (105 mg, 0.24 mmol) was added to a solution of 4 M HCl in 1,4-dioxane (3 mL), and the mixture was stirred at room temperature for 2 h. The mixture was concentrated to afford compound 106 (80 mg, yield: 98%) as a white solid.

### Step 4:

Compound **106** (80 mg, 0.23 mmol), cyclopropylcarboxylic acid (20 mg, 0.23 mmol) and triethylamine (70 mg, 0.69 mmol) were dissolved in N,N-dimethylformamide (3 mL). O-(7-azabenzotriazol-1-yl)-*N,N,N',N'*-tetramethyl uronium hexafluorophosphate (HATU, 87 mg, 0.23 mmol) was added, and the mixture was stirred at room temperature for 2 h. The mixture was purified by preparative HPLC to afford compound **HJM-147** (11 mg, yield: 11.6 %) as a white solid.

¹H NMR (400 MHz, CD₃OD) δ 9.37 (s, 1H), 7.79 (s, 1H), 6.84 (t, *J =* 9.4 Hz, 1H), 6.64 (dd, *J* = 8.6, 3.8 Hz, 1H), 4.74 (s, 2H), 4.58 (t, *J =* 8.7 Hz, 2H), 4.28 (t, *J =* 5.3 Hz, 2H), 3.66 (t, *J =* 5.3 Hz, 2H), 3.38 (t, *J* = 8.7 Hz, 2H), 1.64 - 1.54 (m, 1H), 0.87 - 0.72 (m, 4H). LCMS [M+H]⁺ = 413.3.

### Preparation example 148: synthesis of compound HJM-148

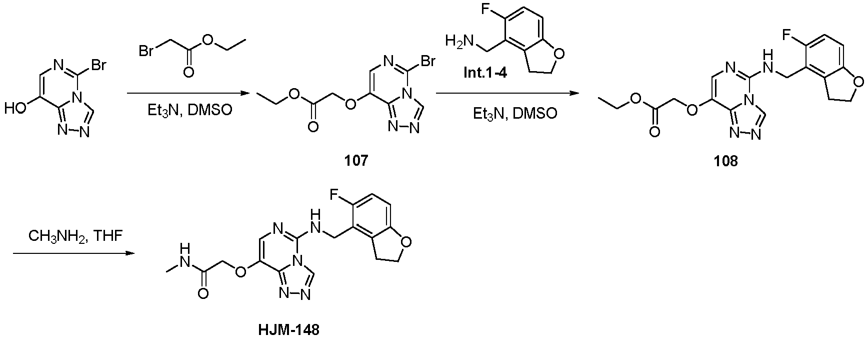

### Step 1:

5-Bromo-[1,2,4]triazolo[4,3-c]pyrimidine-8-ol (150 mg, 0.697 mmol), ethyl bromoacetate (173 mg, 1.04 mmol) and triethylamine (140 mg, 1.39 mmol) were dissolved in dimethyl sulfoxide(5 mL), and the mixture was heated at 80 °C for 2 h. The mixture was cooled, and diluted by adding water. Solid was precipitated. The resulting solid was washed twice with water and dried to afford crude compound 107 (87 mg, yield: 41.8%) as a brown solid.

¹H NMR (400 MHz, DMSO-*d*₆) δ 9.47 (s, 1H), 7.57 (s, 1H), 5.14 (s, 2H), 4.20 (q, *J =* 7.1 Hz, 2H), 1.23 (t, *J =* 7.1 Hz, 3H). LCMS [M+H]⁺ = 300.9.

### Step 2:

Compound **107** (87 mg, 0.29 mmol), **Int.1-4** (53 mg, 0.319 mmol) and triethylamine (59 mg, 0.58 mmol) were dissolved in dimethyl sulfoxide (3 mL), and the mixture was heated at 50 °C for 1 h. The mixture was cooled, diluted with water, and extracted twice with ethyl acetate. The organic phases were combined, dried with anhydrous sodium sulfate, and filtered. The filtrate was concentrated to afford a crude product. The crude product was purified by preparative thin layer chromatography to afford compound 108 (100 mg, yield: 90 %) as a white solid.

¹H NMR (400 MHz, DMSO-*d*₆) δ 9.41 (s, 1H), 8.21 (t, *J =* 5.1 Hz, 1H), 7.31 (s, 1H), 6.93 (t, *J =* 9.5 Hz, 1H), 6.69 (dd, *J =* 8.6, 3.9 Hz, 1H), 4.94 (s, 2H), 4.60 (d, *J =* 5.0 Hz, 2H), 4.53 (t, *J =* 8.7 Hz, 2H), 4.17 (q, *J =* 7.1 Hz, 2H), 3.26 (t, *J =* 8.7 Hz, 2H), 1.20 (t, *J =* 7.1 Hz, 3H). LCMS [M+H]⁺ = 388.1.

### Step 3:

Compound **108** (40 mg, 0.103 mmol) was added to a solution of 2M methylamine in tetrahydrofuran (2 mL). The mixture was sealed, and heated at 80 °C for 2 h. The mixture was concentrated to afford a crude product. The crude product was purified by preparative HPLC to afford compound **HJM-148** (13 mg, yield: 34.2%) as a white solid.

¹H NMR (400 MHz, DMSO-*d*₆) δ 9.42 (s, 1H), 8.25 (t, *J =* 4.0 Hz, 1H), 8.01 (d, *J =* 4.0 Hz, 1H), 7.33 (s, 1H), 6.93 (dd, *J =* 10.3, 8.7 Hz, 1H), 6.69 (dd, *J =* 8.6, 3.9 Hz, 1H), 4.65 (s, 2H), 4.60 (d, *J =* 4.4 Hz, 2H), 4.53 (t, *J* = 8.7 Hz, 2H), 3.28 (t, *J* = 8.9 Hz, 2H), 2.66 (d, *J =* 4.7 Hz, 3H). LCMS [M+H]⁺ = 373.1.

### Preparation example 149: synthesis of compound HJM-149

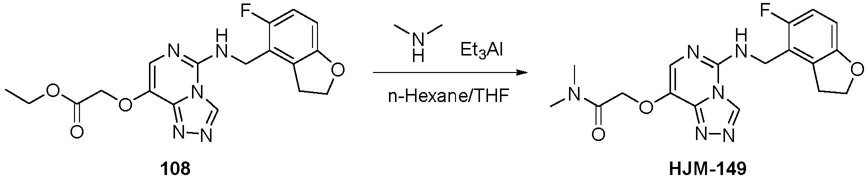

At 0 °C, a solution of 2 M triethylaluminum in n-hexane (1 mL) was added dropwise to a solution of 2 M dimethylamine in tetrahydrofuran (1 mL) under nitrogen protection. After the dropwise addition, the mixture was warmed to room temperature and stirred for 30 min. A solution of compound **108** (40 mg, 0.1 mmol) in tetrahydrofuran (2 mL) was slowly added dropwise. After the dropwise addition, the mixture was heated to 80 °C and stirred for 5 h. The mixture was cooled to 0 °C, and methanol (3 mL) was added dropwise to quench the reaction. The mixture was diluted with water and extracted twice with ethyl acetate. The organic phases were combined, dried with anhydrous sodium sulfate, and filtered. The filtrate was concentrated to afford a crude product. The crude product was purified by preparative HPLC to afford compound **HJM-149** (1.2 mg, yield: 3%) as a white solid.

¹H NMR (400 MHz, CD₃OD) δ 9.25 (s, 1H), 7.38 (s, 1H), 6.83 (dd, *J =* 10.2, 8.7 Hz, 1H), 6.62 (dd, *J =* 8.6, 3.9 Hz, 1H), 5.02 (s, 2H), 4.69 (s, 2H), 4.56 (t, *J =* 8.7 Hz, 2H), 3.35 (t, *J =* 8.7 Hz, 2H), 3.10 (s, 3H), 2.97 (s, 3H). LCMS [M+H]⁺ = 387.5.

### Preparation example 150: synthesis of compound HJM-150

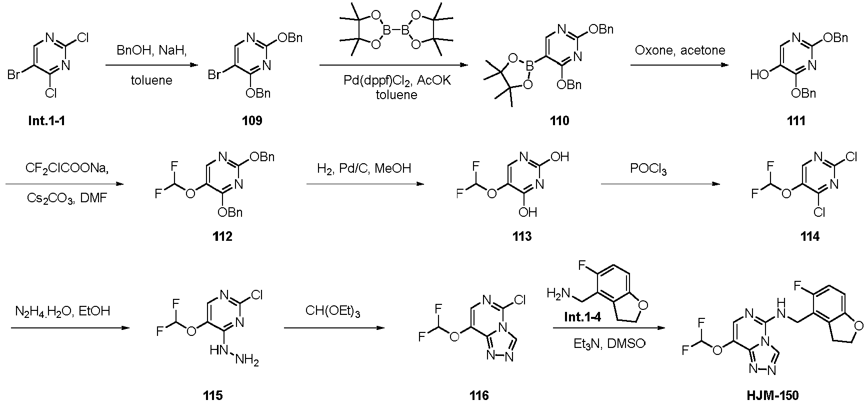

### Step 1:

Under nitrogen protection, 60% sodium hydride (876 mg, 21.94 mmol) was added portionwise to a solution of benzyl alcohol (4.8 g, 43.88 mmol) in anhydrous toluene (50 mL), and the mixture was heated at 80 °C for 2 h. The mixture was cooled to room temperature. A solution of 5-bromo-2,4-dichloropyrimidine **Int.1-1** (5 g, 21.94 mmol) in anhydrous toluene (10 mL) was added dropwise, and the mixture was stirred at room temperature overnight. The mixture was concentrated to afford a crude product. The crude product was purified by silica gel column chromatography (petroleum ether/ethyl acetate, ethyl acetate: 0-50%) to afford compound **109** (3 g, yield: 36.8%) as a white solid. LCMS [M+H]⁺ = 371.3.

### Step 2:

Compound **109** (1 g, 2.69 mmol) and bis(pinacolato)diboron (1 g, 4.04 mmol) were dissolved in toluene (10 mL). Potassium acetate (793 mg, 8.08 mmol) and Pd(dppf)Cl₂ (0.2 g, 0.27 mmol) were added, and the mixture was heated at 100 °C for 2 h. The mixture was cooled, and concentrated to afford a crude product. The crude product was purified by silica gel column chromatography (petroleum ether/ethyl acetate, ethyl acetate: 0-30%) to afford compound **110** (500 mg, yield: 44.4%) as a white solid. LCMS [M+H]⁺ = 419.5.

### Step 3:

Compound **110** (480 mg, 1.15 mmol) was dissolved in acetone (5 mL), and oxone (1.1 g, 1.72 mmol) was added portionwise at 0 °C. After the addition, the mixture was warmed to room temperature and stirred for 2 h. The mixture was concentrated to afford a crude product. The crude product was purified by silica gel column chromatography (petroleum ether/ethyl acetate, ethyl acetate: 0-50%) to afford compound **111** (300 mg, yield: 84.8%) as a white solid. LCMS [M+H]⁺ = 309.4.

### Step 4:

Sodium difluorochloroacetate (207 mg, 1.36 mmol) and cesium carbonate (371 mg, 1.14 mmol) were added to a solution of compound **111** (280 mg, 0.91 mmol) in N,N-dimethylformamide (5 mL), and the mixture was heated at 100 °C for 2 h. The mixture was cooled, poured into water, and extracted twice with ethyl acetate. The organic phases were combined, dried with anhydrous sodium sulfate, and filtered. The filtrate was concentrated to afford a crude product. The crude product was purified by silica gel column chromatography (petroleum ether/ethyl acetate, ethyl acetate: 0-30%) to afford compound **112** (200 mg, yield: 61.2%) as a white solid. LCMS [M+H]⁺ = 359.4.

### Step 5:

Compound **112** (200 mg, 0.56 mmol) was dissolved in methanol (5 mL), and 10% Pd/C (20 mg) was added under nitrogen protection. The atmosphere was replaced three times with hydrogen gas, and the mixture was reacted under a hydrogen balloon for 2 h. The mixture was filtered, and the filter cake was washed with methanol for three times. The filtrates were combined, and concentrated to afford compound **113** (102 mg, yield: 99%) as a white solid. LCMS [M+H]⁺ = 179.1.

### Step 6:

Compound **113** (100 mg, 0.56 mmol) was added to phosphorus oxychloride (2 mL), and the mixture was heated at 105 °C for 12 h. The mixture was cooled, and concentrated to afford a crude product. The crude product was purified by silica gel column chromatography (petroleum ether/ethyl acetate, ethyl acetate: 0-20%) to afford compound **114** (60 mg, yield: 49.7%) as a yellow solid. LCMS [M-H]⁻ = 213.5.

### Step 7:

Compound **114** (60 mg, 0.28 mmol) was dissolved in ethanol (1 mL). A solution of 85% hydrazine hydrate (17.6 mg, 0.30 mmol) in ethanol (1 mL) was added, and the mixture was reacted at room temperature for 2 h. The mixture was concentrated to afford a crude product. The crude product was purified by silica gel column chromatography (dichloromethane/methanol, methanol: 0-5%) to afford compound **115** (40 mg, yield: 68.1%) as a yellow solid. LCMS [M+H]⁺ = 211.0.

### Step 8:

Compound **115** (40 mg, 0.19 mmol) was added to triethyl orthoformate (2 mL), and the mixture was heated at 120 °C and stirred for 2 h. The mixture was cooled, and concentrated to afford a crude product. The crude product was purified by silica gel column chromatography (petroleum ether/ethyl acetate, ethyl acetate: 0-50%) to afford compound **116** (25 mg, yield: 59.7%) as a brown solid. LCMS [M+H]⁺ = 221.3.

### Step 9:

Compound **116** (25 mg, 0.11 mmol), **Int.1-4** (34 mg, 0.20 mmol) and triethylamine (34 mg, 0.34 mmol) were dissolved in dimethyl sulfoxide (1 mL), and the mixture was stirred at room temperature for 2 h. The mixture was purified by preparative HPLC to afford compound **HJM-150** (10 mg, yield: 25.1%) as a white solid.

¹H NMR (400 MHz, CD₃OD) δ 9.29 (s, 1H), 7.67 (s, 1H), 6.95 (t, *J =* 73.7 Hz, 1H), 6.85 (t, *J =* 9.5 Hz, 1H), 6.64 (dd, *J =* 8.7, 3.9 Hz, 1H), 4.75 (s, 2H), 4.57 (t, *J =* 8.7 Hz, 2H), 3.35 (t, *J =* 8.8 Hz, 2H). LCMS [M+H]⁺ = 352.1.

### Preparation example 151: synthesis of compound HJM-151

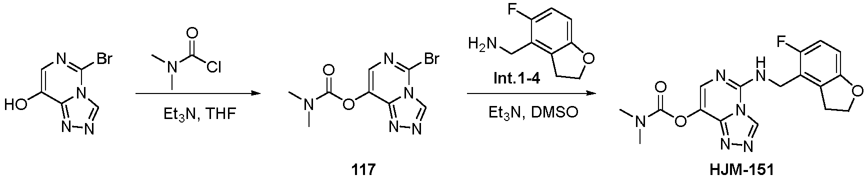

### Step 1:

5-Bromo-[1,2,4]triazolo[4,3-c]pyrimidine-8-ol (100 mg, 0.47 mmol), dimethylcarbamic chloride (50 mg, 0.47 mmol) and triethylamine (141 mg, 1.40 mmol) were dissolved in tetrahydrofuran (3 mL), and the mixture was stirred at room temperature for 4 h. The mixture was concentrated to afford compound **117,** which was directly used in the next step. LCMS [M+H]⁺ = 285.9.

### Step 2:

The above crude product compound **117, Int.1-4** (78 mg, 0.47 mmol) and triethylamine (141 mg, 1.40 mmol) were dissolved in dimethyl sulfoxide (2 mL), and the mixture was heated at 60 °C for 1 h. The mixture was cooled and purified by preparative HPLC to afford compound **HJM-151** (2 mg, yield: 1.2%) as a white solid.

¹H NMR (400 MHz, CD₃OD) δ 9.28 (s, 1H), 7.63 (s, 1H), 6.85 (t, *J =* 9.5 Hz, 1H), 6.64 (dd, *J* = 8.7, 3.9 Hz, 1H), 4.76 (s, 2H), 4.57 (t, *J =* 8.7 Hz, 2H), 3.36 (t, *J =* 8.7 Hz, 3H), 3.19 (s, 3H), 3.03 (s, 3H). LCMS [M+H]⁺ = 373.3.

### Preparation example 152: synthesis of compound HJM-152

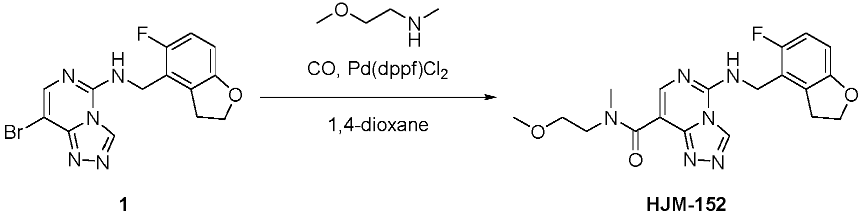

**HJM-152** (22 mg) was prepared as a white solid using *N*-(2-methoxyethyl)methylamine as starting material according to the method similar to that for preparation example **25.**

¹H NMR (400 MHz, CD₃OD) δ 9.30 (s, 1H), 7.87 (s, 1H), 6.86 (dd, *J =* 10.2, 8.7 Hz, 1H), 6.65 (dd, *J =* 8.7, 3.9 Hz, 1H), 4.81 (s, 2H), 4.58 (t, *J =* 8.7 Hz, 2H), 3.85 - 3.63 (m, 2H), 3.59 - 3.43 (m, 2H), 3.43 - 3.22 (m, 5H), 3.21 - 2.99 (m, 3H). LCMS [M+H]⁺ = 401.1.

### Preparation example 153: synthesis of compound HJM-153

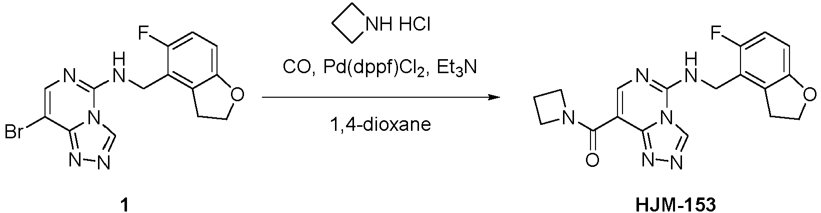

8-Bromo-N-((5-fluoro-2,3-dihydrobenzofuran-4-yl)methyl)-[1,2,4]triazolo[4,3-c]pyrimidine-5-amine 1 (40 mg, 0.11 mmol), cyclobutylamine hydrochloride (103 mg, 1.1 mmol) and triethylamine (167 mg, 1.65 mmol) were dissolved in 1,4-dioxane (2 mL). Pd(dppf)Cl₂ (15 mg, 0.02 mmol) was added. The atmosphere was replaced three times with CO, and the mixture was heated at 100 °C under a CO balloon for 2 h. The mixture was cooled, and concentrated to afford a crude product. The crude product was purified by preparative HPLC to afford compound **HJM-153** (2 mg, yield: 5.0%) as a white solid.

¹H NMR (400 MHz, CD₃OD) δ 9.31 (s, 1H), 8.07 (s, 1H), 6.86 (t, *J =* 9.5 Hz, 1H), 6.65 (dd, *J* = 8.7, 3.9 Hz, 1H), 4.83 (s, 2H), 4.58 (t, *J =* 8.8 Hz, 2H), 4.36 - 4.18 (m, 4H), 3.37 (t, *J* = 9.0 Hz, 2H), 2.42 - 2.30 (m, 2H). LCMS [M+H]⁺ = 369.0.

### Pharmacological activity and application

As the core subunit of the PRC2 complex, EED is necessary for PRC2 to function although EED has no enzymatic activity. EED can specifically bind to H3K27me3, the catalytic product of PRC2, thereby helping PRC2 localize to genomic loci where H3K27me3 already exists on the one hand, and allosterically activating the catalytic activity of EZH2 or EZH1 on the other hand. This positive feedback mechanism enables H3K27me3 to extensively modify chromatin and to be efficiently transmitted between cell generations during the cell cycle, maintaining cell identity and epigenome stability. Small molecules targeting EED can directly bind to EED to allosterically inhibit the enzymatic activity of PRC2. This inhibition mechanism is different from that of PRC2 enzymatic activity inhibitors targeting EZH2, thereby providing a unique new perspective for the development of PRC2 inhibitors, and being able to effectively treat tumors that are resistant to PRC2 enzymatic activity inhibitors. The compounds of the present disclosure can be used to allosterically inhibit the enzymatic activity of PRC2, have a blocking effect on tumor cell proliferation, and can be used to treat EED- or PRC2-mediated diseases or symptoms, especially tumors.

The activity of the compounds of the present disclosure can be demonstrated using any of the following analytical methods. The ability of the compounds of the present disclosure to inhibit PRC2 activity in cells was assessed by analyzing the methylation of histone H3 lysine 27 in human tumor cells. The ability of the compounds of the disclosure to inhibit tumors was assessed by analyzing their effect on the proliferation of PRC2-specifically dependent tumor cells.

### (1) Cellular H3K27 methylation assay

Human cervical cancer cells HeLa were cultured in petri dishes in a sterile incubator with 95% relative humidity and 5% CO₂ at 37 °C. The culture medium was DMEM (Gibco, product No. 12100046) containing 10% fetal bovine serum (FBS, Gibco, product No. 10099141) and 0.1% penicillin/streptomycin solution (P/S). Cells in exponential growth phase were seeded into a 24-well plate (Greiner, product No. 662160) at a density of 2×10⁴ cells/well with 1 mL of medium per well. Subsequently, different concentrations of compounds disclosed herein were added to the wells that had seeded with cells (6 concentrations were set for each compound at 3-fold serial dilution with the highest detection concentration of 10 µM or 1 µM), and the final concentration of DMSO was 0.1 %. After 3 days, cells were lysed in SDS lysis solution (1.45 g SDS, 0.2 g Tris base, 6 mL glycerol, 20 mg bromophenol blue, 310 mg DTT, ddH₂O to 40 mL). H3K27me3 levels were detected, quantified and analyzed by standard molecular biology technique Western Blot immunoblotting. The cell lysate was boiled at 95 °C for 12 minutes. After mixing and centrifugation, the total cell protein in the lysate was separated by SDS-PAGE. The blot was transferred to NC membrane (GE, product No.: 10600002), incubated and blocked in 5% nonfat dry milk-TBST (0.075% Tween-20) at room temperature for 60 minutes, and incubated with primary antibody at 4 °C overnight. Antibodies used were: anti-H3K27me3 (#9733S, 1:1000), anti-H3 (#9715S, 1:1000) and anti-EZH2 (#5246S, 1:1000) (all purchased from Cell Signaling Technology). Washing was carried out three times with TBST at room temperature for 10 minutes per time. After that, incubation was carried out in 5% nonfat dry milk-TBST containing secondary antibody (purchased from Pierce, product No.: 31460, 1:5000) at room temperature for 60 minutes. Washing was carried out three times with TBST at room temperature for 10 minutes per time. Finally, development was carried out using a chemiluminescent substrate, and quantification of imaging and exposure data was carried out on a GE imager 680. The data were fitted to dose-response curves using GraphPad prisim5 to obtain IC₅₀ values of the assay compounds. EED226 was used as a positive control in each assay to eliminate the interference of batch-to-batch error.

**Table 1 shows the IC₅₀ values of some compounds of the present disclosure against cellular H3K27 methylation.**

| Compound No. | PD IC₅₀ (uM) |
|---|---|
| HJM-001 | 0.4837 |
| HJM-002 | 0.0988 |
| HJM-003 | 0.0543 |
| HJM-004 | 0.1397 |
| HJM-005 | 0.454 |
| HJM-006 | 0.0487 |
| HJM-007 | 0.0499 |
| HJM-008 | 0.1119 |
| HJM-009 | 0.563 |
| HJM-010 | 0.0446 |
| HJM-011 | 0.0338 |
| HJM-012 | 0.1122 |
| HJM-013 | 0.2247 |
| HJM-014 | 0.0437 |
| HJM-015 | 0.0254 |
| HJM-016 | 0.0347 |
| HJM-017 | 0.0263 |
| HJM-018 | 0.0592 |
| HJM-019 | 0.0294 |
| HJM-020 | 0.0398 |
| HJM-021 | 0.2418 |
| HJM-022 | 0.2841 |
| HJM-023 | 1.159 |
| HJM-024 | 1.109 |
| HJM-025 | 0.0433 |
| HJM-026 | 0.0756 |
| HJM-027 | 0.0895 |
| HJM-028 | 0.0953 |
| HJM-029 | 0.0822 |
| HJM-030 | 0.0267 |
| HJM-031 | 0.0396 |
| HJM-032 | 0.3174 |
| HJM-033 | 0.0259 |
| HJM-034 | 0.0995 |
| HJM-035 | 0.0536 |
| HJM-036 | 0.1604 |
| HJM-037 | 0.0647 |
| HJM-038 | 0.1871 |
| HJM-039 | 0.0672 |
| HJM-040 | 0.1748 |
| HJM-041 | 0.3337 |
| HJM-042 | 0.0605 |
| HJM-043 | 0.1149 |
| HJM-044 | 0.2877 |
| HJM-045 | 0.013 |
| HJM-046 | 2.0 |
| HJM-047 | 7.0 |
| HJM-048 | 1.621 |
| HJM-049 | 0.2959 |
| HJM-050 | 0.0505 |
| HJM-051 | 0.1673 |
| HJM-052 | 0.01903 |
| HJM-053 | 0.02882 |
| HJM-054 | 0.03112 |
| HJM-055 | 0.00574 |
| HJM-056 | 0.01281 |
| HJM-057 | 0.0405 |
| HJM-058 | 0.131 |
| HJM-059 | 0.03854 |
| HJM-060 | 0.1414 |
| HJM-061 | 0.1097 |
| HJM-062 | 0.445 |
| HJM-063 | 0.6835 |
| HJM-064 | 0.1805 |
| HJM-065 | 0.3627 |
| HJM-066 | 0.2275 |
| HJM-067 | 0.0211 |
| HJM-068 | 0.1377 |
| HJM-069 | 0.3908 |
| HJM-070 | 0.08306 |
| HJM-071 | 0.07277 |
| HJM-072 | 0.03002 |
| HJM-073 | 0.03927 |
| HJM-074 | 0.5535 |
| HJM-075 | 0.6902 |
| HJM-076 | 0.00636 |
| HJM-077 | 0.01495 |
| HJM-078 | 0.01212 |
| HJM-079 | 0.0153 |
| HJM-080 | 0.03505 |
| HJM-081 | 0.0776 |
| HJM-082 | 0.0299 |
| HJM-083 | 0.0285 |
| HJM-084 | 0.084 |
| HJM-085 | 0.0625 |
| HJM-086 | 0.1878 |
| HJM-087 | 0.2346 |
| HJM-088 | 0.6008 |
| HJM-089 | 0.2558 |
| HJM-090 | 0.1984 |
| HJM-091 | 0.2906 |
| HJM-092 | 0.1394 |
| HJM-093 | 0.8139 |
| HJM-094 | 0.2233 |
| HJM-095 | 0.1776 |
| HJM-096 | 0.0899 |
| HJM-097 | 0.7923 |
| HJM-098 | 0.3092 |
| HJM-099 | 1.592 |
| HJM-100 | 0.0543 |
| HJM-101 | 0.0395 |
| HJM-102 | 0.0327 |
| HJM-103 | 0.0942 |
| HJM-104 | 0.0527 |
| HJM-105 | 0.02935 |
| HJM-106 | 0.05766 |
| HJM-107 | 0.04854 |
| HJM-108 | 0.1257 |
| HJM-109 | 0.02473 |
| HJM-110 | 0.03284 |
| HJM-111 | 0.03786 |
| HJM-112 | 0.0294 |
| HJM-113 | 0.0597 |
| HJM-114 | 0.068 |
| HJM-115 | 0.2009 |
| HJM-116 | 0.5505 |
| HJM-117 | 1.297 |
| HJM-118 | 0.7921 |
| HJM-119 | 0.2638 |
| HJM-120 | 0.1787 |
| HJM-121 | 0.494 |
| HJM-122 | 0.05489 |
| HJM-123 | 0.1317 |
| HJM-124 | 0.2498 |
| HJM-125 | 0.4627 |
| HJM-126 | 0.09205 |
| HJM-127 | 0.1075 |
| HJM-128 | 0.06204 |
| HJM-129 | 0.3886 |
| HJM-130 | 0.1044 |
| HJM-131 | 0.1298 |
| HJM-132 | 0.1232 |
| HJM-133 | 0.07436 |
| HJM-134 | 0.1588 |
| HJM-135 | 0.2859 |
| HJM-136 | 0.3935 |
| HJM-137 | 0.062 |
| HJM-138 | 0.04975 |
| HJM-139 | 0.1197 |
| HJM-140 | 0.0214 |
| HJM-141 | 0.0644 |
| HJM-142 | 0.0397 |
| HJM-143 | 0.2036 |
| HJM-144 | 0.0717 |
| HJM-145 | 0.0328 |
| HJM-146 | 0.1505 |
| HJM-147 | 0.0953 |
| HJM-148 | 1.09 |
| HJM-149 | 0.5462 |
| HJM-150 | 0.03847 |
| HJM-151 | 0.0911 |
| HJM-152 | 0.0732 |
| HJM-153 | 0.0932 |
| EED226 | 0.22 |

### (2) Cell proliferation assay

Human B-cell non-Hodgkin's lymphoma cells WSU-DLCL2, Karpas422 and Pfeiffer were cultured in cell culture flasks in a sterile incubator with 95% relative humidity and 5% CO₂ at 37 °C. The medium was RPMI-1640 (Gibco, product No. 31800022) containing 15% fetal bovine serum (FBS, Gibco, product No. 10099141) and 0.1% penicillin/streptomycin solution (P/S). In order to assay the effects of compounds on cell proliferation, cells in exponential growth phase were seeded into a 12-well plate (Nunc, product No. 150628) with 1 mL of medium per well, wherein WSU-DLCL2 and Karpas422 were seeded at a density of 1×10⁵ cells/well, and Pfeiffer was seeded at a density of 2×10⁵ cells/well. At the same time, different concentrations of compounds were added to the wells that had seeded with cells (6 concentrations were set for each compound at 3-fold serial dilution), and the final concentration of DMSO was 0.1%. After that, the number of viable cells was determined by Vi-CELL (Beckman Coulter) for WSU-DLCL2 and Pfeiffer every 3 days, and for Karpas422 on days 4, 7, and 11, respectively. The cells counted each time were seeded into a new 12-well plate at the same density, and supplemented with fresh medium to 1 mL. Different concentrations of compounds were added at the same time. WSU-DLCL2 and Pfeiffer were cultured to the 9th day, and Karpas422 was cultured to the 14th day. Cell data were collected uniformly, and analyzed with GraphPad prisim5. Dose curves were fitted, and IC₅₀ values of the assay compounds were obtained.

**Table 2 shows the IC₅₀ values of the anti-WSU-DLCL2 cell proliferation assay of some compounds of the present disclosure.**

| Compound No. | Anti-proliferation IC₅₀ (uM) |
|---|---|
| HJM-003 | 0.1314 |
| HJM-010 | 0.0778 |
| HJM-011 | 0.0863 |
| HJM-015 | 0.1152 |
| HJM-016 | 0.0864 |
| HJM-017 | 0.1131 |
| HJM-019 | 0.072 |
| HJM-020 | 0.0485 |
| HJM-025 | 0.0264 |
| HJM-027 | 0.058 |
| HJM-030 | 0.0396 |
| HJM-031 | 0.0806 |
| HJM-033 | 0.0947 |
| HJM-037 | 0.1153 |
| HJM-042 | 0.082 |
| HJM-045 | 0.0092 |
| HJM-067 | 0.0294 |
| HJM-072 | 0.1199 |
| HJM-073 | 0.0294 |
| HJM-079 | 0.0368 |
| HJM-082 | 0.1242 |
| HJM-083 | 0.028 |
| HJM-084 | 0.0869 |
| HJM-100 | 0.0548 |
| HJM-101 | 0.0526 |
| HJM-102 | 0.0251 |
| HJM-103 | 0.1484 |
| HJM-104 | 0.0296 |
| HJM-105 | 0.0354 |
| HJM-106 | 0.0398 |
| HJM-114 | 0.2108 |
| HJM-137 | 0.0514 |
| HJM-138 | 0.0432 |
| HJM-145 | 0.0473 |
| HJM-152 | 0.1062 |
| EED226 | 0.1311 |

**Table 3 shows the IC₅₀ values of the anti-Karpas422 cell proliferation assay of some compounds of the present disclosure.**

| Compound No. | Anti-proliferation IC₅₀ (uM) |
|---|---|
| HJM-016 | 0.0749 |
| HJM-030 | 0.0421 |
| HJM-045 | 0.0135 |
| HJM-067 | 0.0510 |
| HJM-102 | 0.0993 |
| HJM-104 | 0.1016 |
| EED226 | 0.1226 |

Multiple compounds of the present disclosure have good anti-proliferative activities on WSU-DLCL2 and Karpas422 cells, which are higher than that of the positive control EED226.

**Table 4 shows the IC₅₀ values of the anti-Pfeiffer cell proliferation assay of some compounds of the present disclosure.**

| Compound No. | Anti-proliferation IC₅₀ (uM) |
|---|---|
| HJM-016 | 0.0557 |
| HJM-045 | 0.0105 |
| HJM-102 | 0.0474 |
| HJM-104 | 0.0816 |

The data show that some compounds of the present disclosure also have good anti-proliferative activity on Pfeiffer cells.

### (3) Pharmacokinetic study in rats

### 1. Assay steps:

Six SPF SD rats, male, weighing 180-220 g, were randomly divided into 2 groups with 3 rats in each group. The compounds of the present disclosure were administered by oral gavage (PO) and intravenous injection (IV), respectively, and the assay compounds were formulated with a vehicle (5% DMSO+10% Solutol+85% pH 4.65 Acetate buffer).

Dosage: 1 mg/kg for intravenous injection and 2 mg/kg for oral administration.

Dosing volume: 5 mL/kg for intravenous injection and 10 mL/kg for oral administration.

The oral administration group was fasted overnight (10-14 h) before the assay, and drank water freely. After 4 hours of administration, they were fed uniformly. They were weighed before administration, and the dosage was calculated according to the body weight.

### 2. Blood collection time point and sample processing:

Intravenous administration: 0.083, 0.25, 0.5, 1, 2, 4, 6, 8, 10 and 24 h after administration;

Oral gavage administration: 0.25, 0.5, 1, 2, 4, 6, 8, 10, 12 and 24 h after administration;

Blood was collected from the jugular vein of rats at the above set time points, about 0.2 mL each time. The blood was anticoagulated with heparin sodium, placed on ice after collection, and centrifuged within 1 hour to separate the plasma (centrifugation conditions: 6800g, 6 minutes, 2-8 °C). Plasma samples were stored in a -80 °C freezer prior to analysis.

### 3. Sample assaying and data analysis

The concentration of each compound in rat plasma was determined by LC/MS/MS method.

Through the plasma drug concentration data at different time points, pharmacokinetic parameters were calculated using Phoenix WinNonlin 7.0, thereby providing parameters such as AUC₀₋ₜ, AUC_{0-∞}, MRT_{0-∞}, Cₘₐₓ, Tₘₐₓ, and T_{1/2} and mean and standard deviation thereof.

**Table 5 shows the rat pharmacokinetic parameters of some compounds of the present disclosure.**

| Compound No. | Way of admi nistra tion | T_{1/2} h | Tₘₐₓ h | Cₘₐₓ ng/mL | AUC_{0-∞} ng·h/mL | MRT_{0-∞} h | Cl mL/min/kg | F % |
|---|---|---|---|---|---|---|---|---|
| HJM-016 | IV | 2.33 | 0.08 | 931.5 | 2055.9 | 2.94 | 8.24 | |
| | PO | 2.80 | 4.00 | 481.6 | 3900.0 | 5.58 | | 98.86 |
| HJM-030 | IV | 1.70 | 0.08 | 1500.8 | 1233.1 | 1.02 | 13.55 | |
| | PO | 1.32 | 0.42 | 393.5 | 1445.1 | 2.93 | | 58.53 |
| HJM-045 | IV | 1.17 | 0.08 | 793.6 | 639.0 | 1.06 | 26.37 | |
| | PO | 1.46 | 2.83 | 119.2 | 628.2 | 3.51 | | 48.40 |
| HJM-067 | IV | 3.00 | 0.08 | 945.6 | 694.8 | 0.80 | 24.08 | |
| | PO | 3.03 | 0.83 | 305.9 | 845.6 | 2.70 | | 60.72 |
| HJM-079 | IV | 0.92 | 0.08 | 750.2 | 856.8 | 1.10 | 19.50 | |
| | PO | 1.72 | 0.42 | 222.3 | 705.5 | 2.94 | | 40.87 |
| HJM-102 | IV | 1.98 | 0.08 | 1181.4 | 1139.7 | 1.40 | 14.77 | |
| | PO | 1.68 | 1.50 | 368.9 | 1887.6 | 3.55 | | 82.62 |
| HJM-104 | IV | 2.70 | 0.08 | 1360.1 | 1976.8 | 2.37 | 8.47 | |
| | PO | 2.21 | 3.33 | 586.3 | 3473.8 | 4.43 | | 89.41 |
| HJM-105 | IV | 1.82 | 0.08 | 1595.3 | 1908.9 | 1.56 | 8.83 | |
| | PO | 1.41 | 2.83 | 342.0 | 1789.5 | 3.29 | | 47.22 |

The data show that some compounds of the present disclosure have better pharmacokinetic properties and bioavailability in rats.

### (4) Pharmacokinetic study in canine

### 1. Assay steps:

Six ordinary Beagle dogs, male, weighing 10-12 kg, were randomly divided into 2 groups with 3 dogs in each group. The compounds of the present disclosure were administered by oral gavage (PO) and intravenous injection (IV), respectively, and the assay compounds were formulated with the corresponding vehicles.

Dosage: 1 mg/kg for intravenous injection and 5 mg/kg for oral administration.

Dosing volume: 5 mL/kg for intravenous injection and 10 mL/kg for oral administration.

The oral administration group was fasted overnight (10-14 h) before the assay, and drank water freely. After 4 hours of administration, they were fed uniformly. They were weighed before administration, and the dosage was calculated according to the body weight.

### 2. Blood collection time point and sample processing:

Intravenous administration: 0.083, 0.25, 0.5, 1, 2, 4, 8, 12 and 24 h after administration;

Oral gavage administration: 0.25, 0.5, 1, 2, 4, 6, 8, 12 and 24 h after administration;

Blood was collected from the veins of extremities, about 0.5-1 mL each time. The blood was anticoagulated with heparin sodium, placed on ice after collection, and centrifuged within 1 hour to separate the plasma (centrifugation conditions: 6800g, 6 minutes, 2-8 °C). Plasma samples were stored in a -80 °C freezer prior to analysis.

### 3. Sample assaying and data analysis

The concentration of each compound in canine plasma was determined by LC/MS/MS method.

Through the plasma drug concentration data at different time points, pharmacokinetic parameters were calculated using Phoenix WinNonlin 7.0, thereby providing parameters such as AUC₀₋ₜ, AUC_{0-∞}, MRT_{0-∞}, Cₘₐₓ, Tₘₐₓ, and T_{1/2} and mean and standard deviation thereof.

**Table 6 shows canine pharmacokinetic parameters of some compounds of the present disclosure.**

| Compound No. | Way of admi nistra tion | T_{1/2} h | Tₘₐₓ h | Cₘₐₓ ng/mL | AUC_{0-∞} ng·h/mL | MRT_{0-∞} h | Cl mL/min/kg | F % |
|---|---|---|---|---|---|---|---|---|
| HJM-016 | IV | 5.87 | 0.08 | 1223.4 | 8840.1 | 8.39 | 1.92 | |
| | PO | 5.18 | 2.00 | 2900.1 | 27166.9 | 7.97 | | 62.50 |
| HJM-045 | IV | 4.13 | 0.08 | 1145.1 | 4501.9 | 5.35 | 3.70 | |
| | PO | 4.29 | 0.50 | 3704.3 | 20674.5 | 5.75 | | 90.95 |
| HJM-104 | IV | 1.67 | 0.08 | 864.02 | 3232.7 | 2.86 | 5.90 | |
| | PO | 2.09 | 1.00 | 3379.7 | 16519.9 | 3.53 | | 102.43 |

Vehicles:
HJM-016: 2% DMSO+5% Solutol+93% Saline
HJM-045: 2% DMSO+98% Saline
HJM-104: 5% DMSO+20% PG+20% PEG400+55% Saline

The data show that some compounds of the present disclosure have better pharmacokinetic properties and bioavailability in canines.

### (5) Pharmacodynamic study

### Mouse Karpas422 CDX Model

Female balb/c nude mice aged 6-8 weeks were selected and housed under SPF conditions (i.e., 20-22 °C; 40-70% relative humidity) in individual ventilated cages at constant temperature and humidity. There were 5 or fewer animals in each cage. Animals had access to dry pelleted food sterilized by irradiation and sterile drinking water. Karpas 422 human B-cell lymphoma cells were cultured in RPMI-1640 (Gibco, product No. 31800022) containing 15% fetal bovine serum (FBS, Gibco, product No. 10099141) and 0.1% penicillin/streptomycin solution (P/S). The cells were cultured in a sterile incubator at a temperature of 37 °C, a relative humidity of 95%, and 5% CO₂. Karpas 422 cells cultured in vitro were maintained at a concentration between 0.5-2 × 10⁶ cells/mL cells in suspension culture and passaged at a 1:3 dilution every 2-4 days. To establish the xenograft model, cells were collected, resuspended in PBS, mixed 1:1 by volume with Matrigel (BD) at a concentration of 5 × 10⁷ cells/mL, and then injected subcutaneously into the right side of balb/c nude mice (Vital River) with 5 × 10⁶ cells per animal. When tumors grew to about 150 mm³, mice were divided into groups with 6 mice in each group. The mice were administered intragastrically, and the tumor size and mouse body weight were measured twice a week. The two largest diameters of the transplanted tumor, width (W) and length (L), were manually measured with a vernier caliper, and the tumor volume was estimated using the formula: 0.5 × L × W². After the assay, the mice were euthanized.

The assay compounds were prepared as a suspension in pure water containing 0.5% HPMC + 0.5% Tween 80 as a solvent. The dosing volume was 10 mL/kg.

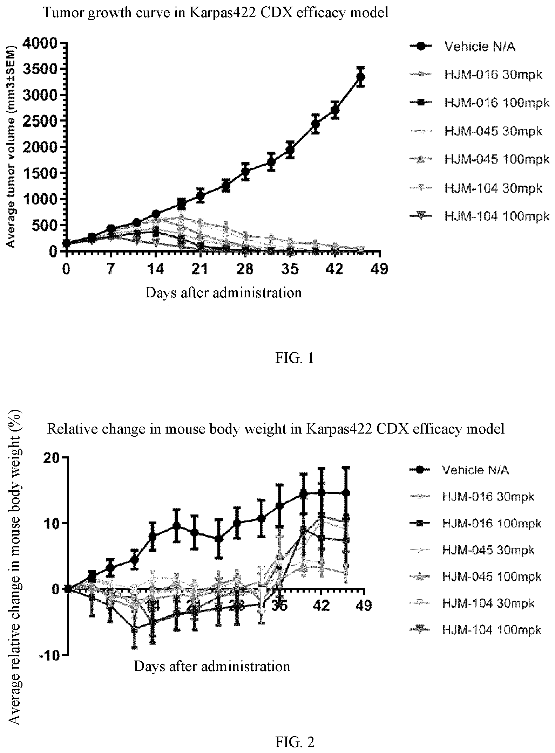

In this study, changes in mouse tumor volume and relative changes in mouse body weight were assayed, and the results were shown in FIGs. 1 and 2.

FIG. 1 and Table 7 show that the compounds HJM-016, HJM-045 and HJM-104 of the present disclosure have inhibitory effects on tumor growth at different dosages, all of which can achieve complete tumor disappearance and have good in vivo efficacy.

**Table 7 shows tumor growth inhibition (TGI%) data of compounds of the present disclosure.**

| Compound No. | Dosage | Day 11 after administration | Day 21 after administration |
|---|---|---|---|
| HJM-016 | 30 mg/kg | 8.5 | 56.1 |
| | 100 mg/kg | 52.3 | 105.39 |
| HJM-045 | 30 mg/kg | 0.25 | 60.06 |
| | 100 mg/kg | 10.7 | 81.43 |
| HJM-104 | 30 mg/kg | 39.4 | 93.29 |
| | 100 mg/kg | 88.9 | 111.9 |

FIG. 2 shows the relative changes in the body weight of mice with different dosages of the compounds HJM-016, HJM-045 and HJM-104 of the present disclosure. The body weight changes of the mice after administration were all less than 10%, indicating that the compounds of the present disclosure have better safety.

The above content is a further detailed description of the present disclosure in conjunction with specific alternative embodiments, and it cannot be assumed that the specific implementation of the present disclosure is limited to these descriptions.

## Claims

1. A compound of formula (X), or a tautomer, stereoisomer, crystal form, pharmaceutically acceptable salt, hydrate or solvate thereof: 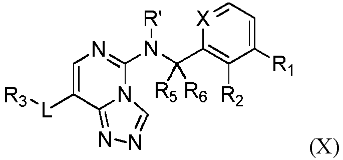 wherein
X is N or C(Rₓ);
wherein Rₓ is selected from H, halogen, C₁₋₆ alkyl, C₁₋₆ haloalkyl or C₁₋₆ alkoxy;
R₁ is selected from H, halogen, C₁₋₆ alkyl, C₁₋₆ haloalkyl or C₁₋₆ alkoxy;
R₂ is selected from H, halogen, C₁₋₆ alkyl, C₁₋₆ haloalkyl or C₁₋₆ alkoxy;
or R₁ and R₂ together form -(CH₂)ₚY-, -Y(CH₂)ₚ- or -Y(CH₂)ₚZ- group;
wherein p is 1, 2, 3, 4 or 5;
Y is selected from O, S, NH or chemical bond;
Z is selected from O, S, NH or chemical bond;
R' is selected from H, C₁₋₆ alkyl or C₁₋₆ haloalkyl;
L is selected from -C(O)-N(R)-, -C(O)-O-, -N(R₃')-C(O)-, -O-C(O)-, -S(O)_{q}-N(R)-, -S(O)_{q}-O-, -O-, -S-, - N(R")-, -C₁₋₄ alkylene- or -C₂₋₄ alkenylene-;
wherein R is selected from H, C₁₋₆ alkyl or C₁₋₆ haloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
R" is selected from H, C₁₋₆ alkyl or C₁₋₆ haloalkyl;
q is 0, 1 or 2;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-NRₐR_{b}, (CH₂)ₙ-ORₐ, (CH₂)ₙ-C₃₋₇ cycloalkyl, (CH₂)ₙ-3- to 10-membered heterocyclyl, (CH₂)ₙ-C₆₋₁₀ aryl or (CH₂)ₙ-5- to 10-membered heteroaryl;
or, R₃, R₃' and the N atom to which they are attached together form a 4- to 7-membered heterocyclyl group;
wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₅ and R₆ are independently selected from H, D, C₁₋₆ alkyl or C₁₋₆ haloalkyl;
wherein R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, -C(O)-5- to 10-membered heteroaryl, -S(O)_{q}-C₁₋₆ alkyl, -S(O)_{q}-C₁₋₆ haloalkyl, -S(O)_{q}-C₁₋₆ alkoxy, -S(O)_{q}-C₃₋₇ cycloalkyl, -S(O)_{q}-4- to 7-membered heterocyclyl, -S(O)_{q}-C₆₋₁₀ aryl or -S(O)_{q}-5- to 10-membered heteroaryl;
wherein R_{c} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
R_{d} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
wherein the alkyl, haloalkyl, alkoxy, haloalkoxy, cycloalkyl, heterocyclyl, aryl, heteroaryl, alkylene and alkenylene are optionally substitued with substituent groups selected from H, D, CN, OH, =O, halogen, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl, or substitued with one or more D, up to complete deuteration.

2. The compound, or the tautomer, stereoisomer, crystal form, pharmaceutically acceptable salt, hydrate or solvate thereof according to claim 1, which is a compound of formula (V): 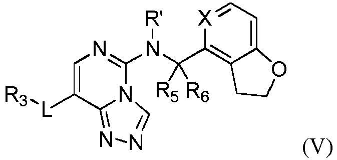 wherein
X is N or C(Rₓ); alternatively, X is C(Rₓ),
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
L is -C(O)-N(R)-, -C(O)-O-, -S(O)_{q}-N(R)- or -S(O)_{q}-O-;
wherein R is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
q is 0, 1 or 2;
R₃ is selected from (CR₇R₈)ₘ-C₁₋₆ alkyl, (CR₇R₈)ₘ-C₁₋₆ haloalkyl, (CR₇R₈)ₙ-C₁₋₆ alkoxy, (CR₇R₈)ₙ-C₁₋₆ haloalkoxy, (CR₇R₈)ₙ-NRₐR_{b}, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl;
wherein m is 0, 1, 2, 3, 4 or 5;
n is 0, 1, 2, 3, 4 or 5;
Rₐ is selected from H, C₁₋₆ alkyl or C₁₋₆ haloalkyl;
R_{b} is selected from H, C₁₋₆ alkyl or C₁₋₆ haloalkyl;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substitued with a group selected from H, D, halogen, CN, OH, C₁₋₆ alkyl, C₁₋₆ haloalkyl, C₃₋₇ cycloalkyl, 4- to 7-membered heterocyclyl, C₆₋₁₀ aryl, 5- to 10-membered heteroaryl, (CR₇R₈)ₙ-C₁₋₆ alkoxy or -S(O)_{q}-C₁₋₆ alkyl;
wherein the alkyl, haloalkyl, alkoxy, haloalkoxy, cycloalkyl, heterocyclyl, aryl and heteroaryl are optionally substitued with one or more D, up to complete deuteration.

3. The compound, or the tautomer, stereoisomer, crystal form, pharmaceutically acceptable salt, hydrate or solvate thereof according to claim 1, which is a compound of formula (VI): 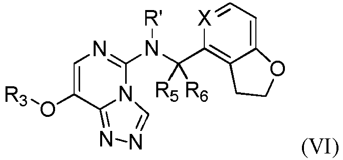 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration; or R₇ and R₈ on the same carbon atom are combined to form =O;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, or -C(O)-5- to 10-membered heteroaryl;
wherein R_{c} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
R_{d} is selected from H, C₁₋₆ alkyl, C₁₋₆ haloalkyl, -C(O)-C₃₋₇ cycloalkyl, -C(O)-4- to 7-membered heterocyclyl, -C(O)-C₆₋₁₀ aryl or -C(O)-5- to 10-membered heteroaryl;
wherein the alkyl, haloalkyl, alkoxy, cycloalkyl, heterocyclyl, aryl and heteroaryl are optionally substitued with substituent groups selected from H, D, CN, OH, =O, halogen, C₁₋₆ alkyl, C₁₋₆ haloalkyl, C₁₋₆ alkoxy, C₃₋₇ cycloalkyl, 4- to 7-membered heterocyclyl, C₆₋₁₀ aryl, 5- to 10-membered heteroaryl, -O-C₃₋₇ cycloalkyl, -O-4- to 7-membered heterocyclyl, -O-C₆₋₁₀ aryl, -O-5- to 10-membered heteroaryl, -C(O)-C₁₋₆ alkyl, -C(O)-C₁₋₆ haloalkyl, -C(O)-C₃₋₇ cycloalkyl, -C(O)-4- to 7-membered heterocyclyl, -C(O)-C₆₋₁₀ aryl, or -C(O)-5- to 10-membered heteroaryl, or substitued with one or more D, up to complete deuteration;
alternatively, wherein
R₃ is optionally substituted by one or more substituent groups selected from H, D, halogen, CN, OH, =O, NR_{c}R_{d}, C₁₋₆ alkyl, C₁₋₆ haloalkyl, C₁₋₆ alkoxy, C₃₋₇ cycloalkyl, -C(O)-C₁₋₆ alkyl, -C(O)-C₃₋₇ cycloalkyl or -O-C₆₋₁₀ aryl;
wherein R_{c} is selected from H, C₁₋₆ alkyl, C₁₋₆ haloalkyl or -C(O)-C₃₋₇ cycloalkyl;
R_{d} is selected from H, C₁₋₆ alkyl, C₁₋₆ haloalkyl or -C(O)-C₃₋₇ cycloalkyl;
wherein the alkyl, haloalkyl, alkoxy, cycloalkyl, heterocyclyl, aryl and heteroaryl are optionally substitued with substituent groups selected from H, D, CN, =O, halogen, C₁₋₆ alkyl, C₁₋₆ haloalkyl, C₁₋₆ alkoxy or -C(O)-C₁₋₆ alkyl, or substitued with one or more D, up to complete deuteration;
alternatively, wherein
X is C(Rₓ);
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl, (CR₇R₈)ₙ-C₃₋₇ cycloalkyl or (CR₇R₈)ₙ-4- to 7-membered heterocyclyl only containing O as heteroatom;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with a group selected from H, D, C₁₋₆ alkyl, C₁₋₆ haloalkyl or C₁₋₆ alkoxy;
alternatively, wherein
X is C(Rₓ);
wherein Rₓ is selected from H or halogen;
R' is selected from H, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₃ is selected from (CR₇R₈)ₘ-C₁₋₆ alkyl, (CR₇R₈)ₘ-C₁₋₆ haloalkyl or (CR₇R₈)ₙ-4- to 7-membered heterocyclyl only containing O as heteroatom;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with a group selected from H, D, C₁₋₆ alkyl, C₁₋₆ haloalkyl or C₁₋₆ alkoxy.

4. The compound, or the tautomer, stereoisomer, crystal form, pharmaceutically acceptable salt, hydrate or solvate thereof according to claim 1, which is a compound of formula (VII): 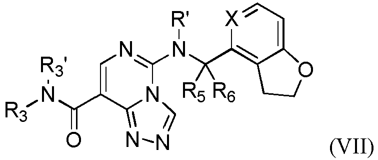 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R₃ is selected from (CR₇R₈)ₘ-C₁₋₆ alkyl, (CR₇R₈)ₘ-C₁₋₆ haloalkyl, (CR₇R₈)ₙ-C₃₋₇ cycloalkyl, (CR₇R₈)ₙ-4- to 7-membered heterocyclyl, (CR₇R₈)ₙ-C₆₋₁₀ aryl or (CR₇R₈)ₙ-5- to 10-membered heteroaryl;
R₃' is selected from H, (CR₇R₈)ₘ-C₁₋₆ alkyl, (CR₇R₈)ₘ-C₁₋₆ haloalkyl or (CR₇R₈)ₙ-C₃₋₇ cycloalkyl;
or, R₃, R₃' and the N atom to which they are attached together form a 4- to 7-membered heterocyclyl group;
wherein m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration; or R₇ and R₈ on the same carbon atom are combined to form =O;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with one or more groups selected from H, D, halogen, C₁₋₆ alkyl, C₁₋₆ haloalkyl or C₁₋₆ alkoxy;
alternatively, wherein
X is C(Rₓ),
wherein Rₓ is selected from H or halogen;
R' is selected from H, C₁₋₆ alkyl or C₁₋₆ haloalkyl;
R₃ is selected from (CR₇R₈)ₘ-C₁₋₆ alkyl, (CR₇R₈)ₘ-C₁₋₆ haloalkyl or (CR₇R₈)ₙ-C₃₋₇ cycloalkyl;
R₃' is selected from H, (CR₇R₈)ₘ-C₁₋₆ alkyl, (CR₇R₈)ₘ-C₁₋₆ haloalkyl or (CR₇R₈)ₙ-C₃₋₇ cycloalkyl;
or, R₃, R₃' and the N atom to which they are attached together form a 4- to 7-membered heterocyclyl group;
m is 0, 1, 2, 3, 4 or 5;
n is 0, 1, 2, 3, 4 or 5;
R₇ and R₈ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
R₅ and R₆ are independently selected from H, D, C₁₋₆ alkyl or C₁₋₆ haloalkyl, wherein the C₁₋₆ alkyl or C₁₋₆ haloalkyl is optionally substituted with 1, 2, 3, 4, 5, 6, 7, 8 or 9 D, up to complete deuteration;
wherein R₃ is optionally substituted with one or more groups selected from H, D, halogen, C₁₋₆ alkyl, C₁₋₆ haloalkyl or C₁₋₆ alkoxy.

5. The compound, or the tautomer, stereoisomer, crystal form, pharmaceutically acceptable salt, hydrate or solvate thereof according to claim 1, which is a compound of formula (I): 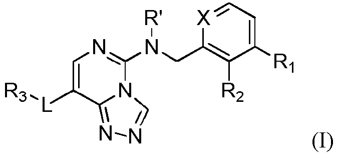 wherein
X is N or C(Rₓ);
wherein Rₓ is selected from H, halogen, C₁₋₆ alkyl, C₁₋₆ haloalkyl or C₁₋₆ alkoxy;
R₁ is selected from H, halogen, C₁₋₆ alkyl, C₁₋₆ haloalkyl or C₁₋₆ alkoxy;
R₂ is selected from H, halogen, C₁₋₆ alkyl, C₁₋₆ haloalkyl or C₁₋₆ alkoxy;
or R₁ and R₂ together form -(CH₂)ₚY-, -Y(CH₂)ₚ- or -Y(CH₂)ₚZ- group;
wherein p is 1, 2, 3, 4 or 5;
Y is selected from O, S, NH or chemical bond;
Z is selected from O, S, NH or chemical bond;
R' is selected from H, C₁₋₆ alkyl or C₁₋₆ haloalkyl;
L is selected from -C(O)-N(R)-, -N(R₃')-C(O)-, -O-, -S-, -N(R")-, -C₁₋₄ alkylene- or -C₂₋₄ alkenylene-;
wherein R is selected from H, C₁₋₆ alkyl or C₁₋₆ haloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
R" is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₇ cycloalkyl, (CH₂)ₙ-3- to 10-membered heterocyclyl, (CH₂)ₙ-C₆₋₁₀ aryl or (CH₂)ₙ-5- to 10-membered heteroaryl;
or, R₃, R₃' and the N atom to which they are attached together form a 4- to 7-membered heterocyclyl group;
wherein m is 0, 1, 2, 3, 4 or 5;
n is 0, 1, 2, 3, 4 or 5.

6. The compound, or the tautomer, stereoisomer, crystal form, pharmaceutically acceptable salt, hydrate or solvate thereof according to claim 5, which is a compound of formula (II): 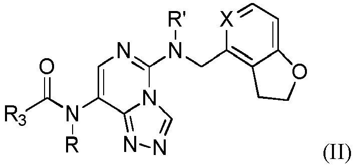 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl;
wherein m is 0, 1, 2, 3, 4 or 5;
n is 0, 1, 2, 3, 4 or 5;
alternatively, wherein
X is N or C(Rₓ); alternatively, X is C(Rₓ);
wherein Rₓ is selected from H, halogen, C₁₋₆ alkyl or C₁₋₆ haloalkyl; alternatively, Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₄ cycloalkyl or (CH₂)ₙ-C₆₋₁₀ aryl;
wherein m is 0, 1, 2, 3, 4 or 5;
n is 0, 1, 2, 3, 4 or 5;
alternatively, wherein
X is N or C(Rₓ); alternatively, X is C(Rₓ);
wherein Rₓ is selected from H, halogen, C₁₋₆ alkyl or C₁₋₆ haloalkyl; alternatively, Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl or (CH₂)ₙ-C₃₋₄ cycloalkyl; alternatively, R₃ is selected from (CH₂)ₘ-C₁₋₆ haloalkyl or (CH₂)ₙ-C₃₋₄ cycloalkyl;
wherein m is 0, 1, 2, 3, 4 or 5;
n is 0, 1, 2, 3, 4 or 5;
alternatively, wherein
X is C(Rₓ);
wherein Rₓ is selected from H or halogen;
R' is selected from H, C₁₋₆ alkyl or C₁₋₆ haloalkyl;
R is selected from H, C₁₋₆ alkyl or C₁₋₆ haloalkyl;
R₃ is selected from (CH₂)ₘ-C₁₋₆ haloalkyl or (CH₂)ₙ-C₃₋₄ cycloalkyl;
wherein m is 0, 1, 2 or 3;
n is 0, 1, 2 or 3.

7. The compound, or the tautomer, stereoisomer, crystal form, pharmaceutically acceptable salt, hydrate or solvate thereof according to claim 5, which is a compound of formula (III): 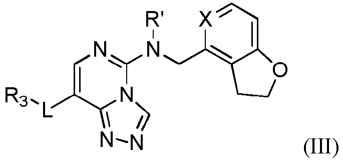 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
L is selected from -O-, -S-, -N(R")-, -C₁₋₄ alkylene- or -C₂₋₄ alkenylene-; alternatively, L is -O-;
wherein R" is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₁₋₆ alkoxy, (CH₂)ₙ-C₁₋₆ haloalkoxy, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl; alternatively, R₃ is selected from (CH₂)ₘ-C₁₋₆ alkyl, (CH₂)ₘ-C₁₋₆ haloalkyl, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl;
wherein m is 0, 1, 2, 3, 4 or 5;
n is 0, 1, 2, 3, 4 or 5;
alternatively, wherein
X is C(Rₓ);
wherein Rₓ is selected from H or halogen;
R' is selected from H, C₁₋₆ alkyl or C₁₋₆ haloalkyl;
L is -O-;
R₃ is selected from (CH₂)ₙ-C₃₋₇ cycloalkyl or (CH₂)ₙ-4- to 7-membered heterocyclyl only containing O as heteroatom;
wherein m is 0, 1, 2, 3, 4 or 5;
n is 0, 1, 2, 3, 4 or 5;
alternatively, wherein
X is N or C(Rₓ); alternatively, X is C(Rₓ);
wherein Rₓ is selected from H, halogen, C₁₋₆ alkyl or C₁₋₆ haloalkyl; alternatively, wherein Rₓ is selected from H or halogen;
R' is selected from H, C₁₋₆ alkyl or C₁₋₆ haloalkyl;
L is O;
R₃ is selected from (CH₂)ₘ-C₁₋₆ alkyl, (CH₂)ₘ-C₁₋₆ haloalkyl, (CH₂)ₙ-C₃₋₇ cycloalkyl or (CH₂)ₙ-5- to 6-membered heteroaryl containing O as heteroatom;
wherein m is 0, 1, 2, 3, 4 or 5;
n is 0, 1, 2, 3, 4 or 5;
alternatively, wherein
X is N or C(Rₓ); alternatively, X is C(Rₓ);
wherein Rₓ is selected from H, halogen, C₁₋₆ alkyl or C₁₋₆ haloalkyl; alternatively, wherein Rₓ is selected from H or halogen;
R' is selected from H, C₁₋₆ alkyl or C₁₋₆ haloalkyl;
L is -O-;
R₃ is selected from (CH₂)ₘ-C₁₋₆ alkyl, (CH₂)ₘ-C₁₋₆ haloalkyl or (CH₂)ₙ-C₃₋₇ cycloalkyl;
wherein m is 0, 1, 2, 3, 4 or 5;
n is 0, 1, 2, 3, 4 or 5.

8. The compound, or the tautomer, stereoisomer, crystal form, pharmaceutically acceptable salt, hydrate or solvate thereof according to claim 5, which is a compound of formula (IV): 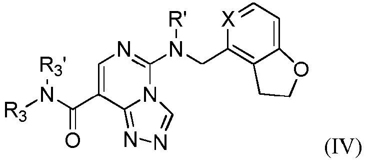 wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl, (CH₂)ₙ-C₃₋₇ cycloalkyl, (CH₂)ₙ-4- to 7-membered heterocyclyl, (CH₂)ₙ-C₆₋₁₀ aryl or (CH₂)ₙ-5- to 10-membered heteroaryl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
or, R₃, R₃' and the N atom to which they are attached together form a 4- to 7-membered heterocyclyl group;
wherein m is 0, 1, 2, 3, 4 or 5;
n is 0, 1, 2, 3, 4 or 5;
alternatively, wherein
X is N or C(Rₓ); alternatively, X is C(Rₓ);
wherein Rₓ is selected from H, halogen, C₁₋₆ alkyl, C₁₋₆ haloalkyl or C₁₋₆ alkoxy; alternatively, Rₓ is selected from H or halogen;
R' is selected from H, C₁₋₆ alkyl or C₁₋₆ haloalkyl;
R₃ is selected from (CH₂)ₘ-C₁₋₆ alkyl, (CH₂)ₘ-C₁₋₆ haloalkyl or (CH₂)ₙ-C₃₋₇ cycloalkyl;
R₃' is selected from H, (CH₂)ₘ-C₁₋₆ alkyl, (CH₂)ₘ-C₁₋₆ haloalkyl or (CH₂)ₙ-C₃₋₇ cycloalkyl;
or, R₃, R₃' and the N atom to which they are attached together form a 4- to 7-membered heterocyclyl group;
wherein m is 0, 1, 2, 3, 4 or 5;
n is 0, 1, 2, 3, 4 or 5.

9. The compound, or the tautomer, stereoisomer, crystal form, pharmaceutically acceptable salt, hydrate or solvate thereof according to any one of claims 1-8, wherein R' is H.

10. The compound, or the tautomer, stereoisomer, crystal form, pharmaceutically acceptable salt, hydrate or solvate thereof according to any one of claims 1-8, wherein R₃ is (CH₂)ₙ-3- to 10-membered heterocyclyl.

11. The compound, or the tautomer, stereoisomer, crystal form, pharmaceutically acceptable salt, hydrate or solvate thereof according to any one of claims 1-8, wherein R₅ and R₆ are H.

12. The compound, or the tautomer, stereoisomer, crystal form, pharmaceutically acceptable salt, hydrate or solvate thereof according to claim 1, wherein the compound is selected from:

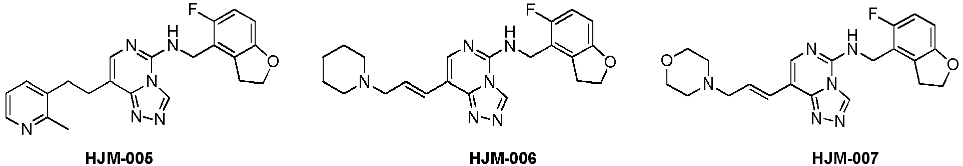

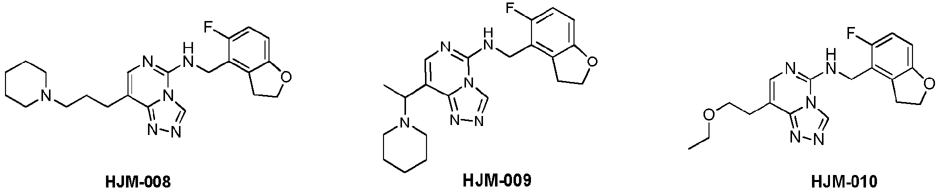

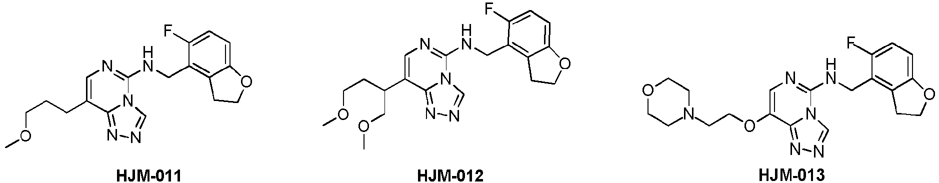

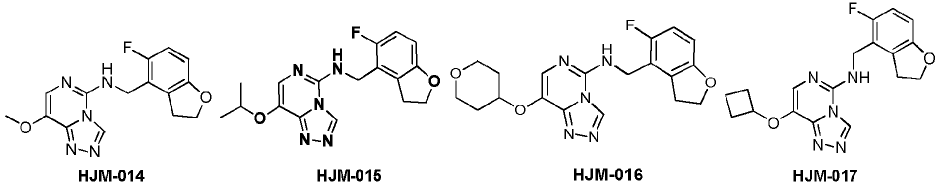

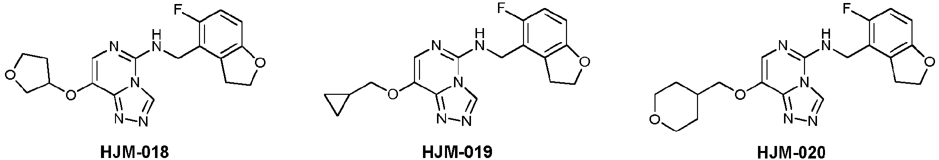

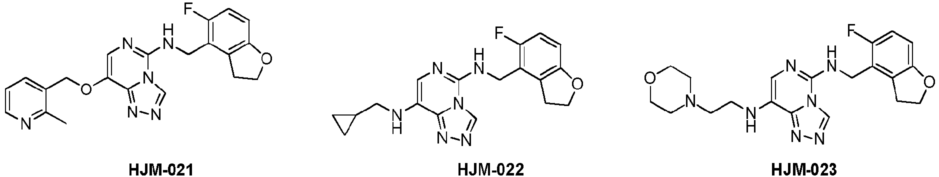

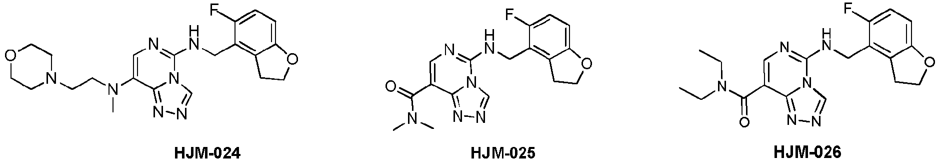

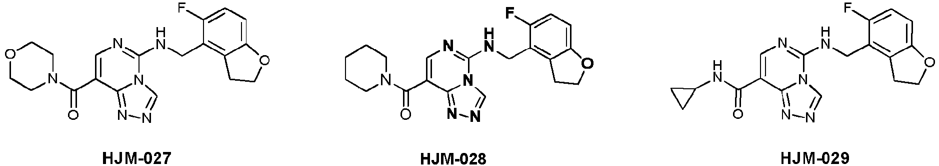

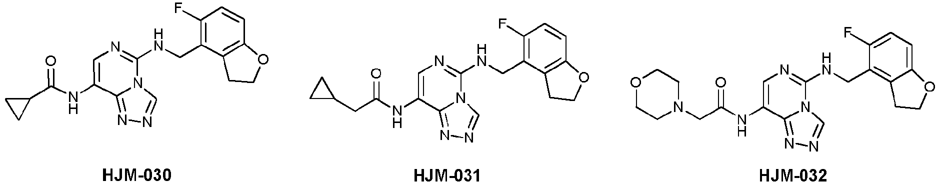

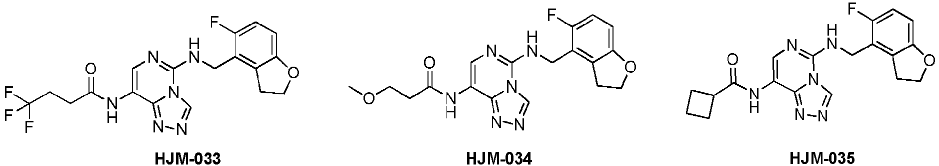

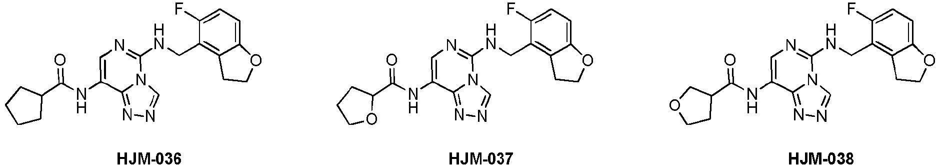

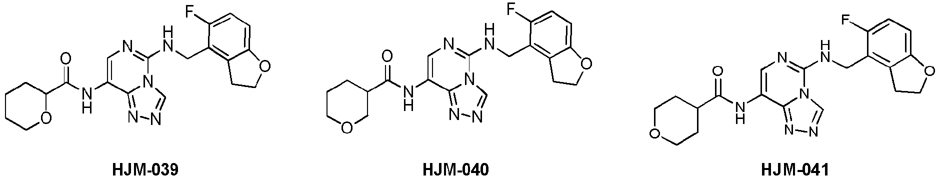

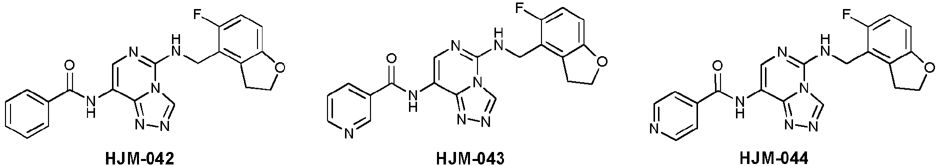

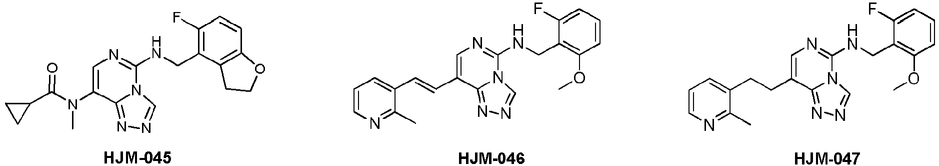

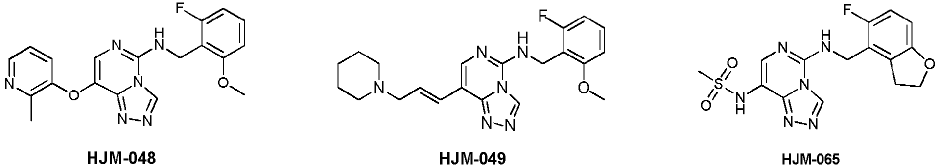

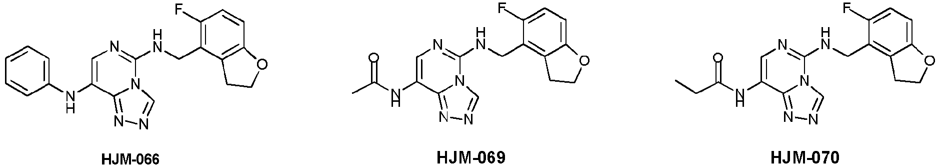

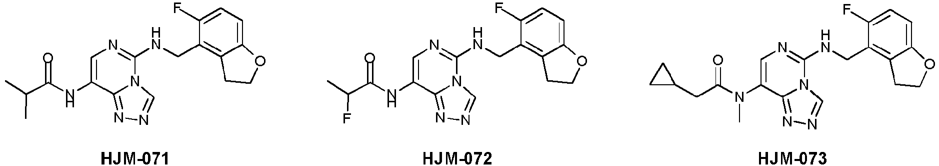

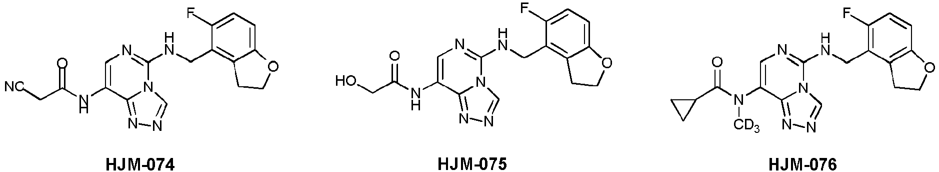

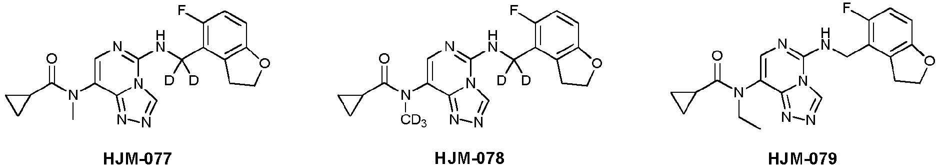

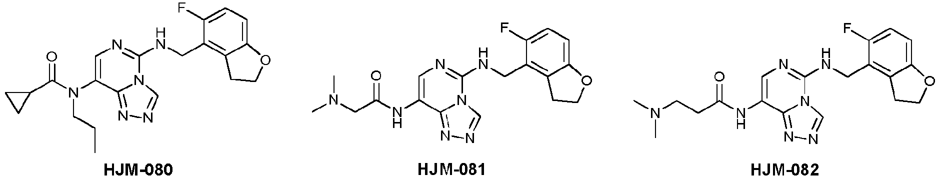

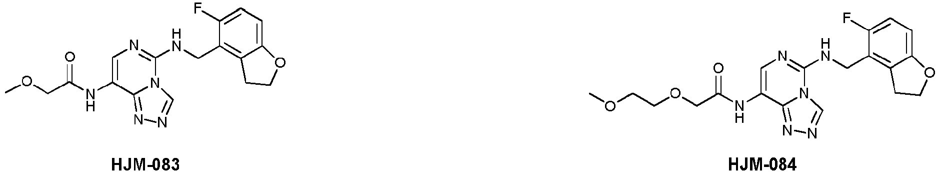

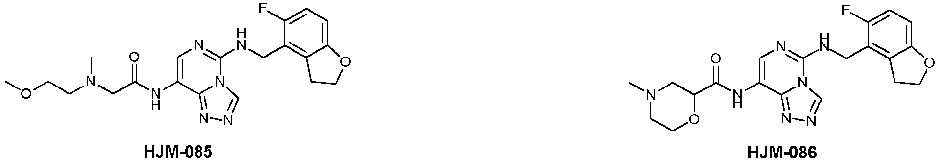

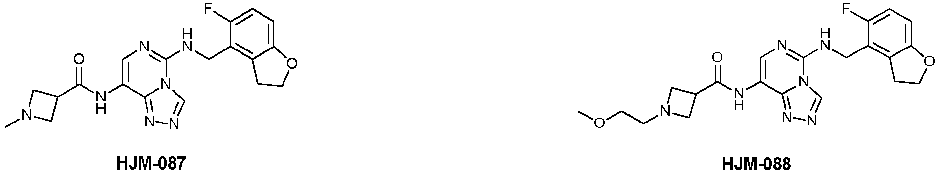

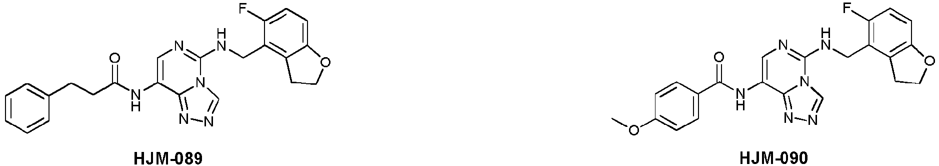

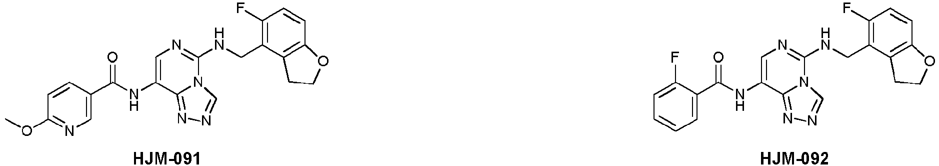

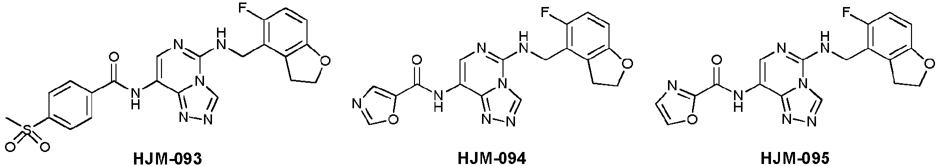

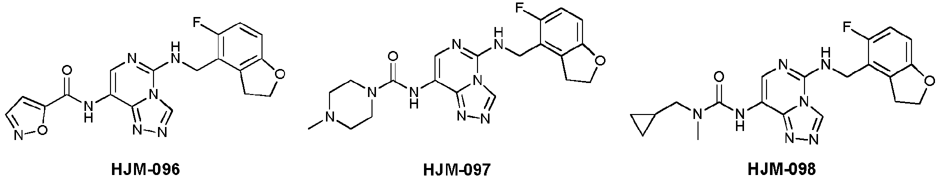

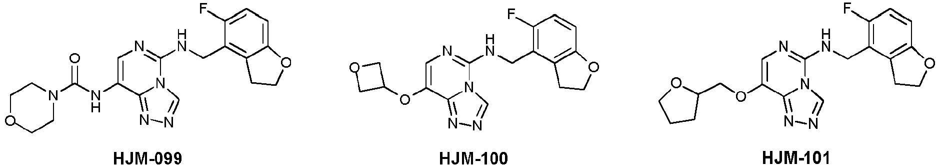

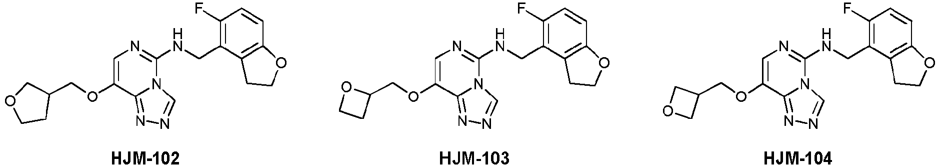

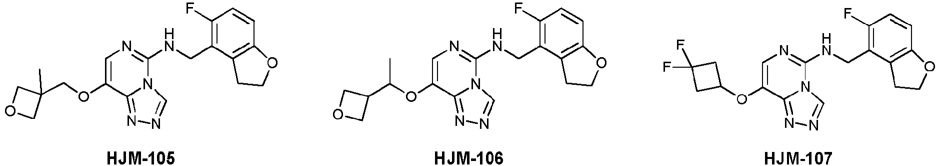

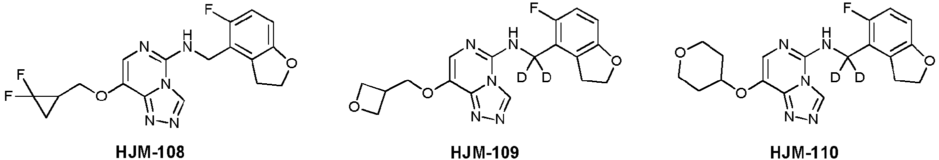

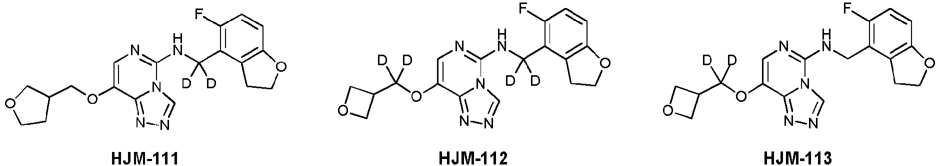

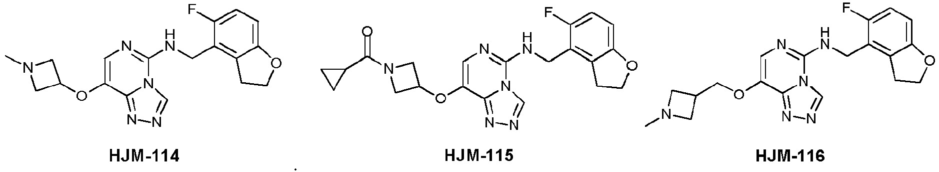

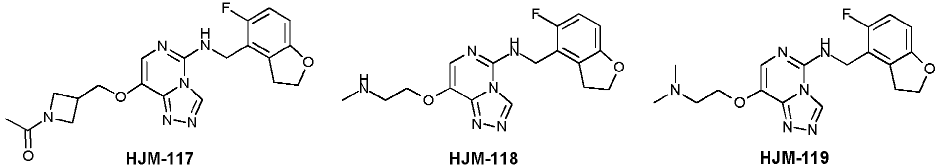

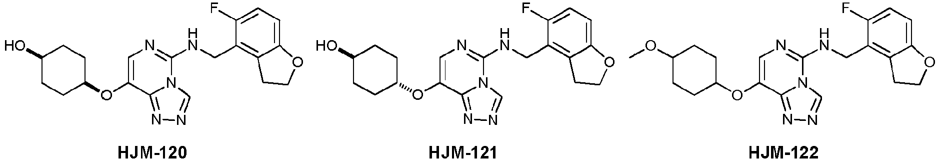

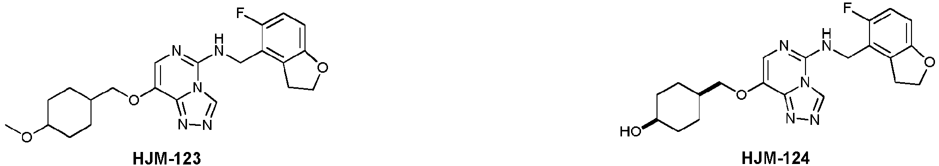

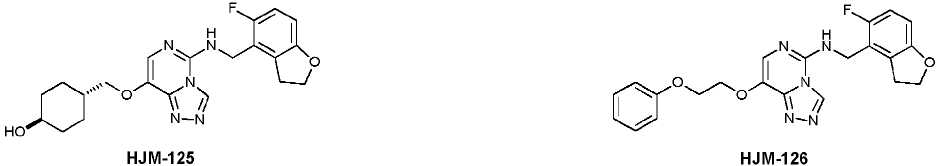

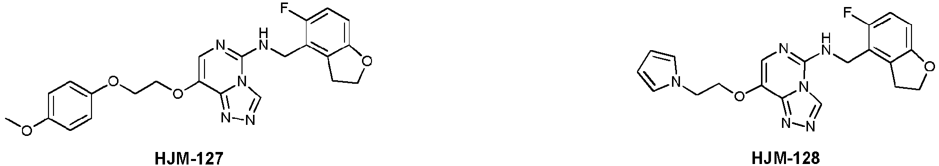

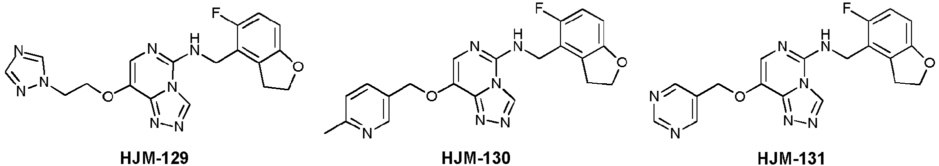

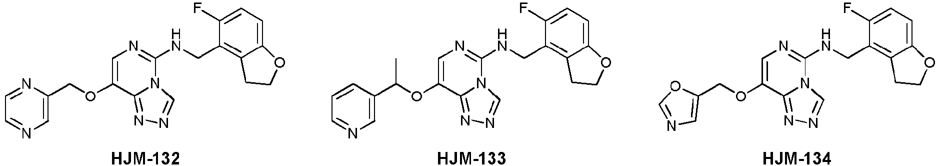

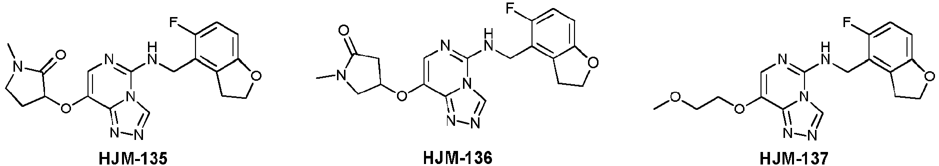

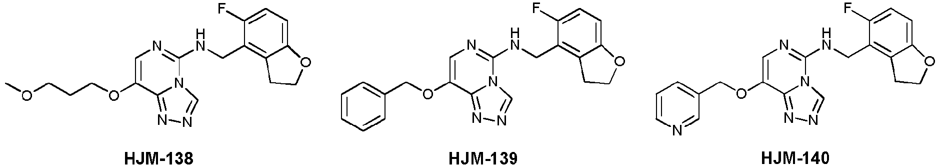

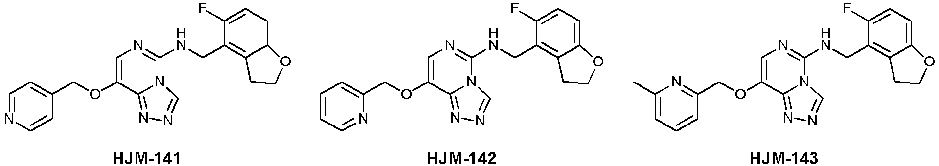

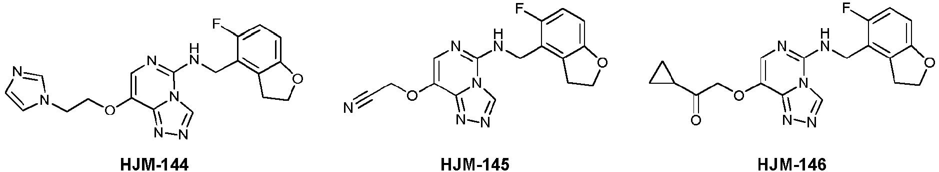

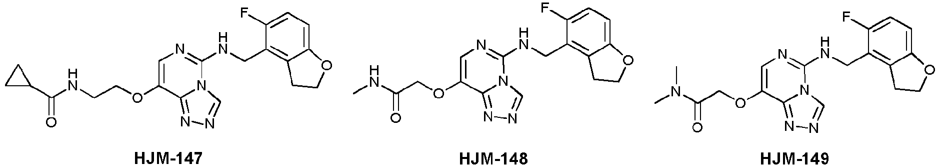

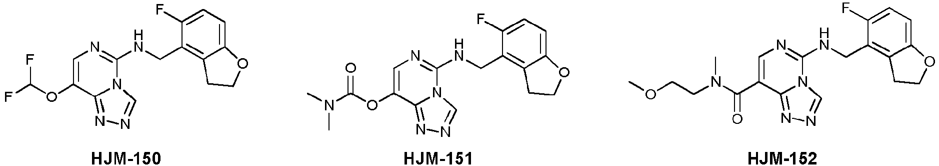

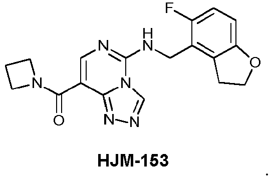

13. A pharmaceutical composition, containing the compound or the tautomer, stereoisomer, crystal form, pharmaceutically acceptable salt, hydrate or solvate thereof according to any one of claims 1-12, and pharmaceutically acceptable excipient (s);
alternatively, wherein the pharmaceutical composition is used in combination with immune checkpoint inhibitors such as PD-1, PD-L1, CTLA-4 antibodies, etc.

14. The compound or the tautomer, stereoisomer, crystal form, pharmaceutically acceptable salt, hydrate or solvate thereof according to any one of claims 1-12, or the pharmaceutical composition according to claim 13, for use in the treatment and/or prevention of an EED- and/or PRC2-mediated disease selected from a tumor-related disease, an immunodeficiency disease or an autoimmune disease; alternatively, the tumor-related disease is selected from diffuse large B-cell lymphoma, follicular lymphoma, other lymphomas, leukemia, multiple myeloma, mesothelioma, gastric cancer, malignant rhabdomyoma, liver cancer, prostate cancer, breast cancer, bile duct and gallbladder cancer, bladder cancer, brain tumors (including neuroblastoma, schwannoma, glioma, glioblastoma and astrocytoma), cervical cancer, colon cancer, melanoma, endometrial cancer, esophageal cancer, head and neck cancer, lung cancer, nasopharyngeal cancer, ovarian cancer, pancreatic cancer, renal cell cancer, rectal cancer, thyroid cancer, parathyroid tumor, uterine tumor and soft tissue sarcoma; wherein the immunodeficiency disease or autoimmune disease includes, but is not limited to, systemic lupus erythematosus, Crohn's disease, ulcerative colitis, multiple sclerosis, aplastic anemia due to autoimmunity, and rejection reaction due to organ transplantation, etc, or alternatively, for use in tumor immunization.

15. A drug combination, comprising the compound or the tautomer, stereoisomer, crystal form, pharmaceutically acceptable salt, hydrate or solvate thereof according to any one of claims 1-12, or the pharmaceutical composition according to claim 13, and immune checkpoint inhibitor(s) such as PD-1, PD-L1, CTLA-4 antibodies, etc.

## Patentansprüche

1. Verbindung der Formel (X), oder ein Tautomer, ein Stereoisomer, eine Kristallform, ein pharmazeutisch annehmbares Salz, ein Hydrat oder ein Solvat davon: 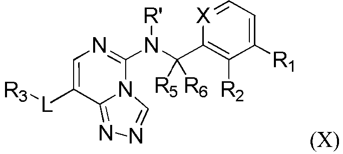 wobei
X N oder C(Rₓ) ist;
wobei Rₓ aus H, Halogen, C₁₋₆ Alkyl, C₁₋₆ Halogenalkyl oder C₁₋₆ Alkoxy ausgewählt ist;
R₁ aus H, Halogen, C₁₋₆ Alkyl, C₁₋₆ Halogenalkyl oder C₁₋₆ Alkoxy ausgewählt ist;
R₂ aus H, Halogen, C₁₋₆ Alkyl, C₁₋₆ Halogenalkyl oder C₁₋₆ Alkoxy ausgewählt ist;
oder R₁ und R₂ zusammen eine -(CH₂)ₚY-, -Y(CH₂)ₚ- oder -Y(CH₂)ₚZ- Gruppe bilden;
wobei p 1, 2, 3, 4 oder 5 ist;
Y aus O, S, NH oder chemischer Bindung ausgewählt ist;
Z aus O, S, NH oder chemischer Bindung ausgewählt ist;
R' aus H, C₁₋₆ Alkyl oder C₁₋₆ Halogenalkyl ausgewählt ist;
L aus -C(O)-N(R)-, -C(O)-O-, -N(R₃')-C(O)-, -O-C(O)-, -S(O)_{q}-N(R)-, -S(O)_{q}-O-, -O-, -S-, -N(R")-, -C₁₋₄ Alkylen- oder -C₂₋₄ Alkenylen- ausgewählt ist;
wobei R aus H, C₁₋₆ Alkyl oder C₁₋₆ Halogenalkyl ausgewählt ist;
R₃' aus H, (CH₂)ₘ-C₁₋₆ Alkyl, (CH₂)ₘ-C₁₋₆ Halogenalkyl oder (CH₂)ₙ-C₃₋₇ Cycloalkyl ausgewählt ist;
R" aus H, C₁₋₆ Alkyl oder C₁₋₆ Halogenalkyl ausgewählt ist;
q 0, 1 oder 2 ist;
R₃ aus (CH₂)ₘ-C₁₋₆ Alkyl, (CH₂)ₘ-C₁₋₆ Halogenalkyl, (CH₂)ₙ-C₁₋₆ Alkoxy, (CH₂)ₙ-C₁₋₆ Halogenalkoxy, (CH₂)ₙ-NRₐR_{b}, (CH₂)ₙ-ORₐ, (CH₂)ₙ-C₃₋₇ Cycloalkyl, (CH₂)ₙ-3- bis 10-gliedrigem Heterocyclo, (CH₂)ₙ-C₆₋₁₀ Aryl oder (CH₂)ₙ-5- bis 10-gliedrigem Heteroaryl ausgewählt ist;
oder R₃, R₃' und das N-Atom, an dem sie hängen, zusammen eine 4- bis 7-gliedrige Heterocyclogruppe bilden;
wobei m 0, 1, 2, 3, 4 oder 5 ist;
n 0, 1, 2, 3, 4 oder 5 ist;
Rₐ aus H, C₁₋₆ Alkyl oder C₁₋₆ Halogenalkyl ausgewählt ist;
R_{b} aus H, C₁₋₆ Alkyl oder C₁₋₆ Halogenalkyl ausgewählt ist;
R₅ und R₆ unabhängig aus H, D, C₁₋₆ Alkyl oder C₁₋₆ Halogenalkyl ausgewählt sind;
wobei R₃ optional substituiert ist durch eine oder mehrere Substituentengruppen ausgewählt aus H, D, Halogen, CN, OH, =O, NR_{c}R_{d}, C₁₋₆ Alkyl, C₁₋₆ Halogenalkyl, C₁₋₆ Alkoxy, C₃₋₇ Cycloalkyl, 4- bis 7-gliedrigem Heterocyclo, C₆₋₁₀ Aryl, 5- bis 10-gliedrigem Heteroaryl, (CH₂)ₘ-C₁₋₆ Alkyl, (CH₂)ₘ-C₁₋₆ Halogenalkyl, (CH₂)ₙ-C₁₋₆ Alkoxy, (CH₂)ₙ₋C₁₋₆ Halogenalkoxy, -O-C₃₋₇ Cycloalkyl, -O-4- bis 7-gliedrigem Heterocyclo, -O-C₆₋₁₀ Aryl, -O-5- bis 10-gliedrigem Heteroaryl, -C(O)-C₁₋₆ Alkyl, -C(O)-C₁₋₆ Halogenalkyl, -C(O)-C₃₋₇ Cycloalkyl, -C(O)-4- bis 7-gliedrigem Heterocyclo, -C(O)-C₆₋₁₀ Aryl, -C(O)-5- bis 10-gliedrigem Heteroaryl, -S(O)_{q}-C₁₋₆ Alkyl, -S(O)_{q}-C₁₋₆ Halogenalkyl, -S(O)_{q}-C₁₋₆ Alkoxy, -S(O)_{q}-C₃₋₇ Cycloalkyl, -S(O)_{q}-4- bis 7-gliedrigem Heterocyclo, -S(O)_{q}-C₆₋₁₀ Aryl oder -S(O)_{q}-5- bis 10-gliedrigem Heteroaryl;
wobei R_{c} aus H, C₁₋₆ Alkyl, C₁₋₆ Halogenalkyl, -C(O)-C₃₋₇ Cycloalkyl, -C(O)-4- bis 7-gliedrigem Heterocyclo, -C(O)-C₆₋₁₀ Aryl oder -C(O)-5- bis 10-gliedrigem Heteroaryl ausgewählt ist;
R_{d} aus H, C₁₋₆ Alkyl, C₁₋₆ Halogenalkyl, -C(O)-C₃₋₇ Cycloalkyl, -C(O)-4- bis 7-gliedrigem Heterocyclo, -C(O)-C₆₋₁₀ Aryl oder -C(O)-5- bis 10-gliedrigem Heteroaryl ausgewählt ist;
wobei das Alkyl, Halogenalkyl, Alkoxy, Halogenalkoxy, Cycloalkyl, Heterocyclo, Aryl, Heteroaryl, Alkylen und Alkenylen optional substituiert sind mit Substituentengruppen ausgewählt aus H, D, CN, OH, =O, Halogen, C₁₋₆ Alkyl, C₁₋₆ Halogenalkyl, C₁₋₆ Alkoxy, C₃₋₇ Cycloalkyl, 4- bis 7-gliedrigem Heterocyclo, C₆₋₁₀ Aryl, 5- bis 10-gliedrigem Heteroaryl, -O-C₃₋₇ Cycloalkyl, -O-4- bis 7-gliedrigem Heterocyclo, -O-C₆₋₁₀ Aryl, -O-5- bis 10-gliedrigem Heteroaryl, -C(O)-C₁₋₆ Alkyl, -C(O)-C₁₋₆ Halogenalkyl, -C(O)-C₃₋₇ Cycloalkyl, -C(O)-4- bis 7-gliedrigem Heterocyclo, -C(O)-C₆₋₁₀ Aryl oder -C(O)-5- bis 10-gliedrigem Heteroaryl, oder mit einem oder mehreren D substituiert sind, bis hin zur vollständigen Deuterierung.

2. Verbindung, oder das Tautomer, das Stereoisomer, die Kristallform, das pharmazeutisch annehmbare Salz, das Hydrat oder das Solvat davon, nach Anspruch 1, welche eine Verbindung der Formel (V) ist: 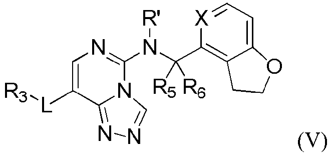 wobei
X N oder C(Rₓ) ist; alternativ wobei X C(Rₓ) ist,
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
L -C(O)-N(R)-, -C(O)-O-, -S(O)_{q}-N(R)- oder -S(O)_{q}-O- ist;
wobei R aus H, C₁₋₆ Alkyl oder C₁₋₆ Halogenalkyl ausgewählt ist, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
q 0, 1 oder 2 ist;
R₃ aus (CR₇R₈)ₘ-C₁₋₆ Alkyl, (CR₇R₈)ₘ-C₁₋₆ Halogenalkyl, (CR₇R₈)ₙ-C₁₋₆ Alkoxy, (CR₇R₈)ₙ-C₁₋₆ Halogenalkoxy, (CR₇R₈)ₙ-NRₐR_{b}, (CR₇R₈)ₙ-C₃₋₇ Cycloalkyl, (CR₇R₈)ₙ-4- bis 7-gliedrigem Heterocyclo, (CR₇R₈)ₙ-C₆₋₁₀ Aryl oder (CR₇R₈)ₙ-5- bis 10-gliedrigem Heteroaryl ausgewählt ist;
wobei m 0, 1, 2, 3, 4 oder 5 ist;
n 0, 1, 2, 3, 4 oder 5 ist;
Rₐ aus H, C₁₋₆ Alkyl oder C₁₋₆ Halogenalkyl ausgewählt ist;
R_{b} aus H, C₁₋₆ Alkyl oder C₁₋₆ Halogenalkyl ausgewählt ist;
R₇ und R₈ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₅ und R₆ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
wobei R₃ optional substituiert ist mit einer Gruppe ausgewählt aus H, D, Halogen, CN, OH, C₁₋₆ Alkyl, C₁₋₆ Halogenalkyl, C₃₋₇ Cycloalkyl, 4- bis 7-gliedrigem Heterocyclo, C₆₋₁₀ Aryl, 5- bis 10-gliedrigem Heteroaryl, (CR₇R₈)ₘ-C₁₋₆ Alkoxy oder -S(O)_{q}-C₁₋₆ Alkyl;
wobei das Alkyl, Halogenalkyl, Alkoxy, Halogenalkoxy, Cycloalkyl, Heterocyclo, Aryl und Heteroaryl optional mit einem oder mehreren D substituiert sind, bis hin zur vollständigen Deuterierung.

3. Verbindung, oder das Tautomer, das Stereoisomer, die Kristallform, das pharmazeutisch annehmbare Salz, das Hydrat oder das Solvat davon, nach Anspruch 1, welche eine Verbindung der Formel (VI) ist: 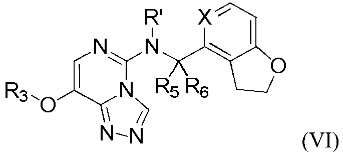 wobei
X N oder C(Rₓ) ist; alternativ wobei X C(Rₓ) ist;
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₃ aus (CR₇R₈)ₘ-C₁₋₆ Alkyl, (CR₇R₈)ₘ-C₁₋₆ Halogenalkyl, (CR₇R₈)ₙ-C₃₋₇ Cycloalkyl, (CR₇R₈)ₙ-4- bis 7-gliedrigem Heterocyclo, (CR₇R₈)ₙ-C₆₋₁₀ Aryl oder (CR₇R₈)ₙ-5- bis 10-gliedrigem Heteroaryl ausgewählt ist;
wobei m 0, 1, 2, 3, 4 oder 5 ist;
n 0, 1, 2, 3, 4 oder 5 ist;
R₇ und R₈ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung; oder R₇ und R₈ an dem gleichen Kohlenstoffatom vereint sind, um =O zu bilden;
R₅ und R₆ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
wobei R₃ optional substituiert ist durch eine oder mehrere Substituentengruppen ausgewählt aus H, D, Halogen, CN, OH, =O, NR_{c}R_{d}, C₁₋₆ Alkyl, C₁₋₆ Halogenalkyl, C₁₋₆ Alkoxy, C₃₋₇ Cycloalkyl, 4- bis 7-gliedrigem Heterocyclo, C₆₋₁₀ Aryl, 5- bis 10-gliedrigem Heteroaryl, -O-C₃₋₇ Cycloalkyl, -O-4- bis 7-gliedrigem Heterocyclo, -O-C₆₋₁₀ Aryl, -O-5- bis 10-gliedrigem Heteroaryl, -C(O)-C₁₋₆ Alkyl, -C(O)-C₁₋₆ Halogenalkyl, -C(O)-C₃₋₇ Cycloalkyl, -C(O)-4- bis 7-gliedrigem Heterocyclo, -C(O)-C₆₋₁₀ Aryl, oder -C(O)-5- bis 10-gliedrigem Heteroaryl;
wobei R_{c} aus H, C₁₋₆ Alkyl, C₁₋₆ Halogenalkyl, -C(O)-C₃₋₇ Cycloalkyl, -C(O)-4- bis 7-gliedrigem Heterocyclo, -C(O)-C₆₋₁₀ Aryl oder -C(O)-5- bis 10-gliedrigem Heteroaryl ausgewählt ist;
R_{d} aus H, C₁₋₆ Alkyl, C₁₋₆ Halogenalkyl, -C(O)-C₃₋₇ Cycloalkyl, -C(O)-4- bis 7-gliedrigem Heterocyclo, -C(O)-C₆₋₁₀ Aryl oder -C(O)-5- bis 10-gliedrigem Heteroaryl ausgewählt ist;
wobei das Alkyl, Halogenalkyl, Alkoxy, Cycloalkyl, Heterocyclo, Aryl und Heteroaryl optional substituiert sind mit Substituentengruppen ausgewählt aus H, D, CN, OH, =O, Halogen, C₁₋₆ Alkyl, C₁₋₆ Halogenalkyl, C₁₋₆ Alkoxy, C₃₋₇ Cycloalkyl, 4- bis 7-gliedrigem Heterocyclo, C₆₋₁₀ Aryl, 5- bis 10-gliedrigem Heteroaryl, -O-C₃₋₇ Cycloalkyl, -O-4- bis 7-gliedrigem Heterocyclo, -O-C₆₋₁₀ Aryl, -O-5- bis 10-gliedrigem Heteroaryl, -C(O)-C₁₋₆ Alkyl, -C(O)-C₁₋₆ Halogenalkyl, -C(O)-C₃₋₇ Cycloalkyl, -C(O)-4- bis 7-gliedrigem Heterocyclo, -C(O)-C₆₋₁₀ Aryl, oder -C(O)-5- bis 10-gliedrigem Heteroaryl, oder mit einem oder mehreren D substituiert sind, bis hin zur vollständigen Deuterierung;
alternativ wobei
R₃ optional durch eine oder mehrere Substituentengruppen substituiert ist ausgewählt aus H, D, Halogen, CN, OH, =O, NR_{c}R_{d}, C₁₋₆ Alkyl, C₁₋₆ Halogenalkyl, C₁₋₆ Alkoxy, C₃₋₇ Cycloalkyl, -C(O)-C₁₋₆ Alkyl, -C(O)-C₃₋₇ Cycloalkyl oder -O-C₆₋₁₀ Aryl;
wobei R_{c} aus H, C₁₋₆ Alkyl, C₁₋₆ Halogenalkyl oder -C(O)-C₃₋₇ Cycloalkyl ausgewählt ist;
R_{d} aus H, C₁₋₆ Alkyl, C₁₋₆ Halogenalkyl oder -C(O)-C₃₋₇ Cycloalkyl ausgewählt ist;
wobei das Alkyl, Halogenalkyl, Alkoxy, Cycloalkyl, Heterocyclo, Aryl und Heteroaryl optional substituiert sind mit Substituentengruppen ausgewählt aus H, D, CN, =O, Halogen, C₁₋₆ Alkyl, C₁₋₆ Halogenalkyl, C₁₋₆ Alkoxy oder -C(O)-C₁₋₆ Alkyl, oder mit einem oder mehreren D substituiert sind, bis hin zur vollständigen Deuterierung;
alternativ wobei
X C(Rₓ) ist;
wobei Rₓ aus H oder Halogen ausgewählt ist;
R' aus H, C₁₋₆ Alkyl oder C₁₋₆ Halogenalkyl ausgewählt ist, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₃ aus (CR₇R₈)ₘ-C₁₋₆ Alkyl, (CR₇R₈)ₘ-C₁₋₆ Halogenalkyl, (CR₇R₈)ₙ-C₃₋₇ Cycloalkyl oder (CR₇R₈)ₙ-4- bis 7-gliedrigem Heterocyclo enthaltend nur O als Heteroatom ausgewählt ist;
wobei m 0, 1, 2, 3, 4 oder 5 ist;
n 0, 1, 2, 3, 4 oder 5 ist;
R₇ und R₈ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₅ und R₆ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
wobei R₃ optional substituiert ist mit einer Gruppe ausgewählt aus H, D, C₁₋₆ Alkyl, C₁₋₆ Halogenalkyl oder C₁₋₆ Alkoxy;
alternativ wobei
X C(Rₓ) ist;
wobei Rₓ aus H oder Halogen ausgewählt ist;
R' aus H, C₁₋₆ Alkyl oder C₁₋₆ Halogenalkyl ausgewählt ist, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₃ aus (CR₇R₈)ₘ-C₁₋₆ Alkyl, (CR₇R₈)ₘ-C₁₋₆ Halogenalkyl oder (CR₇R₈)ₙ-4- bis 7-gliedrigem Heterocyclo enthaltend nur O als Heteroatom ausgewählt ist;
wobei m 0, 1, 2, 3, 4 oder 5 ist;
n 0, 1, 2, 3, 4 oder 5 ist;
R₇ und R₈ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₅ und R₆ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
wobei R₃ optional substituiert ist mit einer Gruppe ausgewählt aus H, D, C₁₋₆ Alkyl, C₁₋₆ Halogenalkyl oder C₁₋₆ Alkoxy.

4. Verbindung, oder das Tautomer, das Stereoisomer, die Kristallform, das pharmazeutisch annehmbare Salz, das Hydrat oder das Solvat davon, nach Anspruch 1, welche eine Verbindung der Formel (VII) ist: 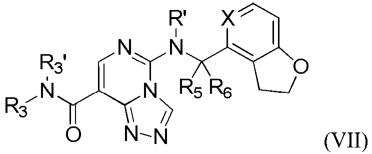 wobei
X N oder C(Rₓ) ist; alternativ wobei X C(Rₓ) ist;
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist;
R₃ aus (CR₇R₈)ₘ-C₁₋₆ Alkyl, (CR₇R₈)ₘ-C₁₋₆ Halogenalkyl, (CR₇R₈)ₙ-C₃₋₇ Cycloalkyl, (CR₇R₈)ₙ-4- bis 7-gliedrigem Heterocyclo, (CR₇R₈)ₙ-C₆₋₁₀ Aryl oder (CR₇R₈)ₙ-5- bis 10-gliedrigem Heteroaryl ausgewählt ist; R₃' aus H, (CR₇R₈)ₘ-C₁₋₆ Alkyl, (CR₇R₈)ₘ-C₁₋₆ Halogenalkyl oder (CR₇R₈)ₙ-C₃₋₇ Cycloalkyl ausgewählt ist; oder R₃, R₃' und das N-Atom, an dem sie hängen, zusammen eine 4- bis 7-gliedrige Heterocyclogruppe bilden;
wobei m 0, 1, 2, 3, 4 oder 5 ist;
n 0, 1, 2, 3, 4 oder 5 ist;
R₇ und R₈ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung; oder R₇ und R₈ an dem gleichen Kohlenstoffatom vereint sind, um =O zu bilden;
R₅ und R₆ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
wobei R₃ optional substituiert ist mit einer oder mehreren Gruppen ausgewählt aus H, D, Halogen, C₁₋₆ Alkyl, C₁₋₆ Halogenalkyl oder C₁₋₆ Alkoxy;
alternativ wobei
X C(Rₓ) ist,
wobei Rₓ aus H oder Halogen ausgewählt ist;
R' aus H, C₁₋₆ Alkyl oder C₁₋₆ Halogenalkyl ausgewählt ist;
R₃ aus (CR₇R₈)ₘ-C₁₋₆ Alkyl, (CR₇R₈)ₘ-C₁₋₆ Halogenalkyl oder (CR₇R₈)ₙ-C₃₋₇ Cycloalkyl ausgewählt ist;
R₃' aus H, (CR₇R₈)ₘ-C₁₋₆ Alkyl, (CR₇R₈)ₘ-C₁₋₆ Halogenalkyl oder (CR₇R₈)ₙ-C₃₋₇ Cycloalkyl ausgewählt ist; oder R₃, R₃' und das N-Atom, an dem sie hängen, zusammen eine 4- bis 7-gliedrige Heterocyclogruppe bilden;
m 0, 1, 2, 3, 4 oder 5 ist;
n 0, 1, 2, 3, 4 oder 5 ist;
R₇ und R₈ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
R₅ und R₆ unabhängig aus H, D, C₁₋₆ Alkyl oder C₁₋₆ Halogenalkyl ausgewählt sind, wobei das C₁₋₆ Alkyl oder C₁₋₆ Halogenalkyl optional mit 1, 2, 3, 4, 5, 6, 7, 8 oder 9 D substituiert ist, bis hin zur vollständigen Deuterierung;
wobei R₃ optional substituiert ist mit einer oder mehreren Gruppen ausgewählt aus H, D, Halogen, C₁₋₆ Alkyl, C₁₋₆ Halogenalkyl oder C₁₋₆ Alkoxy.

5. Verbindung, oder das Tautomer, das Stereoisomer, die Kristallform, das pharmazeutisch annehmbare Salz, das Hydrat oder das Solvat davon, nach Anspruch 1, welche eine Verbindung der Formel (I) ist: 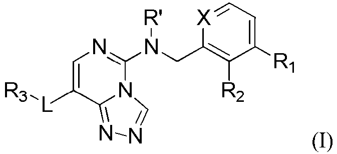 wobei
X N oder C(Rₓ) ist;
wobei Rₓ aus H, Halogen, C₁₋₆ Alkyl, C₁₋₆ Halogenalkyl oder C₁₋₆ Alkoxy ausgewählt ist;
R₁ aus H, Halogen, C₁₋₆ Alkyl, C₁₋₆ Halogenalkyl oder C₁₋₆ Alkoxy ausgewählt ist;
R₂ aus H, Halogen, C₁₋₆ Alkyl, C₁₋₆ Halogenalkyl oder C₁₋₆ Alkoxy ausgewählt ist;
oder R₁ und R₂ zusammen eine -(CH₂)ₚY-, -Y(CH₂)ₚ- oder -Y(CH₂)ₚZ- Gruppe bilden;
wobei p 1, 2, 3, 4 oder 5 ist;
Y aus O, S, NH oder chemischer Bindung ausgewählt ist;
Z aus O, S, NH oder chemischer Bindung ausgewählt ist;
R' aus H, C₁₋₆ Alkyl oder C₁₋₆ Halogenalkyl ausgewählt ist;
L aus -C(O)-N(R)-, -N(R₃')-C(O)-, -O-, -S-, -N(R")-, -C₁₋₄ Alkylen- oder -C₂₋₄ Alkenylen- ausgewählt ist;
wobei R aus H, C₁₋₆ Alkyl oder C₁₋₆ Halogenalkyl ausgewählt ist;
R₃' aus H, (CH₂)ₘ-C₁₋₆ Alkyl, (CH₂)ₘ-C₁₋₆ Halogenalkyl oder (CH₂)ₙ-C₃₋₇ Cycloalkyl ausgewählt ist;
R" aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl, (CH₂)ₙ-C₁₋₆ Alkoxy, (CH₂)ₙ-C₁₋₆ Halogenalkoxy, (CH₂)ₙ-C₃₋₇ Cycloalkyl, (CH₂)ₙ-3- bis 10-gliedrigem Heterocyclo, (CH₂)ₙ-C₆₋₁₀ Aryl oder (CH₂)ₙ-5- bis 10-gliedrigem Heteroaryl ausgewählt ist;
oder R₃, R₃' und das N-Atom, an dem sie hängen, zusammen eine 4- bis 7-gliedrige Heterocyclogruppe bilden;
wobei m 0, 1, 2, 3, 4 oder 5 ist;
n 0, 1, 2, 3, 4 oder 5 ist.

6. Verbindung, oder das Tautomer, das Stereoisomer, die Kristallform, das pharmazeutisch annehmbare Salz, das Hydrat oder das Solvat davon, nach Anspruch 5, welche eine Verbindung der Formel (II) ist: 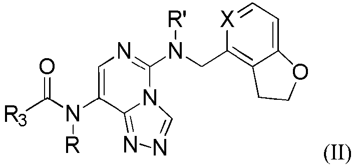 wobei
X is N oder C(Rₓ) ist; alternativ wobei X C(Rₓ) ist;
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist;
R aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl, (CH₂)ₙ-C₁₋₆ Alkoxy, (CH₂)ₙ-C₁₋₆ Halogenalkoxy, (CH₂)ₙ-C₃₋₇ Cycloalkyl, (CH₂)ₙ-4- bis 7-gliedrigem Heterocyclo, (CH₂)ₙ-C₆₋₁₀ Aryl oder (CH₂)ₙ-5- bis 10-gliedrigem Heteroaryl ausgewählt ist;
wobei m 0, 1, 2, 3, 4 oder 5 ist;
n 0, 1, 2, 3, 4 oder 5 ist;
alternativ wobei
X N oder C(Rₓ) ist; alternativ wobei X C(Rₓ) ist;
wobei Rₓ aus H, Halogen, C₁₋₆ Alkyl oder C₁₋₆ Halogenalkyl ausgewählt ist; alternativ wobei Rₓ aus H oder Halogen ausgewählt ist;
R' aus H, C₁₋₆ Alkyl oder C₁₋₆ Halogenalkyl ausgewählt ist;
R aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl, (CH₂)ₙ-C₁₋₆ Alkoxy, (CH₂)ₙ-C₁₋₆ Halogenalkoxy, (CH₂)ₙ-C₃₋₄ Cycloalkyl oder (CH₂)ₙ-C₆₋₁₀ Aryl ausgewählt ist;
wobei m 0, 1, 2, 3, 4 oder 5 ist;
n 0, 1, 2, 3, 4 oder 5 ist;
alternativ wobei
X N oder C(Rₓ) ist; alternativ wobei X C(Rₓ) ist;
wobei Rₓ aus H, Halogen, C₁₋₆ Alkyl oder C₁₋₆ Halogenalkyl ausgewählt ist; alternativ wobei Rₓ aus H oder Halogen ausgewählt ist;
R' aus H, C₁₋₆ Alkyl oder C₁₋₆ Halogenalkyl ausgewählt ist;
R aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl oder (CH₂)ₙ-C₃₋₄ Cycloalkyl ausgewählt ist; alternativ wobei R₃ aus (CH₂)ₘ-C₁₋₆ Halogenalkyl oder (CH₂)ₙ-C₃₋₄ Cycloalkyl ausgewählt ist;
wobei m 0, 1, 2, 3, 4 oder 5 ist;
n 0, 1, 2, 3, 4 oder 5 ist;
alternativ wobei
X C(Rₓ) ist;
wobei Rₓ aus H oder Halogen ausgewählt ist;
R' aus H, C₁₋₆ Alkyl oder C₁₋₆ Halogenalkyl ausgewählt ist;
R aus H, C₁₋₆ Alkyl oder C₁₋₆ Halogenalkyl ausgewählt ist;
R₃ aus (CH₂)ₘ-C₁₋₆ Halogenalkyl oder (CH₂)ₙ-C₃₋₄ Cycloalkyl ausgewählt ist;
wobei m 0, 1, 2 oder 3 ist;
n 0, 1, 2 oder 3 ist.

7. Verbindung, oder das Tautomer, das Stereoisomer, die Kristallform, das pharmazeutisch annehmbare Salz, das Hydrat oder das Solvat davon, nach Anspruch 5, welche eine Verbindung der Formel (III) ist: 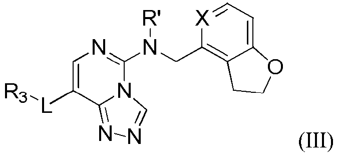 wobei
X N oder C(Rₓ) ist; alternativ wobei X C(Rₓ) ist;
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist;
L aus -O-, -S-, -N(R")-, -C₁₋₄ Alkylen- oder -C₂₋₄ Alkenylen- ausgewählt ist; alternativ wobei L -O- ist;
wobei R" aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl, (CH₂)ₙ-C₁₋₆ Alkoxy, (CH₂)ₙ-C₁₋₆ Halogenalkoxy, (CH₂)ₙ-C₃₋₇ Cycloalkyl, (CH₂)ₙ-4- bis 7-gliedrigem Heterocyclo, (CH₂)ₙ-C₆₋₁₀ Aryl oder (CH₂)ₙ-5- bis 10-gliedrigem Heteroaryl ausgewählt ist; alternativ wobei R₃ aus (CH₂)ₘ-C₁₋₆ Alkyl, (CH₂)ₘ-C₁₋₆ Halogenalkyl, (CH₂)ₙ-C₃₋₇ Cycloalkyl, (CH₂)ₙ-4- bis 7-gliedrigem Heterocyclo, (CH₂)ₙ-C₆₋₁₀ Aryl oder (CH₂)ₙ-5- bis 10-gliedrigem Heteroaryl ausgewählt ist;
wobei m 0, 1, 2, 3, 4 oder 5 ist;
n 0, 1, 2, 3, 4 oder 5 ist;
alternativ wobei
X C(Rₓ) ist;
wobei Rₓ aus H oder Halogen ausgewählt ist;
R' aus H, C₁₋₆ Alkyl oder C₁₋₆ Halogenalkyl ausgewählt ist;
L -O- ist;
R₃ aus (CH₂)ₙ-C₃₋₇ Cycloalkyl oder (CH₂)ₙ-4- bis 7-gliedrigem Heterocyclo enthaltend nur O als Heteroatom ausgewählt ist;
wobei m 0, 1, 2, 3, 4 oder 5 ist;
n 0, 1, 2, 3, 4 oder 5 ist;
alternativ wobei
X N oder C(Rₓ) ist; alternativ wobei X C(Rₓ) ist;
wobei Rₓ aus H, Halogen, C₁₋₆ Alkyl oder C₁₋₆ Halogenalkyl ausgewählt ist; alternativ wobei Rₓ aus H oder Halogen ausgewählt ist;
R' aus H, C₁₋₆ Alkyl oder C₁₋₆ Halogenalkyl ausgewählt ist;
L O ist;
R₃ aus (CH₂)ₘ-C₁₋₆ Alkyl, (CH₂)ₘ-C₁₋₆ Halogenalkyl, (CH₂)ₙ-C₃₋₇ Cycloalkyl oder (CH₂)ₙ-5- bis 6-gliedrigem Heteroaryl enthaltend nur O als Heteroatom ausgewählt ist;
wobei m 0, 1, 2, 3, 4 oder 5 ist;
n 0, 1, 2, 3, 4 oder 5 ist;
alternativ wobei
X N oder C(Rₓ) ist; alternativ wobei X C(Rₓ) ist;
wobei Rₓ aus H, Halogen, C₁₋₆ Alkyl oder C₁₋₆ Halogenalkyl ausgewählt ist; alternativ wobei Rₓ aus H oder Halogen ausgewählt ist;
R' aus H, C₁₋₆ Alkyl oder C₁₋₆ Halogenalkyl ausgewählt ist;
L -O- ist;
R₃ aus (CH₂)ₘ-C₁₋₆ Alkyl, (CH₂)ₘ-C₁₋₆ Halogenalkyl oder (CH₂)ₙ-C₃₋₇ Cycloalkyl ausgewählt ist;
wobei m 0, 1, 2, 3, 4 oder 5 ist;
n 0, 1, 2, 3, 4 oder 5 ist.

8. Verbindung, oder das Tautomer, das Stereoisomer, die Kristallform, das pharmazeutisch annehmbare Salz, das Hydrat oder das Solvat davon, nach Anspruch 5, welche eine Verbindung der Formel (IV) ist: 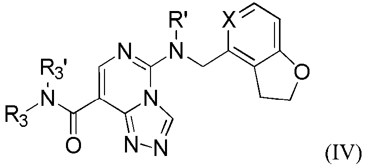 wobei
X N oder C(Rₓ) ist; alternativ wobei X C(Rₓ) ist;
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl, (CH₂)ₙ-C₃₋₇ Cycloalkyl, (CH₂)ₙ-4- bis 7-gliedrigem Heterocyclo, (CH₂)ₙ-C₆₋₁₀ Aryl oder (CH₂)ₙ-5- bis 10-gliedrigem Heteroaryl ausgewählt ist;
R₃' aus H, (CH₂)ₘ-C₁₋₆ Alkyl, (CH₂)ₘ-C₁₋₆ Halogenalkyl oder (CH₂)ₙ-C₃₋₇ Cycloalkyl ausgewählt ist;
oder R₃, R₃' und das N-Atom, an dem sie hängen, zusammen eine 4- bis 7-gliedrige Heterocyclogruppe bilden;
wobei m 0, 1, 2, 3, 4 oder 5 ist;
n 0, 1, 2, 3, 4 oder 5 ist;
alternativ wobei
X N oder C(Rₓ) ist; alternativ wobei X C(Rₓ) ist;
wobei Rₓ aus H, Halogen, C₁₋₆ Alkyl, C₁₋₆ Halogenalkyl oder C₁₋₆ Alkoxy ausgewählt ist; alternativ wobei Rₓ aus H oder Halogen ausgewählt ist;
R' aus H, C₁₋₆ Alkyl oder C₁₋₆ Halogenalkyl ausgewählt ist;
R₃ aus (CH₂)ₘ-C₁₋₆ Alkyl, (CH₂)ₘ-C₁₋₆ Halogenalkyl oder (CH₂)ₙ-C₃₋₇ Cycloalkyl ausgewählt ist;
R₃' aus H, (CH₂)ₘ-C₁₋₆ Alkyl, (CH₂)ₘ-C₁₋₆ Halogenalkyl oder (CH₂)ₙ-C₃₋₇ Cycloalkyl ausgewählt ist;
oder R₃, R₃' und das N-Atom, an dem sie hängen, zusammen eine 4- bis 7-gliedrige Heterocyclogruppe bilden;
wobei m 0, 1, 2, 3, 4 oder 5 ist;
n is 0, 1, 2, 3, 4 oder 5 ist.

9. Verbindung, oder das Tautomer, das Stereoisomer, die Kristallform, das pharmazeutisch annehmbare Salz, das Hydrat oder das Solvat davon, nach einem der Ansprüche 1 bis 8, wobei R' H ist.

10. Verbindung, oder das Tautomer, das Stereoisomer, die Kristallform, das pharmazeutisch annehmbare Salz, das Hydrat oder das Solvat davon, nach einem der Ansprüche 1 bis 8, wobei R₃ (CH₂)ₙ-3- bis 10-gliedriges Heterocyclo ist.

11. Verbindung, oder das Tautomer, das Stereoisomer, die Kristallform, das pharmazeutisch annehmbare Salz, das Hydrat oder das Solvat davon, nach einem der Ansprüche 1 bis 8, wobei R₅ und R₆ H sind.

12. Verbindung, oder das Tautomer, das Stereoisomer, die Kristallform, das pharmazeutisch annehmbare Salz, das Hydrat oder das Solvat davon, nach Anspruch 1, wobei die Verbindung ausgewählt ist aus:

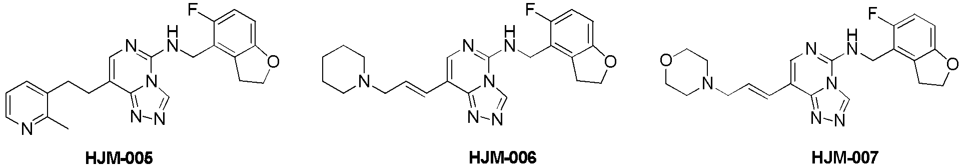

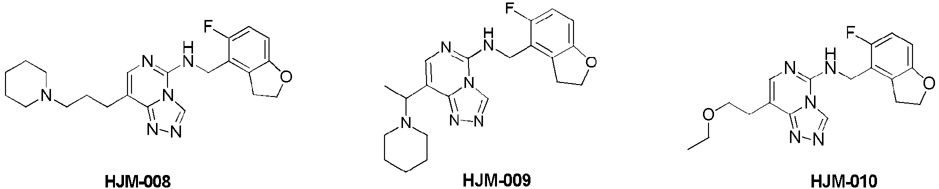

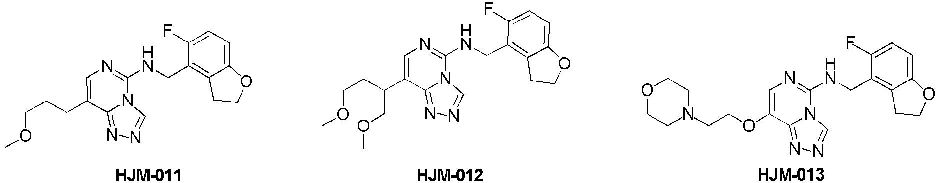

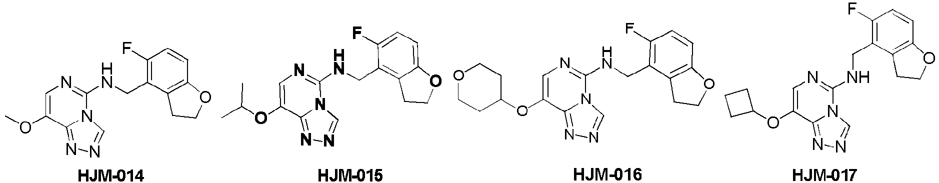

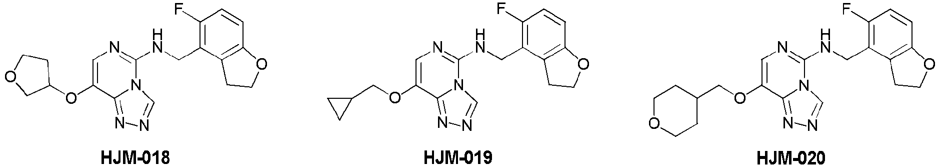

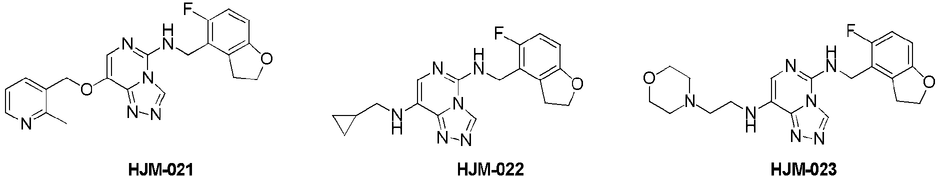

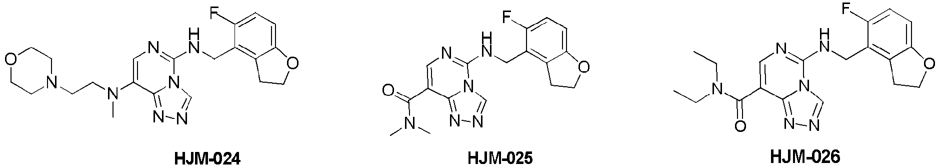

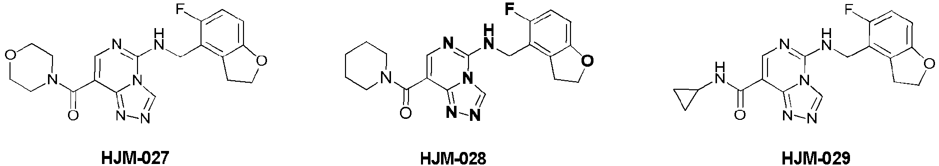

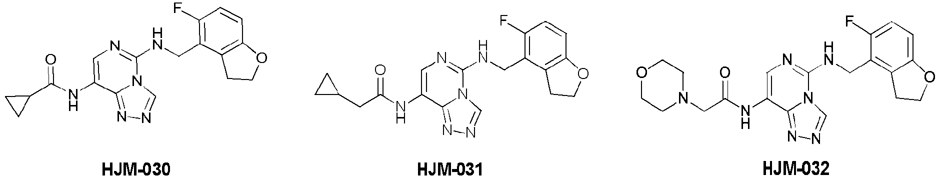

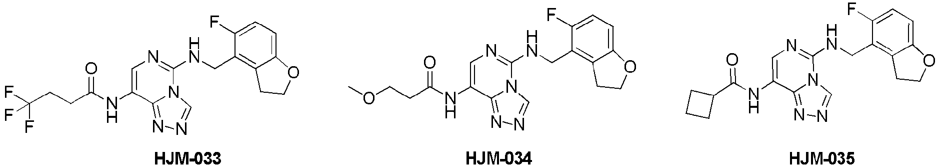

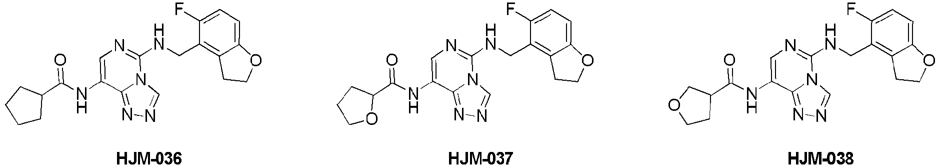

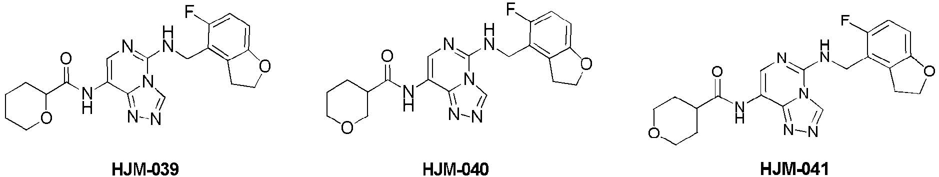

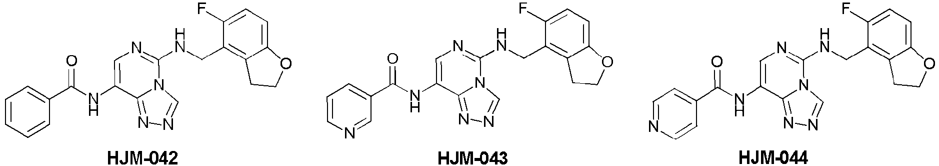

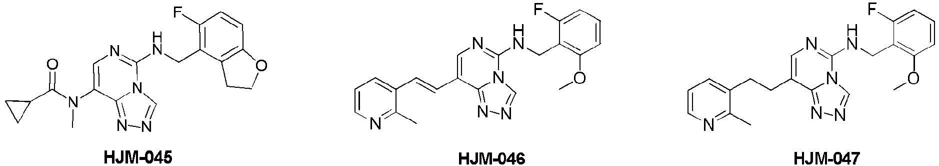

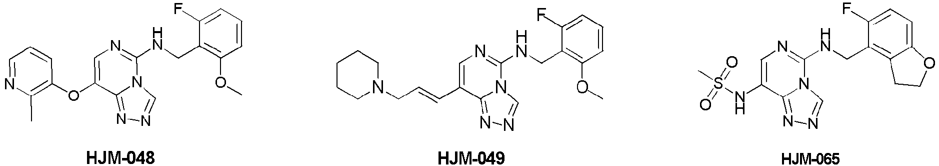

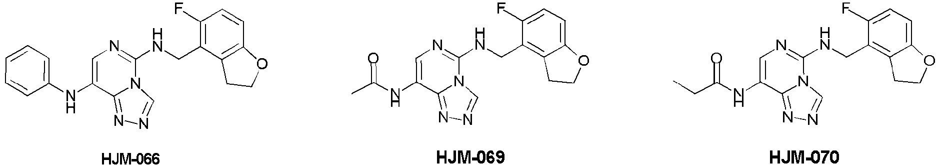

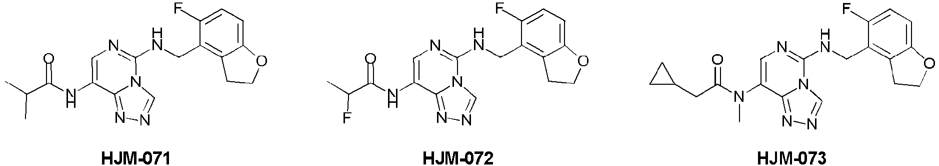

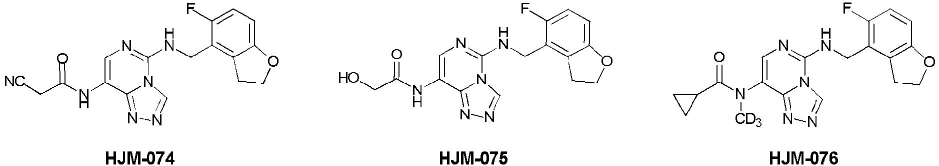

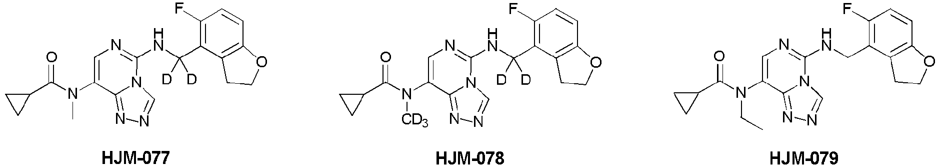

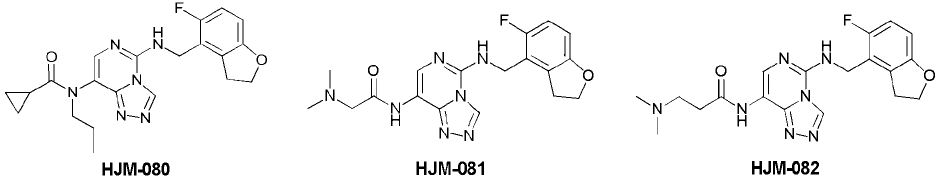

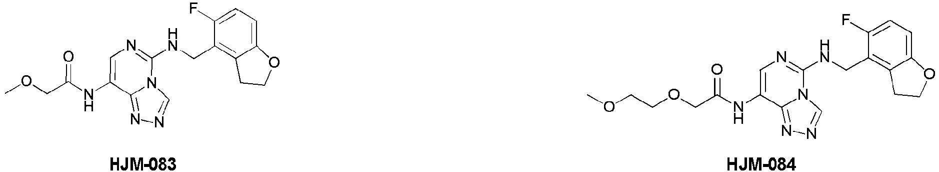

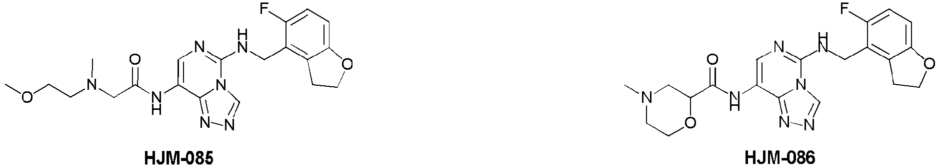

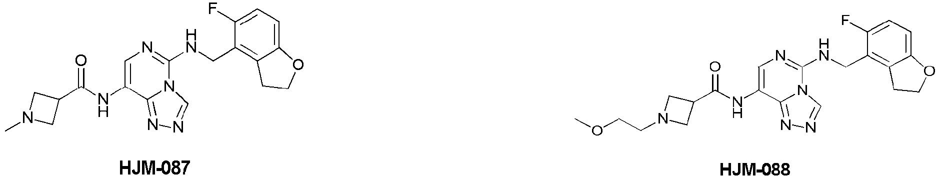

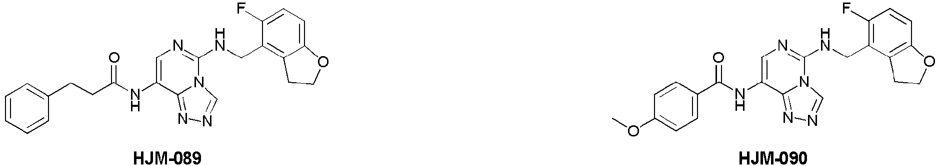

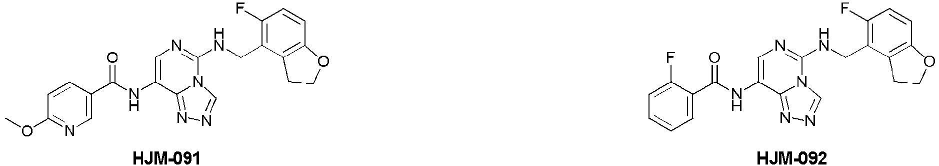

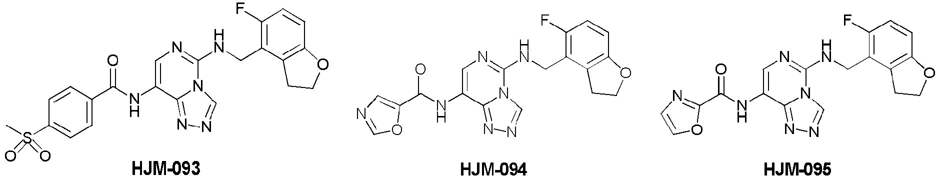

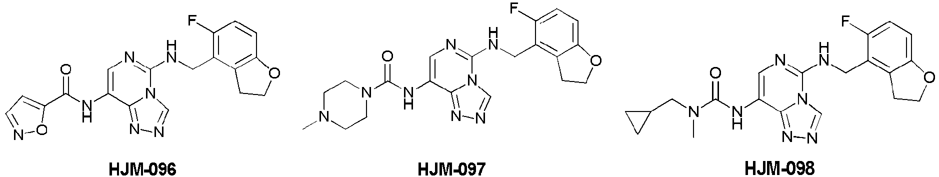

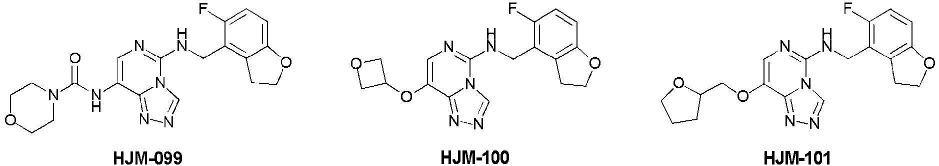

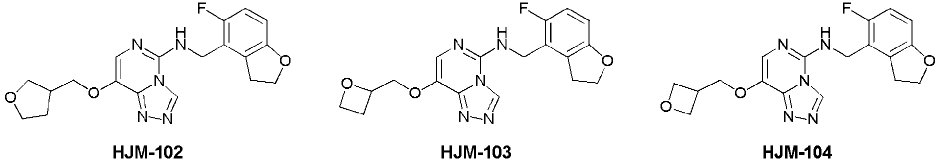

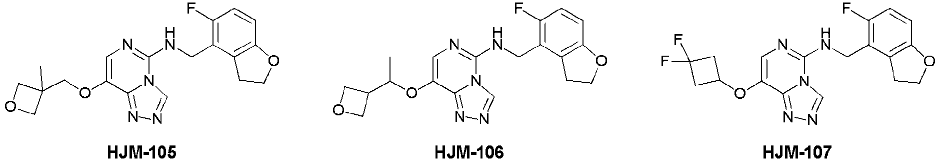

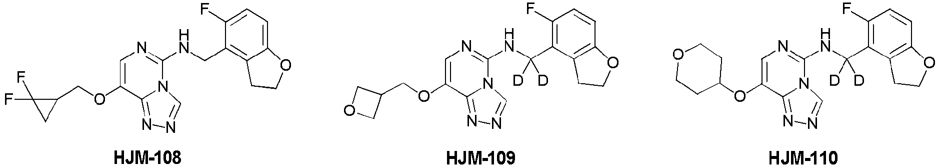

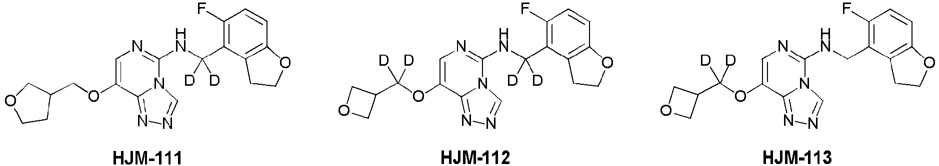

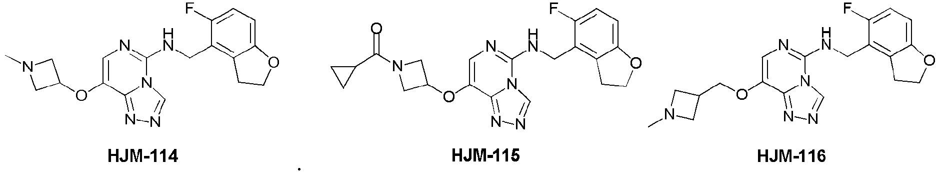

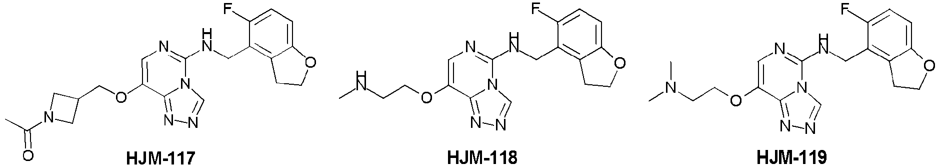

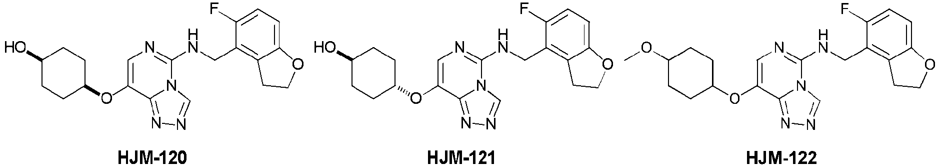

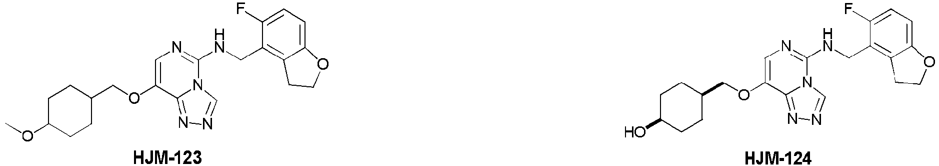

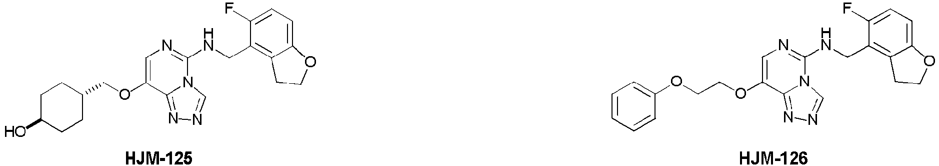

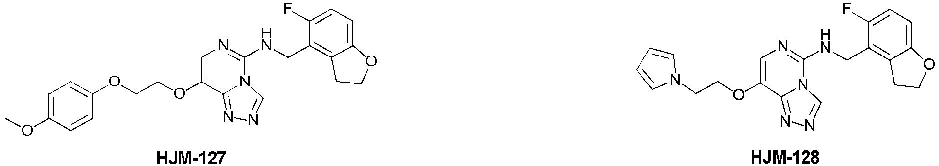

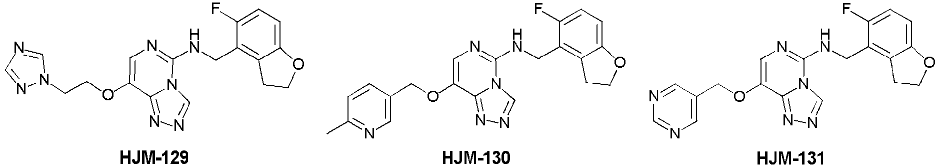

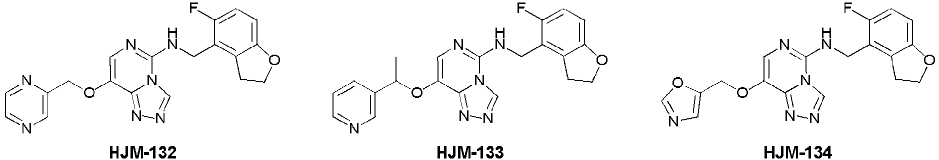

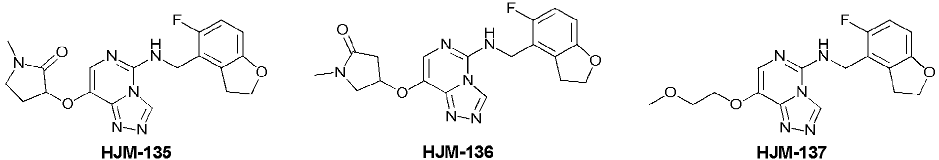

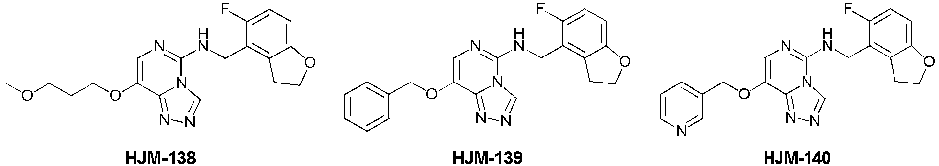

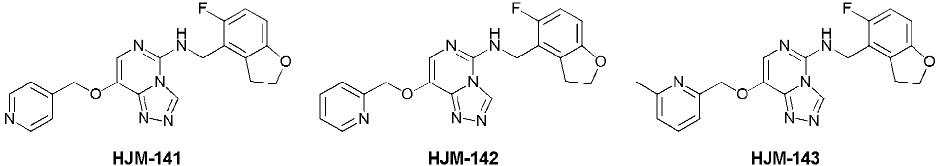

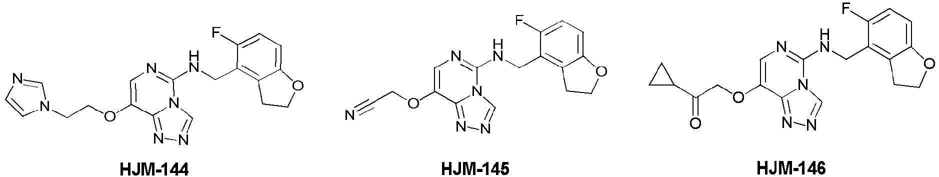

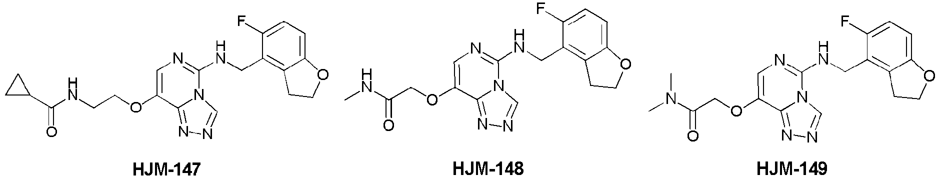

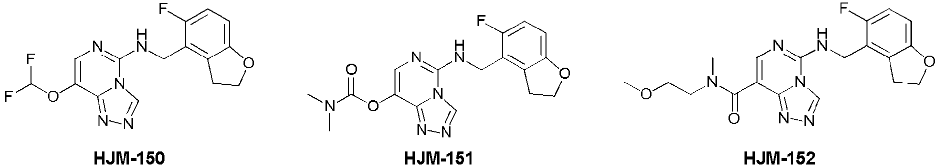

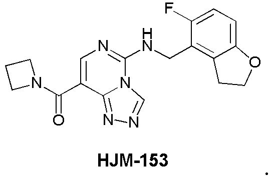

13. Pharmazeutische Zusammensetzung, enthaltend die Verbindung, oder das Tautomer, das Stereoisomer, die Kristallform, das pharmazeutisch annehmbare Salz, das Hydrat oder das Solvat davon, nach einem der Ansprüche 1 bis 12 sowie pharmazeutisch annehmbare(n) Hilfsstoff(e);
alternativ wobei die pharmazeutische Zusammensetzung in Kombination mit Immun-Checkpoint-Inhibitoren wie PD-1, PD-L1, CTLA-4 Antikörpern usw. verwendet wird.

14. Verbindung, oder das Tautomer, das Stereoisomer, die Kristallform, das pharmazeutisch annehmbare Salz, das Hydrat oder das Solvat davon, nach einem der Ansprüche 1 bis 12, oder pharmazeutische Zusammensetzung nach Anspruch 13, zur Verwendung bei der Behandlung und/oder Vorbeugung einer EED- und/oder PRC2-vermittelten Erkrankung, ausgewählt aus einer tumorbedingten Erkrankung, einer Immunschwächeerkrankung oder einer Autoimmunerkrankung; alternativ ist die tumorbezogene Erkrankung ausgewählt aus diffusem großzelligem B-Zell-Lymphom, follikulärem Lymphom, anderen Lymphomen, Leukämie, multiplem Myelom, Mesotheliom, Magenkrebs, malignem Rhabdomyom, Leberkrebs, Prostatakrebs, Brustkrebs, Gallengang- und Gallenblasenkrebs, Blasenkrebs, Hirntumoren (einschließlich Neuroblastom, Schwannom, Gliom, Glioblastom und Astrozytom), Gebärmutterhalskrebs, Darmkrebs, Melanom, Gebärmutterkrebs, Speiseröhrenkrebs, Kopf- und Halskrebs, Lungenkrebs, Nasen-Rachen-Krebs, Eierstockkrebs, Bauchspeicheldrüsenkrebs, Nierenzellkrebs, Enddarmkrebs, Schilddrüsenkrebs, Nebenschilddrüsentumor, Gebärmuttertumor und Weichteilsarkom; wobei die Immunschwächeerkrankung oder Autoimmunerkrankung unter anderem systemischen Lupus erythematodes, Morbus Crohn, Colitis ulcerosa, Multiple Sklerose, aplastische Anämie aufgrund von Autoimmunität und Abstoßungsreaktionen aufgrund von Organtransplantationen usw. einschließt, aber nicht darauf beschränkt ist, oder alternativ zur Verwendung bei der Immunisierung gegen Tumore.

15. Wirkstoffkombination, umfassend die Verbindung, oder das Tautomer, das Stereoisomer, die Kristallform, das pharmazeutisch annehmbare Salz, das Hydrat oder das Solvat davon, nach einem der Ansprüche 1 bis 12, oder die pharmazeutische Zusammensetzung nach Anspruch 13, und Immun-Checkpoint-Inhibitor(en) wie PD-1, PD-L1, CTLA-4 Antikörper usw.

## Revendications

1. Composé de formule (X), ou tautomère, stéréoisomère, forme cristalline, sel pharmaceutiquement acceptable, hydrate ou solvate de celui-ci : 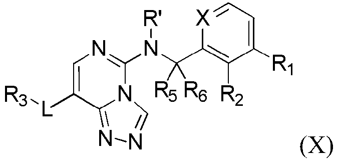
X étant N ou C(Rₓ) ;
Rₓ étant choisi parmi H, un halogène, un alkyle en C₁ à C₆, un haloalkyle en C₁ à C₆ ou un alcoxy en C₁ à C₆ ;
R₁ étant choisi parmi H, un halogène, un alkyle en C₁ à C₆, un haloalkyle en C₁ à C₆ ou un alcoxy en C₁ à C₆ ;
R₂ étant choisi parmi H, un halogène, un alkyle en C₁ à C₆, un haloalkyle en C₁ à C₆ ou un alcoxy en C₁ à C₆ ;
ou R₁ et R₂ formant ensemble un groupe -(CH₂)ₚY-, -Y(CH₂)ₚ- ou -Y(CH₂)ₚZ- ;
p étant 1, 2, 3, 4 ou 5 ;
Y étant choisi parmi O, S, NH ou une liaison chimique ;
Z étant choisi parmi O, S, NH ou une liaison chimique ;
R' étant choisi parmi H, un alkyle en C₁ à C₆ ou un haloalkyle en C₁ à C₆ ;
L étant choisi parmi -C(O)-N(R)-, -C(O)-O-, -N(R₃')-C(O)-, -OC(O)-, -S(O)_{q}-N(R)-, - S(O)_{q}-O-, -O-, -S-, -N(R'')-, -un alkylène en C₁ à C₄ - ou un alcénylène C₂ à C₄- ;
R étant choisi parmi H, un alkyle en C₁ à C₆ ou un haloalkyle en C₁ à C₆ ;
R_{3'} étant choisi parmi H, un (CH₂)ₘ-alkyle en C₁ à C₆, un (CH₂)ₘ-haloalkyle en C₁ à C₆ ou un (CH₂)ₙ-cycloalkyle en C₃ à C₇ ;
R" étant choisi parmi H, un alkyle en C₁ à C₆ ou un haloalkyle en C₁ à C₆ ;
q étant 0, 1 ou 2 ;
R₃ étant choisi parmi un (CH₂)ₘ-alkyle en C₁ à C₆, un (CH₂)ₘ-haloalkyle en C₁ à C₆, un (CH₂)ₙ-alcoxy en C₁ à C₆, un (CH₂)ₙ-haloalcoxy en C₁ à C₆, un (CH₂)ₙ-NRₐR_{b}, un (CH₂)ₙ-ORₐ, un (CH₂)ₙ-cycloalkyle en C₃ à C₇, un (CH₂)ₙ-hétérocyclyle ayant 3 à 10 chaînons, un (CH₂)ₙ-aryle en C₆ à C₁₀ ou un (CH₂)ₙ-hétéroaryle ayant 5 à 10 chaînons ;
ou, R₃, R₃' et l'atome N auquel ils sont attachés formant ensemble un groupe hétérocyclyle ayant 4 à 7 chaînons ;
m étant 0, 1, 2, 3, 4 ou 5 ;
n étant 0, 1, 2, 3, 4 ou 5 ;
Rₐ étant choisi parmi H, un alkyle en C₁ à C₆ ou un haloalkyle en C₁ à C₆ ;
R_{b} étant choisi parmi H, un alkyle en C₁ à C₆ ou un haloalkyle en C₁ à C₆ ;
R₅ et R₆ étant choisis indépendamment parmi H, D, un alkyle en C₁ à C₆ ou un haloalkyle en C₁ à C₆ ;
R₃ étant éventuellement substitué par un ou plusieurs groupes substituants choisis parmi H, D, un halogène, CN, OH, =O, NR_{c}R_{d}, un alkyle en C₁ à C₆, un haloalkyle en C₁ à C₆, un alcoxy en C₁ à C₆, un cycloalkyle en C₃ à C₇, un hétérocyclyle ayant 4 à 7 chaînons, un aryle en C₆ à C₁₀, un hétéroaryle ayant 5 à 10 chaînons, un (CH₂)ₘ-alkyle en C₁ à C₆, un (CH₂)ₘ-haloalkyle en C₁ à C₆, un (CH₂)ₙ-alcoxy en C₁ à C₆, un (CH₂)ₙ-haloalcoxy en C₁ à C₆, un -O-cycloalkyle en C₃ à C₇, un -O-hétérocyclyle ayant 4 à 7 chaînons, un -O-aryle en C₆ à C₁₀, un -O-hétéroaryle ayant 5 à 10 chaînons, un -C(O)-alkyle en C₁ à C₆, un -C(O)-haloalkyle en C₁ à C₆, un -C(O)-cycloalkyle en C₃ à C₇, un -C(O)-hétérocyclyle ayant 4 à 7 chaînons, un -C(O)-aryle en C₆ à C₁₀, un -C(O)-hétéroaryle ayant 5 à 10 chaînons, un - S(O)_{q}-alkyle en C₁ à C₆, un -S(O)_{q}-haloalkyle en C₁ à C₆, un -S(O)_{q}-alcoxy en C₁ à C₆, un - S(O)_{q}-cycloalkyle en C₃ à C₇, un -S(O)_{q}-hétérocyclyle ayant 4 à 7 chaînons, un -S(O)_{q}-aryle en C₆ à C₁₀ ou un -S(O)_{q}-hétéroaryle ayant 5 à 10 chaînons ;
R_{c} étant choisi parmi H, un alkyle en C₁ à C₆, un haloalkyle en C₁ à C₆, un -C(O)-cycloalkyle en C₃ à C₇, un -C(O)-hétérocyclyle ayant 4 à 7 chaînons, un -C(O)-aryle en C₆ à C₁₀ ou un -C(O)-hétéroaryle ayant 5 à 10 chaînons ;
R_{d} étant choisi parmi H, un alkyle en C₁ à C₆, un haloalkyle en C₁ à C₆, un -C(O)-cycloalkyle en C₃ à C₇, un -C(O)-hétérocyclyle ayant 4 à 7 chaînons, un -C(O)-aryle en C₆ à C₁₀ ou un -C(O)-hétéroaryle ayant 5 à 10 chaînons ;
les groupes alkyle, haloalkyle, alcoxy, haloalcoxy, cycloalkyle, hétérocyclyle, aryle, hétéroaryle, alkylène et alcénylène étant éventuellement substitués par des groupes substituants choisis parmi H, D, CN, OH, =O, un halogène, un alkyle en C₁ à C₆, un haloalkyle en C₁ à C₆, un alcoxy en C₁ à C₆, un cycloalkyle en C₃ à C₇, un hétérocyclyle ayant 4 à 7 chaînons, un aryle en C₆ à C₁₀, un hétéroaryle ayant 5 à 10 chaînons, un -O-cycloalkyle en C₃ à C₇, un hétérocyclyle ayant 4 à 7 chaînons, un -O-aryle en C₆ à C₁₀, un hétéroaryle ayant 5 à 10 chaînons, un -C(O)-alkyle en C₁ à C₆, un -C(O)-haloalkyle en C₁ à C₆, un -C(O)-cycloalkyle en C₃ à C₇, un -C(O)-hétérocyclyle ayant 4 à 7 chaînons, un - C(O)-aryle en C₆ à C₁₀ ou un -C(O)-hétéroaryle ayant 5 à 10 chaînons, ou substitué par un ou plusieurs D, jusqu_{'}à deutération complète.

2. Composé, ou tautomère, stéréoisomère, forme cristalline, sel pharmaceutiquement acceptable, hydrate ou solvate de celui-ci selon la revendication 1, lequel est un composé de formule (V) : 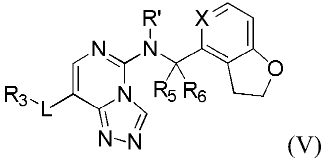
X étant N ou C(Rₓ) ; en variante, X étant C(Rₓ),
Rx étant choisi parmi H, un halogène, un alkyle en C₁ à C₆, un haloalkyle en C₁ à C₆ ou un alcoxy en C₁ à C₆ ; en variante, Rₓ étant choisi parmi H ou un halogène ;
R' étant choisi parmi H, un alkyle en C₁ à C₆ ou un haloalkyle en C₁ à C₆, l'alkyle en C₁ à C₆ ou l_{'}haloalkyle en C₁ à C₆ étant éventuellement substitué par 1, 2, 3, 4, 5, 6, 7, 8 ou 9 D, jusqu_{'}à deutération complète ;
L étant -C(O)-N(R)-, -C(O)-O-, -S(O)_{q}-N(R)- ou -S(O)_{q}-O- ;
R étant choisi parmi H, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
q étant 0, 1 ou 2 ;
R₃ étant choisi parmi un (CR₇R₈)ₘ-alkyle en C₁ à C₆, un (CR₇R₈)ₘ-haloalkyle en C₁ à C₆, un (CR₇R₈)ₙ-alcoxy en C₁ à C₆, un (CR₇R₈)ₙ-haloalcoxy en C₁ à C₆, un (CR₇R₈)ₙ-NRₐR_{b}, un (CR₇R₈)ₙ-cycloalkyle en C₃ à C₇, un (CR₇R₈)ₙ-hétérocyclyle ayant 4 à 7 chaînons, un (CR₇R₈)ₙ-aryle en C₆ C₁₀ ou un (CR₇R₈)ₙ-hétéroaryle ayant 5 à 10 chaînons ;
m étant 0, 1, 2, 3, 4 ou 5 ;
n étant 0, 1, 2, 3, 4 ou 5 ;
Rₐ étant choisi parmi H, un alkyle en C₁ à C₆ ou un haloalkyle en C₁ à C₆ ;
R_{b} étant choisi parmi H, un alkyle en C₁ à C₆ ou un haloalkyle en C₁ à C₆ ;
R₇ et R₈ étant choisis indépendamment parmi H, D, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₅ et R₆ étant choisis indépendamment parmi H, D, un alkyle en C₁ à C₆ ou un haloalkyle en C₁ à C₆, un alkyle en C₁ à C₆ ou un haloalkyle en C₁ à C₆ étant éventuellement substitué par 1, 2, 3, 4, 5, 6, 7, 8 ou 9 D, jusqu'à deutération complète ;
R₃ étant éventuellement substitué par un groupe choisi parmi H, D, un halogène, CN, OH, un alkyle en C₁ à C₆, un haloalkyle en C₁ à C₆, un cycloalkyle en C₃ à C₇, un hétérocyclyle ayant 4 à 7 chaînons, un aryle en C₆ à C₁₀, un hétéroaryle ayant 5 à 10 chaînons, un (CR₇R₈)ₙ-alcoxy en C₁ à C₆ ou un -S(O)_{q}-alkyle en C₁ à C₆ ;
les groupes alkyle, haloalkyle, alcoxy, haloalcoxy, cycloalkyle, hétérocyclyle, aryle et hétéroaryle étant éventuellement substitués par un ou plusieurs D, jusqu_{'}à deutération complète.

3. Composé, ou tautomère, stéréoisomère, forme cristalline, sel pharmaceutiquement acceptable, hydrate ou solvate de celui-ci selon la revendication 1, leuel est un composé de formule (VI) : 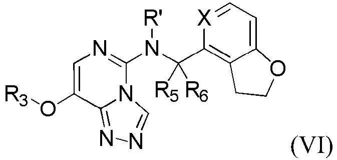
X étant N ou C(Rₓ) ; en variante, X étant C(Rₓ) ;
Rₓ étant choisi parmi H, un halogène, un alkyle en C₁ à C₆, un haloalkyle en C₁ à C₆ ou un alcoxy en C₁ à C₆ ; en variante, Rₓ étant choisi parmi H ou un halogène ;
R' étant choisi parmi H, un alkyle en C₁ à C₆ ou un haloalkyle en C₁ à C₆, l'alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₃ étant choisi parmi un (CR₇R₈)ₘ-alkyle en C₁ à C₆, un (CR₇R₈)ₘ-haloalkyle en C₁ à C₆, un (CR₇R₈)ₙ-cycloalkyle en C₃ à C₇, un (CR₇R₈)ₙ-hétérocyclyle ayant 4 à 7 chaînons, un (CR₇R₈)ₙ-aryle en C₆ à C₁₀ ou un (CR₇R₈)ₙ-hétéroaryle ayant 5 à 10 chaînons ;
m étant 0, 1, 2, 3, 4 ou 5 ;
n étant 0, 1, 2, 3, 4 ou 5 ;
R₇ et R₈ étant choisis indépendamment parmi H, D, un alkyle en C₁ à C₆ ou un haloalkyle en C₁ à C₆, l_{'}alkyle en C₁ à C₆ ou l_{'}haloalkyle en C₁ à C₆ étant éventuellement substitué par 1, 2, 3, 4, 5, 6, 7, 8 ou 9 D, jusqu'à deutération complète ; ou R₇ et R₈ sur le même atome de carbone étant combinés pour former =O ;
R₅ et R₆ étant choisis indépendamment parmi H, D, un alkyle en C₁ à C₆ ou un haloalkyle en C₁ à C₆, un alkyle en C₁ à C₆ ou un haloalkyle en C₁ à C₆ étant éventuellement substitué par 1, 2, 3, 4, 5, 6, 7, 8 ou 9 D, jusqu'à deutération complète ;
R₃ étant éventuellement substitué par un ou plusieurs groupes substituants choisis parmi H, D, un halogène, CN, OH, =O, NR_{c}R_{d}, un alkyle en C₁ à C₆, un haloalkyle en C₁ à C₆, un alcoxy en C₁ à C₆, un cycloalkyle en C₃ à C₇, un hétérocyclyle ayant 4 à 7 chaînons, un aryle en C₆ à C₁₀, un hétéroaryle ayant 5 à 10 chaînons, un -O-cycloalkyle en C₃ à C₇, un hétérocyclyle ayant 4 à 7 chaînons, un -O-aryle en C₆ à C₁₀, un hétéroaryle ayant 5 à 10 chaînons, un -C(O)-alkyle en C₁ à C₆, un -C(O)-haloalkyle en C₁ à C₆, un -C(O)-cycloalkyle en C₃ C₇, hétérocyclyle ayant 4 à 7 chaînons, un -C(O)-aryle en C₆ à C₁₀, ou un -C(O)-hétéroaryle ayant 5 à 10 chaînons;
R_{c} étant choisi parmi H, un alkyle en C₁ à C₆, un haloalkyle en C₁ à C₆, un -C(O)-cycloalkyle en C₃ à C₇, un -C(O)-hétérocyclyle ayant 4 à 7 chaînons, un -C(O)-aryle en C₆ à C₁₀ ou un -C(O)-hétéroaryle ayant 5 à 10 chaînons ;
R_{d} étant choisi parmi H, un alkyle en C₁ à C₆, un haloalkyle en C₁ à C₆, un -C(O)-cycloalkyle en C₃ à C₇, un -C(O)-hétérocyclyle ayant 4 à 7 chaînons, un -C(O)-aryle en C₆ à C₁₀ ou un -C(O)-hétéroaryle ayant 5 à 10 chaînons ;
les groupes alkyle, haloalkyle, alcoxy, cycloalkyle, hétérocyclyle, aryle et hétéroaryle étant éventuellement substitués par des groupes substituants choisis parmi H, D, CN, OH, =O, un halogène, un alkyle en C₁ à C₆, un haloalkyle en C₁ à C₆, un alcoxy en C₁ à C₆, un cycloalkyle en C₃ à C₇, un hétérocyclyle ayant 4 à 7 chaînons, un aryle en C₆ à C₁₀, hétéroaryle ayant 5 à 10 chaînons, un -O-cycloalkyle en C₃ à C₇, un hétérocyclyle ayant 4 à 7 chaînons, un -O-aryle en C₆ à C₁₀, un hétéroaryle ayant 5 à 10 chaînons, un -C(O)-alkyle en C₁ à C₆, un -C(O)-haloalkyle en C₁ à C₆, un -C(O)-cycloalkyle en C₃ à C₇, un hétérocyclyle ayant 4 à 7 chaînons, un -C(O)-aryle en C₆ à C₁₀, ou un -C(O)-hétéroaryle ayant 5 à 10 chaînons, ou substitué par un ou plusieurs D, jusqu'à deutération complète ;
en variante,
R₃ étant éventuellement substitué par un ou plusieurs groupes substituants choisis parmi H, D, un halogène, CN, OH, =O, NR_{c}R_{d}, un alkyle en C₁ à C₆, un haloalkyle en C₁ à C₆, un alcoxy en C₁ à C₆, un cycloalkyle en C₃ à C₇, un -C(O)-alkyle en C₁ à C₆, un -C(O)-cycloalkyle en C₃ à C₇ ou un -O-aryle en C₆ à C₁₀ ;
Rc étant choisi parmi H, un alkyle en C₁ à C₆, un haloalkyle en C₁ à C₆ ou un - C(O)-cycloalkyle en C₃ à C₇ ;
R_{d} étant choisi parmi H, un alkyle en C₁ à C₆, un haloalkyle en C₁ à C₆ ou un - C(O)-cycloalkyle en C₃ à C₇ ;
les groupes alkyle, haloalkyle, alcoxy, cycloalkyle, hétérocyclyle, aryle et hétéroaryle étant éventuellement substitués par des groupes substituants choisis parmi H, D, CN, =O, un halogène, un alkyle en C₁ à C₆, un haloalkyle en C₁ à C₆, un alcoxy en C₁ à C₆ ou un - C(O)-alkyle en C₁ à C₆, ou substitués par un ou plusieurs D, jusqu'à deutération complète ;
en variante,
X étant C(Rₓ);
Rₓ étant choisi parmi H ou halogène ;
R_{'} étant choisi parmi H, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₃ étant choisi parmi un (CR₇R₈)ₘ-alkyle en C₁ à C₆, un (CR₇R₈)ₘ-haloalkyle en C₁ à C₆, un (CR₇R₈)ₙ-cycloalkyle en C₃ à C₇ ou un (CR₇R₈)ₙ-hétérocyclyle ayant 4 à 7 chaînons contenant uniquement O comme hétéroatome ;
m étant 0, 1, 2, 3, 4 ou 5 ;
n étant 0, 1, 2, 3, 4 ou 5 ;
R₇ et R₈ étant choisis indépendamment parmi H, D, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₅ et R₆ étant choisis indépendamment parmi H, D, un alkyle en C₁ à C₆ ou un haloalkyle en C₁ à C₆, un alkyle en C₁ à C₆ ou un haloalkyle en C₁ à C₆ étant éventuellement substitué par 1, 2, 3, 4, 5, 6, 7, 8 ou 9 D, jusqu'à deutération complète ;
R₃ étant éventuellement substitué par un groupe choisi parmi H, D, un alkyle en C₁ à C₆, un haloalkyle en C₁ à C₆ ou un alcoxy en C₁ à C₆ ;
en variante,
X étant C(Rₓ);
Rₓ étant choisi parmi H ou un halogène ;
R' étant choisi parmi H, un alkyle en C₁ à C₆ ou un haloalkyle en C₁ à C₆, l_{'}alkyle en C₁ à C₆ ou l_{'}haloalkyle en C₁ à C₆ étant éventuellement substitué par 1, 2, 3, 4, 5, 6, 7, 8 ou 9 D, jusqu_{'}à deutération complète ;
R₃ étant choisi parmi un (CR₇R₈)ₘ-alkyle en C₁ à C₆, un (CR₇R₈)ₘ-haloalkyle en C₁ à C₆ ou un (CR₇R₈)ₙ-hétérocyclyle ayant 4 à 7 chaînons contenant uniquement O comme hétéroatome ;
m étant 0, 1, 2, 3, 4 ou 5 ;
n étant 0, 1, 2, 3, 4 ou 5 ;
R₇ et R₈ étant choisis indépendamment parmi H, D, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₅ et R₆ étant choisis indépendamment parmi H, D, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₃ étant éventuellement substitué par un groupe choisi parmi H, D, un alkyle en C₁ à C₆, un haloalkyle en C₁ à C₆ ou un alcoxy en C₁ à C₆.

4. Composé, ou tautomère, stéréoisomère, forme cristalline, sel pharmaceutiquement acceptable, hydrate ou solvate de celui-ci selon la revendication 1, lequel est un composé de formule (VII) : 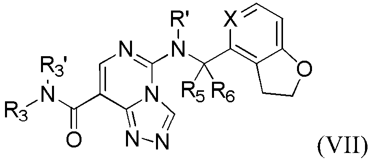
X étant N ou C(Rₓ) ; en variante, X étant C(Rₓ) ;
Rₓ étant choisi parmi H, un halogène, un alkyle en C₁ à C₆, un haloalkyle en C₁ à C₆ ou un alcoxy en C₁ à C₆ ; en variante, Rₓ étant choisi parmi H ou un halogène ;
R' étant choisi parmi H, un alkyle en C₁ à C₆ ou un haloalkyle en C₁ à C₆ ;
R₃ étant choisi parmi un (CR₇R₈)ₘ-alkyle en C₁ à C₆, un (CR₇R₈)ₘ-haloalkyle en C₁ à C₆, un (CR₇R₈)ₙ-cycloalkyle en C₃ à C₇, un (CR₇R₈)ₙ-hétérocyclyle ayant 4 à 7 chaînons, un (CR₇R₈)ₙ-aryle en C₆ à C₁₀ ou un (CR₇R₈)ₙ-hétéroaryle ayant 5 à 10 chaînons ;
R₃' étant choisi parmi H, un (CR₇R₈)ₘ-alkyle en C₁ à C₆, un (CR₇R₈)ₘ-haloalkyle en C₁ à C₆ ou un (CR₇R₈)ₙ-cycloalkyle en C₃ à C₇ ;
ou, R₃, R₃' et l'atome N auquel ils sont attachés formant ensemble un groupe hétérocyclyle ayant 4 à 7 chaînons ;
m étant 0, 1, 2, 3, 4 ou 5 ;
n étant 0, 1, 2, 3, 4 ou 5 ;
R₇ et R₈ étant choisis indépendamment parmi H, D, un alkyle en C₁ à C₆ ou un haloalkyle en C₁ à C₆, l_{'}alkyle en C₁ à C₆ ou l_{'}haloalkyle en C₁ à C₆ étant éventuellement substitué par 1, 2, 3, 4, 5, 6, 7, 8 ou 9 D, jusqu'à deutération complète ; ou R₇ et R₈ sur le même atome de carbone étant combinés pour former =O ;
R₅ et R₆ étant choisis indépendamment parmi H, D, un alkyle en C₁ à C₆ ou haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R3 étant éventuellement substitué par un ou plusieurs groupes choisis parmi H, D, un halogène, un alkyle en C₁ à C₆, un haloalkyle en C₁ à C₆ ou un alcoxy en C₁ à C₆ ;
en variante,
X étant C(Rₓ),
Rₓ étant choisi parmi H ou un halogène ;
R' étant choisi parmi H, un alkyle en C₁ à C₆ ou un haloalkyle en C₁ à C₆ ;
R₃ étant choisi parmi un (CR₇R₈)ₘ-alkyle en C₁ à C₆, un (CR₇R₈)ₘ-haloalkyle en C₁ à C₆ ou un (CR₇R₈)ₙ-cycloalkyle en C₃ à C₇ ;
R₃' étant choisi parmi H, un (CR₇R₈)ₘ-alkyle en C₁ à C₆, un (CR₇R₈)ₘ-haloalkyle en C₁ à C₆ ou un (CR₇R₈)ₙ-cycloalkyle en C₃ à C₇ ;
ou, R₃, R₃, et l'atome N auquel ils sont attachés formant ensemble un groupe hétérocyclyle ayant 4 à 7 chaînons ;
m étant 0, 1, 2, 3, 4 ou 5 ;
n étant 0, 1, 2, 3, 4 ou 5 ;
R₇ et R₈ étant choisis indépendamment parmi H, D, un alkyle en C₁ à C₆ ou un haloalkyle en C₁ à C₆, l_{'}alkyle en C₁ à C₆ ou l'haloalkyle en C₁ à C₆ étant éventuellement substitué par 1, 2, 3, 4, 5, 6, 7, 8 ou 9 D, jusqu'à deutération complète ;
R₅ et R₆ étant choisis indépendamment parmi H, D, un alkyle en C₁ à C₆ ou un haloalkyle en C₁ à C₆, l_{'}alkyle en C₁ à C₆ ou l_{'}haloalkyle en C₁ à C₆ étant éventuellement substitué par 1, 2, 3, 4, 5, 6, 7, 8 ou 9 D, jusqu à deutération complète ;
R₃ étant éventuellement substitué par un ou plusieurs groupes choisis parmi H, D, un halogène, un alkyle en C₁ à C₆, un haloalkyle en C₁ à C₆ ou un alcoxy en C₁ à C₆.

5. Composé, ou tautomère, stéréoisomère, forme cristalline, sel pharmaceutiquement acceptable, hydrate ou solvate de celui-ci selon la revendication 1, lequel est un composé de formule (1) : 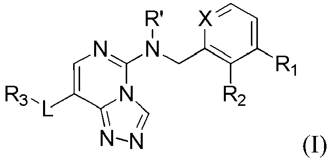
X étant N ou C(Rₓ) ;
Rₓ étant choisi parmi H, un halogène, un alkyle en C₁ à C₆, un haloalkyle en C₁ à C₆ ou un alcoxy en C₁ à C₆ ;
R₁ étant choisi parmi H, un halogène, un alkyle en C₁ à C₆, un haloalkyle en C₁ à C₆ ou un alcoxy en C₁ à C₆ ;
R₂ étant choisi parmi H, un halogène, un alkyle en C₁ à C₆, un haloalkyle en C₁ à C₆ ou un alcoxy en C₁ à C₆ ;
ou R₁ et R₂ formant ensemble un groupe -(CH₂)ₚY-, -Y(CH₂)ₚ- ou -Y(CH₂)ₚZ- ;
p étant 1, 2, 3, 4 ou 5 ;
Y étant choisi parmi O, S, NH ou une liaison chimique ;
Z étant choisi parmi O, S, NH ou une liaison chimique ;
R' étant choisi parmi H, un alkyle en C₁ à C₆ ou un haloalkyle en C₁ à C₆ ;
L étant choisi parmi -C(O)-N(R)-, -N(R3')-C(O)-, -O-, -S-, -N(R")-, -alkylène en C₁ à C₄- ou -alcénylène en C₂ à C₄- ;
R étant choisi parmi H, un alkyle en C₁ à C₆ ou un haloalkyle en C₁ à C₆ ;
R₃' étant choisi parmi H, un (CH₂)ₘ-alkyle en C₁ à C₆, un (CH₂)ₘ-haloalkyle en C₁ à C₆ ou un (CH₂)ₙ-cycloalkyle en C₃ à C₇ ;
R" étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆, un (CH₂)ₙ-alcoxy en C₁ à C₆, un (CH₂)ₙ-haloalcoxy en C₁ à C₆, un (CH₂)ₙ-cycloalkyle en C₃ à C₇, un (CH₂)ₙ-hétérocyclyle ayant 3 à 10 chaînons, un (CH₂)ₙ-aryle en C₆ à C₁₀ ou un (CH₂)ₙ-hétéroaryle ayant 5 à 10 chaînons ;
ou, R₃, R₃' et l'atome N auquel ils sont attachés formant ensemble un groupe hétérocyclyle ayant 4 à 7 chaînons ;
m étant 0, 1, 2, 3, 4 ou 5 ;
n étant 0, 1, 2, 3, 4 ou 5.

6. Composé, ou tautomère, stéréoisomère, forme cristalline, sel pharmaceutiquement acceptable, hydrate ou solvate de celui-ci selon la revendication 5, lequel est un composé de formule (II) : 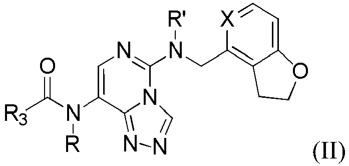
X étant N ou C(Rₓ) ; en variante, X étant C(Rₓ) ;
Rₓ étant choisi parmi H, un halogène, un alkyle en C₁ à C₆, un haloalkyle en C₁ à C₆ ou un alcoxy en C₁ à C₆ ; en variante, Rₓ étant choisi parmi H ou un halogène ;
R' étant choisi parmi H, un alkyle en C₁ à C₆ ou un haloalkyle en C₁ à C₆ ;
R étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆, un (CH₂)ₙ-alcoxy en C₁ à C₆, un (CH₂)ₙ-haloalcoxy en C₁ à C₆, un (CH₂)ₙ-cycloalkyle en C₃ à C₇, un (CH₂)ₙ-hétérocyclyle ayant 4 à 7 chaînons, un (CH₂)ₙ-aryle en C₆ à C₁₀ ou un (CH₂)ₙ-hétéroaryle ayant 5 à 10 chaînons ;
m étant 0, 1, 2, 3, 4 ou 5 ;
n étant 0, 1, 2, 3, 4 ou 5 ;
en variante,
X étant N ou C(Rₓ) ; en variante, X étant C(Rₓ) ;
Rₓ étant choisi parmi H, un halogène, un alkyle en C₁ à C₆ ou un haloalkyle en C₁ à C₆ ; en variante, Rₓ étant choisi parmi H ou un halogène ;
R' étant choisi parmi H, un alkyle en C₁ à C₆ ou un haloalkyle en C₁ à C₆ ;
R étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆, un (CH₂)ₙ-alcoxy en C₁ à C₆, un (CH₂)ₙ-haloalcoxy en C₁ à C₆, un (CH₂)ₙ-cycloalkyle en C₃ à C₄ ou un (CH₂)ₙ-aryle en C₆ à C₁₀ ;
m étant 0, 1, 2, 3, 4 ou 5 ;
n étant 0, 1, 2, 3, 4 ou 5 ;
en variante,
X étant N ou C(Rₓ) ; en variante, X étant C(Rₓ) ;
Rₓ étant choisi parmi H, un halogène, un alkyle en C₁ à C₆ ou un haloalkyle en C₁ à C₆ ; en variante, Rₓ étant choisi parmi H ou un halogène ;
R_{'} étant choisi parmi H, un alkyle en C₁ à C₆ ou un haloalkyle en C₁ à C₆ ;
R étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆ ou un (CH₂)ₙ-cycloalkyle en C₃ à C₄ ; en variante, R₃ étant choisi parmi un (CH₂)ₘ-haloalkyle en C₁ à C₆ ou un (CH₂)ₙ-cycloalkyle en C₃ à C₄ ;
m étant 0, 1, 2, 3, 4 ou 5 ;
n étant 0, 1, 2, 3, 4 ou 5 ;
en variante,
X étant C(Rₓ);
Rx étant choisi parmi H ou un halogène ;
R_{'} étant choisi parmi H, un alkyle en C₁ à C₆ ou un haloalkyle en C₁ à C₆ ;
R étant choisi parmi H, un alkyle en C₁ à C₆ ou un haloalkyle en C₁ à C₆ ;
R3 étant choisi parmi un (CH₂)ₘ-haloalkyle en C₁ à C₆ ou un (CH₂)ₙ-cycloalkyle en C₃ à C₄ ;
m étant 0, 1, 2 ou 3 ;
n étant 0, 1, 2 ou 3.

7. Composé, ou tautomère, stéréoisomère, forme cristalline, sel pharmaceutiquement acceptable, hydrate ou solvate de celui-ci selon la revendication 5, lequel est un composé de formule (III) : 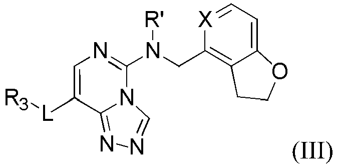
X étant N ou C(Rₓ) ; en variante, X est C(Rₓ) ;
Rₓ étant choisi parmi H, un halogène, un alkyle en C₁ à C₆, un haloalkyle en C₁ à C₆ ou un alcoxy en C₁ à C₆ ; en variante, Rₓ étant choisi parmi H ou un halogène ;
R' étant choisi parmi H, un alkyle en C₁ à C₆ ou un haloalkyle en C₁ à C₆ ;
L étant choisi parmi -O-, -S-, -N(R'')-, -alkylène en C₁ à C₄- ou -alcénylène en C₂ à C₄- ; en variante, L étant -O- ;
R" étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆, un (CH₂)ₙ-alcoxy en C₁ à C₆, un (CH₂)ₙ-haloalcoxy en C₁ à C₆, un (CH₂)ₙ-cycloalkyle en C₃ à C₇, un (CH₂)ₙ-hétérocyclyle ayant 4 à 7 chaînons, un aryle en C₆ à C₁₀ ou un (CH₂)ₙ-hétéroaryle ayant 5 à 10 chaînons ; en variante, R₃ étant choisi parmi un (CH₂)ₘ-alkyle en C₁ à C₆, un (CH₂)ₘ-haloalkyle en C₁ à C₆, un (CH₂)ₙ-cycloalkyle en C₃ à C₇, un (CH₂)ₙ-hétérocyclyle ayant 4 à 7 chaînons, un (CH₂)ₙ-aryle en C₆ à C₁₀ ou un (CH₂)ₙ-hétéroaryle ayant 5 à 10 chaînons ;
m étant 0, 1, 2, 3, 4 ou 5 ;
n étant 0, 1, 2, 3, 4 ou 5 ;
en variante,
X étant C(Rₓ) ;
Rₓ étant choisi parmi H ou un halogène ;
R' étant choisi parmi H, un alkyle en C₁ à C₆ ou un haloalkyle en C₁ à C₆ ;
L étant -O-;
R₃ étant choisi parmi un (CH₂)ₙ-cycloalkyle en C₃ à C₇ ou un (CH₂)ₙ-hétérocyclyle ayant 4 à 7 chaînons contenant uniquement O comme hétéroatome ;
m étant 0, 1, 2, 3, 4 ou 5 ;
n étant 0, 1, 2, 3, 4 ou 5 ;
en variante,
X étant N ou C(Rₓ) ; en variante, X étant C(Rₓ) ;
Rₓ étant choisi parmi H, un halogène, un alkyle en C₁ à C₆ ou un haloalkyle en C₁ à C₆ ; en variante, Rₓ étant choisi parmi H ou un halogène ;
R' étant choisi parmi H, un alkyle en C₁ à C₆ ou un haloalkyle en C₁ à C₆ ;
L étant O ;
R₃ étant choisi parmi un (CH₂)ₘ-alkyle en C₁ à C₆, un (CH₂)ₘ-haloalkyle en C₁ à C₆, un (CH₂)ₙ-cycloalkyle en C₃ à C₇ ou un (CH₂)ₙ-hétéroaryle à 5 à 6 chaînons contenant O comme hétéroatome ;
m étant 0, 1, 2, 3, 4 ou 5 ;
n étant 0, 1, 2, 3, 4 ou 5 ;
en variante,
X étant N ou C(Rₓ) ; en variante, X étant C(Rₓ) ;
Rₓ étant choisi parmi H, un halogène, un alkyle en C₁ à C₆ ou un haloalkyle en C₁ à C₆ ; en variante, Rₓ étant choisi parmi H ou un halogène ;
R_{'} étant choisi parmi H, un alkyle en C₁ à C₆ ou un haloalkyle en C₁ à C₆ ;
L étant -O-;
R₃ étant choisi parmi un (CH₂)ₘ-alkyle en C₁ à C₆, un (CH₂)ₘ-haloalkyle en C₁ à C₆ ou un (CH₂)ₙ-cycloalkyle en C₃ à C₇ ;
m étant 0, 1, 2, 3, 4 ou 5 ;
n étant 0, 1, 2, 3, 4 ou 5.

8. Composé, ou tautomère, stéréoisomère, forme cristalline, sel pharmaceutiquement acceptable, hydrate ou solvate de celui-ci selon la revendication 5, lequel est un composé de formule (IV) : 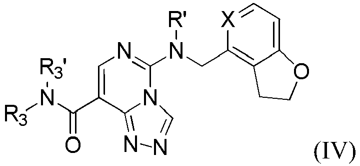
X étant N ou C(Rₓ) ; en variante, X étant C(Rₓ) ;
Rₓ étant choisi parmi H, un halogène, un alkyle en C₁ à C₆, un haloalkyle en C₁ à C₆ ou un alcoxy en C₁ à C₆ ; en variante, Rₓ étant choisi parmi H ou un halogène ;
R' étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆, un (CH₂)ₙ-cycloalkyle en C₃ à C₇, un (CH₂)ₙ-hétérocyclyle ayant 4 à 7 chaînons, un (CH₂)ₙ-aryle en C₆ à C₁₀ ou un (CH₂)ₙ-hétéroaryle ayant 5 à 10 chaînons ;
R₃' étant choisi parmi H, un (CH₂)ₘ-alkyle en C₁ à C₆, un (CH₂)ₘ-haloalkyle en C₁ à C₆ ou un (CH₂)ₙ-cycloalkyle en C₃ à C₇ ;
ou, R₃, R₃' et l'atome N auquel ils sont attachés formant ensemble un groupe hétérocyclyle ayant 4 à 7 chaînons ;
m étant 0, 1, 2, 3, 4 ou 5 ;
n étant 0, 1, 2, 3, 4 ou 5 ;
en variante,
X étant N ou C(Rₓ) ; en variante, X étant C(Rₓ) ;
Rₓ étant choisi parmi H, un halogène, un alkyle en C₁ à C₆, un haloalkyle en C₁ à C₆ ou un alcoxy en C₁ à C₆ ; en variante, Rx étant choisi parmi H ou un halogène ;
R' étant choisi parmi H, un alkyle en C₁ à C₆ ou un haloalkyle en C₁ à C₆ ;
R₃ étant choisi parmi un (CH₂)ₘ-alkyle en C₁ à C₆, un (CH₂)ₘ-haloalkyle en C₁ à C₆ ou un (CH₂)ₙ-cycloalkyle en C₃ à C₇ ;
R₃' étant choisi parmi H, un (CH₂)ₘ-alkyle en C₁ à C₆, un (CH₂)ₘ-haloalkyle en C₁ à C₆ ou un (CH₂)ₙ-cycloalkyle en C₃ à C₇ ;
ou, R₃, R₃' et l'atome N auquel ils sont attachés formant ensemble un groupe hétérocyclyle de 4 à 7 chaînons ;
m étant 0, 1, 2, 3, 4 ou 5 ;
n étant 0, 1, 2, 3, 4 ou 5.

9. Composé, ou tautomère, stéréoisomère, forme cristalline, sel pharmaceutiquement acceptable, hydrate ou solvate de celui-ci selon l_{'}une quelconque des revendications 1 à 8, R' étant H.

10. Composé, ou tautomère, stéréoisomère, forme cristalline, sel pharmaceutiquement acceptable, hydrate ou solvate de celui-ci selon l_{'}une quelconque des revendications 1 à 8, R₃ étant un (CH₂)ₙ-hétérocyclyle ayant 3 à 10 chaînons.

11. Composé, ou tautomère, stéréoisomère, forme cristalline, sel pharmaceutiquement acceptable, hydrate ou solvate de celui-ci selon l_{'}une quelconque des revendications 1 à 8, R₅ et R₆ étant H.

12. Composé, ou tautomère, stéréoisomère, forme cristalline, sel pharmaceutiquement acceptable, hydrate ou solvate de celui-ci selon la revendication 1, le composé étant choisi parmi :

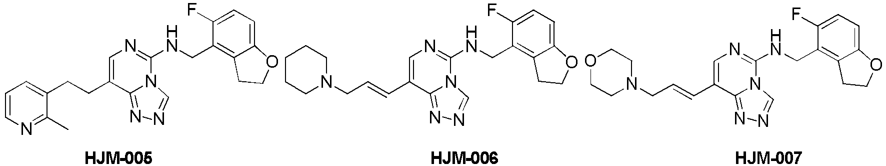

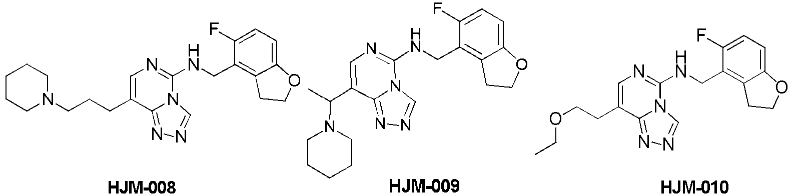

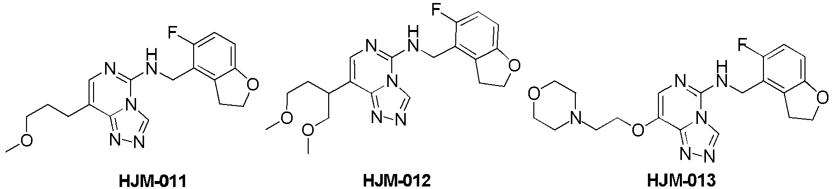

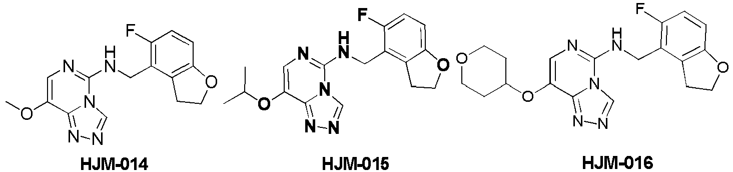

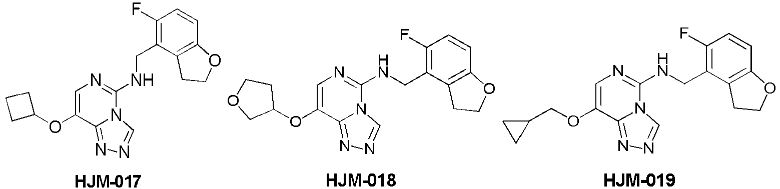

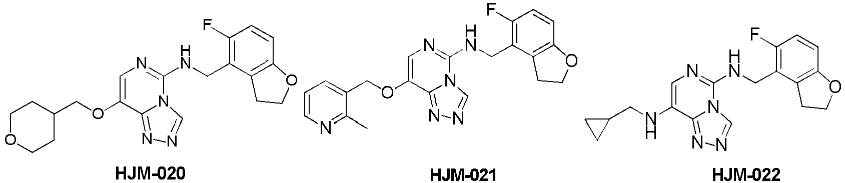

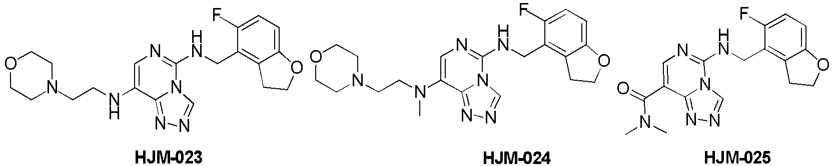

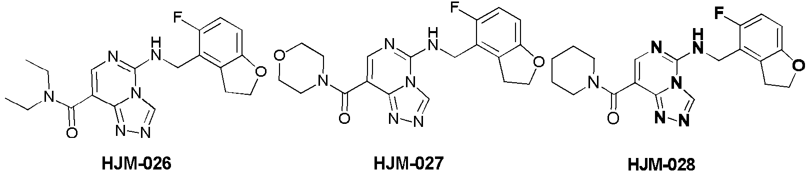

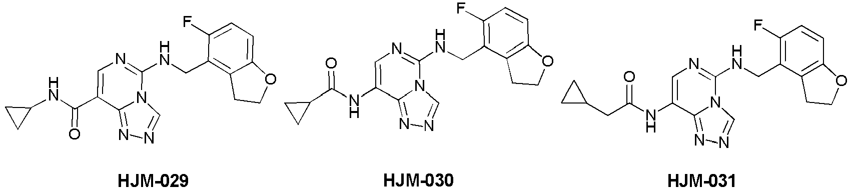

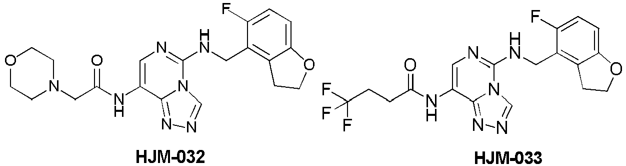

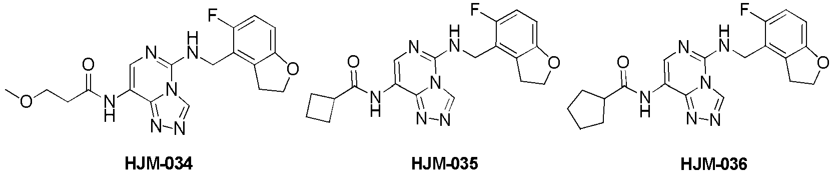

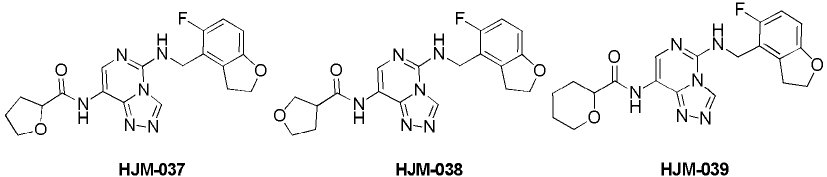

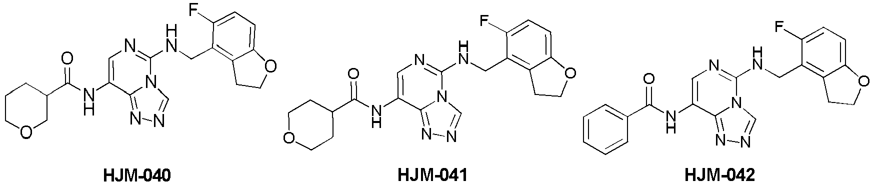

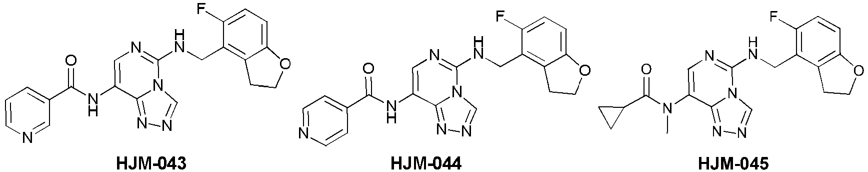

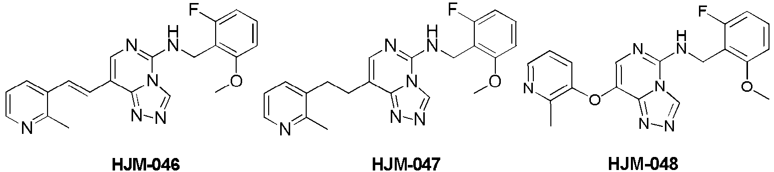

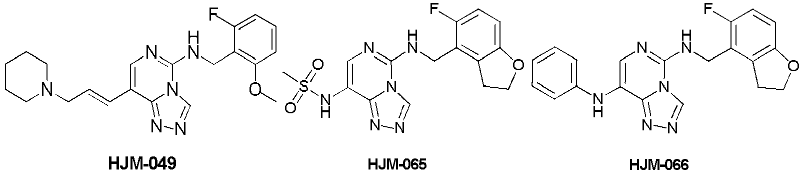

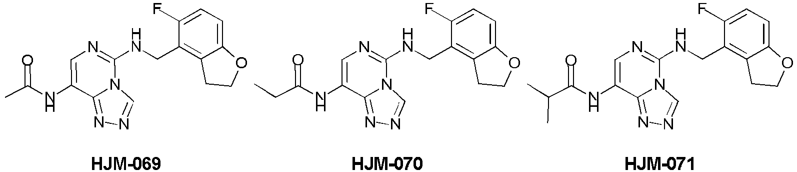

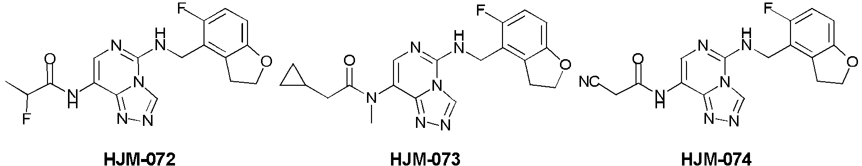

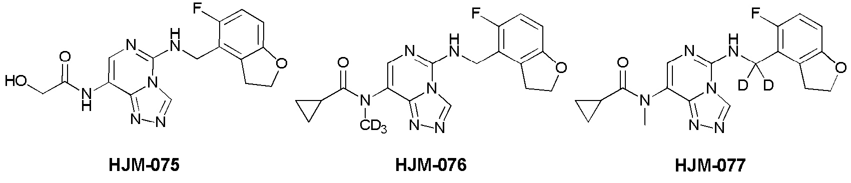

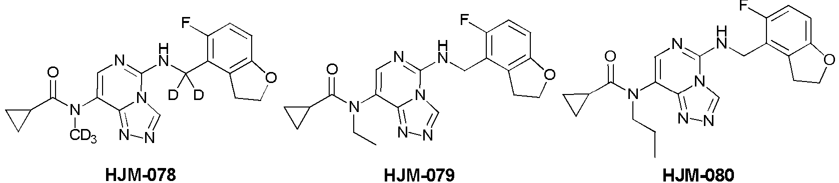

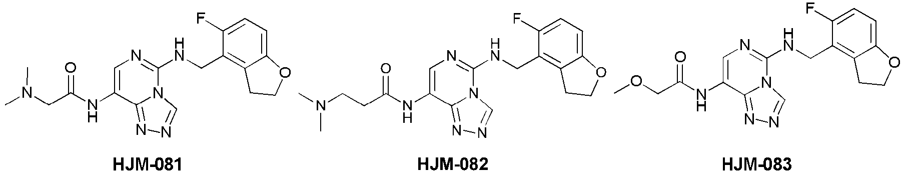

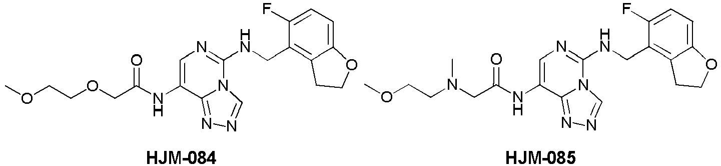

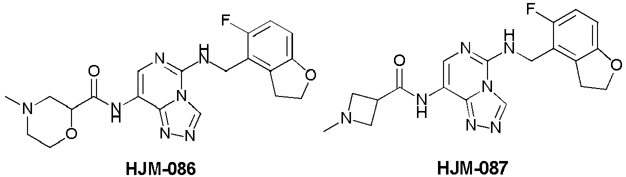

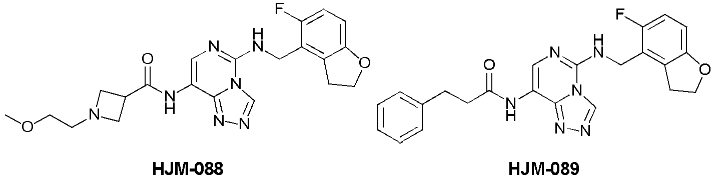

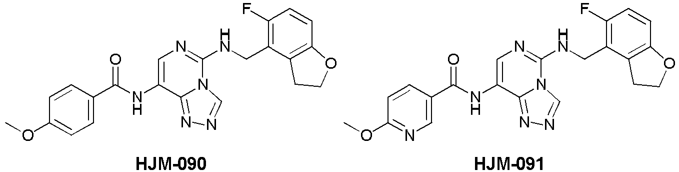

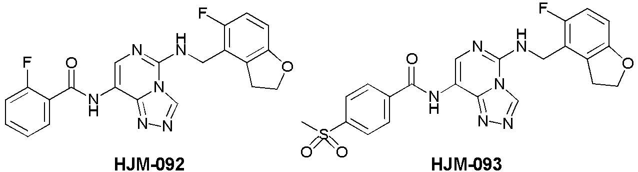

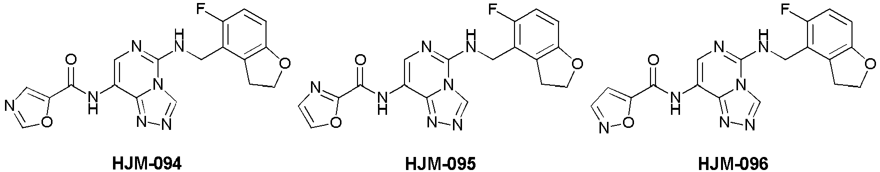

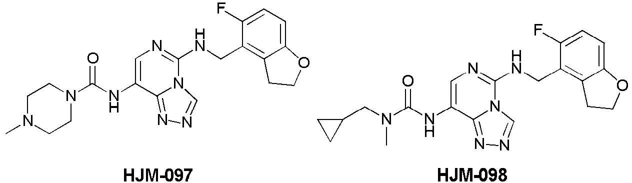

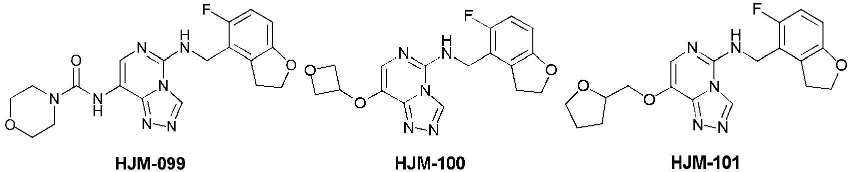

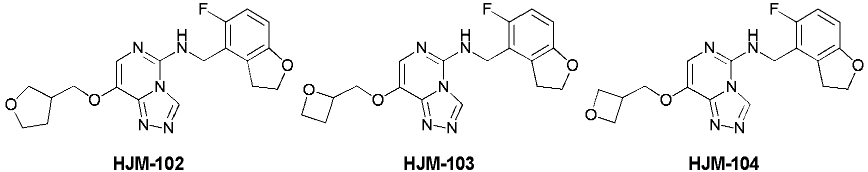

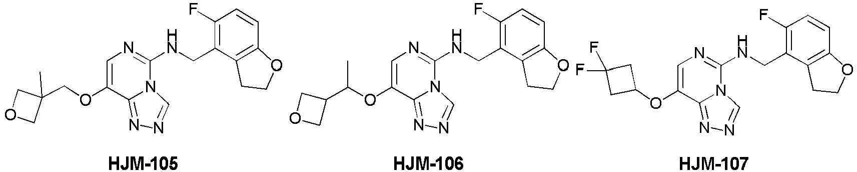

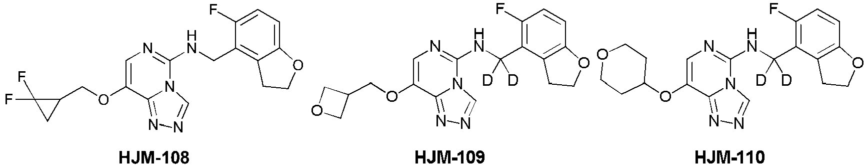

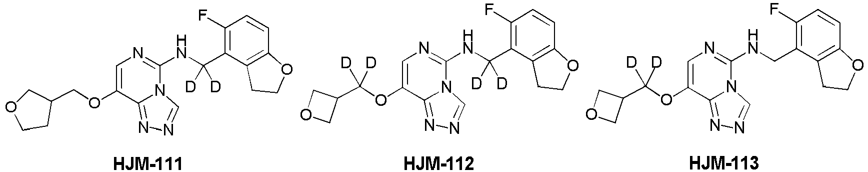

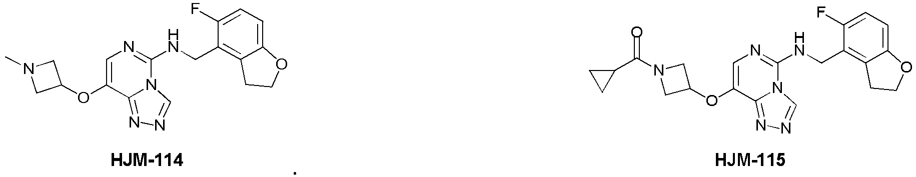

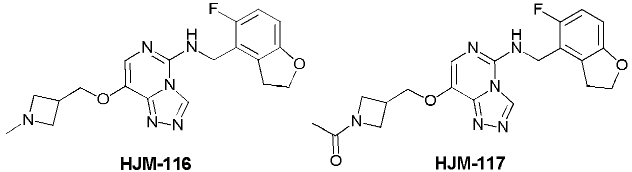

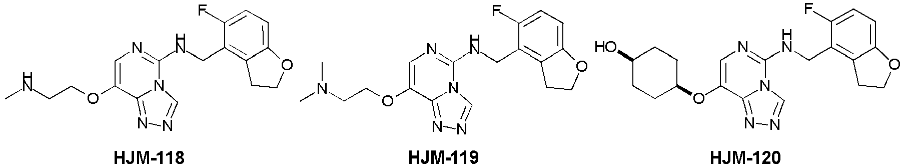

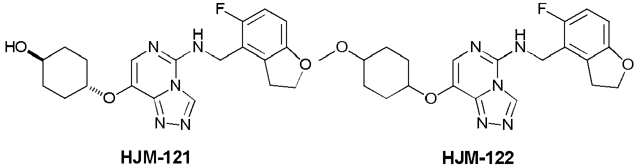

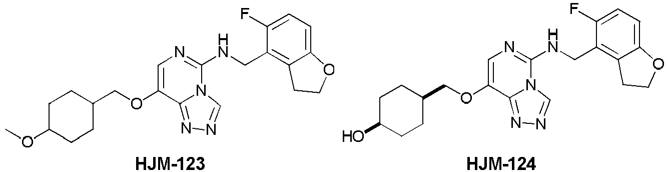

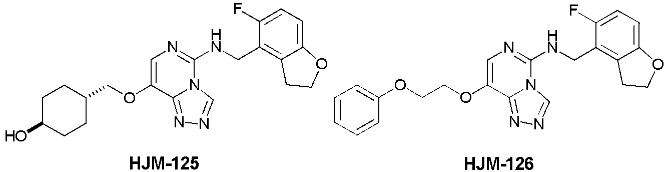

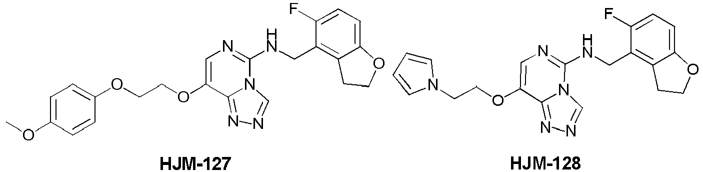

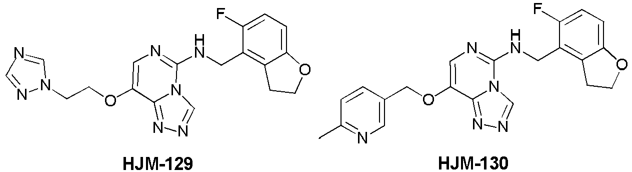

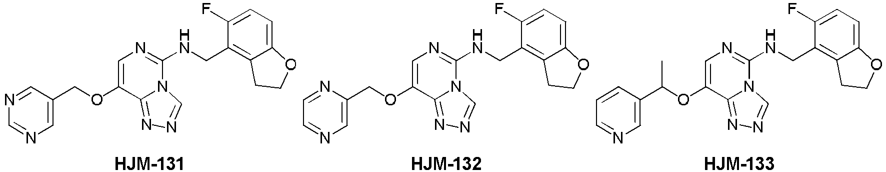

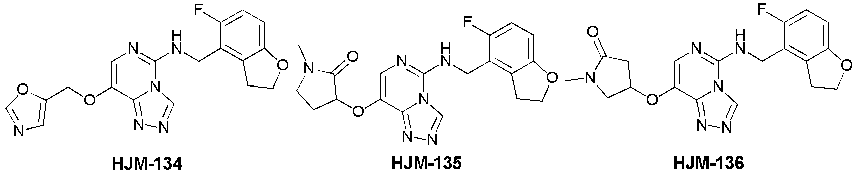

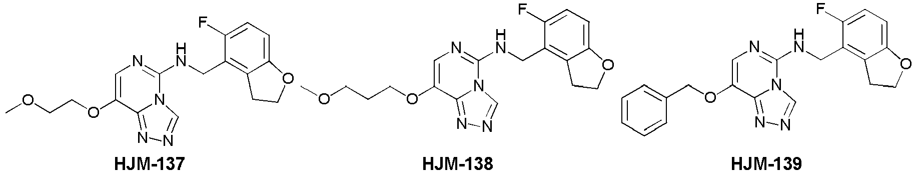

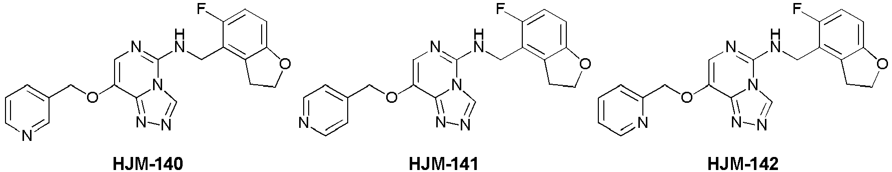

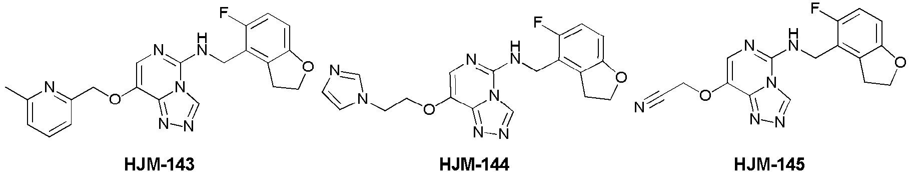

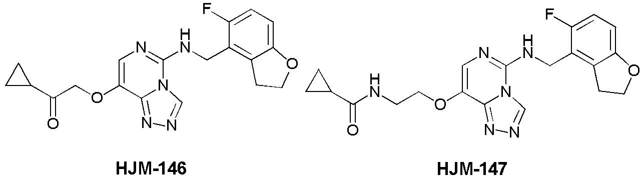

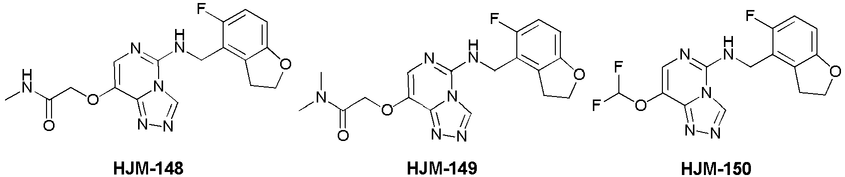

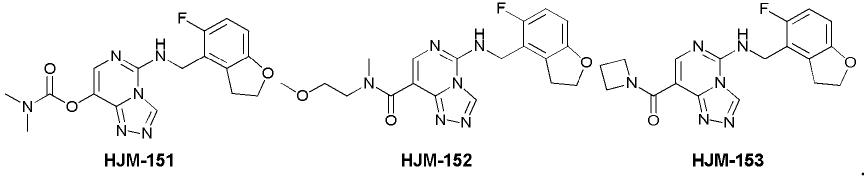

13. Composition pharmaceutique, contenant le composé ou le tautomère, le stéréoisomère, la forme cristalline, le sel pharmaceutiquement acceptable, l'hydrate ou le solvate de celui-ci selon l_{'}une quelconque des revendications 1 à 12, et un ou plusieurs excipients pharmaceutiquement acceptables ;
en variante, la composition pharmaceutique étant utilisée en combinaison avec des inhibiteurs de points de contrôle immunitaires tels que les anticorps PD-1, PD-L1, CTLA-4, etc.

14. Composé ou tautomère, stéréoisomère, forme cristalline, sel pharmaceutiquement acceptable, hydrate ou solvate de celui-ci selon l_{'}une quelconque des revendications 1 à 12, ou composition pharmaceutique selon la revendication 13, destinés à être utilisés dans le traitement et/ou la prévention d_{'}une maladie médiée par EED et/ou PRC2 choisie parmi une maladie liée à une tumeur, une maladie d_{'}immunodéficience ou une maladie auto-immune ; en variante, la maladie liée à une tumeur étant choisie parmi le lymphome diffus à grandes cellules B, le lymphome folliculaire, d'autres lymphomes, la leucémie, le myélome multiple, le mésothéliome, le cancer gastrique, le rhabdomyome malin, le cancer du foie, le cancer de la prostate, le cancer du sein, le cancer des voies biliaires et de la vésicule biliaire, le cancer de la vessie, les tumeurs cérébrales (y compris le neuroblastome, le schwannome, le gliome, le glioblastome et l_{'}astrocytome), le cancer du col de l_{'}utérus, le cancer du côlon, le mélanome, le cancer de l_{'}endomètre, le cancer de l_{'}œsophage, le cancer de la tête et du cou, le cancer du poumon, le cancer du nasopharynx, le cancer de l'ovaire, le cancer du pancréas, le cancer des cellules rénales, le cancer du rectum, le cancer de la thyroïde, la tumeur parathyroïdienne, la tumeur utérine et le sarcome des tissus mous ; la maladie d_{'}immunodéficience ou la maladie auto-immune comprenant, mais sans s_{'}y limiter, le lupus érythémateux disséminé, la maladie de Crohn, la colite ulcéreuse, la sclérose en plaques, l'anémie aplasique due à l_{'}auto-immunité et la réaction de rejet due à une transplantation d_{'}organe, etc., ou en variante, destinés à être utilisé dans l'immunisation tumorale.

15. Combinaison de médicaments, comprenant le composé ou le tautomère, le stéréoisomère, la forme cristalline, le sel pharmaceutiquement acceptable, l'hydrate ou le solvate de celui-ci selon l_{'}une quelconque des revendications 1 à 12, ou la composition pharmaceutique selon la revendication 13, et un ou plusieurs inhibiteurs de point de contrôle immunitaire tels que les anticorps PD-1, PD-L1, CTLA-4, etc.
